(19) Europäisches Patentamt
European Patent Office
Office européen des brevets

(11) EP 4 635 500 A1

(12) **EUROPEAN PATENT APPLICATION**
published in accordance with Art. 153(4) EPC

(43) Date of publication:
**22.10.2025 Bulletin 2025/43**

(21) Application number: **23903522.3**

(22) Date of filing: **12.12.2023**

(51) International Patent Classification (IPC):
***A61K 31/69*** *(2006.01)* ***A61K 31/407*** *(2006.01)*
***A61P 11/00*** *(2006.01)* ***A61P 13/02*** *(2006.01)*
***A61P 31/04*** *(2006.01)* ***A61P 43/00*** *(2006.01)*

(52) Cooperative Patent Classification (CPC):
**A61K 31/407; A61K 31/69; A61P 11/00; A61P 13/02; A61P 31/04; A61P 43/00**

(86) International application number:
**PCT/JP2023/044535**

(87) International publication number:
**WO 2024/128238 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.12.2022 JP 2022198950**
**31.05.2023 JP 2023090353**

(71) Applicants:
- **Sumitomo Pharma Co., Ltd.**
  **Osaka-shi, Osaka 541-0045 (JP)**
- **The Kitasato Institute**
  **Tokyo 108-8641 (JP)**

(72) Inventors:
- **TAKEMOTO, Koji**
  **Osaka-shi, Osaka 554-0022 (JP)**
- **NAKAYAMA, Ryo**
  **Osaka-shi, Osaka 541-0045 (JP)**
- **OKUDA, Yoshiko**
  **Osaka-shi, Osaka 541-0045 (JP)**
- **INOUE, Mami**
  **Osaka-shi, Osaka 554-0022 (JP)**
- **HANAKI, Hideaki**
  **Tokyo 108-8641 (JP)**

(74) Representative: **Vossius & Partner**
**Patentanwälte Rechtsanwälte mbB**
**Siebertstraße 3**
**81675 München (DE)**

(54) **THERAPEUTIC AGENT FOR BACTERIAL INFECTION**

(57) The present disclosure provides a medicament and curing instructions for treating or preventing a bacterial infection. More specifically, the present disclosure is a medicament for treating or preventing a bacterial infection, the medicament comprising Compound A or a pharmaceutically acceptable salt thereof and meropenem or a pharmaceutically acceptable salt thereof, the medicament being characterized in that the medicament is administered to a subject intravenously. Alternatively, the present disclosure provides a medicament for treating or preventing a bacterial infection, the medicament comprising Compound A or a pharmaceutically acceptable salt thereof and meropenem or a pharmaceutically acceptable salt thereof, the medicament being characterized in that the Compound A or a pharmaceutically acceptable salt thereof is administered to a subject in such a manner that the activity of meropenem or a pharmaceutically acceptable salt thereof is improved compared with the case where meropenem or the pharmaceutically acceptable salt thereof is not used in combination.

EP 4 635 500 A1

## Description

[Technical Field]

The present disclosure relates to a suitable dosage and administration of an oxo-substituted compound or a pharmaceutically acceptable salt thereof or a tautomer thereof, useful as a medicament. The medicament of the present disclosure is characterized as broad-spectrum, strong, and effective against a multidrug-resistant bacterium.

[Background Art]

Since discovery of penicillin, antibacterial agents have held an important place in treatment of infectious diseases.

Among them, β-lactam agents (e.g., penicillin antibacterial agents, cephalosporin antibacterial agents, carbapenem antibacterial agents, or monobactam antibacterial agents) have a broad antibacterial spectrum and are the most widely used agents in treatment of bacterial infections because of their strong bactericidal activity and high safety. However, with increased use of the β-lactam agents, emergence and spread of pathogens that have acquired resistance to the β-lactam agents has become a worldwide problem. A mechanism of acquisition of resistance by these pathogens includes production of β-lactamases, conformational changes in target molecules of the β-lactam agents, decreased drug penetration into bacteria, and increased agent efflux. In particular, the production of β-lactamases which degrade and inactivate the β-lactam agents is one of factors having the greatest impact on maintaining efficacy of the β-lactam agents. Various bacteria have evolved β-lactamases that counter efficacy of various β-lactam agents by transferring them among various bacterial strains or species. The β-lactamases can be classified into four classes, that is, Ambler classes A, B, C, and D based on their amino acid sequences. The Class A, C, and D enzymes are called serine-β-lactamases because they have serine residues at active centers of the enzymes, and the Class B enzyme is called a metallo-β-lactamase (zinc-β-lactamase) because it does not have a serine residue at an active center of the enzyme but a metal ion, zinc ($Zn^{2+}$).

A combination of β-lactamase inhibitors with β-lactam agents has already been shown to be effective in solving the problem of acquisition of resistance due to the production of β-lactamases, and it has been known that commercially available β-lactamase inhibitors clavulanic acid, sulbactam, and tazobactam mainly inhibit Class A β-lactamases except Klebsiella pneumoniae carbapenemase (KPC), avibactam inhibits Class A β-lactamases including KPC, Class C β-lactamases such as AmpC, and some Class D β-lactamases including OXA-48, and relebactam and vaborbactam inhibit Class A β-lactamases including KPC and AmpC (Non-Patent Document 1 to 2). However, these existing β-lactamase inhibitors do not effectively and broadly inhibit all β-lactamases produced by various bacteria. For example, they have no effect on Class D β-lactamases of the OXA family produced by Acinetobacter baumannii or Class B metallo-β-lactamases. Pathogenic bacteria producing β-lactamases collectively called extended-spectrum β-lactamases (ESBLs) which can degrade more substrates (β-lactam agents excluding carbapenem antimicrobial agents) than conventional Class A β-lactamases remain a worldwide problem. In addition, emergence and spread of metallo-β-lactamase-producing bacteria is becoming a problem.

Recently, a boronic acid derivative with a β-lactamase inhibitory effect was reported in Patent Document 1.

[Citation List]

[Patent Literature]

[PTL 1]
WO 2019/208797

[Non-Patent Literature]

[NPL 1]
Buynak. JD. Expert Opinion on Therapeutic Patents, 2013, 23(11), 1469-1481.
[NPL 2]
Monogue. ML. Expert Review of Anti-infective Therapy, 2019, 17(8), 571-582.

[Summary of Invention]

[Solution to Problem]

The present disclosure provides the invention relating to dosage and administration of a compound having a superior β-lactamase inhibitory activity and provides dosage and administration of a prophylactic or therapeutic agent effective against a bacterial infection in combination with a β-lactam agent. Specifically, dosage and administration of a prophylactic or therapeutic agent effective against a disease such as sepsis, febrile neutropenia, bacterial meningitis, bacterial endocarditis, otitis media, sinusitis, pneumonia, lung abscess, empyema, a secondary infection of a chronic respiratory tract lesion, pharyngolaryngitis, tonsillitis, osteomyelitis, arthritis, peritonitis, intra-abdominal abscess, cholecystitis, cholangitis, liver abscess, a deep skin infection, lymphangitis and lymphadenitis, a secondary infection such as trauma, burn, and a surgical wound, a urinary tract infection, a genital infection, an ocular or dental infection, a complicated urinary tract infection, a complicated intra-abdominal infection, hospital-acquired pneumonia, and ventilator-associated pneumonia in combination with a β-lactam agent are provided.

More specifically, the inventors provide a technology relating to a compound of Formula (1a), (1b), or (2) below:

[Chem. 1]

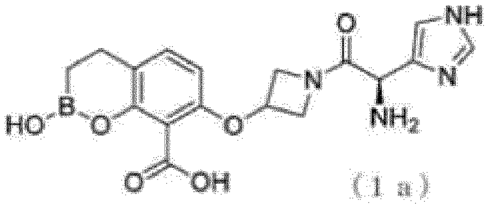

(1 a)

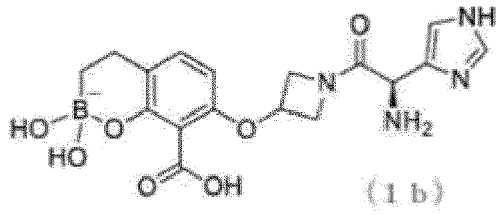

(1 b)

or

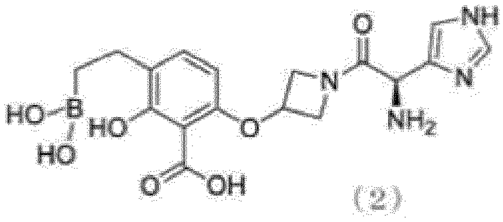

(2)

or a pharmaceutically acceptable salt thereof (hereinafter may also be referred to as "compound of the present disclosure" or "Compound A").

(Item 1)

A medicament for treating or preventing a bacterial infection, the medicament including:

Compound A or a pharmaceutically acceptable salt thereof; and
meropenem or a pharmaceutically acceptable salt thereof,
the Compound A being of Formula (1a), (1b), or (2):

[Chem. 2]

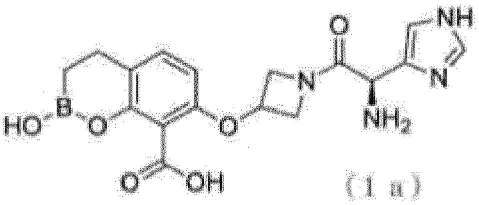

(1 a)

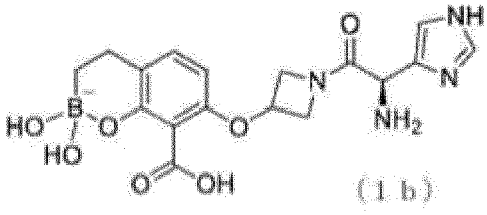

(1 b)

or

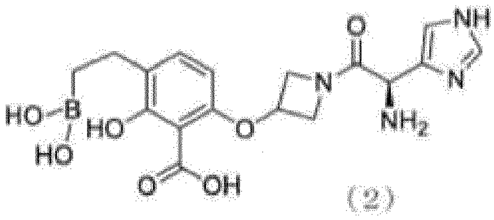

(2)

and
the medicament being administered intravenously to a subject.

(Item 2)

A medicament for treating or preventing a bacterial infection, the medicament including:

Compound A or a pharmaceutically acceptable salt thereof; and
meropenem or a pharmaceutically acceptable salt thereof,
the Compound A or the pharmaceutically acceptable salt thereof being administered to a subject so as to enhance an activity of the meropenem or the pharmaceutically acceptable salt thereof as compared to when not being used in combination, and the Compound A being of Formula (1a), (1b), or (2) above.

(Item 3)

The medicament of any one of the preceding items, in which the medicament is administered intravenously to the subject at a frequency of at least once every 24 hours.

(Item 4)

The medicament of any one of the preceding items, in which the medicament is administered intravenously to the subject at a frequency of at least once every 8 hours.

(Item 5)

The medicament of any one of the preceding items, in which the medicament is administered intravenously to the subject at a frequency of at least once every 6 hours.

(Item 6)

The medicament of any one of the preceding items, in which the Compound A or the pharmaceutically acceptable salt thereof is administered intravenously to the subject at a frequency of at least once every 8 hours.

(Item 7)

The medicament of any one of the preceding items, in which the Compound A or the pharmaceutically acceptable salt thereof is administered intravenously to the subject at a frequency of at least once every 6 hours.

(Item 8)

The medicament of any one of the preceding items, in which the meropenem or the pharmaceutically acceptable salt thereof is administered intravenously to the subject at a frequency of at least once every 8 hours.

(Item 9)

The medicament of any one of the preceding items, in which the meropenem or the pharmaceutically acceptable salt thereof is administered intravenously to the subject at a frequency of at least once every 6 hours.

(Item 10)

The medicament of any one of the preceding items, in which the medicament is administered by intravenous infusion (intravenous drip infusion, drip infusion) for 3 hours or more.

(Item 11)

The medicament of any one of the preceding items, in which the medicament is administered by intravenous infusion for 4 hours or more.

(Item 12)

The medicament of any one of the preceding items, in which the medicament is administered by intravenous infusion for 3 hours or less.

(Item 13)

The medicament of any one of the preceding items, in which the medicament is administered by intravenous infusion for 3 hours.

(Item 14)

The medicament of any one of the preceding items, in which the medicament is administered by intravenous infusion for 4 hours.

(Item 15)

The medicament of any one of the preceding items, in which the Compound A or the pharmaceutically acceptable salt thereof and the meropenem or the pharmaceutically acceptable salt thereof are administered concomitantly or separately.

(Item 16)

The medicament of any one of the preceding items, in which the Compound A or the pharmaceutically acceptable salt thereof and the meropenem or the pharmaceutically acceptable salt thereof are included in a same agent or separate agents.

(Item 17)

The medicament of any one of the preceding items, in which the Compound A or the pharmaceutically acceptable salt thereof and the meropenem or the pharmaceutically acceptable salt thereof are included in a ratio of 1:1 to 1:10 by mass on a free form basis.

(Item 18)

The medicament of any one of the preceding items, in which the Compound A or the pharmaceutically acceptable salt thereof and the meropenem or the pharmaceutically acceptable salt thereof are included in a ratio of 1:2 by mass on a free form basis.

(Item 19)

The medicament of any one of the preceding items, in which the Compound A or the pharmaceutically acceptable salt thereof and the meropenem or the pharmaceutically acceptable salt thereof are included in a ratio of 1:4 on a free form basis.

(Item 20)

The medicament of any one of the preceding items, in which the Compound A or the pharmaceutically acceptable salt thereof and the meropenem or the pharmaceutically acceptable salt thereof are included in a ratio of 1:8 on a free form basis.

(Item 21)

The medicament of any one of the preceding items, in which the medicament includes 500 to 3000 mg of the Compound A on a free form basis.

(Item 22)

The medicament of any one of the preceding items, in which the medicament includes 500 to 1000 mg of the Compound A on a free form basis.

(Item 23)

The medicament of any one of the preceding items, in which the medicament includes 200 to 6000 mg of the meropenem on a free form basis.

(Item 24)

The medicament of any one of the preceding items, in which the medicament includes 250 to 3000 mg of the meropenem on a free form basis.

(Item 25)

The medicament of any one of the preceding items, in which the medicament includes 1500 mg of the meropenem on a free form basis.

(Item 26)

The medicament of any one of the preceding items, in which the medicament includes 2000 mg of the meropenem on a free form basis.

(Item 27)

The medicament of any one of the preceding items, in which the medicament includes 500 mg of the Compound A on a free form basis.

(Item 28)

The medicament of any one of the preceding items, in which the medicament includes 750 mg of the Compound A on a free form basis.

(Item 29)

The medicament of any one of the preceding items, in which the medicament includes 1000 mg of the Compound A on a free form basis.

(Item 30)

The medicament of any one of the preceding items, in which the medicament includes 1000 mg of the Compound A on a free form basis and 2000 mg of the meropenem on a free form basis.

(Item 31)

The medicament of any one of the preceding items, in which the medicament includes 500 mg of the Compound A on a free form basis and 1500 mg of the meropenem on a free form basis.

(Item 32)

The medicament of any one of the preceding items, in which the medicament includes 750 mg of the Compound A on a free form basis and 1500 mg of the meropenem on a free form basis.

(Item 32A)

The medicament of any one of the preceding items, in which the Compound A and/or the meropenem is included as a single dose.

(Item 32B)

The medicament of any one of the preceding items, in which the Compound A and/or the meropenem is included as a daily dose.

(Item 33)

The medicament of any one of the preceding items, in which the bacterial infection is a bacterial infection involving a bacterium that can possess a β-lactamase.

(Item 34)

The medicament of any one of the preceding items, in which the bacterial infection is a bacterial infection involving a bacterium that can possess a serine-β-lactamase.

(Item 35)

The medicament of any one of the preceding items, in which the serine-β-lactamase is an Ambler class A, Ambler class C, or Ambler class D β-lactamase.

(Item 36)

The medicament of any one of the preceding items, in which the bacterial infection is a bacterial infection involving a bacterium that can possess a metallo-β-lactamase.

(Item 37)

The medicament of any one of the preceding items, in which the metallo-β-lactamase is an Ambler class B β-lactamase.

(Item 38)

The medicament of any one of the preceding items, in which a therapeutically effective amount of the Compound A or the pharmaceutically acceptable salt thereof and the meropenem or the pharmaceutically acceptable salt thereof results in %fT > 1 μg/mL of 30% or more in terms of a plasma concentration of the Compound A.

(Item 39)

The medicament of any one of the preceding items, in which a therapeutically effective amount of the Compound A or the pharmaceutically acceptable salt thereof and the meropenem or the pharmaceutically acceptable salt thereof results in %fT > 2 μg/mL of 20% or more in terms of a plasma concentration of the Compound A.

(Item 40)

The medicament of any one of the preceding items, in which a therapeutically effective amount of the Compound A or the pharmaceutically acceptable salt thereof and the meropenem or the pharmaceutically acceptable salt thereof results in %fT > 4 μg/mL of 10% or more in terms of a plasma concentration of the Compound A.

(Item 41)

The medicament of any one of the preceding items, in which a therapeutically effective amount of Compound A or a pharmaceutically acceptable salt thereof and meropenem or a pharmaceutically acceptable salt thereof results in an fAUC of 70 μg·h/mL or more in terms of a plasma concentration of the Compound A.

(Item 42)

The medicament of any one of the preceding items, in which the bacterial infection is a bacterial infection involving a bacterium selected from Enterobacteriales, Acinetobacter baumannii complex, Pseudomonas aeruginosa, and Bacteroides spp.

(Item 43)

The medicament of any one of the preceding items, in which the bacterial infection is a bacterial infection involving Escherichia coli, Klebsiella pneumoniae, Enterobacter cloacae complex, or Acinetobacter baumannii complex.

(Item 44)

The medicament of any one of the preceding items, in which the bacterial infection is a complicated urinary tract infection, a complicated intra-abdominal infection, hospital-acquired pneumonia, or ventilator-associated pneumonia.

(Item 45)

The medicament of any one of the preceding items, in which the medicament is used for a patient suffering from a bacterium susceptible to a combination of the Compound A or the pharmaceutically acceptable salt thereof and the meropenem or the pharmaceutically acceptable salt thereof.

(Item 46)

The medicament of any one of the preceding items, in which the medicament has a metallo-β-carbapenemase inhibitory activity.

(Item 47)

The medicament of any one of the preceding items, in which the medicament has an antibacterial activity against Escherichia coli, Klebsiella pneumoniae, Enterobacter cloacae complex, or Acinetobacter baumannii complex.

(Item 48)

The medicament of any one of the preceding items, in which the medicament has an antibacterial activity against Acinetobacter baumannii complex.

(Item A1)

A method for treating or preventing a bacterial infection, the method including

intravenously administering an effective amount of Compound A or a pharmaceutically acceptable salt thereof and meropenem or a pharmaceutically acceptable salt thereof to a subject,
the Compound A being of Formula (1a), (1b), or (2) above.

(Item A2)

A method for treating or preventing a bacterial infection, the method including

administering an effective amount of Compound A or a pharmaceutically acceptable salt thereof and meropenem or a pharmaceutically acceptable salt thereof to a subject,
the Compound A or the pharmaceutically acceptable salt thereof being administered to the subject so as to enhance an activity of the meropenem or the pharmaceutically acceptable salt thereof as compared to when not being used in combination, and the Compound A being of Formula (1a), (1b), or (2) above.

(Item B1)

A combination of Compound A or a pharmaceutically acceptable salt thereof and meropenem or a pharmaceutically acceptable salt thereof for treating or preventing a bacterial infection, the combination being administered intravenously to a subject, and the Compound A being of Formula (1a), (1b), or (2) above.

(Item B2)

A combination of Compound A or a pharmaceutically acceptable salt thereof and meropenem or a pharmaceutically acceptable salt thereof for treating or preventing a bacterial infection, the Compound A or the pharmaceutically acceptable salt thereof being administered to a subject so as to enhance an activity of the meropenem or the pharmaceutically acceptable salt thereof as compared to when not being used in combination, and the Compound A being of Formula (1a), (1b), or (2) above.

(Item BA1)

Compound A or a pharmaceutically acceptable salt thereof for treating or preventing a bacterial infection, the Compound A or the pharmaceutically acceptable salt thereof being used in combination with meropenem or a pharmaceutically acceptable salt thereof, the combination being administered intravenously to a subject, and the Compound A being of Formula (1a), (1b), or (2) above.

(Item BA2)

Compound A or a pharmaceutically acceptable salt thereof for treating or preventing a bacterial infection, the Compound A or the pharmaceutically acceptable salt thereof being used in combination with meropenem or a pharmaceutically acceptable salt thereof, the Compound A or the pharmaceutically acceptable salt thereof being administered to a subject so as to enhance an activity of the meropenem or the pharmaceutically acceptable salt thereof as compared to when not being used in combination, and the Compound A being of Formula (1a), (1b), or (2) above.

(Item BB1)

Meropenem or a pharmaceutically acceptable salt thereof for treating or preventing a bacterial infection, the meropenem or the pharmaceutically acceptable salt thereof being used in combination with Compound A or a pharmaceutically acceptable salt thereof, the combination being administered intravenously to a subject, and the Compound A being of Formula (1a), (1b), or (2) above.

(Item BB2)

Meropenem or a pharmaceutically acceptable salt thereof for treating or preventing a bacterial infection, the meropenem or the pharmaceutically acceptable salt thereof being used in combination with Compound A or a pharmaceutically acceptable salt thereof, the Compound A or the pharmaceutically acceptable salt thereof being administered to a subject so as to enhance an activity of the meropenem or the pharmaceutically acceptable salt thereof as compared to when not being used in combination, and the Compound A being of Formula (1a), (1b), or (2) above.

(Item BC1)

A combination drug of meropenem or a pharmaceutically acceptable salt thereof and Compound A or a pharmaceutically acceptable salt thereof for treating or preventing a bacterial infection, the combination being administered intravenously to a subject, and the Compound A being of Formula (1a), (1b), or (2) above.

(Item BC2)

A combination drug of meropenem or a pharmaceutically acceptable salt thereof and Compound A or a pharmaceutically acceptable salt thereof for treating or preventing a bacterial infection, the Compound A or the pharmaceutically acceptable salt thereof being administered to a subject so as to enhance an activity of the meropenem or the pharmaceutically acceptable salt thereof as compared to when not being used in combination, and the Compound A being of Formula (1a), (1b), or (2) above.

(Item C1)

A use of a combination of Compound A or a pharmaceutically acceptable salt thereof and meropenem or a pharmaceutically acceptable salt thereof for producing a medicament for treating or preventing a bacterial infection, the medicament being administered intravenously to a subject, and the Compound A being of Formula (1a), (1b), or (2) above.

(Item C2)

A use of a combination of Compound A or a pharmaceutically acceptable salt thereof and meropenem or a pharmaceutically acceptable salt thereof in producing a medicament for treating or preventing a bacterial infection, the Compound A or the pharmaceutically acceptable salt thereof being administered to a subject so as to enhance an activity of the meropenem or the pharmaceutically acceptable salt thereof as compared to when not being used in combination, and the Compound A being of Formula (1a), (1b), or (2) above.

(Item D)

The method according to Item A1 or A2, the combination according to Item B1 or B2, the composition according to Item BA1, BA2, BB1, or BB2, the combination drug according to Item BC1 or BC2, or the use according to Item C1 or C2, further including any one or more features of Items 1 to 48.

Some embodiments described herein relate to a method for treating a bacterial infection, the method including administering a composition including Compound A (Compound A is of Formula (1a), (1b), or (2) above, as similarly interpreted herein) or a pharmaceutically acceptable salt thereof and a carbapenem antibacterial agent to a subject in need thereof so as to give a proportion of time for which a free drug concentration is above 0.25 to 8 μg/mL (%fT > 0.25 to 8 μg/mL) in a dosing interval of the Compound A is about 5% to about 70%.

In some embodiments, the carbapenem antibacterial agent is selected from the group consisting of imipenem, biapenem, doripenem, meropenem, panipenem, tebipenem, and ertapenem. In some such embodiments, the carbapenem antibacterial agent is meropenem.

In some embodiments, the compound meropenem is administered in a dose range of about 10 mg/kg to about 120 mg/kg body weight on a free form basis. In some further embodiments, the Compound A is administered in a dose range of about 1 mg/kg to about 120 mg/kg body weight on a free form basis.

In some embodiments, the composition is administered intravenously.

In some embodiments, the infection is caused by a bacterium selected from

[Chem. 3]

*Pseudomonas aeruginosa, Pseudomonas fluorescens, Stenotrophomonas maltophilia, Escherichia coli, Citrobacter freundii, Salmonella typhimurium, Salmonella typhi, Salmonella paratyphi, Salmonella senftenberg, Shigella dysenteriae, Shigella flexneri. Shigella sonnei, Enterobacter cloacae, Enterobacter aerogenes, Klebsiella pneumoniae, Klebsiella oxytoca. Klebsiella ozaenae, Kluyvera ascorbata, Morganella morganii, Proteus mirabilis, Serratia marcescens, Acinetobacter baumannii , Providencia rettgeri , Raoultella ornithinolytica , Acinetobacter calcoaceticus, Acinetobacter haemolyticus, Yersinia enterocolitica, Yersinia pestis, Yersinia pseudotuberculosis, Haemophilus influenzae, Haemophilus parainfluenzae, Campylobacter jejuni, Vibrio cholerae, Vibrio parahaemolyticus, Neisseria gonorrhoeae, Neisseria meningitidis, Moraxella catarrhalis, Bacteroides fragilis, Bacteroides valgatus, Bacteroides ovalus, Bacteroides thetaiotaomicron.*

In some embodiments, the composition further includes an additional drug selected from an antibacterial agent, an antifungal agent, an antiviral agent, an anti-inflammatory agent, or an antiallergic agent.

In some embodiments, the subject to be treated by the above method is a mammal. In some further embodiments, the subject is human.

In the present disclosure, it is intended that one or more of the above features may be provided in further combinations in addition to explicit combinations. Those skilled in the art will be recognized still further embodiments and advantages of the present disclosure upon reading and understanding the following detailed description, if necessary.

[Advantageous Effects of Invention]

The technology of the present disclosure is useful as a therapeutic and/or prophylactic agent for a bacterial infection involving a bacterium that can possess a β-lactamase, specifically, sepsis, febrile neutropenia, bacterial meningitis, bacterial endocarditis, otitis media, sinusitis, pneumonia, lung abscess, empyema, a secondary infection of a chronic respiratory tract lesion, pharyngolaryngitis, tonsillitis, osteomyelitis, arthritis, peritonitis, intra-abdominal abscess, cholecystitis, cholangitis, liver abscess, a deep skin infection, lymphangitis and lymphadenitis, a secondary infection such as trauma, burn, and a surgical wound, a urinary tract infection, a genital infection, an ocular or dental infection, a complicated urinary tract infection, a complicated intra-abdominal infection, hospital-acquired pneumonia, and ventilator-associated pneumonia by using in combination with a β-lactam agent in dosage and administration provided herein. Without limitation, the technology of the present disclosure can be used as a variety of therapeutic and/or prophylactic agents to be administered in a dose that has been confirmed or expected to be tolerated in a human.

[Brief Description of Drawings]

[FIG. 1] FIG. 1 shows types of β-lactamases and inhibition spectra of various β-lactamase inhibitors based on Examples 1 and 7. ESBL stands for Extended-Spectrum β-Lactamase, KPC stands for Klebsiella Pneumoniae Carbapenemase, OXA stands for oxacillinase, NDM stands for New Delhi Metallo-β-lactamase, VIM stands for Verona Integron-encoded Metallo-β-lactamase, and IMP stands for Imipenemase.

[FIG. 2] FIG. 2 shows individual (circle) bacterial growth or reduction and average (bar) ± standard deviation 24 hours after initiation of treatment as indicated by $\log_{10}$ CFU/kidney in a complicated urinary tract infection model of a neutropenic mouse (Example 16). Average kidney bacterial loads at the start of treatment in E. coli CDC-150 and Klebsiella pneumoniae ATCC BAA-2344 infection models were 5.30 $\log_{10}$ CFU/kidney and 5.99 $\log_{10}$ CFU/kidney, respectively. The treatment was initiated 3 to 5 hours after infection by subcutaneous administration and continued for 24 hours (n = 4). MEPM stands for meropenem.

[FIG. 3] FIG. 3 shows efficacy of Compound A in combination with meropenem and cilastatin in an A. baumannii CDC-277 lung infection model (Example 17). A change (average and standard error) in bacterial count in the lung of each of drug-treated groups relative to a bacteria count in the lung (baseline count) at the start of treatment, that is, at 2 hours after infection is shown. The nasal cavity of each of mice in which neutropenia was induced was inoculated with about 1 x $10^6$ CFU/mouse (0.02 mL/mouse) of A. baumannii CDC-277. Starting 2 hours after infection, Compound A at 30 and 60 mg/kg every 3 hours for 8 doses, Compound A at 60 and 120 mg/kg every 6 hours for 4 doses, or Compound A at 120 and 240 mg/kg every 12 hours for 2 doses were administered subcutaneously in combination with subcutaneous administration of meropenem at 100 mg/kg and cilastatin at 100 mg/kg every 3 hours for 8 doses. Colistin at 30 mg/kg was administered subcutaneously every 12 hours for 2 doses. A bacterial count in the lung was measured at 2 or 26 hours after infection. (*): Significant difference ($p < 0.05$) compared to the baseline count as determined by one-way ANOVA and Dunnett's test. Each group contained 8 animals.

[FIG. 4] FIG. 4 shows a schematic diagram of PK/PD parameters.

[FIG. 5] FIG. 5 shows a summary of a testing procedure for an intramuscular infection model of a neutropenic mouse used in Examples 18 and 19. A check mark denotes administration of meropenem at 5 or 100 mg/kg and cilastatin at 100 mg/kg and ¶ denotes administration of Compound A at various doses.

[FIG. 6-1] FIG. 6-1 shows an in vivo activity of MEPM at 5 mg/kg administered every 3 hours alone and in combination with Compound A against A) KPC-2-, B) KPC-3-, and C) NDM-1-producing bacteria (Example 18). An average value of growth or reduction in $\log_{10}$ CFU/thigh at 24 hours compared to the start of treatment in an intramuscular infection model of a neutropenic mouse is shown. Treatment was initiated 2 hours after infection (subcutaneous administration) and continued for 24 hours (n = 4).

[FIG. 6-2] FIG. 6-2 shows an in vivo activity of MEPM at 5 or 100 mg/kg administered every 3 hours alone and in combination with Compound A against D) IMP-1- and E) KPC-38-producing bacteria (Example 18). An average value of growth or reduction in $\log_{10}$ CFU/thigh at 24 hours compared to the start of treatment in an intramuscular infection model of a neutropenic mouse is shown. Treatment was initiated 2 hours after infection (subcutaneous administration) and continued for 24 hours (n = 4).

[FIG. 7-1] FIG. 7-1 shows an in vivo activity of MEPM at 100 mg/kg administered every 3 hours alone and in combination with Compound A against A) Acinetobacter baumannii CDC-83 (producing NDM-1, OXA-23, and OXA-69), B) CDC-277 (producing OXA-24 and OXA-65), and C) CDC-301 (producing OXA-66 and OXA-72) (Example 19). An average value of growth or reduction in $log_{10}$ CFU/thigh at 24 hours compared to the start of treatment in an intramuscular infection model of a neutropenic mouse is shown. Treatment was initiated 2 hours after infection (subcutaneous administration) and continued for 24 hours (n = 4).

[FIG. 7-2] FIG. 7-2 shows an in vivo activity of MEPM at 100 mg/kg administered every 3 hours alone and in combination with Compound A against D) CDC-293 (producing OXA-66 and OXA-72) and E) CDC-289 (producing OXA-66 and OXA-72) (Example 19). An average value of growth or reduction in $log_{10}$ CFU/thigh at 24 hours compared to the start of treatment in an intramuscular infection model of a neutropenic mouse is shown. Treatment was initiated 2 hours after infection (subcutaneous administration) and continued for 24 hours (n = 4).

[FIG. 8] FIG. 8 shows a relationship between a change in the number of bacteria in the muscle and %fT > 1 ug/mL of Compound A and a relationship between a change in the number of bacteria in the muscle and fAUC of Compound A (Example 22). Black square: KPC-2-producing bacterium, black circle: KPC-3-producing bacterium, black triangle: KPC-38-producing bacterium, gray triangle: NDM-1-producing bacterium, and gray circle: IMP-1-producing bacterium.

[FIG. 9] FIG. 9 shows a relationship between a change in the number of bacteria in the muscle and %fT > 4 ug/mL of Compound A and a relationship between a change in the number of bacteria in the muscle and fAUC of Compound A (Example 23). Black square: CDC-301, black circle: CDC-293, black triangle: CDC-277, gray triangle: CDC-289, and gray circle: CDC-83.

[FIG. 10-1] FIG. 10-1 shows an in vivo activity of MEPM at 50 or 100 mg/kg administered every 3 hours alone and in combination with Compound A against A) VIM-27-, B) KPC-3-, and C) NDM-7-producing bacteria (Example 29). An average value of growth or reduction in $log_{10}$ CFU/thigh at 24 hours compared to the start of treatment in an intramuscular infection model of a neutropenic mouse is shown. Treatment was initiated 2 hours after infection (subcutaneous administration) and continued for 24 hours (n = 4).

[FIG. 10-2] FIG. 10-2 shows an in vivo activity of MEPM at 100 mg/kg administered every 3 hours alone and in combination with Compound A against D) NDM-1- and OXA-232-producing bacteria (Example 29). An average value of growth or reduction in $log_{10}$ CFU/thigh at 24 hours compared to the start of treatment in an intramuscular infection model of a neutropenic mouse is shown. Treatment was initiated 2 hours after infection (subcutaneous administration) and continued for 24 hours (n = 4).

[FIG. 11]FIG. 11 shows A) a relationship between a change in the number of bacteria in the muscle and %fT > 2 μg/mL of Compound A in 9 strains of Klebsiella pneumoniae producing a carbapenemase, B) a relationship between a change in the number of bacteria in the muscle and %fT > 2 μg/mL of Compound A in 4 strains of Klebsiella pneumoniae producing a serine-carbapenemase, C) a relationship between a change in the number of bacteria in the muscle and %fT > 2 ug/mL of Compound A in 5 strains of Klebsiella pneumoniae producing a metallo-carbapenemase and %fT > 2 ug/mL of Compound A (Example 29). Black square: ATCC BAA-2344 (KPC-2-producing bacterium), black circle: ATCC BAA-1902 (KPC-3-producing bacterium), black diamond: CDC-113 (KPC-3-producing bacterium), black triangle: KUB3606 (KPC-38-producing bacterium), gray circle: KUB3166 (IMP-1-producing bacterium), gray triangle: ATCC BAA-2473 (NDM-1-producing bacterium), gray cross: CDC-40 (VIM-27-producing bacterium), gray square: CDC-68 (NDM-1- and OXA-232-producing bacterium), and gray diamond: CDC-138 (NDM-7-producing bacterium) .

[Description of Embodiments]

The present disclosure will be described in detail.

It should be understood that throughout this specification, singular expressions also include the concept of their plural forms, unless otherwise noted. Thus, it should be understood that singular articles (e.g., "a", "an", "the", etc. in English) also include the concept of their plural forms, unless otherwise noted. It should also be understood that terms used herein are to be used in the sense normally used in the art, unless otherwise noted. Therefore, unless otherwise defined, all technical and scientific terms used herein have the same meaning as commonly understood by those skilled in the art to which the present disclosure pertains. In the event of a conflict, this specification (including definitions) shall prevail.

(Definition and general description)

First, terms and general techniques used herein will be described.

A compound provided herein may encompass a variety of stereochemical forms. A compound of the present disclosure (also referred to as Compound A) also encompasses an optical isomer thereof, a diastereomer thereof, a mixture of enantiomers including, for example, a racemic mixture, as well as an individual enantiomer and an individual

diastereomer resulting from structural asymmetry in a specific compound. Individual isomers are separated or selectively synthesized by applying various methods known to those skilled in the art.

The term "group" means a monovalent group unless otherwise specified. Examples of a non-momovalent group include an alkylene group (divalent), etc. The term "group" may be omitted in the description of substituents, etc. below.

In this specification, the number of substituents when defined as "optionally substituted" or "substituted" is not particularly limited herein, as long as a substitution is possible. The number of substituents is one or multiple substituents. Unless specifcally indicated otherwise, the description of each substituent also applies when that substituent is a part of or a substituent of another substituent.

In some embodiments, a subject to be treated by the above method is a mammal. The present disclosure may be used for a mammal, and the term "mammal" is used herein in its common biological sense. Thus, specifically, the mammal includes a human, cattle, a horse, a dog, a cat, a rat, and a mouse, and also many other species.

In some further embodiments, the subject is a human.

The phrase "serine-β-lactamase" refers to an enzyme that hydrolyzes a β-lactam in a multistep reaction via a serine residue at a catalytic site, although it has a variety in terms of structure and hydrolysis profile. A negatively charged carboxylic acid group or a similarly charged group on a side chain of an azetidinone ring in a β-lactam antibacterial agent is attracted to an active site of a β-lactamase by a positively charged residue. When properly arranged, a β-lactam forms a key hydrogen bond with a β-lactamase. The β-lactamase acylates with the β-lactam via a conserved serine residue serving as a reactive nucleophile. Then a water molecule is activated, a β-lactam-β-lactamase complex is deacylated, and the β-lactam is ring-opened to produce a molecule that has lost its effect. The serine-β-lactamase includes, for example, Ambler classes A, C, and D β-lactamases (Bush. K. Nature Reviews Microbiology, 2019, 17, 295-306).

A "metallo-β-lactamase" possesses at least one and usually two $Zn^{2+}$ ions. The ions are cross-linked by a hydroxide ion, which promotes a nucleophilic substitution reaction to a carbonyl oxygen atom in a β-lactam ring. As a result, a β-lactam is hydrolyzed and ring-opened to produce a molecule that has lost its effect. The first $Zn^{2+}$ is fixed by three histidine residues. The second zinc ligand is aspartic acid, cysteine, and/or histidine, and the histidine is also involved in the hydrolysis reaction. The metallo-β-lactamase includes, for example, Ambler class B β-lactamase (Bush. K. Nature Reviews Microbiology, 2019, 17, 295-306).

An "Ambler class A β-lactamase" is an enzyme with a very broad spectrum of activity and includes the largest number of enzymes. A β-lactamase called penicillinase which can hydrolyze a penicillin antibacterial agent is mainly included. An extended-spectrum β-lactamase which can hydrolyze not only a penicillin antibacterial agent but also various cephalosporin antibacterial agents as a result of an amino acid substitution in the penicillinase is also classified as the Class A β-lactamase. A carbapenemase, including KPC, which can hydrolyze a carbapenem antibacterial agent, is also classified therein (Bush. K. Nature Reviews Microbiology, 2019, 17, 295-306). The Ambler class A β-lactamase include, for example, Temoneiera (TEM), Sulfhydryl variable (SHV), Cefotaximase-Munich (CTX-M), Guiana extended-spectrum (GES), Pseudomonas extended resistance (PER), Vietnamese extended-spectrum β-lactamase (VEB), Brazilian ESBL (BES), Belgium ESBL (BEL), Serratia fonticola (SFO), Tlahuicas (TLA), KPC, Serratia marcescens enzyme (SME), Not metalloenzyme carbapenemase A (NMC-A), Imipenem-hydrolyzing β-lactamase (IMI), etc. (Reference: Bush. K. Nature Reviews Microbiology, 2019, 17, 295-306).

The phrase "Ambler class B β-lactamase" refers to a metallo-β-lactamase. It hydrolyzes most β-lactams, including a carbapenem antibacterial agent, but does not hydrolyze a monobactam antibacterial agent (Bush. K. Nature Reviews Microbiology, 2019, 17, 295-306). The Ambler class B β-lactamase includes, for example, New Delhi metallo-β-lactamase (NDM), Verona Integron-encoded metallo-β-lactamase (VIM), Imipenemase (IMP), Sao Paulo metallo-β-lactamase (SPM), German Imipenemase (GIM), etc. (Reference: Bush. K. Nature Reviews Microbiology, 2019, 17, 295-306).

The "Ambler Class C β-lactamase" mainly includes a β-lactamase called cephalosporinase which hydrolyzes a cephalosporin antibacterial agent. It can hydrolyze a penicillin antibacterial agent and some monobactam antibacterial agents. The Ambler class C β-lactamase includes, for example, AmpC and Cephamycinase (CMY) (Reference: Bush. K. Nature Reviews Microbiology, 2019, 17, 295-306).

The phrase "Ambler class D β-lactamase" refers to an OXA-β-lactamase that was formerly classified as an oxacillin hydrolase. It is now considered a group of broad enzymes that have variability in a substrate property and an amino acid sequence. It has an amino acid homology to the Class A or Class C β-lactamase of less than 20%. The OXA-β-lactamase often has a higher hydrolytic activity against oxacillin than penicillin G, and some of this group of enzymes can hydrolyze a cephalosporin antibacterial agent or a carbapenem antibacterial agent (Reference: Bush. K. Nature Reviews Microbiology, 2019, 17, 295-306).

The term "Enterobacterales" means bacteria belonging to Enterobacterales. In 2016, some of the species classified in Enterobacteriaceae were reclassified to other families, and it was proposed to use, as a synonym of Enterobacteriaceae, Enterobacterales which is a term indicating a higher-order taxonomic level (order). The Enterobacterales includes seven families: Enterobacteriaceae, Erwiniaceae, Pectobacteriaceae, Yersiniaceae, Hafniaceae, Morganellaceae, Budviciaceae. Enterobacteriaceae includes Escherichia coli, Klebsiella spp., Enterobacter spp., Citrobacter spp., Salmonella spp., Shigella spp., etc., Morganellaceae includes Morganella spp., Proteus spp., Providencia spp., etc., Yersiniaceae includes Yersinia spp. and Serratia spp., etc., and Hafniaceae includes Hafnia spp., Edwardsiella spp., etc. (Reference: Adeolu M. International Journal of Systematic and Evolutionary Microbiology, 2016, 66, 5575-5599).

For "Acinetobacter baumannii complex", Acinetobacter spp. has been confirmed to include at least 22 species and 11 putative species, of which two species A. baumannii and Acinetobacter genomic species 13TU are very important from the viewpoint of an infectious disease. Since it is not possible to distinguish among 4 species A. calcoaceticus, A. baumannii, Acinetobacter genomic species 13TU, and Acinetobacter genomic species 3 by Gram staining, morphological observation, or biochemical properties commonly used in the laboratory, they are comprehensively referred to as Acinetobacter calcoaceticus-Acinetobacter baumannii complex (ACB complex) or Acinetobacter baumannii complex (Peleg AY. Clinical Microbiology Reviews. 2008. 21. 538-582).

For "Enterobacter cloacae complex", Enterobacter spp. has been reported to include 22 species. Since it is difficult to distinguish among E. asburiae, E. cloacae, E. hormaechei, E. kobei, E. ludwigii, E. mori, E. nimipressuralis, etc. by biochemical properties, they are comprehensively referred to as Enterobacter cloacae complex (Reference: Rino Matsui. The Journal of the Japanese Society for Clinical Microbiology. 31 (2). 2021. 113-115).

The phrase "Estimated glomerular filtration rate (eGFR)" is an alternative indicator of a glomerular filtration rate (GFR), and a value corrected for body surface area (unit: mL/min/1.73 $m^2$) is used as one of indicators for diagnosis and severity assessment of a chronic kidney disease. A value calculated using the Japanese GFR estimation formula based on a serum creatinine value, gender, and age has been widely used in Japan. Internationally, the MDRD or CKD-EPI formula has been used (Reference: the Japanese Society of Nephrology ed., TOKYO IGAKUSHA, Evidence-based CKD Clinical Practice Guidelines 2018. 2018.)

The phrase "eGFR uncorrected for body surface area" refers to eGFR converted to a value uncorrected for body surface area (unit: mL/min), which is used for setting a drug dosage for a patient with renal impairment (Reference: the Japanese Society of Nephrology ed., TOKYO IGAKUSHA, CKD Clinical Practice Guidelines 2012. 2012).

In the field of an antibacterial agent, pharmacokinetics (PK)/pharmacodynamics (PD) results of a non-clinical pharmacology test are often used to design an optimal clinical dosage and administration. The term PD refers to a pharmacological effect varying with a drug concentration and time including a minimal inhibitory concentration (MIC) and the term PK refers to a change in drug concentration in the blood or tissue. A combination of PK and PD, PK/PD, is important for an antibacterial agent to maximize its effect. A maximum free drug concentration ($fC_{max}$) divided by MIC ($fC_{max}$/MIC), an area under a free drug concentration curve (fAUC) divided by MIC (fAUC/MIC), and a proportion of time for which a free drug concentration is above MIC in a dosing interval (%fT > MIC) are three main parameters used as the PK/PD parameter.

The PK/PD parameter includes a maximum free drug concentration ($fC_{max}$), a $fC_{max}$ divided by MIC ($fC_{max}$/MIC), an area under a free drug concentration curve (fAUC), a fAUC divided by MIC (fAUC/MIC), and proportion of time for which a free drug concentration is above MIC or a certain threshold concentration in a dosing interval (%fT > MIC, %fT > $C_T$), etc. The PK/PD parameter includes $C_{max}$, $C_{max}$/MIC, AUC, AUC/MIC, %T>MIC, %T>CT), etc. when considering a total drug concentration.

The phrase "%fT > $C_T$" indicates a proportion of time for which a free drug concentration is above a certain threshold concentration ($C_T$) in a dosing interval. For example, %fT > 1 μg/mL indicates a proportion of time for which a free drug concentration is above 1 μg/mL in a dosing interval.

The term fAUC refers to an area under a free drug concentration-time curve.

The phrase "Compound A or a pharmaceutically acceptable salt thereof enhances an activity of meropenem or a pharmaceutically acceptable salt thereof as compared to when not being used in combination" means that the Compound A suppresses degradation of the meropenem by inhibiting a β-lactamase, thereby enhancing an antibacterial activity of the meropenem against a bacterium that can possess a β-lactamase.

As used herein, the phrase "microbial infection disease" or "microbial infection" is one of diseases that the present disclosure targets and refers to invasion of a pathogenic microorganism into a host organism and the host organism may be any of a vertebrate, an invertebrate, fish, a plant, a bird, or a mammal. The phrase also includes overgrowth of a microorganism normally present in the body of an animal, etc. More generally, the microbial infection can be any situation in which the presence of a microbial population is damaging a host animal. Thus, a mammal is said to be "suffering from" a microbial infection if an excessive number of microbial populations are present in the body of the mammal or if the mammal is affected by the presence of the microbial population. If the microorganism is a bacterium, it can be referred to as a bacterial infection or a bacterial infection disease. Specifically, the specification applies to the bacterial infection. A compound of a preferred embodiment is also useful for treating microbial growth or contamination in a cell culture or another medium or on an inanimate surface or an object, and it should be noted that nothing herein should limit the preferred embodiment to a treatment of a higher organism unless expressly defined in the claims.

As used herein, the term "subject" or "test subject" means a human or a non-human mammal, e.g., a dog, a cat, a mouse, a rat, cattle, a sheep, a pig, a goat, a non-human primate or a bird, e.g., chicken, or another vertebrate or

invertebrate. The "subject" may be referred to as a "patient" if the subject is suffering from a disease.

In the present disclosure, a therapeutic effect refers to alleviation of one or more symptoms of an infection to some extent, including a cure of the infection. The term "cure" means elimination of a symptom of an active infection, including elimination of an excess symptom. Even after the cure is achieved, a certain long-term or permanent effect of the infection (e.g., extensive tissue damage) may still be present, but this case is also included in the cure.

As used herein, the term "treat", "care", or "administer" generally refers to administration of a pharmaceutical composition for a prophylactic and/or therapeutic purpose. The phrase "prophylactic treatment" refers to a treatment of a patient who has not been infected yet but who is susceptible to or otherwise at risk for a specific infection, and can decrease a likelihood of developing the infection. The phrase "therapeutic treatment" refers to a treatment of a patient who has already been suffering from an infection.

(Lactamase inhibitory active compound of the present disclosure)

In one aspect of the present disclosure, a compound provided in combination as an antibiotic may include specifically a compound represented by Formula (1a), (1b), or (2) below: or

[Chem. 4]

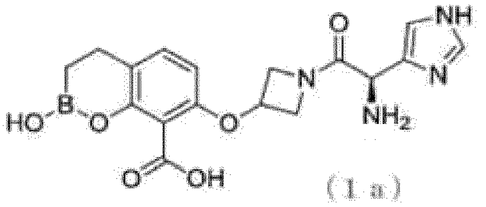

(1a)

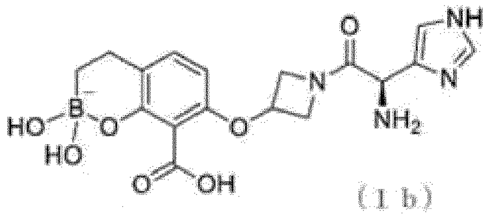

(1b)

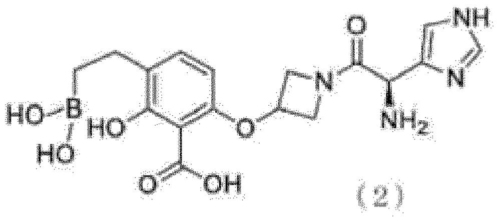

(2)

or a pharmaceutically acceptable salt thereof. The compound of Formula (2) can be interconverted with the compound of Formula (1a) through equilibrium reactions in aqueous solutions or in vivo, and thus, can be biologically equivalent to the compound of Formula (1a). The compound of Formula (1a), (1b), or (2) may also be referred to herein as "Compound A".

The Compound A, when in a form of Formula (2), can exist as a condensate by intermolecular condensation of a phenol group and a carboxyl group on a benzene ring with a boronic acid moiety. This condensation can occur between two or more Compound A molecules.

Preferably, an embodiment of the compound of the present disclosure (Compound A) may be specifically exemplified as a compound represented by Formula (1a) or (2) below:

[Chem. 5]

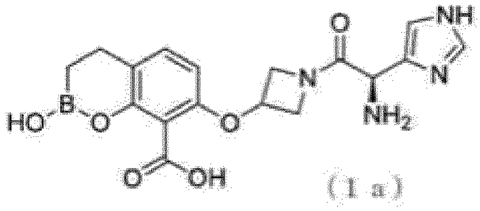

(1 a)

or

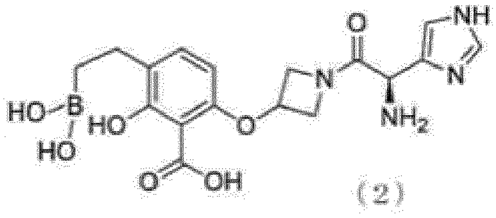

(2)

or a pharmaceutically acceptable salt thereof.

More preferably, an embodiment of the compound of the present disclosure (Compound A) may also be specifically exemplified as a compound represented by Formula (1a) below:

[Chem. 6]

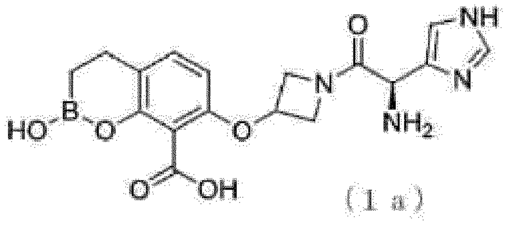

(1 a)

or a pharmaceutically acceptable salt thereof.

The compound (Compound A) of the present disclosure will be further described.

The compound of the present disclosure may include tautomers, stereoisomers such as geometric isomers and optical isomers , depending on a type of substituent. The present disclosure encompasses them as well. Or, in case the compound of the present disclosure has one or more asymmetric carbon atoms, diastereomers and an optical isomers thereof may exist. In such cases, any mixtures and/or isolated products of the diastereomers and the optical isomers are also included in the compound of the present disclosure.

The compound of the present disclosure (Compound A) can also exist in the structure represented by Formula (2) under equilibrium conditions, due to environmental factors such as temperature or humidity, or a physical factor such as being in solid, liquid, or solution form. The compound of the present disclosure also encompasses them.

[Chem. 7]

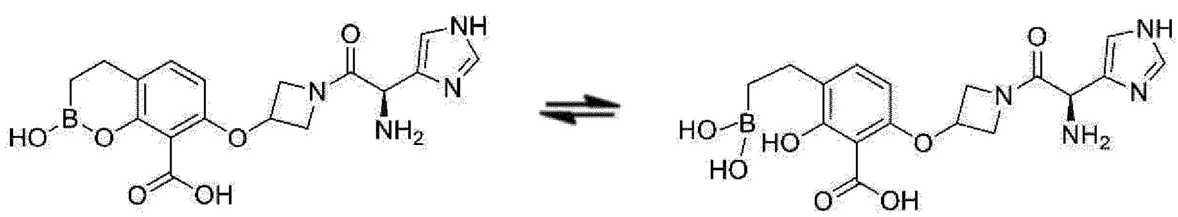

(1 a) (2)

Furthermore, for example, the structures of the compound (Compound A) of Examples in the present application arebased on estimation which is considered to be the most reasonable by those skilled in the art by using proton nuclear magnetic resonance spectroscopy (1H-NMR), liquid chromatography-mass spectrometry (LCMS), etc. On the other

hand, the structure estimation is based on each of the specific measurement conditions. In particular, the structures of Formulae (1a), (1b), and (2) may be interconverted with each other or may partially be converted into any one of them and become mixture due to properties of individual compounds, various environmental conditions such as temperature or humidity, or a physical factors such as being in solid, liquid, or solution form.

The compound of the present disclosure (Compound A) also includes various hydrates, solvates, and crystalline polymorphs.

Furthermore, the compound of the present disclosure (Compound A) may be substituted with isotopes (e.g., 2H (or D), 3H (or T), 11C, 13C, 14C, 13N, 15N, 150, 35S, 18F, 125I, etc.) and these compounds are also included in the compound of the present disclosure.

Furthermore, the scope of the present disclosure also includes prodrugs of the compound of the present disclosure (Compound A). In the present disclosure, the term "prodrug(s)"refers to a derivative that is degraded in vivo by hydrolysis or enzymatically to give the compound of Formula (1a), (1b), or (2). For example, in case the compound of Formula (1a), (1b), or (2) has a hydroxyl group, an amino group, or a carboxyl group, these groups can be modified according to conventional methods to produce a prodrug.

For example, in case of the compound having a carboxy group, the examples may include each compound where the carboxyl group is converted to an alkoxycarbonyl group, an alkylthiocarbonyl group, or an alkylaminocarbonyl group.

For example, in case of the compound having an amino group, the examples may include each compound where the amino group is substituted with an alkanoyl group to form an alkanoylamino group, substituted with an alkoxycarbonyl group to form an alkoxycarbonylamino group, substituted with an alkanoyloxy methylamino group, or converted to hydroxylamine.

For example, in case of the compound having a hydroxyl group, the examples may include each compound where the hydroxyl group is substituted with an alkanoyl group to form an alkanoyloxy group, converted to a phosphate ester, or substituted with an alkanoyloxy methyl group to form an alkanoyloxy methyl group.

An alkyl moiety of the group to be used for forming the prodrug may be the aforementioned alkyl group, which may be substituted with, for example, an alkoxy group. Preferred examples thereof include those described below.

Examples of the compound in which the carboxyl group is converted to an alkoxycarbonyl group include alkoxycarbonyl such as methoxycarbonyl or ethoxycarbonyl, or alkoxycarbonyl substituted by an alkoxy group such as methoxymethoxycarbonyl, ethoxymethoxycarbonyl, 2-methoxyethoxycarbonyl, 2-methoxyethoxymethoxycarbonyl, or pivaloyloxymethoxycarbonyl.

As used herein, the phrase "pharmaceutically acceptable salt" refers to a salt that retains a biological effect and property of the compound of the preferred embodiment and is biologically or otherwise undesirable. The compound of the preferred embodiment can often form an acid salt and/or a base salt due to the presence of an amino group and/or a carboxyl group or similar groups. A pharmacologically acceptable acid addition salt can be formed with an inorganic acid and an organic acid. Examples of an inorganic acid that can form a salt include, for example, hydrochloric acid, hydrobromic acid, sulfuric acid, nitric acid, phosphoric acid, etc. Examples of an organic acid from which a salt can be derived include acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, etc. A pharmacologically acceptable base addition salt can be formed with an inorganic base and an organic base. Examples of an inorganic base which can form a salt include, for example, sodium, potassium, lithium, ammonium, calcium, magnesium, iron, zinc, copper, manganese, and aluminum, with an ammonium salt, a potassium salt, a sodium salt, a calcium salt, and a magnesium salt being particularly preferred. Examples of an organic base that can form a salt include, for example, a primary, secondary, and tertiary amine, a substituted amine including a naturally occurring substituted amine, a cyclic amine, a basic ion exchange resin, etc. Specifically, isopropylamine, trimethylamine, diethylamine, triethylamine, tripropylamine, or ethanolamine may be used. Many such salts are known in the art as described in WO 87/05297 by Johnston et al., published September 11, 1987 (incorporated herein by reference in its entirety). Thus, the "pharmaceutically acceptable salt" representatively means an acid addition salt and a base addition salt that are acceptable for pharmacological use. Examples of the "pharmaceutically acceptable salt" include, but are not limited to, an acid addition salt such as an acetate, a propionate, a butyrate, a formate, a trifluoroacetate, a maleate, a fumarate, a tartrate, a citrate, a stearate, a succinate, an ethyl succinate, a malonate, a lactobionate, a gluconate, a glucoheptonate, a benzoate, a methanesulfonate, a benzenesulfonate, a paratoluenesulfonate (tosylate), a lauryl sulfate, a malate, an ascorbate, a mandelate, a saccharinate, a xinafoate, a pamoate, a cinnamate, an adipate, a cysteine salt, an N-acetylcysteine salt, a hydrochloride, a hydrobromide, a phosphate, a sulfate, a hydroiodide, a nicotinate, an oxalate, a picrate, a thiocyanate, a undecanoate, an acrylic acid polymer salt, and a carboxyvinyl polymer; an inorganic base addition salt such as a lithium salt, a sodium salt, a potassium salt, and a calcium salt; an organic base addition salt such as morpholine and piperidine; and an addition salt with an amino acid such as aspartic acid and glutamic acid. Examples of the pharmaceutically acceptable salt of the compound of the present disclosure include, but are not limited to, a hydrochloride salt and a sodium salt.

As used herein, the phrase "pharmaceutically acceptable carrier" or "pharmaceutically acceptable excipient" is

intended to include any and all solvents, dispersing media, coating agents, antibacterial and antifungal agents, tonicity agents, absorption retardants, inert components, etc. A use of such media and agents for a pharmaceutically active substance is well-known in the art. However, this does not apply if they are incompatible with conventional media or agents. The "pharmaceutically acceptable carrier" or "pharmaceutically acceptable excipient" is contemplated for use with an active component in a therapeutic composition. A supplemental active component can also be incorporated into the composition. In addition, various adjuvants may be included as commonly used in the art. These and other such compounds are described in the literature, e.g., Merck Index, Merck & Company, Rahway, N.J. A consideration for inclusion of various components in a pharmaceutical composition is described, for example, in Gilman et al. (Eds) (1990); Goodman and Gilman's. The Pharmacological Basis of Therapeutics, 8th Ed., Pergamon Press.

As used herein, the term "solvate" is a compound formed by interaction of a solvent with an EPI, a metabolite, or a salt thereof. A pharmaceutically acceptable solvate including a hydrate is a suitable solvate.

As used herein, the phrase "XX mg of Compound A on a free form basis" or "XX mg of Compound A in a free form", etc. means that XX mg of Compound A in whatever form that is disclosed herein is included in terms of a free form (liberated form) of the compound represented by Formula (1a) among the Compound A, i.e., in terms of a form that is not a solvate such as a pharmaceutically acceptable salt or hydrate.

As used herein, the phrase "XX mg of meropenem on a free form basis" or "XX mg of meropenem in a free form", etc. means that XX mg of meropenem in whatever form is included in terms of a free form (liberated form) of the meropenem, i.e., in terms of a form that is not a solvate such as a pharmaceutically acceptable salt or hydrate.

The compound of the present disclosure can be formed into a formulation, medicament, or pharmaceutical composition and administered by oral or parenteral administration, either directly or by using an appropriate dosage form. Specific examples of the dosage form include, but are not limited to, a tablet, a capsule, a powder, a granule, a solution, a suspension, an injection, a patch, and a poultice. The formulation can be produced by a known method using an additive that is used as an ordinary pharmaceutical additive.

The additive may be an excipient, a disintegrant, a binder, a fluidizer, a lubricant, a coating agent, a dissolving agent, a dissolution aid, a thickener, a dispersant, a stabilizer, a sweetener, and a flavoring agent depending on a purpose. Specific examples of the additive include, but are not limited to, lactose, mannitol, crystalline cellulose, low-substituted hydroxypropyl cellulose, corn starch, partially pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, talc, etc.

The compound included in the present disclosure is a compound with an inhibitory activity against a β-lactamase. Therefore, when the compound is used in combination with an antibacterial agent, a useful prophylactic or therapeutic agent for a bacterial infection can be obtained. Specific examples of the bacterial infection include sepsis, febrile neutropenia, bacterial meningitis, bacterial endocarditis, otitis media, sinusitis, pneumonia, lung abscess, empyema, a secondary infection of a chronic respiratory tract lesion, pharyngolaryngitis, tonsillitis, osteomyelitis, arthritis, peritonitis, intra-abdominal abscess, cholecystitis, cholangitis, liver abscess, a deep skin infection, lymphangitis and lymphadenitis, a secondary infection such as trauma, burn, and a surgical wound, a urinary tract infection, a genital infection, an ocular or dental infection, a complicated urinary tract infection, a complicated intra-abdominal infection, hospital-acquired pneumonia, ventilator-associated pneumonia, etc., in particular, a complicated urinary tract infection, a complicated intra-abdominal infection, hospital-acquired pneumonia, ventilator-associated pneumonia.

(Treatment method)

In one aspect, the present disclosure provides a method for treatment or prevention.

Some embodiments described herein relate to a method for treating a bacterial infection, the method including administering a composition including Compound A or a pharmaceutically acceptable salt thereof and a β-lactam agent to a subject in need thereof to achieve a plasma concentration $C_{max}$ of Compound A of about 2 mg/L to about 300 mg/L. In some embodiments, the plasma concentration $C_{max}$ of Compound A achieved by the method described herein is about 5 mg/L to 200 mg/L. In some embodiments, the plasma concentration $C_{max}$ of Compound A achieved by the method described herein is about 10 mg/L to 150 mg/L. In some embodiments, the plasma concentration $C_{max}$ of Compound A achieved by the method described herein is about 20 mg/L to 100 mg/L.

Some embodiments described herein relate to a method for treating a bacterial infection, the method including administering a composition including Compound A or a pharmaceutically acceptable salt thereof and a carbapenem antibacterial agent to a subject in need thereof so as to give a 24-hour AUC of Compound A of about 20 mg*h/L to about 3000 mg*h/L. In some embodiments, the 24-hour AUC of Compound A achieved by the method described herein is about 50 mg*h/L to 2700 mg*h/L. In some embodiments, the AUC of Compound A achieved by the method described herein is about 100 mg*h/L to 2000 mg*h/L. In some embodiments, the 24-hour AUC of Compound A achieved by the method described herein is about 250 mg*h/L to 1000 mg*h/L.

The compound of the present disclosure can be used in combination with at least one or more drugs selected from

an antibacterial drug, an antifungal drug, an antiviral drug, an anti-inflammatory drug, or an antiallergic drug to treat one or more bacterial infections described herein. The drug is preferably an antibacterial drug, further preferably a β-lactam agent, specifically meropenem.

Specifically, another preferred embodiment of the β-lactam agent includes amoxicillin, ampicillin (pivampicillin, hetacillin, bacampicillin, metampicillin, talampicillin), epicillin, carbenicillin (calindacillin), ticarcillin, temocillin, azulocillin, piperacillin, mezulocillin, mesilinam (pivmecillinam), sulbenicillin, benzylpenicillin (G), clometocillin, benzathine benzylpenicillin, procaine benzylpenicillin, azidocillin, penamecillin, phenoxymethylpenicillin (V), propicillin, benzathine phenoxymethylpenicillin, phenethycillin, cloxacillin (dicloxacillin, flucloxacillin), oxacillin, methicillin, nafcillin, faropenem, biapenem, doripenem, ertapenem, tebipenem, imipenem, panipenem, tomopenem, rasupenem, cephazolin, cephacetril, cephadroxil, cephalexin, cephaloglycine, cephalonium, cepharolidine, cephalothin, cephapirin, cephatridine cefazedone, cefazafur, cefurazine, cefuroxazine, ceftezole, cefaclor, cefamandole, cefominox, cefoniside, ceforanide, cefothiam, cefprozil, cefbuperazone, cefuroxime, cefzonum, cefoxitin, cefotetan, cefmetazole, loracarbef, cefixime, ceftazidime, ceftriaxone, cefcapene, cefdaroxime, cefdinil, cefditoren, cefetamet, cefmenoxime, cefodizime, cefoperazone, cefotaxime, cefpimizole, cefpyramide, cefpodoxime, cefthrogin, ceftelam, ceftibuten, ceftioren, ceftizoxime, flomoxef, latamoxef, cefepime, cefozopran, cefpirom, cefquinom, ceftobiprole, ceftaroline, CXA-101, RWJ-54428, MC-04546, ME1036, BAL30072, SYN2416, ceftiofur, cefquinom, cefovecin, aztreonam, tigenonam, carmonam, RWJ-442831, RWJ-333441, or RWJ-3334442. An administration timing of the compound and the therapeutic agent is not limited, and they may be administered to a subject at the same time or at different times. They can also be used as a mixture. A dosage of the therapeutic agent can be appropriately selected based on a clinically used dose by taking the same approach as in the present disclosure. A compounding ratio of the compound to the therapeutic agent can be appropriately selected according to a subject to be administered, a route of administration, a target disease, a symptom, a combination, etc.

In some embodiments, the carbapenem antibacterial agent is selected from the group consisting of imipenem, panipenem, biapenem, tebipenem, doripenem, meropenem, and ertapenem. In some such embodiments, the carbapenem antibacterial agent is meropenem.

As used herein, a dose of Compound A is a dose in terms of a free form (free form corresponding to the compound of Formula 1a) unless otherwise noted.

In some embodiments, Compound A is administered in a dose range of about 0.2 mg/kg to about 200 mg/kg body weight. In some further embodiments, Compound A is administered in a dose range of about 0.5 mg/kg to about 150 mg/kg body weight. In some embodiments, Compound A is administered in a dose range of about 0.5 mg/kg to about 100 mg/kg. In some embodiments, Compound A is administered in a dose range of about 2.5 mg/kg to about 100 mg/kg.

In some embodiments, plasma clearance of Compound A achieved by the method described herein is about 0.001 L/h/kg to about 1 L/h/kg. In some embodiments, plasma clearance of Compound A achieved by the method described herein is 0.01 L/h/kg to about 0.5 L/h/kg. In some embodiments, plasma clearance of Compound A achieved by the method described herein is about 0.02 L/h/kg to about 0.2 L/h/kg.

In some embodiments, a half-life of Compound A achieved by the method described herein is about 0.5 hours to about 100 hours. In some embodiments, a half-life of Compound A achieved by the method described herein is about 1 hour to about 6 hours. In some embodiments, a half-life of Compound A achieved by the method described herein is about 1.5 hours to about 3 hours.

The above-described pharmacokinetic parameters can be achieved through a variety of methods including selection of a dose, duration of administration (e.g., intravenous infusion rate), and selection of formulation (e.g., selection of immediate or sustained release formulation).

In some embodiments, the composition is administered intravenously.

Some embodiments include co-administering Compound A, an enantiomer, a diastereomer, a tautomer, or a pharmaceutically acceptable salt thereof, a composition, and/or a pharmaceutical composition described herein with a carbapenem antibacterial agent. The "co-administration" means that two or more agents may be found simultaneously in the bloodstream of a patient, regardless of when or how they are actually administered. In one embodiment, the agents are administered simultaneously. In one such an embodiment, administration by a combination is accomplished by combining the agents in a single dosage form. When the agents are combined into a single dosage form, they may be physically mixed (e.g., by co-dissolution or dry mixing), or they may form an adduct or be covalently bonded so that they are separated into two or more active components upon administration to a patient. In another embodiment, the agents are administered sequentially. In one embodiment, the agents are administered by the same route such as orally or intravenously. In another embodiment, the agents are administered by different routes, for example, one is administered orally and the other intravenously.

A further embodiment includes administering a combination of Compound A, a carbapenem antibacterial agent, and an additional pharmaceutical combination selected from an antibacterial agent, an antifungal agent, an antiviral agent, an anti-inflammatory agent, or an antiallergic agent to a subject in need thereof.

In one embodiment of the medicament of the present disclosure, a therapeutically effective amount of Compound

A or a pharmaceutically acceptable salt thereof and meropenem or a pharmaceutically acceptable salt thereof result in 30% or more of %fT > 1 μg/mL, 100 or more of %fT > 4 ug/mL, or 70 μg·h/mL or more of an fAUC in terms of a plasma concentration of the Compound A. In one embodiment, the bacterial infection is a bacterial infection involving a bacterium selected from:

[Chem. 8]

***Enterobacteriales, Acinetobacter* baumannii complex, *Pseudomonas aeruginosa* and *Bacteroides* spp.**

In one embodiment, the bacterial infection is a bacterial infection involving:

[Chem. 9]

***Escherichia coli, Klebsiella pneumoniae, Enterobacter cloacae* complex or *Acinetobacter baumannii* complex**

In one embodiment, the bacterial infection is a complicated urinary tract infection, a complicated intra-abdominal infection, a hospital-acquired pneumonia, or a ventilator-associated pneumonia.

In one embodiment, the medicament of the present disclosure is used for a patient suffering from a bacterium susceptible to a combination of Compound A or a pharmaceutically acceptable salt thereof and meropenem or a pharmaceutically acceptable salt thereof. In one embodiment, the medicament of the present disclosure has a metallo-β-carbapenemase inhibitory activity. In one embodiment, the medicament of the present disclosure has an antibacterial activity against Acinetobacter baumannii.

In another embodiment of the present disclosure, when a pharmaceutical composition including an antibacterial agent such as a β-lactam agent is used, the compound of the present disclosure can be administered in combination therewith at the same time or at different times. Such a pharmaceutical composition including a β-lactam agent is also within the scope of the present disclosure and can be used for treating or preventing a bacterial infection such as sepsis, febrile neutropenia, bacterial meningitis, bacterial endocarditis, otitis media, sinusitis, pneumonia, lung abscess, empyema, a secondary infection of a chronic respiratory tract lesion, pharyngolaryngitis, tonsillitis, osteomyelitis, arthritis, peritonitis, intra-abdominal abscess, cholecystitis, cholangitis, liver abscess, a deep skin infection, lymphangitis and lymphadenitis, a secondary infection such as trauma, burn, and a surgical wound, a urinary tract infection, a genital infection, an ocular or dental infection, a complicated urinary tract infection, a complicated intra-abdominal infection, hospital-acquired pneumonia, and ventilator-associated pneumonia.

Such a medicament, formulation, or pharmaceutical composition can be produced by mixing the compound of the present disclosure and/or an additional agent (e.g., an antibacterial agent such as a β-lactam agent) with any appropriate component concomitantly or separately as a mixture or as separate agents using any technique known in the art and can be formulated into an appropriate formulation such as a tablet, a capsule, a powder, a granule, a solution, a suspension, an injection, a patch, or a poultice using any technique known in the art. If the compound of the present disclosure and/or an additional agent (e.g., an antibacterial agent such as a β-lactam agent) is prepared as separate agents, they may be provided as a kit of two agents, and may be provided with an instruction (e.g., package insert) instructing that one component is provided as a single agent and the other component (the additional agent (e.g., β-lactam agent) in the case of the compound of the present disclosure or the compound of the present disclosure in the case of the additional agent (e.g., β-lactam agent) is administered in combination at the same time or at different times.

When the compound of the present disclosure is used as an active component in a medicament, it is not intended for use only in a human, but can also be used in another animal other than a human (a cat, a dog, cattle, a chicken, fish, etc.).

A dosage of the compound of the present disclosure is appropriately selected depending on a nature of an animal (human) to be administered, a route of administration, a disease, age, weight, and symptom of a patient according to a measure described herein. It can also be appropriately determined taking the PK/PD parameters of the compound of the present disclosure into consideration. For example, those skilled in the art may determine appropriate dosage and administration with reference to description, for example, in Examples for the dosage and administration of the present disclosure appropriately taking a measure such as a maximum free drug concentration ($fC_{max}$), a $fC_{max}$ divided by MIC ($fC_{max}$/MIC), an area under a free drug concentration curve (fAUC), a fAUC divided by MIC (fAUC/MIC), or a proportion of time for which a free drug concentration is above MIC or a certain threshold concentration in a dosing interval (%fT > MIC, %fT > $C_T$) into consideration.

In one embodiment, the medicament of the present disclosure is administered intravenously to a subject at a frequency of at least once every 24 hours. In one embodiment, the medicament of the present disclosure is administered intravenously to the subject at a frequency of at least once every 8 hours. In one embodiment, Compound A of the present disclosure or a pharmaceutically acceptable salt thereof is administered intravenously to the subject at a frequency of at least once every 8 hours. In one embodiment, meropenem or a pharmaceutically acceptable salt thereof is administered intravenously to the subject at a frequency of at least once every 8 hours.

In one embodiment, the medicament of the present disclosure is administered by intravenous infusion for 3 hours. In one embodiment, the medicament of the present disclosure is administered by intravenous infusion for 3 hours or more. In one embodiment, the medicament of the present disclosure is administered by intravenous infusion for 3 hours or less. In one embodiment, the medicament of the present disclosure is administered by intravenous infusion for 4 hours. In one embodiment, the medicament of the present disclosure is administered by intravenous infusion for 4 hours or more. In

one embodiment, the medicament of the present disclosure is administered by intravenous infusion for 4 hours or less. In one embodiment, Compound A or a pharmaceutically acceptable salt thereof and meropenem or a pharmaceutically acceptable salt thereof are administered concomitantly or separately. In one embodiment, Compound A or a pharmaceutically acceptable salt thereof and meropenem or a pharmaceutically acceptable salt thereof are included in a same agent or separate agents.

In one embodiment, the medicament of the present disclosure includes Compound A or a pharmaceutically acceptable salt thereof and meropenem or a pharmaceutically acceptable salt thereof in a ratio of 1:1 to 1:10 by mass on a free form basis. In one embodiment, the medicament of the present disclosure includes Compound A or a pharmaceutically acceptable salt thereof and meropenem or a pharmaceutically acceptable salt thereof in a ratio of 1:1, 1:1.3, 1:2, 1:2.7, 1:3, 1:4, 1:5, 1:6, 1:7, 1:8, 1:9, or 1:10 by mass on a free form basis.

For example, for intravenous administration, a lower limit is 10 mg (preferably 100 mg) and an upper limit is 10000 mg (preferably 6000 mg) per day for an adult, and such an amount can be administered once a day or divided into several doses.

In one embodiment, the medicament of the present disclosure can be used for the same application as AVYCAZ (ceftazidime and avibactam) and RECARBRIO (imipenem-cilastatin-relebactam) or for identifying a target bacterium.

In one preferred embodiment, dosage and administration may be advantageously adjusted for a renal disorder patient with eGFR of less than 50 mL/min/1.73 $m^2$.

[Table A]

| eGFR (mL/m in/1. 73$m^2$) | Recommended dosage and administration Meropenem · Compound A of the present disclosure: **Dose combination** | Dosing interval |
|---|---|---|
| 30~49 | Meropenem · Compound A of the present disclosure: **Dose combination** (Compound A/Meropenem : (1g/2g), (0.5g/1g), (0.75g/2g), or (0.375g/ 1g) | Every 8 hours or every 12 hours |
| 15~29 | Meropenem · Compound A of the present disclosure: **Dose combination** (Compound A/Meropenem : (0.5g/1g), (0.25g/1 g), (0.375g/1g), or (0.1 88g/1g) | Every 8 hours or every 12 hours |
| Less than 15 | Meropenem · Compound A of the present disclosure: **Dose combination** (Compound A/Meropenem : (0.25g/0.5g), (0.12 5g/0.5g), (0.188g/0.5 g), or (0.094g/0.5g) | every 12 hours |

In one embodiment, the medicament of the present disclosure is used for a patient with renal impairment by adjusting doses of Compound A and meropenem using an eGFR uncorrected for body surface area as an indicator.

In one embodiment, the medicament of the present disclosure is used for a patient with renal impairment by adjusting doses of Compound A and meropenem using an estimated glomerular filtration rate (eGFR) as an indicator.

In one embodiment, the medicament of the present disclosure is used for a patient with renal impairment by adjusting doses of Compound A and meropenem using creatinine clearance (mL/min) as an indicator.

In one embodiment, the medicament of the present disclosure is used for a patient with an estimated glomerular filtration rate (eGFR) of 50 mL/min/1.73 $m^2$ or more in doses of 1 g of Compound A and 2 g of meropenem every 8 hours.

In one embodiment, the medicament of the present disclosure is used for a patient with an eGFR uncorrected for body surface area of 50 mL/min or more in doses of 1 g of Compound A on a free form basis and 2 g of meropenem on a free form basis every 8 hours.

In one embodiment, the medicament of the present disclosure is used for a patient with an estimated glomerular filtration rate (eGFR) of 50 mL/min/1.73 $m^2$ or more in doses of 1 g of Compound A on a free form basis and 2 g of meropenem on a free form basis every 12 hours.

In one embodiment, the medicament of the present disclosure is used for a patient with an estimated glomerular filtration rate (eGFR) of 50 mL/min/1.73 $m^2$ or more in doses of 0.5 g of Compound A on a free form basis and 1 g of meropenem on a free form basis every 8 hours.

In one embodiment, the medicament of the present disclosure is used for a patient with an estimated glomerular filtration rate (eGFR) of 50 mL/min/1.73 $m^2$ or more in doses of 0.5 g of Compound A on a free form basis and 1 g of meropenem on a free form basis every 12 hours.

In one embodiment, the medicament of the present disclosure is used for a patient with an estimated glomerular filtration rate (eGFR) of 30 to 49 mL/min/1.73 $m^2$ in doses of 1 g of Compound A on a free form basis and 2 g of meropenem on a free form basis every 12 hours.

In one embodiment, the medicament of the present disclosure is used for a patient with an estimated glomerular

filtration rate (eGFR) of 30 to 49 mL/min/1.73 $m^2$ in doses of 0.5 g of Compound A on a free form basis and 1 g of meropenem on a free form basis every 8 hours.

In one embodiment, the medicament of the present disclosure is used for a patient with an eGFR uncorrected for body surface area of 30 to 49 mL/min in doses of 0.5 g of Compound A on a free form basis and 1 g of meropenem on a free form basis every 8 hours.

In one embodiment, the medicament of the present disclosure is used for a patient with an estimated glomerular filtration rate (eGFR) of 30 to 49 mL/min/1.73 $m^2$ in doses of 0.5 g of Compound A on a free form basis and 1 g of meropenem on a free form basis every 12 hours.

In one embodiment, the medicament of the present disclosure is used for a patient with an estimated glomerular filtration rate (eGFR) of 15 to 29 mL/min/1.73 $m^2$ in doses of 0.5 g of Compound A on a free form basis and 1 g of meropenem on a free form basis every 8 hours.

In one embodiment, the medicament of the present disclosure is used for a patient with an estimated glomerular filtration rate (eGFR) of 15 to 29 mL/min/1.73 $m^2$ in doses of 0.5 g of Compound A on a free form basis and 1 g of meropenem on a free form basis every 12 hours.

In one embodiment, the medicament of the present disclosure is used for a patient with an eGFR uncorrected for body surface area of 15 to 29 mL/min in doses of 0.5 g of Compound A on a free form basis and 1 g of meropenem on a free form basis every 12 hours.

In one embodiment, the medicament of the present disclosure is used for a patient with an estimated glomerular filtration rate (eGFR) of 15 to 29 mL/min/1.73 $m^2$ in doses of 0.25 g of Compound A on a free form basis and 1 g of meropenem on a free form basis every 8 hours.

In one embodiment, the medicament of the present disclosure is used for a patient with an estimated glomerular filtration rate (eGFR) of 15 to 29 mL/min/1.73 $m^2$ in doses of 0.25 g of Compound A on a free form basis and 1 g of meropenem on a free form basis every 12 hours.

In one embodiment, the medicament of the present disclosure is used for a patient with an eGFR uncorrected for body surface area of 15 to 29 mL/min in doses of 0.25 g of Compound A on a free form basis and 1 g of meropenem on a free form basis every 12 hours.

In one embodiment, the medicament of the present disclosure is used for a patient with an estimated glomerular filtration rate (eGFR) of less than 15 mL/min/1.73 $m^2$ in doses of 0.25 g of Compound A on a free form basis and 0.5 g of meropenem on a free form basis every 12 hours.

In one embodiment, the medicament of the present disclosure is used for a patient with an eGFR uncorrected for body surface area of less than 15 mL/min in doses of 0.25 g of Compound A on a free form basis and 0.5 g of meropenem on a free form basis every 12 hours.

In one embodiment, the medicament of the present disclosure is used for a patient with an estimated glomerular filtration rate (eGFR) of less than 15 mL/min/1.73 $m^2$ in doses of 0.125 g of Compound A on a free form basis and 0.5 g of meropenem on a free form basis every 12 hours.

In one embodiment, the medicament of the present disclosure is used for a patient with an eGFR uncorrected for body surface area of less than 15 mL/min in doses of 0.125 g of Compound A on a free form basis and 0.5 g of meropenem on a free form basis every 12 hours.

In one embodiment, the medicament of the present disclosure is used for a patient with creatinine clearance of 40 mL/min or more in doses of 1 g of Compound A on a free form basis and 2 g of meropenem on a free form basis every 8 hours.

In one embodiment, the medicament of the present disclosure is used for a patient with creatinine clearance of 20 to 39 mL/min in doses of 0.5 g of Compound A on a free form basis and 1 g of meropenem on a free form basis every 8 hours.

In one embodiment, the medicament of the present disclosure is used for a patient with creatinine clearance of 10 to 19 mL/min in doses of 0.5 g of Compound A on a free form basis and 1 g of meropenem on a free form basis every 12 hours.

In one embodiment, the medicament of the present disclosure is used for a patient with creatinine clearance of less than 10 mL/min in doses of 0.25 g of Compound A on a free form basis and 0.5 g of meropenem on a free form basis every 12 hours.

In one embodiment, the medicament of the present disclosure is used for a patient with an estimated glomerular filtration rate (eGFR) of 50 mL/min/1.73 $m^2$ or more in doses of 0.75 g of Compound A on a free form basis and 2 g of meropenem on a free form basis every 8 hours.

In one embodiment, the medicament of the present disclosure is used for a patient with an eGFR uncorrected for body surface area of 50 mL/min or more in doses of 0.75 g of Compound A on a free form basis and 2 g of meropenem on a free form basis every 8 hours.

In one embodiment, the medicament of the present disclosure is used for a patient with an estimated glomerular filtration rate (eGFR) of 30 to 49 mL/min/1.73 $m^2$ in doses of 0.75 g of Compound A on a free form basis and 2 g of meropenem on a free form basis every 12 hours.

In one embodiment, the medicament of the present disclosure is used for a patient with an estimated glomerular filtration rate (eGFR) of 30 to 49 mL/min/1.73 $m^2$ in doses of 0.375 g of Compound A on a free form basis and 1 g of meropenem on a free form basis every 8 hours.

In one embodiment, the medicament of the present disclosure is used for a patient with an eGFR uncorrected for body surface area of 30 to 49 mL/min in doses of 0.375 g of Compound A on a free form basis and 1 g of meropenem on a free form basis every 8 hours.

In one embodiment, the medicament of the present disclosure is used for a patient with an estimated glomerular filtration rate (eGFR) of 15 to 29 mL/min/1.73 $m^2$ in doses of 0.375 g of Compound A on a free form basis and 1 g of meropenem on a free form basis every 12 hours.

In one embodiment, the medicament of the present disclosure is used for a patient with an eGFR uncorrected for body surface area of 15 to 29 mL/min in doses of 0.375 g of Compound A on a free form basis and 1 g of meropenem on a free form basis every 12 hours.

In one embodiment, the medicament of the present disclosure is used for a patient with an estimated glomerular filtration rate (eGFR) of less than 15 mL/min/1.73 $m^2$ in doses of 0.1875 g of Compound A on a free form basis and 0.5 g of meropenem on a free form basis every 12 hours.

In one embodiment, the medicament of the present disclosure is used for a patient with an eGFR uncorrected for body surface area of less than 15 mL/min in doses of 0.1875 g of Compound A on a free form basis and 0.5 g of meropenem on a free form basis every 12 hours.

In one embodiment, the medicament of the present disclosure is used for a patient with creatinine clearance of 40 mL/min or more in doses of 0.75 g of Compound A on a free form basis and 2 g of meropenem on a free form basis every 8 hours.

In one embodiment, the medicament of the present disclosure is used for a patient with creatinine clearance of 20 to 39 mL/min in doses of 0.375 g of Compound A on a free form basis and 1 g of meropenem on a free form basis every 8 hours.

In one embodiment, the medicament of the present disclosure is used for a patient with creatinine clearance of 10 to 19 mL/min in doses of 0.375 g of Compound A on a free form basis and 1 g of meropenem on a free form basis every 12 hours.

In one embodiment, the medicament of the present disclosure is used for a patient with creatinine clearance of less than 10 mL/min in doses of 0.1875 g of Compound A on a free form basis and 0.5 g of meropenem on a free form basis every 12 hours.

In one embodiment, a dose of Compound A to be administered ranges from 100 mg to 6000 mg on a free form basis. In one embodiment, a dose of Compound A to be administered falls within a range between a certain lower limit and a certain upper limit, examples of the lower limit include, but are not limited to, about 100, about 125, about 150, about 200, about 250, about 300, about 350, about 400, about 450, about 500, about 550, about 600, about 650, about 700, about 750, about 800, about 850, about 900, about 950, and about 1000 mg and examples of the upper limit include, but are not limited to, about 250, about 300, about 350, about 400, about 450, about 500, about 550, about 600, about 650, about 700, about 750, about 800, about 850, about 900, about 950, about 1000, about 1250, about 1500, about 2000, about 2500, about 3000, about 3500, about 4000, about 4500, about 5000, about 5500, about 6000. In one embodiment, the dose of the Compound A is a single dose. In one embodiment, the dose of the Compound A is a daily dose.

In one embodiment, examples of a range of a dose of Compound A to be administered include, but are not limited to, about 100 to about 250 mg, about 100 to about 500 mg, about 100 to about 1000 mg, about 125 to about 1000 mg, about 125 to about 2000 mg, about 250 to about 1000 mg, about 500 to about 1000 mg, about 500 to about 1500 mg, about 500 to about 2000 mg, about 500 to about 3000 mg, about 500 to about 4500 mg, about 500 to about 6000 mg, about 500 to about 2000 mg, about 750 to about 3000 mg, about 1000 to about 4000 mg, and about 1000 to about 6000 mg on a free form basis. In one embodiment, the dose of the Compound A is a single dose. In one embodiment, the dose of the Compound A is a daily dose.

In one embodiment, a dose of Compound A to be administered ranges from 1 mg to 120 mg per kg of body weight of a subject on a free form basis. In one embodiment, a dose of Compound A to be administered falls within a range between a certain lower limit and a certain upper limit, examples of a lower limit per kg of body weight of a subject include, but are not limited to, about 1, about 2.5, about 5, about 10, about 15, about 20, about 25, about 30, about 35, and about 40 mg on a free form basis and examples of an upper limit per kg of body weight of a subject include, but are not limited to, about 5, about 10, about 15, about 20, about 25, about 30, about 35, about 40, about 45, about 50, about 55, about 60, about 65, about 70, about 75, about 80, about 85, about 95, about 100, about 105, about 110, about 115, and about 120 mg on a free form basis. In one embodiment, the dose of the Compound A is a single dose. In one embodiment, the dose of the Compound A is a daily dose.

In one embodiment, examples of a range of a dose of Compound A to be administered include, but are not limited to, about 1 to about 5mg, about 1 to about 10 mg, about 1 to about 20 mg, about 1 to about 40 mg, about 2.5 to about 10 mg, about 2.5 to about 20 mg, about 2.5 to about 40 mg, about 5 to about 20 mg, about 5 to about 40 mg, about 10 to about 40

mg, about 20 to about 40 mg, about 30 to about 60 mg, about 30 to about 90 mg, about 30 to about 120 mg, about 40 to about 90 mg, and about 40 to about 120 mg per kg of body weight of a subject on a free form basis. In one embodiment, the dose of the Compound A is a single dose. In one embodiment, the dose of the Compound A is a daily dose.

In one embodiment, the medicament of the present disclosure includes about 100, about 125, about 150, about 200, about 250, about 300, about 350, about 400, about 450, about 500, about 550, about 600, about 650, about 700, about 750, about 800, about 850, about 900, about 950, about 1000 mg, about 1250, about 1500, about 2000, about 2150, about 2500, about 3000, about 3500, about 4000, about 4500, about 5000, about 5500, or about 6000 mg of Compound A on a free form basis. In one embodiment, the dose of the Compound A is a single dose. In one embodiment, the dose of the Compound A is a daily dose.

In one embodiment, a dose of meropenem to be administered ranges from 100 mg to 6000 mg on a free form basis. In one embodiment, a dose of meropenem to be administered falls within a range between a certain lower limit and a certain upper limit, examples thereof include, but are not limited to, about 100, about 125, about 150, about 200, about 250, about 300, about 350, about 400, about 450, about 500, about 550, about 600, about 650, about 700, about 750, about 800, about 850, about 900, about 950, about 1000 mg, and about 2000 mg on a free form basis and examples of the upper limit include, but are not limited to, about 1000, about 1250, about 1500, about 2000, about 2500, about 3000, about 3500, about 4000, about 4500, about 5000, about 5500, and about 6000 mg on a free form basis. In one embodiment, the dose of the meropenem is a single dose. In one embodiment, the dose of the meropenem is a daily dose.

In one embodiment, examples of a range of a dose of meropenem to be administered include, but are not limited to, about 100 to about 1000 mg, about 100 to about 2000 mg, about 250 to about 1000 mg, about 250 to about 2000 mg, about 500 to about 2000 mg, about 500 to about 3000 mg, about 500 to about 6000 mg, about 1000 to about 2000 mg, about 1000 to about 3000 mg, about 1000 to about 6000 mg, about 2000 to about 3000 mg, and about 3000 to about 6000 mg on a free form basis. In one embodiment, the dose of the meropenem is a single dose. In one embodiment, the dose of the meropenem is a daily dose.

In one embodiment, a dose of meropenem to be administered ranges from 5 mg to 120 mg per kg of body weight of a subject on a free form basis. In one embodiment, a dose of meropenem to be administered falls within a range between a certain lower limit and a certain upper limit, examples of a lower limit per kg of body weight of a subject include, but are not limited to, about 5, about 7.5, about 10, about 12.5, about 15, about 20, about 25, about 30, about 40, about 50, about 60, about 90, and about 120 mg on a free form basis, and examples of upper limit per kg of body weight of a subject include, but are not limited to, about 10, about 12.5, about 15, about 20, about 25, about 30, about 35, about 40, about 45, about 50, about 60, about 70, about 80, about 90, about 100, and about 120 mg on a free form basis. In one embodiment, the dose of the meropenem is a single dose. In one embodiment, the dose of the meropenem is a daily dose.

In one embodiment, examples of a range of a dose of meropenem to be administered include, but are not limited to, about 5 to about 10 mg, about 5 to about 20 mg, about 5 to about 40 mg, about 15 to about 30 mg, about 15 to about 40 mg, about 15 to about 60 mg, about 15 to about 90 mg, about 15 to about 120 mg, about 20 to about 30 mg, about 20 to about 40 mg, about 20 to about 60 mg, about 20 to about 90 mg, about 20 to about 120 mg, about 30 to about 40 mg, about 30 to about 60 mg, about 30 to about 90 mg, about 30 to about 120 mg, about 40 to about 60 mg, about 40 to about 90 mg, and about 40 to about 120 mg per kg of body weight of a subject on a free form basis. In one embodiment, the dose of the meropenem is a single dose. In one embodiment, the dose of the meropenem is a daily dose.

In one embodiment, the medicament of the present disclosure includes about 100, about 125, about 150, about 200, about 250, about 300, about 350, about 400, about 450, about 500, about 550, about 600, about 650, about 700, about 750, about 800, about 850, about 900, about 950, about 1000, about 1250, about 1500, about 2000, about 2500, about 3000, about 3500, about 4000, about 4500, about 5000, about 5500, or about 6000 mg of the meropenem on a free form basis. In one embodiment, the dose of the meropenem is a single dose. In one embodiment, the dose of the meropenem is a daily dose.

In one embodiment, the medicament of the present disclosure includes 500 mg of Compound A on a free form basis and 1000 mg of meropenem on a free form basis. In one embodiment, the doses of the Compound A and the meropenem are single doses. In one embodiment, the doses of the Compound A and the meropenem are daily doses.

In one embodiment, the medicament of the present disclosure includes 500 mg of Compound A on a free form basis and 1500 mg of meropenem on a free form basis. In one embodiment, the doses of the Compound A and the meropenem are single doses. In one embodiment, the doses of the Compound A and the meropenem are daily doses.

In one embodiment, the medicament of the present disclosure includes 500 mg of Compound A on a free form basis and 2000 mg of meropenem on a free form basis. In one embodiment, the doses of the Compound A and the meropenem are single doses. In one embodiment, the doses of the Compound A and the meropenem are daily doses.

In one embodiment, the medicament of the present disclosure includes 750 mg of Compound A on a free form basis and 1000 mg of meropenem on a free form basis. In one embodiment, the doses of the Compound A and the meropenem are single doses. In one embodiment, the doses of the Compound A and the meropenem are daily doses.

In one embodiment, the medicament of the present disclosure includes 750 mg of Compound A on a free form basis and 1500 mg of meropenem on a free form basis. In one embodiment, the doses of the Compound A and the

meropenem are single doses. In one embodiment, the doses of the Compound A and the meropenem are daily doses.

In one embodiment, the medicament of the present disclosure includes 750 mg of Compound A on a free form basis and 2000 mg of meropenem on a free form basis. In one embodiment, the doses of the Compound A and the meropenem are single doses. In one embodiment, the doses of the Compound A and the meropenem are daily doses.

In one embodiment, the medicament of the present disclosure includes 1000 mg of Compound A on a free form basis and 1000 mg of meropenem on a free form basis. In one embodiment, the doses of the Compound A and the meropenem are single doses. In one embodiment, the doses of the Compound A and the meropenem are daily doses.

In one embodiment, the medicament of the present disclosure includes 1000 mg of Compound A on a free form basis and 1500 mg of meropenem on a free form basis. In one embodiment, the doses of the Compound A and the meropenem are single doses. In one embodiment, the doses of the Compound A and the meropenem are daily doses.

In one embodiment, the medicament of the present disclosure includes 1000 mg of Compound A on a free form basis and 2000 mg of meropenem on a free form basis. In one embodiment, the doses of the Compound A and the meropenem are single doses. In one embodiment, the doses of the Compound A and the meropenem are daily doses.

In one embodiment, a daily dose of the medicament of the present disclosure includes 2000 mg of Compound A on a free form basis and 6000 mg of meropenem on a free form basis.

In one embodiment, a daily dose of the medicament of the present disclosure includes 2250 mg of Compound A on a free form basis and 6000 mg of meropenem on a free form basis.

In one embodiment, a daily dose of the medicament of the present disclosure includes 3000 mg of Compound A on a free form basis and 6000 mg of meropenem on a free form basis.

Some embodiments may include a dose that has been confirmed to be tolerated in a human.

Some embodiments may include a dose that is expected to be tolerated in a human.

The compound of the present disclosure is a compound with an inhibitory activity against a β-lactamase. Therefore, when the compound is used in combination with an antibacterial agent, a useful prophylactic or therapeutic agent for a bacterial infection can be obtained. Target bacteria may include the below-described bacteria.

Examples of the target bacteria include, but are not limited to, bacteria belonging to the following families:

[Chem. 10]

*Campylobacteraceae, Vibrionaceae, Enterobacteriaceae, Budviciaceae, Morganellaceae, Hafniaceae, Yersiniaceae, Pectobacteriaceae. Erwiniaceae, Neisseriaceae, Pseudomonadaceae, Moraxellaceae, Francisellaceae, Bacillaceae, Corynebacteriaceae, Enterococcaceae, Pasteurellaceae, Burkholderiaceae*, *Nocardiaceae*, *Streptococcaceae*, *Staphylococcaceac*, *Actinomycetaceae, Clostridiaceae, Peptoniphilaceae*, *Peptostreptococcaceas*, *Eggerthellaceae. Atopobiaceae*, *Erysipelotrichidae, Propionibacteriaceae*, *Bacteroidaceae*, *Prevatellaceae, Porphyromonadaceae, Fusobacteriaceae*

Examples of the target bacteria include, but are not limited to, bacteria belonging to the following genera:

[Chem. 11]

*Campylobacter, Vibrio, Escherichia, Neisseria, Klebsiella, Enterobacter, Serratia, Citrobacter, Budvicia*, *Leminorella, Pragia*, *Hafnia, Edwardsiella*, *Obesumbacterium*, *Yersinia, Chania, Ewingella, Rahnella, Rouxiella, Pectobacterium, Brenneria, Dickeya, Lonsdalea, Sodalis, Erwinia, Pantoea, Phaseolibacter, Tatumella, Morganella. Arsenophonus, Maellerella, Photorhabdus, Proteus*, *Providencia*, *Xenorhabdus*, *Kluyvera*, *Raoultella*, *Haemophilus*, *Pseudomonas*, *Moraxella*, *Acinetobacter, Burkholderia, Salmonella, Shigella* , *Atlentibacter, Buttiauxella*, *Cedecea*, *Cronobacter, Francombacter, Kosakonia*, *Leclercia*, *Lelliottia*, *Mangrovibacter, Pluralibacter, Raoultella, Shimwellia, Siccibacter, Trabulsiella*, *Yokenella*, *Francisella*, *Bacillus*, *Corynebacterium*, *Enterococcus*, *Nocardia*, *Streptococcus*, *Staphylococcus*, *Actinomyces*, *Clostridium*, *Clostridioides*, *Peptoniphilus*, *Peptostreptococcus*, *Eggerthella*, *Atopobium*, *Bulleidia*, *Propionibacterium*, *Bacteroides*, *Prevotella* , *Porphyromonas* , *Fusobacterium* , *Parvimonas, Finegoldia. Olsenella*

Examples of the target bacteria include, but are not limited to, the following species:

[Chem. 12]

*Campylobacter coli, Campylobacter fetus, Campylobacter jejuni*, *Vibrio cholerae, Vibrio fluvialis, Vibrio parahaemolyticus*, *Vibrio vulnificus*, *Escherichia albertii. Escherichia coli. Escherichia fergusonii*, *Escherichia hermannii*, *Escherichia marmotae*, *Escherichia vulneris*, *Neisseria gonorrhoeae*, *Neisseria meningitidis*, *Klebsiella aerogenes*, *Klebsiella granulomatis*, *Klebsiella grimontii, Klebsiella huaxiensis*, *Klebsiella kielensis*, *Klebsiella michiganensis, Klebsiella milletis, Klebsiella oxytoca, Klebsiella pneumoniae, Klebsiella quasipneumoniae*, *Klebsiella quasivariicola*, *Klebsiella senegalensis, Klebsiella steroids*, *Klebsiella variicola, Klebsiella ozaenae, Enterobacter amnigenus*, *Enterobacter arachidis*, *Enterobacter asburiae*, *Enterobacter cancerogenous*, *Enterobacter cloacae*, *Enterobacter cowanii*, *Enterobacter dissolvens*, *Enterobacter gergoviae*, *Enterobacter helveticus*, *Enterobacter hormaechel*, *Enterobacter intermedius*, *Enterobacter kobei*, *Enterobacter ludwigii*, *Enterobacter mori*, *Enterobacter nimipressurali's*, *Enterobacter oryzae, Enterobacter pulveris, Enterobacter pyrinus. Enterobacter radicincitans*, *Enterobacter taylorae*, *Enterobacter turicensis, Enterobacter soli, Serratia aquatilis*, *Serratia entomophila*, *Serratia ficaria, Serratia fonticala*, *Serratia glossinae*, *Serratia grimesii*, *Serratia liquefaciens*, *Serratia marcescens*, *Serratia myotis*, *Serratia nematodiphila*, *Serratia odorifera*, *Serratia plymuthica, Serratia proteamaculans, Serratia quinivorans*, *Serratia rubidaea, Serratia symbiotica, Serratia ureilytica*,

*Serratia vespertilionis*, *Citrobacter amalonaticus*, *Citrobacter breakii*, *Citrobacter europaeus*, *Citrobacter farmers*, *Citrobacter freundii*, *Citrobacter gillenii*, *Citrobacter hoseri*, *Citrobacter murliniae*, *Citrobacter pasteurii*, *Citrobacter portucalensis*, *Citrobacter rodentium, Citrobacter sedlakii*, *Citrobacter werkmanii*, *Citrobacter youngae, Budvicia aquatica*, *Budvicia diplopodorum*, *Leminorella grimontii, Pragia fontium, Hafnia alvei*, *Edwardsiella tarda*, *Edwardsiella ictaluri*, *Edwardsiella hoshinae*, *Obesumbacterium proteus*, *Yersinia aldovae*, *Yersinia aleksiciae, Yersinia alsatica*, *Yersinia artesiana, Yersinia bercovieri*, *Yersinia canariae*, *Yersinia enterocolitica, Yersinia entomophaga*, *Yersinia frederiksenii*, *Yersinia hibernica, Yersinia intermedia. Yersinia kristensenji, Yersirn'a massifiensis*, *Yersinia mollaretti*, *Yersinia nurmli*, *Yersinia pekkanenii*, *Yersinia pestis*, *Yersinia proxima*, *Yersinia pseudotuberculosis*, *Yersinia rochesterensis*, *Yersinia rohdei, Yersinia ruckeri*, *Yersinia simills*, *Yersinia thracica. Yersinia vastinensis*, *Yersinia wautersfi*, *Yersinia perhemolyticus. Chania multitudinisentens*, *Ewingella americana*, *Rafinella aquatitis*, *Rouxiella chamberiensis*, *Pectobacterium aroidearum*, *Pectobacterium atrosepticum*, *Pectobacterium betavasculorum*, *Pectobacterium cacticida*, *Pectobacterium carnegieana*, *Pectobacterium carotovorum*, *Pectobacterium cypripedii*, *Pectobacterium rhapontici*, *Pectobacterium wasabiae*, *Brenneria alni*, *Brenneria corticis*, *Brenneria goodwinii*, *Brenneria nigrifluens*, *Brenneria populi*, *Brenneria roseae*, *Brenneria rubrifaciens*, *Brenneria salicis*, *Dickeya aquatica. Dickeya chrysanthemi*, *Dickeya dadantii*, *Dickeya dianthicola*, *Dickeya dieffenbachise*, *Dickeya paradisiaca*, *Dickeya solani*, *Dickeya zeae*, *Lonsdalea quercina*, *Sodalis glossinidrus* , *Sodalis praecaptivus*, *Erwinia amylovora*, *Erwinia*

[Chem. 13]

*aphidicola. Erwinia billingiae*, *Erwinia endophytica*, *Erwinia gerundensis*, *Erwinia injecta*, *Erwinia mallotivora*, *Erwinia oleae*, *Erwinia papayae*, *Erwinia persicina*, *Erwinia piriflorinigrans*, *Erwinia psidii*, *Erwinia pyrifoliae*, *Erwinia rhapontici*, *Erwinia tasmaniensis*, *Erwinia teleogryili*, *Erwinia toletana*, *Erwinia tracheiphila*, *Erwinia typographi*, *Erwinia uzenensis. Pantoea agglomerans*, *Pantoea ananatis*, *Pantoea citrea*, *Pantoea dispersa*, *Pantoea punctata*, *Pantoea stewartii*, *Pantoea terrea*, *Pantoea vagans*, *Pantoea alhagi*, *Pantoea allii*, *Pantoea anthophila*, *Pantoea brenneri*, *Pantoea coffeiphila*, *Pantoea conspicua*, *Pantoea cypripedii*, *Pantoea deleyi*, *Pantoea endophytica*, *Pantoea eucalypti*, *Pantoea eucrina*, *Pantoea hericii*, *Pantoea jilinensis*, *Pantoea latae*, *Pantoea pleuroti*, *Pantoea rodasii*, *Pantoea rwandensis*, *Pantoea septica*, *Pantoea sesami*, *Pantoea wallisii*, *Phaseolibacter flectens*, *Tatumella ptyseos*, *Morganella morganii*, *Arsenophonus nasoniae*, *Moellerella wisconsensis*, *Photorhabdus asymbiotica*, *Photorhabdus luminescens*, *Photorhabdus temperata*, *Proteus hauseri*, *Proteus mirabilis*, *Proteus myxofaciens*, *Proteus penneri*, *Proteus vulgaris*, *Proteus alimentorum*, *Proteus cibi*, *Proteus columbae*, *Proteus faecis, Proteus inconstans*, *Proteus terrae*, *Providencia stuartii*, *Providencia sneebia*, *Providencia rettgeri*, *Providencia rustigianji*, *Providencia heimbachae*, *Providencia burhodogranarica*, *Providencia alcalifaciens*, *Providencia entomophila*, *Providencia huaxiensis*, *Providencia thailandensis*, *Providencia vermicola*, *Xenorhabdus beddingii*, *Xenorhabdus bovienii*, *Xenorhabdus btidapestensis*, *Xenorhabdus cabanillasti*, *Xenorhabdus doucetiae*, *Xenorhabdus eapokensis*, *Xenorhabdus ehlersii*, *Xenorhabdus griffiniae*, *Xenorhabdus hominickii*, *Xenorhabdus indica*, *Xenorhabdus innexi*, *Xenorhabdus ishibashii*, *Xenorhabdus japonica*, *Xenorhabdus khoisanae*, *Xenorhabdus kappenhoeferi*, *Xenorhabdus kozodoli*, *Xenorhabdus lircayensis*, *Xenorhabdus magdalenensis*, *Xenorhabdus mauleonii, Xenorhabdus miraniensis*, *Xenorhabdus nematophila*, *Xenorhabdus poinarii*, *Xenorhabdus romanii*, *Xenorhabdus stockiae*, *Xenorhabdus szentirmaii*, *Xenorhabdus thuongxuanensis*, *Xenorhabdus vietnamensis*, *Kluyvera ascorbata*, *Kluyvera cryocrescens*, *Kluyvera georgiana*, *Kluyvera intermedia*, *Kluyvera sichuanensis*, *Raoultella electrica*, *Raoultella ornithinolytica*, *Raoultella planticola*, *Raoultella terrigena*, *Haemophilus aegyptius*, *Haemophilus ducreyi*, *Haemophilus felis*, *Haemophilus haemoglobinophilus*, *haemolyticus*, *Haemophilus influenzae*, *Haemophilus parainfluenzae*, *Haemophilus paracuniculus*, *Haemophilus parahaemolyticus*, *Haemophilus paraphrohaemalyticus*, *Haemophilus pittmaniae*, *Haemophilus piscium* , *Haemophilus sputorum*, *Pseudomonas aeruginosa*, *Pseudomonas fluorescens*, *Pseudomonas putida*, *Moraxella catarrhalis*, *Acinetobacter albensis*, *Acinetobacter apis*, *Acinetobacter baumannii*, *Acinetobacter* genomic species 13TU, *Acinetobacter genomic* species 3, *Acinetobacter baylyi*, *Acinetobacter bejerinckil*, *Acinetobacter bereziniae*, *Acinetobacter bohemicus*, *Acinetobacter boissieri*, *Acinetobacter bauvetii*, *Acinetobacter brisouii*, *Acinetobacter calcoaceticus*, *Acinetobacter celticus*, *Acinetobacter chengduensis*, *Acinetobacter colistiniresistens*, *Acinetobacter courvalinii*, *Acinetobacter*

[Chem. 14]

*cumulans*, *Acinetobacter defluvii*, *Acinetobacter dispersus*, *Acinetobacter dijkshoorniae*, *Acinetobacter equi*, *Acinetobacter gandensis*, *Acinetobacter gerneri*, *Acinetobacter guangdongensis*, *Acinetobacter guerrae*, *Acinetobacter guillouiae*, *Acinetobacter gyllenbergii*, *Acinetobacter haemolyticus*, *Acinetobacter harbinensis*, *Acinetobacter indicus*, *Acinetobacter junit*, *Acinetobacter kookii, Acinetobacter lactucae, Acinetobacter lanii, Acinetobacter larvae, Acinetobacter lwoffii*, *Acinetobacter modestus*, *Acinetobacter nectaris*, *Acinetobacter nosocomialis*, *Acinetobacter oryzae*, *Acinetobacter parvus*, *Acinetobacter pakistanensis*, *Acinetobacter populi*, *Acinetobacter portensis*, *Acinetobacter proteolyticus*, *Acinetobacter pittii*, *Acinetobacter piscicola, Acinetobacter pragensis. Acinetobacter proteolyticus, Acinetobacter pseudolwoffii*, *Acinetobacter pullicarnis*, *Acinetobacter pullorum*, *Acinetobacter puyangensis*, *Acinetobacter qingfengensis, Acinetobacter radioresistens. Acinetobacter rudis, Acinetobacter schindleri, Acinetobacter seifertii, Acinetobacter shaoyimingii, Acinetobacter soli, Acinetobacter stercoris*, *Acinetobacter tandoii*, *Acinetobacter tjernbergiae*, *Acinetobacter towneri*, *Acinetobacter ursingii*, *Acinetobacter variabilis*, *Acinetobacter venetianus*, *Acinetobacter vivianii*, *Acinetobacter*

*wanghuae*, *Acinetobacter wuhouensis*, *Salmonella bongori*, *Salmonella enterica subsp. arizonae, Salmonella enterica subsp. diarizonae, Salmonella enterica subsp. enterica, Salmonella enterica subsp. houtenae, Salmonella enterica subsp. indica, Salmonella enterica subsp. salamae*, *Salmonella* Typhimurium, *Salmonella* Typhi, *Salmonella* Paratyphi, *Salmonella* Enteritidis, *Salmonella* Senftenberg, *Shigella boydii*, *Shigella dysenteriae, Shigella flexneri*, *Shigella sonnei*, *Atlantibacter hermannii, Atlantibacter subterranea*, *Buttiauxella agrestis*, *Buttiauxella brenneree*, *Buttiauxella ferragutiae*, *Buttiauxella gaviniae*, *Buttiauxella izardii*, *Buttiauxella noackiae*, *Buttiauxella warmboldiae*, *Buttiauxella chrysanthemi*, *Buttiauxella masslliensis*, *Cedecea davisae, Cedecea lapegei*, *Cedecea neteri*, *Cedecea colo*, *Cronobacter sakazakii*, *Cronobacter malonaticus*, *Cronobacter turicensis*, *Cronobacter muytjensii*, *Cronobacter dublinensis*, *Cronobacter universalis*, *Cronobacter condimentiFranconibacter daqui*, *Franconibacter helveticus*, *Franconibacter pulveris*, *Kosakonia arachidis, Kosakonia cowanii*, *Kosakonia oryzae*, *Kosakonia oryzendophytica*, *Kosakonia oryziphila*, *Kosakonia pseudosacchari*, *Kosakonia quasisacchari*, *Kosakonia radicincitans*, *Kosakonia sacchari*, *Leclercia adecarboxylata*, *Leclercia pneumoniae*, *Lelliottia amnigena*, *Lelliottia aquatitis*, *Lelliottia jeotgali*, *Lelliottia nimipressuralis*, *Mangrovibacter plantisponsor*, *Mangrovibacter phragmitis*, *Mangrovibacter yixingensis*, *Pluralibacter gergoviae*, *Pluralibacter pyrinus*, *Shimwellia blattae*, *Shimwellia pseudoproteusSiccibacter colletis*, *Siccibacter turicensis*, *rabulsielle guamensis*, *Trabulsiella odontotennitis, Yokenella regensburgei. Francisella tularensis. Francisella no vicida*, *Francisella hispaniensis*, *Francisella persica. Francisella noatunensis*, *Francisella philormiragia*, *Francisella halioticida*, *Francisella endociliophora*, *Francisella guangzhouensis*, *Francisella piscicida*, *Francisella*
[Chem. 15]

*adeliensis*, *Francisella frigiditurris*, *Francisella marina*, *Francisella opportunistica*, *Francisella orientalis*, *Francisella salimarina*, *Francisella salina*, *Francisella uliginis*, *Bacillus anthracis*, *Bacillus cereus*, *Bacillus subtilis*, *Corynebacterium xerαsr's*, *Enterococcus avium, Enterococcus casseliflavus*, *Enterococcus faecalis. Enterococcus faecium, Enterococcus gallinarum, Nocardia asteroides, Nocardia brasiliensis*, *Nocardia farcinica*, *Nocardia nova, Streptococcus agalactiae*, *Streptococcus anginosus*, *Streptococcus constellatus*, *Streptococcus gordonii*, *Streptococcus intermedius*, *Streptococcus mitis*, *Streptococcus mutans*, *Streptococcus oralis*, *Streptococcus parasanguinis*, *Streptococcus pneumoniae*, *Streptococcus porcinus*, *Streptococcus pseudoporcinus, Streptococcus pyogenes, Streptococcus sanguinis, Streptococcus viridans, Staphylococcus aureus*, *Staphylococcus epidermidis*, *Staphylococcus haemolyticus*, *Staphylococcus lugdunensis*, *Staphylococcus saccharolyticus*, *Staphylococcus saprophyticus. Actinomyces odontolyticus*, *Actinomyces meyeri*, *Actinomyces pyogenes, Actinomyces bovis*, *Actinomyces israelii*, *Actinomyces naeslundii*, *Actinomyces viscosus*, *Clostridium histolyticum*, *Clostridium indolis*, *Clostridium innocuum*, *Clostridium croticum*, *Clostridium perfringens*, *Clostridium ramosum*, *Clostridium septicum*, *Clostridium sordellii*, *Clostridium sporogenes*, *Clostridium symbiosum*, *Clostridium tetani,*
*Clostridioides difficile. Clostridioides mangenotii, Peptoniphilus asaccharolyticus*, *Peptoniphifus indolicus*, *Peptostreptococcus anaerobius*, *Peptostreptococcus tetradius*, *Peptostreptococcus stomatis*, *Eggerthella lenta*, *Atopobium rimae*, *Bulleidia extructa*, *Propionibacterium acres*, *Propionibacterium avidum*, *Propionibacterium thoenii*, *Propionibacterium propionicum*, *Bacteroides distasonis*, *Bacteroides fragilis*, *Bacteroides ovatus*, *Bacteroides thetaiotaomicron*, *Bacteroides uniformis*, *Bacteroides vulgatus*, *Bacteroides stercoris*, *Bacteroides caccae*, *Bacteroides salversiae*, *Bacteroides ovalus*, *Bacteroides eggerthii, Bacteroides splanchnicus*, *Prevotella bivia*, *Prevotella disiens*, *Prevotella intermedia*, *Prevotella melaninogenica*, *Prevotella oralis*, *Prevotella oris*, *Prevotella loescheii*, *Prevotella corporis*, *Prevotella heparinolytica*, *Prevotella nigrescens, Prevotella salivae, Prevotella buccae*, *Prevotella nanceiensis, Prevotella denticola* , *Prevotella pallens*, *Prevotella baroniae* , *Prevotella jejuni*, *Prevotella histicola*, *Porphyromonas assaccharolytica*, *Porphyromonas gingivalis*, *Fusobacterium nucleatum*, *Fusobacterium necrophorum, Fusobacterium varium, Fusobacterium mortiferum*, *Fusobacterium gonidiaformans*, *Parvimonas micra, Finegoldia magna. Olsenella uli*,

Examples of the target bacteria include, but are not limited to, the following strains:
[Chem. 16]

*E. coli* O157:H7
*Clostridioides* (formerly *Clostridium) difficile*
*Salmonella enterica* subsp. *enterica* serovar Typhi

Indication

A composition including Compound A and a carbapenem compound described herein can be used to treat a bacterial infection. The bacterial infection that can be treated with a combination of the Compound A and the carbapenem antibacterial agent described herein can include a broad-spectrum bacterium. Exemplary organisms include a Gram-positive bacterium, a Gramnegative bacterium, an aerobic bacterium, and an anaerobic bacterium, for example, an

aerobic bacterium and an anaerobic bacterium such as
[Chem. 17]
*Staphylococcus*, *Lactobacillus*, *Streptococcus*, *Escherichia*, *Enterobacter*, *Klebslella*,*Pseudomonas*, *Acinetobacter, Proteus, Campylobacter*, *Citrobacter*, *Nisseria*, *Baccillus*, *Bacteroides, Peptococcus, Clostridium, Salmonella, Shigella, Serratia, Haemophilus.*

More specific examples of the bacterial infection are any of
[Chem. 18]
*Pseudomonas aeruginosa*, *Pseudomonas fluorescens, Pseudomonas putida, Stenotrophomonas maltophilia, Burkholderia cepacia, Escherichia coli, Citrobacter freundii, Salmonella typhimurium, Salmonella typhi, Salmonella paratyphi. Shigella dysenteriae, Shigella flexneri*, *Shigella sonnei*, *Enterobacter cloacae*, *Enterobacter aerogenes*, *Klebsiella pneumoniae, Klebsiella oxytoca*, *Klebsiella ozaenae, Serratia marcescens. Francisella tularensis, Morganella morganii, Proteus mirabilis, Proteus vulgaris, Providencia alcalifaciens, Providencia rettaeri*, *Providencia stuartii*, *Acinetobacter baumannii*, *Acinetobacter calcoaceticus*, *Acinetobacter haemolyticus*, *Yersinia enterocolitica*, *Yersinia pestis*, *Yersinia pseudo tuberculosis*, *influenzae*, *Haemophilus parainfluenzae, Moraxella catarrhalis, Campylobacter jejuni, Vibrio cholerae, Vibrio parahaemolyticus*, *Neisseria gonorrhoeae*, *Neisseria meningitidis*, *Bacteroides fragilis*, *Bacteroides vulgatus*, *Bacteroides ovalus. Clostridium difficile, Streptococcus pneumoniae*, *Streptococcus agalactiae*, *Streptococcus pyogenes*, *Enterococcus faecalis*, *Enterococcus faecium, Staphylococcus aureus, Staphylococcus epidermidis, Staphylococcus saprophyticus*, *Staphylococcus haemolyticus*

Examples of the target bacterium of interest include, but are not limited to,
[Chem. 19]

Enterobacteriales, *Acinetobacter baumannii* complex, *Pseudomonas aeruginosa* **and** *Bacteroides* spp.
**or**
*Escherichia coli*, *Klebsiella pneumoniae*, *Enterobacter cloacae* complex, *Pseudomonas aeruginosa* **or** *Acinetobacter baumannii* complex

[Chem. 20]
Examples of *Enterobacteriales* include, but are not limited to

*Citrobacter freundii*
*Enterobacter aerogenes*
*Enterobacter cloacae*
*Escherichia coli*
*Klebsiella oxytoca*
*Klebsiella ozaenae*
*Klebsiella pneumoniae*
*Kluyvera ascorbate*
*Morganella morganii*
*Proteus mirabilis*
*Providencia rettgeri*
*Raoultella ornithinolytica*
*Salmonella* Senftenberg
*Serratia marcescens*

In one embodiment, a bacterial infection that the present disclosure targets is a bacterial infection involving a bacterium that can possess a β-lactamase. In one embodiment, the bacterial infection is a bacterial infection involving a bacterium that can possess a serine-β-lactamase. In one embodiment, the serine-β-lactamase is an Ambler class A, Ambler class C, or Ambler class D β-lactamase. In one embodiment, the bacterial infection is a bacterial infection involving a bacterium that can possess a metallo-β-lactamase. In one embodiment, the metallo-β-lactamase is an Ambler class B β-lactamase.

In one embodiment, a medicament of the present disclosure is used as a therapeutic and/or prophylactic agent for sepsis, febrile neutropenia, bacterial meningitis, bacterial endocarditis, otitis media, sinusitis, pneumonia, lung abscess, empyema, a secondary infection of a chronic respiratory tract lesion, pharyngolaryngitis, tonsillitis, osteomyelitis, arthritis, peritonitis, intra-abdominal abscess, cholecystitis, cholangitis, liver abscess, a deep skin infection, lymphangitis and lymphadenitis, a secondary infection such as trauma, burn, and a surgical wound, a urinary tract infection, a genital infection, an ocular or dental infection, a complicated urinary tract infection, a complicated intra-abdominal infection, hospital-acquired pneumonia, and/or ventilator-associated pneumonia.

In one embodiment, the medicament of the present disclosure is typically taken by an adult at 2.5 to 3 g per dose

every 8 hours.

In one embodiment, the medicament of the present disclosure is administered in a total daily dose of 7.5 to 9 g (6 g of meropenem on a free form basis/ 1.5 to 3 g of Compound A on a free form basis).

In one embodiment, the medicament of the present disclosure is administered at a dose of 0.5 to 1 g of Compound A as a free form every 8 hours for treating a complicated urinary tract infection, a complicated intra-abdominal infection, hospital-acquired pneumonia, and/or ventilator-associated pneumonia.

In one embodiment, the medicament of the present disclosure is administered to a patient with renal dysfunction and a dialysis patient at a reduced dose depending on the degree of renal dysfunction.

In one embodiment, the medicament of the present disclosure is administered to a child at a dose of 2.5 to 20 mg per kg of body weight of Compound A as a free form every 8 hours. In one embodiment, the medicament of the present disclosure is administered to a 2- to 9-month-old child at a dose of 2.5 to 20 mg per kg of body weight of Compound A as a free form every 8 hours.

In one embodiment, Compound A and meropenem are dissolved, diluted, and administered separately. In one embodiment, the medicament of the present disclosure can be administered in combination under a certain condition.

In one embodiment, the medicament of the present disclosure is provided as a sterile powder-filled product or a lyophilized product, preferably for intravenous infusion. In one embodiment, the medicament of the present disclosure is provided in a single-dose vial. In one embodiment, the medicament of the present disclosure is provided in a pharmacy bulk vial.

A combination of Compound A with meropenem, etc. of the present disclosure is preferably administered with caution to a patient with a previous history of allergy to a penicillin antibacterial drug, a cephalosporin antibacterial drug, or a cephem antibacterial drug. For example, it is preferably administered with caution to a patient with a previous history of allergy to a penicillin antibiotic, a cephalosporin antibiotic, or a β-lactamase inhibitor.

In one embodiment, the medicament of the present disclosure is administered as a free form via intravenous infusion for 30 minutes or more. In one embodiment, the medicament of the present disclosure is typically administered to an adult at a total dose of 2.5 to 3 g per day as a free form every 8 hours. In one embodiment, a duration of administration of the medicament of the present disclosure is usually 5 to 10 days. In one embodiment, the medicament of the present disclosure is administered by intravenous infusion for 3 hours.

In one embodiment, the medicament of the present disclosure is administered to a subject with a complicated urinary tract infection, a complicated intra-abdominal infection, hospital-acquired pneumonia, and/or ventilator-associated pneumonia at a dose of 2.5 to 3 g as a free form every 8 hours. In one embodiment, a duration of administration of the medicament of the present disclosure is 7 to 14 days.

In one embodiment, an intravenous dosage of the medicament of the present disclosure is reduced in a renal disorder patient and a dialysis patient (hemodialysis, CAPI) depending on the degree of renal dysfunction.

In one embodiment, in the case of a pediatric patient, a recommended dose of Compound A of the present disclosure for a 4 or more-week-old pediatric patient with a body weight of 40 kg or less can be 2.5 to 40 mg per kg of body weight on a free form basis when renal function is normal. A combination of meropenem and Compound A of the present disclosure can be administered at a dose of 12.5 to 80 mg per kg of body weight on a free form basis every 8 to 12 hours.

In one embodiment, a dosage based on a pharmacokinetic model is set for a pediatric patient. In one embodiment, without wishing to be bound by any theory, a recommended dose based on pharmacokinetic modeling is 40 mg of meropenem per kg body weight on a free form basis/ 10 to 40 mg of Compound A of the present disclosure on a free form basis and can be administered every 8 hours.

In one embodiment, a pediatric patient with a body weight of more than 40 kg and with a normal renal function can be administered with an adult dose.

In one embodiment, Compound A of the present disclosure, as a formulation with meropenem, may be used for treating an 18 or more-year-old patient with a complicated urinary tract infection including pyelonephritis, a complicated intra-abdominal infection, hospital-acquired pneumonia, and/or ventilator-associated pneumonia caused by a susceptible bacterium (e.g., Escherichia coli, Klebsiella pneumoniae, Enterobacter cloacae complex, Pseudomonas aeruginosa, or Acinetobacter baumannii complex).

In one embodiment, in order to prevent emergence of a drug-resistant bacterium and to maintain an effect of Compound A of the present disclosure as an antibacterial agent, the Compound A of the present disclosure may be used only to treat or prevent an infection that has been proven or strongly presumed to be caused by the susceptible bacterium.

In one embodiment, the medicament of the present disclosure may be administered by intravenous infusion for 3 hours to an 18 or more-year-old patient with an estimated glomerular filtration rate (eGFR) of 50 mL/min/1.73 m or more at a dose of 2.5 to 4 g as a free form every 8 hours for up to 14 days.

In one embodiment, the medicament of the present disclosure is provided in a form of a sterile powder in a single-dose vial containing 0.5 g of Compound A as a free form and 2 g of the meropenem. In one embodiment, the medicament of the present disclosure is provided in a form of a sterile powder in a single-dose vial containing 0.75 g of Compound A as a free form and 2 g of the meropenem as a free form. In one embodiment, the medicament of the present disclosure is

provided in a form of a sterile powder in a single-dose vial containing 1 g of Compound A as a free form and 2 g of the meropenem as a free form. In one embodiment, the medicament of the present disclosure is provided, without limitation, in a form of a sterile powder in a single-dose vial containing 1.5 g of Compound A as a free form and 2 g of the meropenem as a free form.

In one embodiment, the medicament of the present disclosure is characterized in that it is administered in the case of an infection or suspected infection of a bacterium that is susceptible to a combination of Compound A and meropenem or a pharmaceutically acceptable salt thereof in order to prevent emergence of a bacterium resistant to the combination of Compound A and meropenem or a pharmaceutically acceptable salt thereof.

In one embodiment, the medicament of the present disclosure is characterized in that it is administered when presence or absence of development of severe hypersensitivity observed with a β-lactam drug including meropenem is checked and no signs of the severe hypersensitivity appear.

In one embodiment, the medicament of the present disclosure is characterized in that it is not administered to a patient with a previous history of hypersensitivity to a β-lactam drug including meropenem since severe hypersensitivity to the β-lactam drug may develop.

In one embodiment, the medicament of the present disclosure is characterized in that it is not administered to a patient receiving valproic acid since a blood level of the valproic acid may decrease to cause relapse of an epileptic fit.

Some embodiments include co-administration of a combination of Compound A and a carbapenem antibacterial agent with an additional antibacterial agent such as a β-lactam. In some embodiments, the carbapenem antibacterial agent is meropenem.

Preferred embodiments of an additional medicament include a β-lactam such as ceftazidime, doripenem, ertapenem, imipenem, biapenem, razupenem, panipenem, ceftolozane, and cefiderocol.

Some embodiments include co-administration of the combination of Compound A and a carbapenem antibacterial agent described herein with an additional agent including monobactam. Examples of the monobactam include aztreonam, tigemonam, and carumonam. Some embodiments include co-administration of the combination of Compound A and the carbapenem antibacterial agent described herein with an additional agent including a Class A, B, C, or D β-lactamase inhibitor.

Although implementation of the present disclosure will be described hereinafter with reference to Examples, the present disclosure is not limited thereto. In Examples below, Compound A was used in a free form and meropenem was used in a hydrate form. A dose of the meropenem was described on a free form basis.

[Example]

Compound A was synthesized by reaction, work-up, and purification in the same manner as described in WO 2019/208797.

(Example 1) Biochemical property of Compound A against various β-lactamases using apparent inhibition constant as indicator (Purpose)

Biochemical properties of Compound A and a control agent against representative Class A, B, C, and D β-lactamases were evaluated using an apparent inhibition constant as an indicator for the purpose of demonstrating that the Compound A is a broad-spectrum inhibitor of various β-lactamases.

(Method)

Each of the Compound A and the control agents was dissolved with distilled water, DMSO, or an equimolar sodium carbonate solution to prepare a 10 mmol/L stock solution. As a reporter substrate, Nitrocefin and a meropenem hydrate were dissolved in DMSO and distilled water to prepare a 40 mmol/L stock solution and a 6 mmol/L stock solution (in terms of meropenem), respectively. In the case of evaluating Class A, C, and D β-lactamases, a 1/15 mol/L phosphate buffer was in principle used and a bovine serum albumin solution was added to be 0.01% as necessary. In the case of evaluating Class B β-lactamase, a 1/15 mol/L phosphate buffer containing 400 μmol/L zinc sulfate was used. The stock solutions of the Compound A and the control agents were diluted with a buffer solution to prepare solutions of 1.2 nmol/L to 400 μmol/L. The stock solution of the reporter substrate was diluted with a buffer solution to prepare a 50 to 200 μmol/L solution. The β-lactamase solutions were diluted with a buffer solution to 10 to 600 nmol/L. Each of the Compound A and the control agent (designated as inhibitor) solutions having the above-described concentrations was dispensed at 50 μL/well and a buffer solution for background and a positive control was dispensed at 50 μL/well into a 96-well plate, and then each of the β-lactamase solutions was added thereto at 50 μL/well (a buffer solution at 50 μL/well for background). After stirring, the resultants were preincubated for 30 minutes at 37°C. The reporter substrate solution was incubated at 37°C for 15 minutes or more, dispensed at 100 μL/well, and measured for a change in absorbance by a microplate reader

at 37°C. For each measured concentration of the positive control and the inhibitor, an initial rate (μmol/L/sec) was determined from a time to be analyzed and a change in product amount. Expression 1 was plotted using an initial rate that showed 50% or more inhibition compared to the positive control, and a slope was calculated for each inhibitor concentration. $K_{i,app}$ for each inhibitor concentration was calculated from the slope, the inhibitor concentration, an enzyme concentration, $K_m$, and $k_{cat}$ using Expression 2. If the initial rate was determined at two or more concentrations for each inhibitor and a plurality of $K_{i,app}$ could be obtained, an average value was used as the $K_{i,app}$ for the inhibitor.

$$t - t_c = K_m / V_{max} \times ([\text{Inhibitor}] / K_{i,app}) \times \text{Ln}[S]_0 / [S]$$

(Expression 1)

$$K_{i,app} = [\text{Inhibitor}] \times K_m / (k_{cat} \times [\text{Enzyme}]) \times 1 / \text{slope}$$

(Expression 2)

An inhibitory activity of each of the Compound A and the control agents (vaborbactam, avibactam, relebactam, tazobactam, sulbactam, clavulanate) was determined from a degradation activity of the β-lactamase on the reporter substrate, and $K_m$ and $k_{cat}$ values were used to calculate an apparent inhibition constant ($K_{i,app}$) of the β-lactamase (Table 1). The experiment was performed in duplicate. An average and a standard deviation of the $K_{i,app}$ were calculated from three generally independent experiments.

(Results)

The Compound A inhibited Class A and D carbapenemases KPC-2, 3, 6, 8, 31, 33, 38, and OXA-23, 24, 48, 65, 66, 72 with the $K_{i,app}$ values ranging from 0.621 nM to 2.33 μM, and the $K_{i,app}$ values for most enzyme species were equal to or lower than $K_{i,app}$ values for the control agents. The Compound A also inhibited Class B metallo-carbapenemases (VIM-1, 2, IMP-1, 6, NDM-1, 4, 5, 6, 7) at 31.6 nM to 19.9 μM, but no control agents had a MBL inhibitory activity. Among Class A extended-spectrum β-lactamases and Class C cephalosporinases showing a weak carbapenemase activity, the Compound A showed $K_{i,app}$ of 0.0664 to 0.79 nM for CTX-M-14, 15, 27, and PDC-11, and these values were equal to or lower than those of all control agents. $K_{i,app}$ of the Compound A for SHV-2, 5, 12, TEM-10, and ACT-17 were 6.9 to 361 nM, which were higher than those of avibactam, relebactam and tazobactam.

These results confirm that the Compound A is the only compound that inhibits Class A, B, C, and D β-lactamases (Table 1).

[Table 1-1]

| **Table 1. $K_{i,app}$ values of Compound A and various control agents for β - lactamases** | | | | | | | | | |
|---|---|---|---|---|---|---|---|---|---|
| Ambler class | CPase | **Enzyme** | *K*i app (μ mol/L) | | | | | | |
| | | | Compo und A | **Vaborb actam** | **Avibact am** | **Releba ctam** | **Tazoba ctam** | **Sulbact am** | **Clavula nate** |
| class A | - | CTX -M-14 | 0.0001 53 ± 0.0000 235 | 0.392 ± 0.0203 | 0.0023 5 ± 0.0007 63 | 0.0059 6 ± 0.0032 2 | 0.0000 793 ± 0.0000 256 | n.t. | n.t. |
| class A | - | CTX -M-15 | 0.0000 664 ± 0.0000 15 | 0.0781 ± 0.0066 3 | 0.0003 69 ± 0.0000 569 | 0.0008 41 ± 0.0002 35 | 0.0000 387 ± 0.0000 0434 | 0.0008 8 ± 0.0002 48 | 0.0000 882 ± 0.0000 496 |
| class A | - | CTX -M-27 | 0.0001 59± 0.0000 228 | 0.351 ± 0.0287 | 0.0012 9± 0.0006 63 | 0.0036 2± 0.0012 | 0.0000 403 ± 0.0000 162 | n.t. | n.t. |
| class A | - | SHV -2 | 0.361 ± 0.127 | n.d. | 0.0022 6± 0.0014 4 | 0.0003 73± 0.0000 856 | 0.0001 87± 0.0001 04 | n.t. | n.t. |
| class A | - | SHV -5 | 0.127 ± 0.016 | 1.42 ±0.03 75 | 0.0021 5 ± 0.0002 64 | 0.0062 6 ± 0.0015 3 | 0.0018 4 ± 0.0002 43 | 0.0209 ± 0.0045 1 | 0.0005 15 ± 0.0000 929 |

(continued)

| Table 1. $K_{i,app}$ values of Compound A and various control agents for $\beta$ - lactamases | | | | | | | | | |
|---|---|---|---|---|---|---|---|---|---|
| Ambler class | CPase | **Enzyme** | *K*i app ($\mu$ mol/L) | | | | | | |
| | | | Compound A | **Vaborbactam** | **Avibactam** | **Relebactam** | **Tazobactam** | **Sulbactam** | **Clavulanate** |
| class A | - | SHV -12 | 0.0535 ± 0.0122 | 0.755 ± 0.103 | 0.00255 ± 0.0006 96 | 0.0193 + 0.0063 2 | 0.0016 8± 0.0002 66 | n.t. | n.t. |
| class A | - | TEM -10 | 0.0069 ± 0.0008 79 | 0.219 ± 0.0678 | 0.0002 3 ± 0.0000 587 | 0.0068 3 ± 0.0034 8 | 0.0009 38 ± 0.0001 49 | 0.0069 8 ± 0.0036 2 | 0.0004 85 ± 0.0004 56 |
| class C | - | ACT -17 | 0.0383 + 0.0069 6 | 0.427 ± 0.0393 | 0.0009 8 ± 0.0001 62 | 0.0004 98 ± 0.0000 114 | 0.0121 ± 0.0030 3 | 0.135 ± 0.0489 | 3.23 ± 0.328 |

[Table 1-2]

| | | | | | | | | | |
|---|---|---|---|---|---|---|---|---|---|
| class C | - | PDC -11 | 0.0007 9 ± 0.0000 379 | 3.86 ± 0.163 | 0.107 ± 0.0202 | 0.048 ± 0.0119 | 0.0405 ± 0.02 | 0.668 ± 0.26 | n.d. |
| class A | ± | KPC -2 | 0.0014 6 ± 0.0000 574 | 0.0637 + 0.0249 | 0.0039 6 ± 0.0021 6 | 0.0050 3 ± 0.0007 34 | 0.159 + 0.0482 | 0.42 ± 0.0992 | 0.0767 ± 0.0383 |
| class A | + | KPC -3 | 0.0021 9 ± 0.0006 87 | 0.0883 ± 0.0303 | 0.0057 ± 0.0011 9 | 0.0076 7 ± 0.0016 4 | 0.27 ± 0.0808 | 0.674 ± 0.239 | 0.128 ± 0.0532 |
| class A | ± | KPC -6 | 0.0029 5 ± 0.0007 9 | 0.109 ± 0.0217 | 0.0107 ± 0.0050 6 | 0.012 ± 0.0048 2 | 0.0799 ± 0.0165 | n.t. | n.t. |
| class A | + | KPC -8 | 0.0019 4 ± 0.0004 48 | 0.114 ± 0.0221 | 0.0097 9 ± 0.0051 | 0.0137 ± 0.0058 8 | 0.052 ± 0.0118 | n.t. | n.t. |
| class A | ± | KPC -31 | 2.33 ± 0.602 | 13.3 ± 8.59 | 0.43 ± 0.114 | 0.862 ± 0.0753 | 0.0119 ± 0.0096 5 | n.t. | n.t. |
| class A | + | KPC -33 | 0.041 + 0.0234 | 4.6 ± 0.437 | 0.029 ± 0.0251 | 0.074 ± 0.0291 | 0.0037 4 ± 0.0012 1 | n.t. | n.t. |
| class A | + | KPC -38 | 0.0008 36 ± 0.0004 14 | 0.0483 + 0.0132 | 0.0016 6 ± 0.0003 29 | 0.0029 4 ± 0.0009 82 | 0.112 ± 0.0145 | 0.405 ± 0.0996 | 0.0782 ± 0.0205 |

[Table 1-3]

| | | | | | | | | | |
|---|---|---|---|---|---|---|---|---|---|
| class D | + | OXA -23 | 0.0040 7 ± 0.0002 37 | n.d. | 0.161 ± 0.0363 | 5.64 ± 0.522 | 0.014 ± 0.0053 1 | 0.681 ± 0.0743 | 0.394 ± 0.169 |
| class D | + | OXA -24 | 0.0010 9 ± 0.0003 87 | n.d. | 0.112 ± 0.0712 | n.d. | 0.1 ± 0.0456 | n.t. | n.t. |

(continued)

| | | | | | | | | | |
|---|---|---|---|---|---|---|---|---|---|
| class D | + | OXA -48 | 0.0008 61 ± 0.0000 767 | n.d. | 0.0024 1 ± 0.0016 2 | 0.0893 ± 0.0467 | 0.0053 2 ± 0.0003 29 | 0.264 ± 0.116 | 0.103 ± 0.0365 |
| class D | + | OXA -65 | 0.0054 4 ± 0.00121 | n.d. | 5.9 ± 1.05 | n.d. | n.d. | n.t. | n.t. |
| class D | + | OXA -66 | 0.0057 8 ± 0.0006 54 | n.d. | 7.35 ± 0.963 | n.d. | n.d. | n.t. | n.t. |
| class D | + | OXA -72 | 0.0006 21 ± 0.0000 248 | n.d. | 0.691 ± 0.0638 | ≥37.4 * | 0.0789 ± 0.0089 6 | n.t. | n.t. |
| class B | + | VIM -1 | 0.35 ± 0.0454 | n.d. | n.d. | n.d. | n.d. | n.d. | n.d. |
| class B | + | VIM -2 | 1.21 ± 0.337 | n.d. | n.d. | n.d. | n.d. | n.t. | n.t. |
| class B | + | IMP -1 | 0.512 ± 0.119 | n.d. | n.d. | n.d. | n.d. | n.d. | n.d. |
| class B | + | IMP -6 | 19.9 ± 3.17 | n.d. | n.d. | n.d. | n.d. | n.t. | n.t. |
| class B | + | NDM -1 | 0.126 ± 0.0581 | n.d. | n.d. | n.d. | n.d. | n.d. | n.d. |

[Table 1-4]

| | | | | | | | | | |
|---|---|---|---|---|---|---|---|---|---|
| class B | + | NDM -4 | 0.153 ± 0.0452 | n.d. | n.d. | n.d. | n.d. | n.t. | n.t. |
| class B | + | NDM -5 | 0.0316 ± 0.0123 | n.d. | n.d. | n.d. | n.d. | n.d. | n.d. |
| class B | + | NDM -6 | 1.05 ± 0.0375 | n.d. | n.d. | n.d. | n.d. | n.t. | n.t. |
| class B | + | NDM -7 | 0.053 ± 0.0302 | n.d. | n.d. | n.d. | n.d. | n.t. | n.t. |
| **This test was performed under conditions under which an enzyme and an inhibitor were preincubated for 30 minutes. Average ± standard deviation in three independent experiments was described. Cpase, Carbapenemase. n.t., Not tested. n.d., Not determined due to too low inhibitor activity at the highest set concentration of 100 μ mol/L. *, A value obtained in one of three independent experiments because of n.d. in two of the three independent experiments.** | | | | | | | | | |

(Example 2) Kinetic properties of Compound A for various β-lactamases ($k_2/K_i$, $k_{off}$)

(Purpose)

An inhibitory activity of Compound A and control agents against serine- and metallo-β-lactamases, KPC-2 and VIM-1, was tested using kinetic properties ($k_2/K_i$, $k_{off}$) as indicators. In other words, as part of the study of an inhibitory mechanism of various β-lactamases by the Compound A, a binding rate constant ($k_2/K_i$) and a dissociation rate constant ($k_{off}$) for interaction of the KPC-2 with the Compound A and for interaction of the VIM-1 with the Compound A were determined. For KPC-2, Compound A was compared with control agents vaborbactam (VAB), avibactam (AVI), and tazobactam (TAZ).

(Method)

(Measurement of $k_2/K_i$)

Measurement was performed according to the method described in John F. Morrison, Christopher T. Walsh, The Behavior AND Significance OF Slow-Binding Enzyme Inhibitors. Adv Enzymol Relat Areas Mol Biol. 1988; 61: 201-301.

A phosphate buffer (1/15 mol/L, pH 7.0) containing 0.01% Bovine Serum Albumin Solution (BSA) and a phosphate buffer containing 0.01% BSA and 100 μmol/L zinc sulfate were used as buffers for evaluating the KPC-2 and the VIM-1, respectively. As inhibitor drugs for evaluating a binding rate constant with the KPC-2, a 50 to 800 nmol/L

Compound A solution, 1.56 to 50 μmol/L VAB and AVI solutions, and a 20 to 640 μmol/L TAZ solution were prepared. As inhibitor drugs for evaluating a binding rate constant with the VIM-1, a 2-fold dilution series of Compound A solution at 0.625 to 20 μmol/L was prepared. As enzyme solutions, a 10 nmol/L KPC-2 solution and a 20 nmol/L VIM-1 solution were prepared using a buffer solution. As a reporter substrate solution, a 200 μmol/L Nitrocefin solution was prepared using a buffer solution.

The buffer solution was dispensed at 70 μL/well, and each inhibitor solution for each concentration (for measuring an inhibitory activity) was dispensed at 20 μL/well or a buffer solution for background and a positive control was dispensed at 20 μL/well. Then, the reporter substrate solution was added thereto at 100 μL/well. After stirring, the resultants were incubated at 37°C for 10 minutes. The buffer solution and the enzyme solutions were dispensed into a background well and other wells at 10 μL/well, respectively, and absorbance at 490 nm was measured at 15 second intervals for 20 minutes at 37°C using a microplate reader. Absorbance of the background was subtracted from absorbance of the positive control or for measuring an inhibitory activity, and absorbance at time 0 was subtracted from absorbance at each time point. The absorbance was converted to a product amount (μmol/L) using the Lambert-Beer law. The analysis software Prism8 was used to calculate a false first-order reaction rate constant $k_{obs}$ at each inhibitor concentration by approximating a progress curve for time and product at each inhibitor concentration with Expression 1:

$$P = v_s \times t + (v_i - v_s) \times (1 - e\hat{}\ (-k_{obs} \times t))/k_{obs} \ldots$$

(Expression 1)

in which an amount of product (P), an initial reaction rate ($v_i$) and a steady state rate ($v_s$) of a positive control, and a reaction time (t).

A relationship between a pseudo-first-order reaction rate constant and a binding rate constant $k_2/K_i$ for β-lactamase inhibition in a two-step reversible inhibition model (Compound A, VAB, AVI) is shown in Expression 2.

$$k_{obs} = k_{-2} + (k_2 \times I) / (K_i \times (1 + S/K_m) + I) \ldots$$

(Expression 2)

When $K_i$ is larger than I, Expression 2 is simplified to Expression 3, which was used to derive an association rate constant $k_2/K_i$ as a slope of a line of $k_{obs}$ versus an inhibitor concentration.

$$k_{obs} = k_{-2} + (k_2 \times I) / (K_i \times (1 + S/K_m) \ldots \text{(Expression 3)}$$

in which I is an inhibitor concentration, S is a nitrocefin concentration (100 μmol/L), and $K_m$ is a Michaelis constant of nitrocefin for the KPC-2 and the VIM-1 (88.2 and 63.9 μmol/L, respectively).

$K_{iobs}$ and $k_{inact}$ in an irreversible inhibition model (TAZ) were determined by the Prism8 using Expression 4.

$$k_{obs} = (k_{inact} \times I) / (K_{iobs} + I) \ldots \text{(Expression 4)}$$

$k_{iobs}$ was corrected by Expression 5 taking affinity of the nitrocefin for the KPC-2 into account.

$$K_i = K_{iobs} / (1 + S/K_m) \ldots \text{(Expression 5)}$$

A binding rate constant $k_{inact}/K_i$ was calculated using the $k_{inact}$ and the $K_i$.

(Measurement of $k_{off}$)

Measurement was performed according to the method described in Robert A Copeland, Aravind Basavapathruni, Mikel Moyer, Margaret Porter Scott, Impact of enzyme concentration and residence time on apparent activity recovery in jump dilution analysis. Anal Biochem. 2011 15; 416 (2): 206-10. For evaluation of the KPC-2, 10, 2.22 μmol/L and 1.11 nmol/L Compound A solutions, 100, 22.2 μmol/L, and 11.1 nmol/L VAB solutions, 10, 2.22 μmol/L, and 1.11 nmol/L AVI solutions, and 300, 66.7 μmol/L, and 33.3 nmol/L TAZ solutions were prepared. For evaluation of the VIM-1, 300, 66.7 μmol/L, and 33.3 nmol/L Compound A solutions were prepared. Such inhibitor solutions are designated as A, B, and C in order of increasing concentration. A control buffer containing sodium carbonate or DMSO having the same concentration as an inhibitor solution A was also prepared.

Enzyme solutions at 0.444 μmol/L and 0.667 μmol/L were prepared by diluting KPC-2 and VIM-1 solutions with

the buffer solution, respectively. A 400 $\mu$mol/L Nitrocefin solution was prepared by dilution with the buffer solution.

The buffer solution and inhibitor solutions B and C were dispensed at 90 $\mu$L/well and a 400 $\mu$mol/L Nitrocefin solution was added thereto at 100 $\mu$L/well. After stirring, the resultant was incubated at 37°C for 10 minutes or more. An inhibitor solution A and the control buffer were diluted 10-fold with the enzyme solution, stirred, and then incubated at 37°C for 5 minutes. The thus-diluted solution was diluted 100-fold with a buffer solution and dispensed at 10 $\mu$L/well and absorbance at 490 nm was measured at 20-second intervals for up to 4 hours using a microplate reader at 37°C. Absorbance of the background was subtracted from absorbance of the positive control, for measuring $v_s$, for measuring $v_i$, and for evaluating $k_{off}$, and absorbance at time 0 was subtracted from absorbance at each time point. The absorbance was converted to a product amount ($\mu$mol/L) by the Lambert-Beer law. A coefficient of determination of a linear regression equation was calculated by fitting a change in absorbance per measurement time to a linear regression equation in a data series for measuring $v_s$. When the coefficient of determination is lower than 0.99, a time to be analyzed was shortened in 20-second increments from the longest measurement time to a point at which the coefficient of determination first reached 0.99 or higher, which was determined as a time to be analyzed in this study. The Prism8 was used to calculate a dissociation rate constant ($k_{off}$) for each inhibitor and enzyme by approximating a curve for a time to be analyzed and a product in a data series for evaluating $k_{off}$ with Expression 3. If the thus-calculated $k_{off}$ exceeded 0.00385 ($sec^{-1}$), it was treated as > 0.00385 ($sec^{-1}$).

$$P = v_s \times t + (v_i - v_s) \times (1 - e\hat{\ } (-k_{off} \times t)) / k_{off} \ldots$$

(Expression 3)

in which amount of product (P), rates of formation at time 0 (vi) and at complete dissociation of enzyme/inhibitor (vs) for evaluating $k_{off}$, and reaction time (t).

(Results)

For KPC-2, Compound A was the most efficient inhibitor, with a binding rate for the Compound A (1.31 x $10^5$ $M^{-1}sec^{-1}$) being 24 times or more than those for other inhibitors. A dissociation rate constant ($k_{off}$) of a KPC-2-Compound A complex was slow of 3.39 x $10^{-4}$ $sec^{-1}$, which showed a speed difference of two times compared with that of two-step reversible inhibitors VAB and AVI and a speed difference of nine times compared with that of an irreversible inhibitor TAZ. These results suggest that strong inhibition of the KPC-2 by the Compound A can be described by a two-step reversible inhibition model.

For a metallo-$\beta$-lactamase VIM-1, the Compound A showed a typical linear inactivation profile of a fast on-fast off type. Dissociation of the Compound A from the VIM-1 with a high $k_{off}$ value of 3.85 x $10^{-3}$ $sec^{-1}$ or more supports the above-mentioned interpretation.

In summary, the Compound A is a potent inhibitor with a fast binding rate and a slow dissociation rate against the KPC-2, and the Compound A is a fast on-fast off inhibitor against the VIM-1.

[Table 2]

**Table 2. Rate constants of Compound A and control agent $\beta$ -lactamase**

| | β-lactamase | CompoundA | VAB | AVI | TAZ |
|---|---|---|---|---|---|
| $k_2/K_1$* ($M^{-1}sec^{-1}$) | KPC-2 | $1.31\times10^5$ ± $0.338\times10^5$ | $1.01\times10^3$ ± $0.0774\times10^3$ | $5.48\times10^3$ ± $1.52\times10^3$ | $5.27\times10^3$ ± $1.18\times10^3$ |
| | VIM-1 | n.d. | n.t. | n.t. | n.t. |
| $k_{off}$ ($sec^{-1}$) | KPC-2 | $3.39\times10^{-4}$ ± $0.781\times10^{-4}$ | $1.52\times10^{-4}$ ± $0.328\times10^{-4}$ | $1.52\times10^{-4}$ ± $0.210\times10^4$ | $3.02\times10^{-3}$ ± $1.91\times10^{-3}$ |
| | VIM-1 | $>3.85\times10^{-3}$ | n.t. | n.t. | n.t. |
| $t_{1/2}$ (min) | KPC-2 | 35.3 + 8.31 | 78.4 ± 18.0 | 77.2 ± 9.97 | 5.15 ± 3.39 |
| | VIM-1 | <3.0 | n.t. | n.t. | n.t. |

**Data are shown as average ± SD. *: $k_{inact}/K_i$ ($M^{-1}sec^{-1}$) is shown for TAZ. n.t.: Not tested due to too low inhibitory activity. n.d.: Not determined due to different inhibitory patterns.**

(Example 3) Occurrence frequency of spontaneous resistance of carbapenemase-producing Enterobacteriaceae (CPE) to meropenem (MEPM)/Compound A

An occurrence frequency of spontaneous resistance of CPE to MEPM/Compound A was examined. Test strains were cultured on a Mueller Hinton agar (MHA) at 35°C for 1 day. Colonies were then collected from the agar medium, suspended in cationadjusted Mueller Hinton Broth (2 to 10 mL), and cultured at 35°C. After overnight culture, the resultant was washed twice with buffered saline with gelatin (BSG) and suspended in the BSG to about 1.0 McFarland ($10^8$ CFU/mL). The resulting bacterial suspension was further diluted 10-fold with the BSG ($10^7$ CFU/mL). Then, 100 μL of a $10^4$ to $10^6$-fold dilution of a 1.0 McFarland unit bacterial suspension was smeared on a drugfree MHA. A plate was incubated at 35°C for 1 day and viable counts were determined by counting colonies.

Compound A was dissolved in water containing sodium carbonate and diluted to 80 and 320 μg/mL with water. A MEPM hydrate was dissolved in water and diluted to a concentration of 5 to 320 μg/mL in terms of MEPM. Equal volumes of a MEPM solution and a Compound A solution were mixed to a target concentration. Autoclaved MHA was cooled to about 55 to 60°C. One milliliter of a MEPM/Compound A solution at each concentration was mixed with 9 mL of MHA which remained dissolved in a petri dish, and solidified at room temperature. Then, 100 μL of an inoculum ($10^7$ to $10^8$ CFU/mL) was smeared on an MHA containing the MEPM and the Compound A. For each drug concentration in each test bacterium, 60 to 100 MHAs were used. After culturing at 35°C for 1 day, an emergence frequency of resistance was calculated from a ratio of the number of colonies on a drug-containing MHA to the number of inoculated bacteria. A likelihood of resistance development was determined to be low when a development frequency of resistance was less than 1 x $10^{-8}$.

For E. coli ATCC BAA-2469 strain and Klebsiella pneumoniae CDC-505 strain, a combination of 0.25 μg/mL of the MEPM and 4 μg/mL of the Compound A resulted in a resistance frequency equal to or lower than a threshold (< 1 x $10^{-8}$). Klebsiella pneumoniae CDC-112, ATCC BAA-2344, ATCC BAA-2470, and CDC-148 strains required the Compound A at 16 μg/mL and the MEPM at 0.25, 1, 1, and 4 ug/mL or more to achieve a resistance acquisition frequency of less than 1 x $10^{-8}$, respectively. A combination of the MEPM at 4 μg/mL and the Compound A at 16 μg/mL was able to suppress the occurrence frequency of resistance to less than 1 x $10^{-8}$ in all tested strains.

(Example 4) Minimum bactericidal concentration for carbapenemase-producing Enterobacteriaceae (CPE) and Acinetobacter baumannii (CPA)

A minimum bactericidal concentration (MBC) of Meropenem(MEPM)/Compound A (4 μg/mL) was determined for 17 CPE strains (6 serine-carbapenemase strains and 11 metallo-β-lactamase strains) and 4 CPA strains (3 serine-carbapenemase strains and 1 metallo-β-lactamase strain). MEPM/vaborbactam (8 μg/mL), ceftazidime, and ceftazidime/avibactam (4 μg/mL) were used as control agents. A minimal inhibitory concentration (MIC) assay was performed by a broth microdilution method according to the Clinical and Laboratory Standards Institute (CLSI) method. Test bacteria were cultured on a Mueller Hinton agar (MHA) at 35°C for 21 hours. The resultant was then suspended in buffered saline with gelatin (BSG) to about 0.5 McFarland. A 0.5 McFarland unit bacterial suspension was diluted 20-fold with the BSG to make an inoculum. Then, 100 μL of a $10^5$-fold dilution of the 0.5 McFarland unit bacterial suspension was smeared on an MHA and cultured at 35°C for 20 hours, and viable counts were determined by counting colonies. Then, 100 μL of an antibacterial drug at each concentration or 100 pL of a mixed solution of an antibacterial drug and a β-lactamase inhibitor prepared using a cation-adjusted Mueller Hinton Broth was added to wells of a 96-well round-bottom microplate. The 96-well plate was inoculated with 10 μL of the bacterial suspension and cultured at 35°C for 20 hours. The MIC was defined as the lowest concentration exhibiting no visible growth. A culture solution was collected from each well where no growth was observed, smeared on an MHA, cultured at 35°C for 18 hours, and then colonies were counted. A minimum bactericidal concentration (MBC) was defined as the lowest drug concentration that killed 99.9% or more of inoculated bacteria.

MEPM/Compound A (4 μg/mL) showed a bactericidal activity against all four CPA strains and 16 of 17 CPE strains for which an MBC/MIC ratio was less than 4 (Table 3). An in vitro antibacterial activity of the MEPM/Compound A against CPE and CPA was equal to or greater than that of MEPM/vaborbactam (8 μg/mL) and superior to ceftazidime or ceftazidime/avibactam (4 μg/mL) .

[Table 3-1]

| Organism (Carbapenemase) | Drug | MIC (μg/mL) | MBC (μg/mL) | MBC/MIC ratio |
|---|---|---|---|---|
| *Klebsiella pneumoniae* | MEPM | 16 | 32 | 2 |
| ATCC BAA-2344 | MEPM/CompoundA(4) | 0.03 | 0.03 | 1 |
| (KPC-2) | MEPM/vaborbactam (8) | 0.03 | 0.03 | 1 |
| | CAZ | >128 | >128 | ND |
| | CAZ/avibactam (4) | 1 | 1 | 1 |

(continued)

| Organism (Carbapenemase) | Drug | MIC (μg/mL) | MBC (μg/mL) | MBC/MIC ratio |
|---|---|---|---|---|
| *Escherichia coli* | MEPM | 4 | 8 | 2 |
| ATCC BAA-2340 | MEPM/CompoundA(4) | 0.03 | 0.03 | 1 |
| (KPC-3) | MEPM/vaborbactam (8) | 0.03 | 0.03 | 1 |
| | CAZ | 128 | 128 | 1 |
| | CAZ/avibactam (4) | 1 | 1 | 1 |
| *Klebsiella pneumoniae* | MEPM | 32 | 32 | 1 |
| CDC-112 | MEPM/CompoundA(4) | 0.015 | 0.03 | 2 |
| (KPC-3) | MEPM/vaborbactam (8) | 0.03 | 0.06 | 2 |
| | CAZ | >128 | >128 | ND |
| | CAZ/avibactam (4) | 2 | 2 | 1 |
| *Klebsiella pneumoniae* | MEPM | 1 | 4 | 4 |
| ATCC BAA-2524 | MEPM/CompoundA(4) | 0.03 | 0.03 | 1 |
| (OXA-48) | MEPM/vaborbactam (8) | 0.5 | 4 | 8 |
| | CAZ | 0.5 | 0.5 | 1 |
| | CAZ/avibactam (4) | ≤0.12 | ≤0.12 | ND |
| *Serratia marcescens* | MEPM | 64 | 128 | 2 |
| CDC- 124 | MEPM/CompoundA(4) | 0.06 | 0.06 | 1 |
| (SME-3) | MEPM/vaborbactam (8) | 0.06 | 0.06 | 1 |
| | CAZ | 0.25 | 64 | 256 |
| | CAZ/avibactam (4) | 0.5 | 0.5 | 1 |
| *Enterobacter cloacae* | MEPM | 32 | 32 | 1 |

[Table 3-2]

| Organism (Carbapenemase) | Drug | MIC (μg/mL) | MBC (μg/mL) | MBC/MIC ratio |
|---|---|---|---|---|
| *complex* | MEPM/CompoundA(4) | 0.03 | 0.06 | 2 |
| CDC-164 | MEPM/vaborbactam (8) | 0.06 | 0.25 | 4 |
| (NMC-A) | CAZ | 32 | 32 | 1 |
| | CAZ/avibactam (4) | 1 | 2 | 2 |
| *Escherichia coli* | MEPM | 32 | 32 | 1 |
| ATCC BAA-2469 | MEPM/CompoundA(4) | 0.03 | 0.03 | 1 |
| (NDM-1) | MEPM/vaborbactam (8) | 32 | 32 | 1 |
| | CAZ | >128 | >128 | ND |
| | CAZ/avibactam (4) | >128 | >128 | ND |
| *Klebsiella pneumoniae* | MEPM | 32 | 32 | 1 |
| ATCC BAA-2470 | MEPM/CompoundA(4) | 0.06 | 0.06 | 1 |
| (NDM-1) | MEPM/vaborbactam (8) | 32 | 32 | 1 |
| | CAZ | >128 | >128 | ND |
| | CAZ/avibactam (4) | >128 | >128 | ND |
| *Klebsiella pneumoniae* | MEPM | 64 | 64 | 1 |
| CDC-505 | MEPM/CompoundA(4) | 0.03 | 0.03 | 1 |
| (NDM-1) | MEPM/vaborbactam (8) | 64 | 64 | 1 |
| | CAZ | >128 | >128 | ND |
| | CAZ/avibactam (4) | >128 | >128 | ND |
| *Citrobacter freundii* | MEPM | 128 | 128 | 1 |
| CDC-157 | MEPM/CompoundA(4) | 0.25 | 0.25 | 1 |

(continued)

| Organism (Carbapenemase) | Drug | MIC (μg/mL) | MBC (μg/mL) | MBC/MIC ratio |
|---|---|---|---|---|
| (NDM-1) | MEPM/vaborbactam (8) | 128 | 128 | 1 |
| | CAZ | >128 | >128 | ND |
| | CAZ/avibactam (4) | >128 | >128 | ND |
| *Morganella morganii* CDC-57 (NDM-1) | MEPM | 8 | 8 | 1 |
| | MEPM/CompoundA(4) | 0.12 | 0.12 | 1 |
| | MEPM/vaborbactam (8) | 8 | 8 | 1 |

[Table 3-3]

| Organism (Carbapenemase) | Drug | MIC (μg/mL) | MBC (μg/mL) | MBC/MIC ratio |
|---|---|---|---|---|
| | CAZ | >128 | >128 | ND |
| | CAZ/avibactam (4) | >128 | >128 | ND |
| *Escherichia coli* CDC-150 (NDM-5) | MEPM | 128 | 128 | 1 |
| | MEPM/CompoundA(4) | 0.12 | 0.12 | 1 |
| | MEPM/vaborbactam (8) | 128 | 128 | 1 |
| | CAZ | >128 | >128 | ND |
| | CAZ/avibactam (4) | >128 | >128 | ND |
| *Escherichia coli* CDC-162 (NDM-7) | MEPM | 128 | 128 | 1 |
| | MEPM/CompoundA(4) | 0.12 | 0.12 | 1 |
| | MEPM/vaborbactam (8) | 128 | >128 | ≥2 |
| | CAZ | >128 | >128 | ND |
| | CAZ/avibactam (4) | >128 | >128 | ND |
| *Klebsiella pneumoniae* NCTC13440 (VIM-1) | MEPM | 16 | 64 | 4 |
| | MEPM/CompoundA(4) | 0.06 | 0.06 | 1 |
| | MEPM/vaborbactam (8) | 32 | 32 | 1 |
| | CAZ | >128 | >128 | ND |
| | CAZ/avibactam (4) | >128 | >128 | ND |
| *Escherichia coli* NCTC13476 (IMP-1) | MEPM | 4 | 8 | 2 |
| | MEPM/CompoundA(4) | 0.06 | 0.06 | 1 |
| | MEPM/vaborbactam (8) | 4 | 8 | 2 |
| | CAZ | >128 >128 | >128 | ND |
| | CAZ/avibactam (4) | >128 | >128 | ND |
| *Klebsiella pneumoniae* KUB3607 (IMP-1) | MEPM | 8 | 8 | 1 |
| | MEPM/CompoundA(4) | 0.06 | 0.12 | 2 |
| | MEPM/vaborbactam (8) | 8 | 16 | 2 |
| | CAZ | >128 | >128 | ND |
| | CAZ/avibactam (4) | >128 | >128 | ND |
| *Enterobacter aerogenes* CDC-161 | MEPM | 4 | 8 | 2 |
| | MEPM/CompoundA(4) | 0.06 | 0.5 | 8 |

[Table 3-4]

| Organism (Carbapenemase) | Drug | MIC (μg/mL) | MBC (μg/mL) | MBC/MIC ratio |
|---|---|---|---|---|
| (IMP-4) | MEPM/vaborbactam (8) | 4 | 8 | 2 |
| | CAZ | 128 | 128 | 1 |
| | CAZ/avibactam (4) | >128 | >128 | ND |
| *Acinetobacter baumannii* CDC-303 | MEPM | 64 | 64 | 1 |
| | MEPM/CompoundA(4) | 4 | 16 | 4 |

(continued)

| Organism (Carbapenemase) | Drug | MIC (μg/mL) | MBC (μg/mL) | MBC/MIC ratio |
|---|---|---|---|---|
| (OXA-23, OXA-66) | MEPM/vaborbactam (8) | 64 | 64 | 1 |
| | CAZ | 64 | 128 | 2 |
| | CAZ/avibactam (4) | 16 | 64 | 4 |
| *Acinetobacter baumannii* | MEPM | 128 | 128 | 1 |
| CDC-101 | MEPM/CompoundA(4) | 8 | 8 | 1 |
| (OXA-24, OXA-65) | MEPM/vaborbactam (8) | 128 | 128 | 1 |
| | CAZ | >128 | >128 | ND |
| | CAZ/avibactam (4) | 64 | 128 | 2 |
| *Acinetobacter baumannii* | MEPM | 64 | 64 | 1 |
| CDC-304 | MEPM/Comp) oundA (4) | 4 | 8 | 2 |
| (OXA-66, OXA-72) | MEPM/vaborbactam (8) | 64 | 64 | 1 |
| | CAZ | 64 | 64 | 1 |
| | CAZ/avibactam (4) | 32 | 64 | 2 |
| *Acinetobacter baumannii* | MEPM | >128 | >128 | ND |
| CDC-33 | MEPM/CompoundA(4) | 8 | 16 | 2 |
| (NDM-1, OXA-94) | MEPM/vaborbactam (8) | 128 | >128 | ≥2 |
| | CAZ | >128 | >128 | ND |
| | CAZ/avibactam (4) | >128 | >128 | ND |

ND. Not determined
ME PM. meropenem ; (CAZ. ceftazidime
**Concentrations of Compound A, vaborbactam, and avibactam were fixed at 4, 8, and 4 μg/mL and shown in parentheses.**

(Example 5) Antibacterial activity of MEPM/Compound A against carbapenem-resistant Enterobacteriaceae and carbapenemase-producing Enterobacteriaceae from standard strain and Japanese clinical isolate

(Purpose)

An in vitro antibacterial activity of MEPM/Compound A and control agents against carbapenemase-producing Enterobacteriaceae from the National Collection of Type Cultures (NCTC) and American Type Culture Collection (ATCC), and carbapenem-producing Enterobacteriaceae and carbapenem-resistant Enterobacteriaceae clinically isolated in Japan were tested by in an vitro study. Standard strains harboring KPC, OXA, VIM, and NDM genotypes from the NCTC and the ATCC, and strains harboring IMP, KPC, and NDM genes from clinical isolates in Japan were evaluated. Nineteen serine carbapenemase gene-harboring strains and 27 metallocarbapenemase gene-harboring strains were included.

(Method)

A minimal inhibitory concentration (MIC) was determined by a broth microdilution method according to the CLSI (M07). The test strains were inoculated on a Modified Drigalski agar or a sheep blood agar at 36 ± 1°C for 16 to 24 hours, and colonies on the agar medium were inoculated on the same fresh agar medium and cultured under the same conditions. Colonies on the agar medium were suspended in saline to a turbidity of about 0.5 McF and further diluted 10-fold with saline, which was used as an inoculum. A measured concentration of MEPM was 512 to 0.004 μg/mL. For MEPM/-Compound A and MEPM/vaborbactam, MEPM at the above concentrations was combined with Compound A at fixed concentrations of 4, 8, and 16 μg/mL or vaborbactam at a fixed concentration of 8 μg/mL, respectively. A measured concentration of cefiderocol was 64 to 0.06 μg/mL. Measured concentrations of ceftolozane and piperacillin were 64 to 0.12 and 64 to 0.06 μg/mL, respectively, and both ceftolozane and piperacillin were used in combination with tazobactam at a fixed concentration of 4 μg/mL. Measured concentrations of aztreonam, tigecycline, colistin, amikacin, ciprofloxacin, levofloxacin, and cefozopron were 64 to 0.03 μg/mL. Measured concentrations of ceftazidime and imipenem were 32 to 0.06 μg/mL, and ceftazidime/avibactam and imipenem/relebactam were used in combination with avibactam and relebactam at a fixed concentration of 4 μg/mL, respectively. A cation-adjusted Mueller-Hinton broth (CAMHB) was used to prepare a drug-containing medium, and an iron-deficient CAMHB was used only for cefiderocol. The inoculum was

inoculated into each well with a drug solution using a 96-pin plate (STEM Corporation, P96003S). In order to confirm growth of the test strains and a quality control (QC) strain, the inoculum was inoculated on a drug-free medium in the same manner, which was used as a positive control for growth. The MIC was defined as the lowest concentration exhibiting no visible growth of each of the test strains according to the CLSI (M07-ED11). If the MIC for the QC strain was within an acceptable MIC range described in the CLSI (M100-ED31), the MIC was determined to have been measured accurately.

(Results)

Individual MICs for strains are shown in Table 4, and $MIC_{range}$, $MIC_{50}$, $MIC_{80}$, and $MIC_{90}$ are summarized in Table 5.

The $MIC_{50}$, the $MIC_{80}$, and the $MIC_{90}$ of MEPM against the 53 CRE and CPE strains were 4, 16, and 64 μg/mL, respectively. Compound A evaluated at fixed concentrations of 4 to 16 μg/mL restored an antibacterial activity of MEPM and the $MIC_{50}$, the $MIC_{80}$ and the $MIC_{90}$ of MEPM/Compound A (4 μg/mL) were decreased to 0.03, 1, and 8 μg/mL, respectively. The $MIC_{50}$, the $MIC_{80}$, and the $MIC_{90}$ were decreased to 0.03, 0.5, and 4 μg/mL for MEPM/Compound A (8 μg/mL) and 0.03, 0.25, and 2 μg/mL for MEPM/Compound A (16 μg/mL). The $MIC_{90}$ for MEPM and Compound A at 4 μg/mL was lower than those for comparators such as MEPM/vaborbactam, ceftazidime/avibactam, imipenem/relebactam, and colistin, which are main treatment options for CRE and CPE infections. Only tigecycline (2 μg/mL) and cefiderocol (4 μg/mL) showed lower $MIC_{90}$ values (Table 5).

[Table 4-1]

| Table 4 MIC of meropenem/Compound A and control agents against test strains | | | | | | | |
|---|---|---|---|---|---|---|---|
| KUB No. | Organism | Origin | Harbored carbapenemase genes | Meropenem | Meropenem/CompoundA(4) | Meropenem/CompoundA(8) | Meropenem/CompoundA(16) |
| KUB3148 | *Escherichia coli* | ATCC BAA-2340 | KPC-3 | 0.5 | 0.015 | 0.015 | 0.015 |
| KUB3149 | *Escherichia coli* | ATCC BAA-2469 | NDM-1 | 2 | 0.015 | 0.015 | 0.015 |
| KUB3150 | *Escherichia coli* | ATCC BAA-2523 | OXA-48 | 0.06 | 0.015 | 0.015 | 0.008 |
| KUB3151 | *Escherichia coli* | NCTC13476 | IMP-1 | 1 | 0.03 | 0.015 | 0.015 |
| KUB3152 | *Klebsiella pneumoniae* | ATCC BAA-1705 | KPC-2 | 4 | 0.015 | 0.015 | 0.015 |
| KUB3153 | *Klebsiella pneumoniae* | ATCC BAA-1902 | KPC-3 | 64 | 0.12 | 0.12 | 0.06 |
| KUB3154 | *Klebsiella pneumoniae* | ATCC BAA-1903 | KPC-2 | 4 | 0.015 | 0.015 | 0.015 |
| KUB3155 | *Klebsiella pneumoniae* | ATCC BAA-1905 | KPC-2 | 8 | 0.015 | 0.015 | 0.015 |
| KUB3156 | *Klebsiella pneumoniae* | ATCC BAA-2344 | KPC-2 | 4 | 0.015 | 0.015 | 0.015 |
| KUB3157 | *Klebsiella pneumoniae subsp. pneumoniae* | ATCC BAA-2470 | NDM-1 | 16 | 0.03 | 0.03 | 0.03 |
| KUB3158 | *Klebsiella pneumoniae subsp. pneumoniae* | ATCC BAA-2524 | OXA-48 | 0.25 | 0.03 | 0.03 | 0.03 |
| KUB3159 | *Klebsiella pneumoniae* | NCTC13439 | VIM-1 | 0.5 | 0.03 | 0.03 | 0.03 |
| KUB3160 | *Klebsiella pneumoniae* | NCTC13440 | VIM-1 | 0.5 | 0.03 | 0.03 | 0.03 |

(continued)

| Table 4 MIC of meropenem/Compound A and control agents against test strains | | | | | | | |
|---|---|---|---|---|---|---|---|
| KUB No. | Organism | Origin | Harbored carbapene mase genes | Mer open em | Meropen em/ Compou ndA(4) | Meropen em/ Compou ndA(8) | Meropen em/ Compou ndA(16) |
| KUB3163 | *Klebsiella pneu-moniae* | Clinical iso-late | IMP-1 | 0.5 | 0.03 | 0.015 | 0.015 |
| KUB3164 | *Klebsiella pneu-moniae* | Clinical iso-late | IMP-6 | 2 | 0.5 | 0.5 | 0.12 |
| KUB3165 | *Enterobacter cloacae* | Clinical isolate | IMP-34 | 0.25 | 0.03 | 0.03 | 0.015 |
| KUB3166 | *Klebsiella pneu-moniae* | Clinical iso-late | IMP-1 | 0.5 | 0.03 | 0.03 | 0.015 |
| KUB3167 | *Enterobacter cloacae* | Clinical isolate | IMP-1 | 0.25 | 0.03 | 0.03 | 0.015 |
| KUB3168 | *Enterobacter cloacae* | Clinical isolate | - | 0.06 | 0.03 | 0.03 | 0.03 |
| KUB3169 | *Serratia marces-cens* | Clinical iso-late | Unidentifi ed | 2 | 1 | 0.25 | 0.12 |
| KUB3170 | *Serratia marces-cens* | Clinical iso-late | Unidentifi ed | 2 | 1 | 0.25 | 0.12 |
| KUB3171 | *Serratia marces-cens* | Clinical iso-late | - | 2 | 2 | 2 | 2 |
| KUB3605 | *Escherichia coli* | Clinical isolate | NDM-1 | 32 | 0.015 | 0.015 | 0.015 |
| KUB3606 | *Klebsiella pneu-moniae* | Clinical iso-late | KPC-38 | 256 | 32 | 0.5 | 0.25 |
| KUB3607 | *Klebsiella pneu-moniae* | Clinical iso-late | IMP-1 | 0.5 | 0.03 | 0.03 | 0.015 |
| KUB3609 | *Enterobacter cloacae* | Clinical isolate | IMP-1 | 16 | 8 | 4 | 2 |
| KUB3610 | *Enterobacter cloacae* | Clinical isolate | IMP-1 | 4 | 4 | 4 | 4 |
| KUB3614 | *Klebsiella aero-genes* | Clinical iso-late | - | 2 | 0.5 | 0.5 | 0.25 |
| KUB3615 | *Klebsiella aero-genes* | Clinical iso-late | - | 0.12 | 0.03 | 0.015 | 0.015 |
| KUB3620 | *Enterobacter cloacae* | Clinical isolate | - | 16 | 8 | 8 | 2 |
| KUB3628 | *Klebsiella pneu-moniae* | Clinical iso-late | IMP-1 | 8 | 0.25 | 0.12 | 0.06 |
| KUB3629 | *Klebsiella pnem-noniae* | Clinical iso-late | IMP-1 | 0.5 | 0.03 | 0.015 | 0.015 |
| KUB3630 | *Klebsiella pneu-moniae* | Clinical iso-late | IMP-1 | 1 | 0.015 | 0.015 | 0.015 |
| KUB3631 | *Klebsiella pneu-moniae* | Clinical iso-late | IMP-1 | 4 | 0.12 | 0.06 | 0.03 |

(continued)

| Table 4 MIC of meropenem/Compound A and control agents against test strains | | | | | | | |
|---|---|---|---|---|---|---|---|
| KUB No. | Organism | Origin | Harbored carbapenemase genes | Meropenem | Meropenem/ CompoundA(4) | Meropenem/ CompoundA(8) | Meropenem/ CompoundA(16) |
| KUB3633 | *Klebsiella pneumoniae* | Clinical isolate | IMP-6 | 1 | 0.5 | 0.25 | 0.25 |
| KUB3634 | *Klebsiella pneumoniae* | Clinical isolate | IMP-6 | 1 | 0.5 | 0.25 | 0.12 |
| KUB3635 | *Klebsiella pneumoniae* | Clinical isolate | IMP-1 | 32 | 16 | 16 | 8 |
| KUB3996 | *Klebsiella pneumoniae* | ATCC BAA-1898 | KPC-2 | 32 | 0.03 | 0.03 | 0.015 |
| KUB3997 | *Klebsiella pneumoniae* | ATCC BAA-1899 | KPC-2 | 8 | 0.015 | 0.03 | 0.015 |
| KUB3998 | *Klebsiella pneumoniae* | ATCC BAA-1900 | KPC-3 | 8 | 0.015 | 0.03 | 0.03 |
| KUB3999 | *Klebsiella pneumoniae* | ATCC BAA-1904 | KPC-3 | 2 | 0.03 | 0.03 | 0.015 |
| KUB4000 | *Klebsiella pneumoniae* | ATCC BAA-2078 | KPC-3 | 4 | 0.015 | 0.015 | 0.015 |
| KUB4001 | *Enterobacter hormaechei* | ATCC BAA-2082 | KPC-2 | 16 | 0.12 | 0.06 | 0.03 |
| KUB4002 | *Enterobacter cloacae* | ATCC BAA-2341 | KPC-3 | 16 | 0.03 | 0.03 | 0.03 |
| KUB4003 | *Klebsiella pneumoniae* | ATCC BAA-2342 | KPC-3 | 4 | 0.015 | 0.015 | 0.015 |
| KUB4004 | *Klebsiella pneumoniae* | ATCC BAA-2343 | KPC-3 | 16 | 0.03 | 0.03 | 0.03 |

[Table 4-2]

| KUB No. | Organism | Origin | Harbored carbapenemase genes | Meropenem | Meropenem/ CompoundA(4) | Meropenem/ CompoundA(8) | Meropenem/ CompoundA(16) |
|---|---|---|---|---|---|---|---|
| KUB4005 | *Escherichia coli* | ATCC BAA-245-2 | NDM-1 | 1 | 0.015 | 0.015 | 0.015 |
| KUB4006 | *Enterobacter cloacae* | ATCC BAA-246-8 | NDM-1 | 128 | 128 | 8 | 2 |
| KUB4007 | *Escherichia coli* | ATCC BAA-247-1 | NDM-6 | 32 | 0.12 | 0.12 | 0.06 |

(continued)

| KUB No. | Organism | Origin | Harbored carbapenemase genes | Meropenem | Meropenem/ CompoundA(4) | Meropenem/ CompoundA(8) | Meropenem/ CompoundA(16) |
|---|---|---|---|---|---|---|---|
| KUB4008 | *Klebsiella pneumoniae subsp. Pneumoniae* | ATCC BAA-247-2 | NDM-1 | 128 | 64 | 16 | 1 |
| KUB4009 | *Klebsiella pneumoniae* | ATCC BAA-247-3 | NDM-1 | 64 | 16 | 4 | 0.25 |
| KUB4010 | *Providencia rettgeri deposited as P. rettgeri* | ATCC BAA-252-5 | OXA-181 | 2 | 0.12 | 0.12 | 0.12 |
| KUB4011 | *Klebsiella pneumoniae* | ATCC BAA-214-6 | NDM-1 | 64 | 0.5 | 0.12 | 0.06 |

[Table 4-3]

Table 4 continued

| KUB No. | Harbored carbapene mase genes | Meropen em/ Vaborba ctam(8) | Ceftaz idime | Ceftaz idime/ Avibac tam(4) | Imipen em | Imipene m/ Relebact am(4) | Ceftoloz ane/ Tazobact am(4) | Cefide rocol | Piperacil lin/ Tazobact am(4) | Aztreo nam |
|---|---|---|---|---|---|---|---|---|---|---|
| KUB3148 | KPC-3 | 0.015 | >32 | 0.25 | 1 | 0.12 | 16 | 1 | >64 | 64 |
| KUB3149 | NDM-1 | 4 | >32 | >32 | 4 | 4 | >64 | 2 | >64 | 8 |
| KUB3150 | OXA-48 | 0.12 | 1 | 0.12 | 0.5 | 0.25 | 0.5 | ≤0.06 | 32 | 8 |
| KUB3151 | IMP-1 | 1 | >32 | 32 | 0.5 | 1 | >64 | 1 | 4 | 0.25 |
| KUB3152 | KPC-2 | 0.015 | 32 | 1 | 8 | 0.12 | >64 | 2 | >64 | >64 |
| KUB3153 | KPC-3 | 0.5 | >32 | 1 | 16 | 0.12 | >64 | 2 | >64 | >64 |
| KUB3154 | KPC-2 | 0.015 | 32 | 0.5 | 1 | 0.12 | 64 | 4 | >64 | >64 |
| KUB3155 | KPC-2 | 0.015 | >32 | 1 | 8 | ≤0.06 | 64 | 4 | >64 | >64 |
| KUB3156 | KPC-2 | 0.015 | >32 | 0.5 | 8 | 0.12 | 64 | 2 | >64 | >64 |
| KUB3157 | NDM-1 | 16 | >32 | >32 | 16 | 8 | >64 | 8 | >64 | >64 |
| KUB3158 | OXA-48 | 0.25 | 0.5 | 0.12 | 1 | 4 | 1 | 1 | >64 | 0.12 |
| KUB3159 | VIM-1 | 0.25 | >32 | >32 | 4 | 8 | >64 | 4 | 64 | >64 |
| KUB3160 | VIM-1 | 0.5 | >32 | >32 | 4 | 8 | >64 | 0.5 | >64 | 64 |
| KUB3163 | IMP-1 | 0.5 | >32 | 32 | 2 | 2 | >64 | 0.5 | 8 | 0.12 |
| KUB3164 | IMP-6 | 2 | 32 | 16 | 0.5 | 0.5 | 32 | 0.12 | 8 | 16 |
| KUB3165 | IMP-34 | 0.25 | >32 | >32 | 0.5 | 1 | >64 | 1 | 2 | ≤0.03 |
| KUB3166 | IMP-1 | 0.5 | >32 | >32 | 1 | 1 | >64 | 2 | 4 | 4 |
| KUB3167 | IMP-1 | 1 | >32 | >32 | 1 | 0.5 | >64 | 1 | 4 | 0.25 |
| KUB3168 | - | 0.03 | 1 | 0.25 | 1 | 0.25 | 0.25 | n.d. | 2 | 0.25 |
| KUB3169 | Unidentifi ed | 0.5 | 8 | 0.5 | 1 | 0.25 | 4 | 1 | 64 | 8 |
| KUB3170 | Unidentified | 0.25 | 8 | 0.5 | 1 | 0.5 | 4 | 1 | 64 | 8 |
| KUB3171 | - | 2 | 8 | 8 | 4 | 4 | 8 | 1 | 64 | 8 |
| KUB3605 | NDM-1 | 32 | >32 | >32 | 4 | 4 | >64 | 1 | >64 | >64 |
| KUB3606 | KPC-38 | 2 | >32 | 4 | >32 | 8 | >64 | 1 | >64 | >64 |

(continued)

Table 4 continued

| KUB No. | Harbored carbapene mase genes | Meropen em/ Vaborba ctam(8) | Ceftaz idime | Ceftaz idime/ Avibac tam(4) | Imipen em | Imipene m/ Relebact am(4) | Ceftoloz ane/ Tazobact am(4) | Cefide rocol | Piperacil lin/ Tazobact am(4) | Aztreo nam |
|---|---|---|---|---|---|---|---|---|---|---|
| KUB3607 | IMP-1 | 0.5 | >32 | >32 | 1 | 1 | 64 | 2 | 8 | 64 |
| KUB3609 | IMP-1 | 16 | >32 | >32 | 16 | 16 | >64 | 2 | >64 | 64 |
| KUB3610 | IMP-1 | 8 | >32 | >32 | 16 | 8 | >64 | n.d. | >64 | >64 |
| KUB3614 | - | 1 | >32 | 2 | 16 | 0.5 | 8 | 1 | >64 | 64 |
| KUB3615 | - | 0.03 | >32 | 1 | 0.5 | 0.5 | 8 | 0.5 | 64 | 32 |
| KUB3620 | - | 1 | >32 | 1 | 32 | 1 | 8 | 1 | 32 | 64 |
| KUB3628 | IMP-1 | 4 | >32 | >32 | 32 | 8 | >64 | 0.25 | 32 | 0.06 |
| KUB3629 | IMP-1 | 0.25 | >32 | >32 | 8 | 0.25 | >64 | 2 | 16 | 64 |
| KUB3630 | IMP-1 | 1 | >32 | >32 | 1 | 0.5 | >64 | 2 | 16 | 64 |

[Table 4-4]

| KUB No. | Harbored carbapenemase genes | Meropenem/ Vaborbactam(8) | Ceftazidime | Ceftazidime/ Avibactam(4) | Imipenem | Imipenem/ Relebactam(4) | Ceftolozane/ Tazobactam(4) | Cefiderocol | Piperacillin/ Tazobactam(4) | Aztreonam |
|---|---|---|---|---|---|---|---|---|---|---|
| KUB3631 | IMP-1 | 8 | >32 | >32 | 1 | 1 | >64 | 2 | >64 | >64 |
| KUB3633 | IMP-6 | 1 | 32 | 16 | 0.12 | 0.12 | 64 | 1 | 4 | 8 |
| KUB3634 | IMP-6 | 1 | 16 | 32 | 0.12 | 0.12 | 32 | 2 | 4 | 16 |
| KUB3635 | IMP-1 | 32 | >32 | >32 | >32 | >32 | >64 | l | >64 | >64 |
| KUB3996 | KPC-2 | 0.06 | >32 | 1 | 32 | 0.25 | 64 | 2 | >64 | >64 |
| KUB3997 | KPC-2 | 0.015 | >32 | 1 | 8 | 0.12 | 64 | 2 | >64 | >64 |
| KUB3998 | KPC-3 | 0.03 | >32 | 2 | 32 | 0.12 | >64 | 0.25 | >64 | >64 |
| KUB3999 | KPC-3 | 0.03 | 16 | 0.25 | 4 | 0.12 | 32 | 1 | >64 | *64* |
| KUB4000 | KPC-3 | 0.015 | >32 | 1 | 8 | 0.12 | >64 | 2 | >64 | >64 |
| KUB4001 | KPC-2 | 0.12 | >32 | 1 | 32 | 0.25 | 64 | 2 | >64 | >64 |
| KUB4002 | KPC-3 | 0.12 | >32 | 2 | 16 | 0.5 | >64 | 0.5 | >64 | >64 |
| KUB4003 | KPC-3 | 0.015 | >32 | 2 | 8 | 0.12 | >64 | 0.5 | >64 | >64 |
| KUB4004 | KPC-3 | 0.03 | >32 | 2 | 16 | 0.5 | >64 | 0.25 | >64 | >64 |
| KUB4005 | NDM-I | 2 | >32 | >32 | 4 | 4 | >64 | 0.5 | >64 | 4 |
| KUB4006 | NDM-1 | 128 | >32 | >32 | >32 | >32 | >64 | 1 | >64 | >64 |
| KUB4007 | NDM-6 | 32 | >32 | >32 | 32 | 16 | >64 | 4 | >64 | >64 |
| KUB4008 | NDM-1 | 128 | >32 | >32 | 32 | 32 | >64 | 8 | >64 | >64 |
| KUB4009 | NDM-1 | 64 | >32 | >32 | 32 | 32 | >64 | 8 | >64 | >64 |
| KUB4010 | OXA-181 | 1 | >32 | >32 | 4 | 4 | 64 | 2 | >64 | 1 |
| KUB4011 | NDM-1 | 32 | >32 | >32 | >32 | >32 | >64 | 2 | >64 | >64 |

[Table 5]

| **Table 5 Activity of MEPM/Compound A and control agents for 53 bacterial isolates** | | | | | | | |
|---|---|---|---|---|---|---|---|
| **Antibacterial agent** | **MIC (μg/mL)** | | | | **CLSI breakpoint (μg/mL)** | | |
| | $MIC_{range}$ | $MIC_{50}$ | $MIC_{80}$ | $MIC_{90}$ | **Susceptible** | **Intermediate** | Resistant |
| **MEPM** | **0.06 - 256** | **4** | **16** | **64** | **≤1** | **2** | **≥4** |
| **MEPM/Compound A(4)** | **0.015 - 128** | **0.03** | **1** | **8** | **-** | **-** | **-** |
| **MEPM/Compound A(8)** | **0.015 - 16** | **0.03** | **0.5** | **4** | **-** | **-** | **-** |
| **MEPM/Compound A(16)** | **0.008 - 8** | **0.03** | **0.25** | **2** | **-** | **-** | **-** |
| **MEPM/ vaborbactam (8)** | **0.015 - 128** | **0.5** | **8** | **32** | **≤4** | **8** | **≥16** |
| **Ceftazidime** | **0.5 - >32** | **>32** | **>32** | **>32** | **≤4** | **8** | **≥16** |
| **Ceftazidime/avibactam (4)** | **0.12 - >32** | **16** | **>32** | **>32** | **≤8** | **-** | **≥16** |
| **Imipenem** | **0.12 - >32** | **4** | **32** | **32** | **≤1** | **2** | **≥4** |
| **Imipenem/relebactam (4)** | **≤0.06 - >32** | **0.5** | **8** | **16** | **≤1*** | **2*** | **≥4*** |
| **Ceftolozane/tazobactam (4)** | **0.25 - >64** | **>64** | **>64** | **>64** | **≤2** | **4** | **≥8** |
| **Cefiderocol** | ≤0.06 - 8 | l | 2 | 4 | ≤4 | 8 | ≥16 |
| **Piperacillin/tazobactam (4)** | 2 - >64 | >64 | >64 | >64 | ≤16 | 32 - 64 | ≥128 |

(continued)

| **Table 5 Activity of MEPM/Compound A and control agents for 53 bacterial isolates** | | | | | | | |
|---|---|---|---|---|---|---|---|
| **Antibacterial agent** | **MIC (μg/mL)** | | | | **CLSI breakpoint (μg/mL)** | | |
| | $MIC_{range}$ | $MIC_{50}$ | $MIC_{80}$ | $MIC_{90}$ | **Susceptible** | **Intermediate** | Resistant |
| **Aztreonam** | ≤0.03 - >64 | 64 | >64 | >64 | ≤4 | 8 | ≥16 |
| **Tigecycline** | 0.12 - 16 | 0.5 | 1 | 2 | ≤2* | 4* | ≥8* |
| **Colistin** | 0.25 - >64 | 0.5 | 1 | 32 | - | ≤2 | ≥4 |
| **Amikacin** | 0.5 - >64 | 2 | 32 | >64 | ≤16 | 32 | ≥64 |
| **Ciprofloxacin** | ≤0.03 - >64 | 32 | >64 | >64 | ≤0.25 | 0.5 | ≥1 |
| **Levofloxacin** | 0.06 - >64 | 16 | 64 | 64 | ≤0.5 | 1 | ≥2 |
| **Cefozopran** | 0.06 - >64 | 32 | >64 | >64 | - | - | - |
| Concentration of β -lactamase inhibitor (μg/mL) in combination with β -lactam is shown in parentheses.<br>* Breakpoint provided by FDA was referenced since breakpoint is not described in CLSI<br>[4 Antibacterial Susceptibility Test Interpretive Criteria. FDA. https://www.fda.gov/drugs/development-resources antibacterial-susceptibilitytestinterpretive-criteria].<br>**Data in bold represent main treatment options for CPE and CRE infections** | | | | | | | |

The MIC of MEPM/Compound A against the serine-carbapenemase- and metallo-β-lactamase-producing strains was almost equal to those against all 53 strains (Table 6). The $MIC_{90}$ of the MEPM/Compound A (at a fixed concentration of 4 μg/mL) against the serine carbapenemase-producing strains was lower than that of all controls, and the $MIC_{90}$ (16 μg/mL) of the MEPM/Compound A (at a fixed concentration of 4 μg/mL) against the metallo-β-lactamase-producing strains was lower than those of all control agents except for cefiderocol (8 μg/mL), tigecycline (4 μg/mL), or colistin (1 μg/mL). The $MIC_{90}$ value of the MEPM/Compound A was the lowest among all drugs except for colistin when used in combination with Compound A at 16 μg/mL.

[Table 6]

| **Table 6. Activity of MEPM/Compound A and control agents for carbapenemase-producing isolates** | | | | | | |
|---|---|---|---|---|---|---|
| | **Antibacterial agent** | **$MIC_{50}$ (mg/mL)** | **$MIC_{90}$ (mg/mL)** | **Antibacterial agent** | **$MIC_{50}$ (mg/mL)** | **$MIC_{90}$ (mg/mL)** |
| **Serine-carbape ne-mase [a] (N = 19)** | **MEPM** | **4** | **64** | **Cefiderocol** | **2** | **4** |
| | MEPM/Compound A (4) | 0.015 | 0.12 | **Piperacillin/ tazobactam (4)** | >64 | >64 |
| | MEPM/Conpound A (8) | 0.03 | 0.12 | **Aztreonam** | >64 | >64 |
| | MEPM/Compound A (16) | 0.015 | 0.12 | **Tigecycline** | 0.5 | 1 |
| | **MEPM/Vaborbactam (8)** | 0.03 | 1 | **Colistin** | 0.25 | 2 |
| | **Ceftazidime** | >32 | >32 | **Amikacin** | 4 | 64 |
| | **Ceftazidime/avibactam(4)** | 1 | 4 | **Ciprofloxacin** | 64 | >64 |
| | **Imipenem** | 8 | 32 | **Levofloxacin** | 32 | 64 |
| | **Imipenem/relebactam(4)** | 0.12 | 4 | **Cefozopran** | 32 | >64 |
| | **Ceftolozane/tazobactam(4)** | 64 | >64 | | | |
| | MEPM | 2 | 64 | **Cefiderocol** | 2 | 8 |
| | MEPM/Compound A (4) | 0.03 | 16 | **Piperacillin/ tazobactam(4)** | >64 | >64 |
| | **MEPM/Compound A** (8) | 0.03 | 8 | **Aztreonam** | 64 | >64 |

(continued)

| Table 6. Activity of MEPM/Compound A and control agents for carbapenemase-producing isolates | | | | | | |
|---|---|---|---|---|---|---|
| | **Antibacterial agent** | **$MIC_{50}$ (mg/mL)** | **$MIC_{90}$ (mg/mL)** | **Antibacterial agent** | **$MIC_{50}$ (mg/mL)** | **$MIC_{90}$ (mg/mL)** |
| **Metallo-$\beta$-lactamase (N = 27)** | MEPM/Compound A (16) | 0.03 | 2 | **Tigecycline** | 0.5 | 4 |
| | MEPM/**Vaborbactam**(8) | **2** | 64 | **Colistin** | 0.25 | 1 |
| | **Ceftazidime** | **>32** | >32 | **Amikacin** | 2 | >64 |
| | **Ceftazidime/avibactam(4)** | >32 | >32 | **Ciprofloxacin** | 32 | >64 |
| | **Imipenem** | 4 | >32 | **Levofloxacin** | 16 | 64 |
| | **Imipenem/relebactam(4)** | 4 | >32 | **Cefozopran** | 64 | >64 |
| | **Ceftolozane/tazobactom(4)** | >64 | >64 | | | |
| **[a]KPC-2 (7), KPC-3 (8), KPC-38 (1), OXA-48 (2), OXA-181 (1). [b]IMP-1 (12), IMP-6 (3), IMP-34 (1), NDM-1 (8), NDM-6 (1), VIM-1 (2)** | | | | | | |

(Example 6) In vitro antibacterial activity of MEPM/Compound A against A. baumannii

A MIC measurement test was performed by a broth microdilution method according to the Clinical and Laboratory Standards Institute (CLSI) M07-A10 method. Test strains were cultured on an agar medium for 18 to 24 hours and colonies were collected and suspended in a cation-adjusted Mueller Hinton Broth (CAMHB) to achieve a target bacterial concentration using an optical density measurement ($OD_{620}$). Then, 196 μL of the resulting bacterial suspension was added to wells of a 96-well plate (final bacterial count was 2 to 8 x $10^5$ CFU/mL). Mero·BR>Ynem (MEPM) and colistin sulfate were dissolved in distilled water for injection and adjusted to various concentrations with distilled water for injection. Compound A was dissolved in an equimolar sodium carbonate aqueous solution and adjusted to various concentrations with distilled water for injection. Stock solutions of Compound A and MEPM (100x each, 2 μL each) or colistin (50x each, 4 μL each) were added to wells of the above 96-well plate. A concentration of MEPM ranged from 128 to 0.125 μg/mL, and Compound A at fixed concentrations of 4, 8, and 16 μg/mL were used in combination. The 96-well plate was cultured at 35 to 37°C for 18 hours. The MIC was defined as the lowest drug concentration that completely inhibited visble growth of a pathogen in a culture medium.

When the Compound A at fixed concentrations of 4, 8, and 16 μg/mL was added to the MEPM, the MIC of the MEPM was decreased to 1 to 8, 0.5 to 4, and 0.25 to 2 μg/mL against 5 MEPM-resistant **A.** baumannii strains, respectively. For one MEPM-susceptible strain, the MIC of the MEPM was 2 ug/mL and was not significantly changed even when Compound A was added thereto (Table 7).

[Table 7]

| Table 7. MIC values of MEPM/Compound A against 6 strains of A. *baumannii* | | | | | | |
|---|---|---|---|---|---|---|
| **AMR gene** | **Strain information** | **MEPM** | **MEPM / Compound A 4 μg/mL** | **MEPM/ Compou nd A 8 μg/mL** | **MEPM/ Compou nd A 16 μg/mL** | **Colistin sulfate (refere nce)** |
| ADC-25, OXA-66, TEM-1D | **Carbapenem-susceptible** | 2 | 2 | 2 | 1 | 2 |
| ADC-25, XA-23, OXA-66, strA, strB, sul2, TEM-1D | **Carbapenem-resistant** | 64 | 8 | 4 | 2 | 1 |
| ADC-25, OXA-23, OXA-66, TEM-ID | **Carbapenem-resistant** | 64 | 8 | 4 | 2 | 1 |
| OXA-23, OXA-69, TEM-1D, | **Carbapenem-resistant** | 16 | 1 | 0.5 | 0.25 | 1 |

(continued)

| Table 7. MIC values of MEPM/Compound A against 6 strains of A. *baumannii* | | | | | | |
|---|---|---|---|---|---|---|
| **AMR gene** | **Strain information** | **MEPM** | **MEPM / Compound A 4 μg/mL** | **MEPM/ Compou nd A 8 μg/mL** | **MEPM/ Compou nd A 16 μg/mL** | **Colistin sulfate (refere nce)** |
| OXA-24, OXA-65 | **Carbapenem-resistant** | 128 | 4 | 4 | 1 | 1 |
| NDM-1, OXA-64 | **Carbapenem-resistant** | >128 | 4 | 2 | 1 | 1 |

(Example 7) Antibacterial activity of MEPM/Compound A against Gram-negative bacilli from Centers for Disease Control and Prevention (CDC) and U.S. Food and Drug Administration (FDA) Antibiotic Resistance Isolate Bank

(Purpose)

An antibacterial activity of MEPM/Compound A and control agents was examined against Enterobacterales, A. baumannii, and P. aeruginosa including a carbapenem-resistant strain.

(Method)

An antibacterial activity of MEPM/Compound A and control agents against 305 Gram-negative bacilli strains from the Centers for Disease Control and Prevention (CDC) and U.S. Food and Drug Administration (FDA) Antibiotic Resistance Isolate Bank was examined in an in vitro study. A minimal inhibitory concentration (MIC) was determined by a broth microdilution method according to the Clinical and Laboratory Standards Institute (CLSI, M07).

Test strains were 154 strains of Enterobacterales (61 serine-carbapenemase producing strains and 47 metallo-β-lactamase producing strains), 55 strains of Acinetobacter baumannii (50 serine-carbapenemase producing strains and 4 metallo-β-lactamase producing strains), 96 strains of P. aeruginosa (7 serine-carbapenemase producing strains and 26 metallo-β-lactamase producing strains). The test strains were inoculated on a modified Drigalski agar a sheep blood agar at 36 ± 1°C for 16 to 24 hours, and colonies on the agar medium were inoculated on the same fresh agar medium and cultured under the same conditions. The colonies on the agar medium were suspended in saline to a turbidity of about 0.5 McF and further diluted 10-fold with saline, which was used as an inoculum. Measured concentrations of MEPM were 32 to 0.06 μg/mL for Enterobacterales and 128 to 0.25 μg/mL for A. baumannii and P. aeruginosa. For MEPM/Compound A and MEPM/vaborbactam, the MEPM at the above concentrations was combined with Compound A at fixed concentrations of 4, 8, and 16 ug/mL or vaborbactam at a fixed concentration of 8 μg/mL, respectively. A measured concentration of cefiderocol was 64 to 0.06 μg/mL. Measured concentrations of ceftolozane and piperacillin were 64 to 0.12 and 64 to 0.06 μg/mL, respectively, and both ceftolozane and piperacillin were used in combination with tazobactam at a fixed concentration of 4 μg/mL. Measured concentrations of aztreonam, tigecycline, colistin, amikacin, ciprofloxacin, levofloxacin, and cefozopron were 64 to 0.03 μg/mL. Measured concentrations of ceftazidime and imipenem were 32 to 0.06 μg/mL, and ceftazidime and imipenem were used in combination with avibactam and relebactam at a fixed concentration of 4 μg/mL, respectively. A cation-adjusted Mueller-Hinton medium (CAMHB) was used to prepare a drug-containing medium, and an iron-deficient CAMHB was used only for cefiderocol. An inoculum was inoculated into each well with a drug solution using a 96-pin plate (STEM Corporation, P96003S). In order to confirm growth of the test strains and a quality control (QC) strain, the inoculum was inoculated on a drug-free medium in the same manner, which was used as a positive control for growth. The MIC was defined as the lowest concentration exhibiting no visible growth of each of the test strains according to the CLSI (M07-ED11). If the MIC for the QC strain was within an acceptable MIC range described in the CLSI (M100-ED31), the MIC was determined to have been measured accurately.

(Results)

MICs of MEPM/Compound A and control agents against the test strains are summarized in Tables 8 to 10.

[Table 8-1]

**Table 8 MIC of MEPM/Compound A and control agents** against ***Enterobacterales***

| Organism | KUB No. | AR Bank # | Harbored carbapenemase genes | | MIC (μg/mL) | | | | | | |
|---|---|---|---|---|---|---|---|---|---|---|---|
| | | | Serine -type | Metallo -type | Meropenem | Meropenem/ CompoundA(4) | Meropenem/ CompoundA(8) | Meropenem/ CompoundA(16) | Meropenem/ Vaborbactam(8) | Ceftazidime/ Avibactam(4) | Imipenem/ Relebactam(4) |
| *Enterobacter cloacae* | 5529 | 32 | KPC-3 | - | 0.12 | ≤0.06 | ≤0.06 | ≤0.06 | ≤0.06 | ≤0.06 | ≤0.06 |
| *Klebsiella pneumoniae* | 5531 | 34 | - | IMP-4 | 0.25 | ≤0.06 | ≤0.06 | ≤0.06 | 0.25 | >32 | 0.5 |
| *Enterobacter cloacae* | 5535 | 38 | - | NDM-1 | >32 | >32 | >32 | >32 | >32 | >32 | >32 |
| *Klebsiella pneumoniae* | 5536 | 39 | OXA-181 | - | 1 | ≤0.06 | ≤0.06 | ≤0.06 | 1 | 0.5 | 0.5 |
| *Klebsiella pneumoniae* | 5537 | 40 | - | VIM-27 | >32 | 16 | 8 | 1 | >32 | >32 | >32 |
| *Klebsiella pneumoniae* | 5538 | 41 | - | NDM-1 | 8 | ≤0.06 | 0.06 | ≤0.06 | 16 | >32 | 4 |
| *Klebsiella pneumoniae* | 5540 | 43 | - | - | 0.5 | 0.5 | 0.25 | 0.12 | 0.5 | 1 | 0.25 |
| *Klebsiella pneumoniae* | 5541 | 44 | - | - | 2 | 0.25 | 0.25 | 0.12 | 0.5 | 1 | 0.5 |
| *Klebsiella pneumoniae* | 5543 | 46 | - | VIM-27 | 32 | 8 | 2 | 0.5 | 32 | >32 | 32 |
| *Klebsiella pneumoniae* | 5544 | 47 | - | - | 4 | 2 | 1 | 0.5 | 2 | 2 | 0.5 |
| *Escherichia coli* | 5545 | 48 | - | NDM-1 | 16 | ≤0.06 | ≤0.06 | ≤0.06 | 16 | >32 | 16 |
| *Klebsiella pneumoniae* | *5546* | 49 | - | NDM-1 | 32 | 0.25 | ≤0.06 | ≤0.06 | 32 | >32 | 32 |
| *Enterobacter cloacae* | 5547 | 50 | KPC-4 | - | 0.5 | ≤0.06 | ≤0.06 | ≤0.06 | 0.25 | 8 | 0.25 |
| *Klebsiella ozaenae* | 5548 | 51 | OXA-181 | - | 1 | ≤0.06 | ≤0.06 | ≤0.06 | 1 | 1 | 2 |
| *Enterobacter cloacae* | 5550 | 53 | KPC-3 | - | 2 | ≤0.06 | ≤0.06 | ≤0.06 | ≤0.06 | 2 | 0.12 |
| *Escherichia coli* | 5552 | 55 | - | NDM-1 | 2 | ≤0.06 | ≤0.06 | ≤0.06 | 8 | >32 | 4 |
| *Morganella morganii* | 5554 | 57 | - | NDM-1 | 2 | ≤0.06 | ≤0.06 | ≤0.06 | 2 | >32 | 8 |
| *Escherichia coli* | 5555 | 58 | - | - | 0.25 | ≤0.06 | ≤0.06 | ≤0.06 | 0.25 | 0.25 | ≤0.06 |
| *Proteus mirabilis* | 5556 | 59 | - | - | 0.12 | 0.12 | 0.12 | 0.12 | 0.12 | 1 | 4 |
| *Enterobacter cloacae* | 5557 | 60 | - | - | ≤0.06 | ≤0.06 | ≤0.06 | ≤0.06 | ≤0.06 | 0.5 | 0.12 |
| *Escherichia* | 5558 | 61 | KPC- | - | 1 | ≤0.06 | ≤0.06 | ≤0.06 | ≤0.06 | 0.5 | 0.12 |

[Table 8-2]

| Orgamism | KUB No. | AR Bank # | Harbored carbapenemase genes | | MIC (μg/mL) | | | | | | |
|---|---|---|---|---|---|---|---|---|---|---|---|
| | | | Serine -type | Metallo -type | Meropenem | Meropenem/ CompoundA(4) | Meropenem/ CompoundA(8) | Meropenem/ CompoundA(16) | Meropenem/ Vaborbactam(8) | Ceftazidime/ Avibactam(4) | Imipenem/ Relebactam(4) |
| *coli* | | | 3 | | | | | | | | |
| *Enterobacter aerogenes* | 5559 | 62 | - | - | 0.12 | ≤0.06 | ≤0.06 | ≤0.06 | ≤0.06 | 0.5 | 0.12 |
| *Enterobacter cloacae* | 5562 | 65 | - | - | 0.25 | ≤0.06 | ≤0.06 | ≤0.06 | ≤0.06 | 1 | 0.12 |
| *Klebsiella pneumoniae* | 5563 | 66 | OXA-232 | - | 8 | 1 | 0.25 | 0.25 | 8 | 4 | 1 |
| *Escherichia coli* | 5564 | 67 | - | - | ≤0.06 | ≤0.06 | ≤0.06 | ≤0.06 | ≤0.06 | 0.12 | 0.12 |
| *Klebsiella pneumoniae* | 5565 | 68 | OXA-232 | NDM-1 | >32 | 32 | 1 | 0.25 | >32 | >32 | >32 |
| *Escherichia coli* | 5566 | 69 | - | NDM-1 | 1 | ≤0.06 | ≤0.06 | ≤0.06 | 2 | >32 | 4 |
| *Klebsiella oxytoca* | 5568 | 71 | - | - | 8 | 8 | 2 | 0.25 | 4 | 2 | 0.12 |
| *Enterobacter cloacae* | 5569 | 72 | - | - | 0.12 | ≤0.06 | ≤0.06 | ≤0.06 | ≤0.06 | 1 | ≤0.06 |
| *Enterobacter cloacae* | 5570 | 73 | - | - | ≤0.06 | ≤0.06 | ≤0.06 | ≤0.06 | ≤0.06 | 0.25 | 0.12 |
| *Enterobacter aerogenes* | 5571 | 74 | OXA-48 | - | 0.5 | ≤0.06 | ≤0.06 | ≤0.06 | 0.25 | 0.25 | 2 |
| *Klebsiella pnenmoniae* | 5572 | 75 | OXA-232 | - | 8 | 1 | ≤0.06 | ≤0.06 | 8 | 1 | 0.5 |
| *Klebsiella pneumoniae* | 5573 | 76 | - | VIM-1 | 2 | ≤0.06 | ≤0.06 | ≤0.06 | 1 | >32 | 2 |
| *Escherichia coli* | 5574 | 77 | - | - | ≤0.06 | ≤0.06 | ≤0.06 | ≤0.06 | ≤0.06 | 0.12 | 0.12 |
| *Klebsiella pneumoniae* | 5576 | 79 | - | - | 4 | 4 | 2 | 0.25 | 2 | 2 | 0.25 |
| *Klebsiella pneumoniae* | 5577 | 80 | - | IMP-4 | 4 | 0.25 | ≤0.06 | ≤0.06 | 4 | >32 | 8 |
| *Escherichia coli* | 5578 | 81 | - | - | ≤0.06 | ≤0.06 | ≤0.06 | ≤0.06 | ≤0.06 | 0.12 | 0.12 |
| *Providencia rettgeri* | 5579 | 82 | - | NDM-1 | 8 | ≤0.06 | ≤0.06 | ≤0.06 | 8 | >32 | 32 |
| *Escherichia coli* | 5581 | 84 | - | - | ≤0.06 | ≤0.06 | ≤0.06 | ≤0.06 | ≤0.06 | 0.5 | 0.5 |
| *Escherichia coli* | 5582 | 85 | - | - | 0.25 | ≤0.06 | ≤0.06 | ≤0.06 | 0.12 | 0.25 | 0.12 |
| *Escherichia coli* | 5583 | 86 | - | - | 0.12 | ≤0.06 | ≤0.06 | ≤0.06 | ≤0.06 | 0 | ≤0.06 |
| *Klebsiella pneumoniae* | 5584 | 87 | - | - | ≤0.06 | ≤0.06 | ≤0.06 | ≤0.06 | 0.12 | 2 | 0.25 |

(continued)

| Orgamism | KUB No. | AR Ban k # | Harbored carbapenemase genes | | MIC ($\mu$g/mL) | | | | | | |
|---|---|---|---|---|---|---|---|---|---|---|---|
| | | | Serine -type | Metallo -type | Merop enem | Merop enem/ Compo undA(4 ) | Merop enem/ Compo undA(8 ) | Merop enem/ Compo undA(1 6) | Merop enem/ Vaborb actam( 8) | Ceftazi dime/ Avibac tam(4) | Imipen em/ Releba ctam(4 ) |
| *Escherichia coli* | 5586 | 89 | - | - | ≤0.06 | ≤0.06 | ≤0.06 | ≤0.06 | ≤0.06 | 0.25 | 0.25 |
| *Serratia marcescens* | 5588 | 91 | SME-3 | - | 16 | ≤0.06 | ≤0.06 | ≤0.06 | ≤0.06 | 0.12 | 2 |
| *Enterobacte r cloacae* | 5590 | 93 | KPC-6 | - | 1 | ≤0.06 | ≤0.06 | ≤0.06 | ≤0.06 | 1 | 0.12 |
| *Klebsiella ozaenae* | 5593 | 96 | KPC-3 | - | 32 | ≤0.06 | ≤0.06 | ≤0.06 | <0.06 | 0.5 | 0.5 |
| *Klebsiella pneumoniae* | 5594 | 97 | KPC-3 | - | 32 | ≤0.06 | ≤0.06 | ≤0.06 | 0.25 | 2 | 1 |

[Table 8-3]

| Organism | KUB No. | AR Bank # | Harbored carbapenemase genes | | MIC (μg/mL) | | | | | | |
|---|---|---|---|---|---|---|---|---|---|---|---|
| | | | Serine -type | Metallo -type | Meropenem | Meropenem/ CompoundA(4) | Meropenem/ CompoundA(8) | Meropenem/ CompoundA(16) | Meropenem/ Vaborbactam(8) | Ceftazidime/ Avibactam(4) | Imipenem/ Relebactam(4) |
| *Klebsiella pneumoniae* | 5595 | 98 | KPC-2 | - | 8 | ≤0.06 | ≤0.06 | ≤0.06 | ≤0.06 | 0.25 | ≤0.06 |
| *Serratia marcescens* | 5596 | 99 | SME-3 | - | 16 | ≤0.06 | ≤0.06 | ≤0.06 | ≤0.06 | 0.12 | 2 |
| *Escherichia coli* | 5601 | 104 | KPC-4 | - | 0.5 | ≤0.06 | ≤0.06 | ≤0.06 | ≤0.06 | 4 | 0.12 |
| *Klebsiella pneumoniae* | 5603 | 106 | - | NDM-1 | >32 | >32 | 2 | 1 | >32 | >32 | >32 |
| *Klebsiella pneumoniae* | 5604 | 107 | - | - | 16 | 4 | 0.5 | 0.12 | 16 | 2 | 4 |
| *Klebsiella pneumoniae* | 5606 | 109 | - | - | 8 | 4 | 1 | 0.25 | 2 | 2 | 0.25 |
| *Klebsiella pneumoniae* | 5609 | 112 | KPC-3 | - | 4 | ≤0.06 | ≤0.06 | ≤0.06 | ≤0.06 | 2 | 0.25 |
| *Klebsiella pneumoniae* | 5610 | 113 | KPC-3 | - | >32 | 16 | 0.12 | 0.12 | 1 | 4 | 2 |
| *Escherichia coli* | 5611 | 114 | KPC-3 | - | 2 | ≤0.06 | ≤0.06 | ≤0.06 | ≤0.06 | 1 | 0.5 |
| *Klebsiella pneumoniae* | 5612 | 115 | KPC-3 | - | 8 | ≤0.06 | ≤0.06 | ≤0.06 | ≤0.06 | 0.5 | ≤0.06 |
| *Citrobacter freundii* | 5613 | 116 | KPC-2 | - | 16 | ≤0.06 | ≤0.06 | ≤0.06 | ≤0.06 | 1 | 0.25 |
| *Klebsiella pneumoniae* | 5614 | 117 | KPC-3 | - | 16 | ≤0.06 | ≤0.06 | ≤0.06 | 0.12 | 1 | 0.12 |
| *Escherichia coli* | 5615 | 118 | - | NDM-1 | 32 | ≤0.06 | ≤0.06 | ≤0.06 | 32 | >32 | 32 |
| *Escherichia coli* | 5616 | 119 | - | NDM-1 | 32 | ≤0.06 | ≤0.06 | ≤0.06 | 16 | >32 | 4 |
| *Klebsiella pneumoniae* | 5617 | 120 | KPC-2 | - | 16 | ≤0.06 | ≤0.06 | ≤0.06 | ≤0.06 | 2 | 0.12 |
| *Serratia marcescens* | 5618 | 121 | SME-3 | - | 16 | ≤0.06 | ≤0.06 | ≤0.06 | ≤0.06 | 0.12 | 2 |
| *Serratia marcescens* | 5619 | 122 | SME-3 | - | 32 | ≤0.06 | ≤0.06 | ≤0.06 | ≤0.06 | 0.12 | 1 |
| *Serratia marcescens* | 5620 | 123 | SME-3 | - | 2 | ≤0.06 | ≤0.06 | ≤0.06 | ≤0.06 | 0.12 | 1 |
| *Serratia marcescens* | 5621 | 124 | SME-3 | - | 32 | ≤0.06 | ≤0.06 | ≤0.06 | ≤0.06 | 0.25 | 2 |
| *Klebsiella pneumoniae* | 5622 | 125 | KPC-3 | - | 16 | ≤0.06 | ≤0.06 | ≤0.06 | ≤0.06 | 2 | 0.12 |
| *Klebsiella pneumoniae* | 5623 | 126 | KPC-2 | - | 4 | ≤0.06 | ≤0.06 | ≤0.06 | ≤0.06 | 0.25 | 0.25 |
| *Salmonella* Senftenberg | 5624 | 127 | - | NDM-1 | 16 | ≤0.06 | ≤0.06 | ≤0.06 | 16 | >32 | 4 |

(continued)

| Organism | KUB No. | AR Bank # | Harbored carbapenemase genes | | MIC (μg/mL) | | | | | | |
|---|---|---|---|---|---|---|---|---|---|---|---|
| | | | Serine -type | Metallo -type | Meropenem | Meropenem/ CompoundA(4 ) | Meropenem/ CompoundA(8 ) | Meropenem/ CompoundA(1 6) | Meropenem/ Vaborbactam( 8) | Ceftazidime/ Avibactam(4) | Imipenem/ Relebactam(4 ) |
| *Escherichia coli* | 5625 | 128 | - | NDM-1 | 32 | ≤0.06 | ≤0.06 | ≤0.06 | 32 | >32 | 32 |
| *Klebsiella pneumoniae* | 5626 | 129 | KPC-3 | - | 4 | ≤0.06 | ≤0.06 | ≤0.06 | ≤0.06 | 2 | 0.12 |
| *Serratia marcescens* | 5627 | 130 | SME-3 | - | 8 | ≤0.06 | ≤0.06 | ≤0.06 | ≤0.06 | 0.25 | 1 |
| *Serratia marcescens* | 5628 | 131 | SME-3 | - | 0.5 | ≤0.06 | ≤0.06 | ≤0.06 | ≤0.06 | 0.12 | 2 |
| *Enterobacte* | 5629 | 132 | NMC | - | 2 | ≤0.06 | ≤0.06 | ≤0.06 | \| ≤0.06 | 0.25 | 1 |

[Table 8-4]

| Orgamism | KUB No. | AR Ban k# | Harbored carbapenemase genes | | MIC (μg/mL) | | | | | | |
|---|---|---|---|---|---|---|---|---|---|---|---|
| | | | Serine -type | Metallo -type | Merop enem | Merop enem/ Compo undA(4 ) | Merop enem/ Compo undA(8 ) | Merop enem/ Compo undA(1 6) | Merop enem/ Vaborb actam( 8) | Ceftazi dime/ Avibac tam(4) | Imipen em/ Releba ctam(4 ) |
| *r cloacae group* | | | -A | | | | | | | | |
| *Morganella morganii* | 5630 | 133 | KPC-2 | - | 1 | ≤0.06 | ≤0.06 | ≤0.06 | ≤0.06 | 0.5 | 1 |
| *Raoultella ornithinolyti ca* | 5631 | 134 | KPC-3 | - | 0.25 | ≤0.06 | ≤0.06 | ≤0.06 | ≤0.06 | 0.12 | 0.25 |
| *Klebsiella pneumoniae* | 5632 | 135 | - | VIM-1 | 0.5 | ≤0.06 | ≤0.06 | ≤0.06 | 2 | >32 | 4 |
| *Enterobacte r cloacae* | 5633 | 136 | KPC-3 | - | 8 | ≤0.06 | <0.06 | ≤0.06 | ≤0.06 | 1 | 0.12 |
| *Escherichia coli* | 5634 | 137 | - | NDM-6 | 32 | 0.12 | 0.12 | 0.12 | 32 | >32 | 16 |
| *Klebsiella pneumoniae* | 5635 | 138 | - | NDM-7 | >32 | >32 | 32 | 1 | >32 | >32 | >32 |
| *Klebsiella pneumoniae* | 5636 | 139 | - | NDM-1 | 0.12 | ≤0.06 | ≤0.06 | ≤0.06 | ≤0.06 | 0.5 | 0.12 |
| *Klebsiella pneumoniae* | 5637 | 140 | OXA-181 | - | 1 | ≤0.06 | ≤0.06 | ≤0.06 | 1 | 1 | 1 |
| *Klebsiella pneumonine* | 5638 | 141 | OXA-181 | - | 2 | ≤0.06 | ≤0.06 | ≤0.06 | 1 | 1 | 1 |
| *Klebsiella pneumoniae* | 5639 | 142 | OXA-181 | - | 1 | ≤0.06 | ≤0.06 | 0.12 | 1 | 1 | 2 |
| *Klebsiella pneumoniae* | 5640 | 143 | - | NDM-L | >32 | 32 | 8 | 1 | >32 | >32 | >32 |
| *Kluyvera ascorbata* | 5641 | 144 | KPC-3 | - | 2 | ≤0.06 | ≤0.06 | ≤0.06 | ≤0.06 | 0.5 | 0.12 |
| *Klebsiella pneumoniae* | 5642 | 145 | - | NDM-1 | 8 | ≤0.06 | ≤0.06 | ≤0.06 | 8 | >32 | 8 |
| *Klebsiella pneumoniae* | 5643 | 146 | - | NDM-1 | 8 | ≤0.06 | ≤0.06 | ≤0.06 | 8 | >32 | 8 |
| *Klebsiella oxytoca* | 5644 | 147 | KPC-3 | - | 0.25 | ≤0.06 | ≤0.06 | ≤0.06 | ≤0.06 | ≤0.06 | 0.12 |
| *Klebsiella pneumoniae* | 5645 | 148 | - | NDM-1 | 8 | ≤0.06 | ≤0.06 | ≤0.06 | 8 | >32 | 4 |
| *Escherichia coli* | 5646 | 149 | - | NDM-7 | 8 | 0.5 | 0.5 | 0.5 | 8 | >32 | 16 |
| *Escherichia coli* | 5647 | 150 | - | NDM-5 | >32 | ≤0.06 | ≤0.06 | ≤0.06 | 32 | >32 | 16 |
| *Escherichia coli* | 5648 | 151 | - | NDM-5 | 8 | ≤0.06 | ≤0.06 | ≤0.06 | 8 | >32 | 16 |
| *Klebsiella pneumonice* | 5649 | 152 | - | NDM-1 | 8 | ≤0.06 | ≤0.06 | ≤0.06 | 4 | >32 | 4 |
| *Klebsiella pneumoniae* | 5650 | 153 | OXA-232 | NDM-1 | >32 | 16 | 1 | 0.5 | >32 | >32 | 32 |

(continued)

| Orgamism | KUB No. | AR Ban k# | Harbored carbapenemase genes | | MIC (μg/mL) | | | | | | |
|---|---|---|---|---|---|---|---|---|---|---|---|
| | | | Serine -type | Metallo -type | Merop enem | Merop enem/ Compo undA(4 ) | Merop enem/ Compo undA(8 ) | Merop enem/ Compo undA(1 6) | Merop enem/ Vaborb actam( 8) | Ceftazi dime/ Avibac tam(4) | Imipen em/ Releba ctam(4 ) |
| *Enterobacte r cloacae* | 5651 | 154 | - | VIM-1 | 4 | ≤0.06 | ≤0.06 | ≤0.06 | 4 | >32 | 8 |
| *Proteus mirabilis* | 5652 | 155 | KPC-6 | - | 1 | ≤0.06 | ≤0.06 | ≤0.06 | ≤0.06 | 0.25 | 0.5 |
| *Proteus mirabilis* | 5653 | 156 | KPC-2 | - | 0.12 | ≤0.06 | ≤0.06 | ≤0.06 | ≤0.06 | ≤0.06 | 1 |
| *Citrobacter freundii* | 5654 | 157 | - | NDM-1 | 32 | 0.12 | 0.12 | 0.12 | 32 | >32 | 16 |

[Table 8-5]

| Orgamism | KUB No. | AR Bank# | Harbored carbapenemase genes | | MIC (μg/mL) | | | | | | |
|---|---|---|---|---|---|---|---|---|---|---|---|
| | | | Serine -type | Metallo -type | Meropenem | Meropenem/ CompoundA(4) | Meropenem/ CompoundA(8) | Meropenem/ CompoundA(16) | Meropenem/ Vaborbactam(8) | Ceftazidime/ Avibactam(4) | Imipenem/ Relebactam(4) |
| *Klebsiella pneumoniae* | 5655 | 158 | - | NDM-1 | 8 | ≤0.06 | ≤0.06 | ≤0.06 | 8 | >32 | 8 |
| *Proteus mirabilis* | 5656 | 159 | - | NDM-1 | 2 | ≤0.06 | ≤0.06 | ≤0.06 | 2 | >32 | 8 |
| *Klebsiella pneumoniae* | 5657 | 160 | OXA-48 | - | 8 | ≤0.06 | ≤0.06 | ≤0.06 | 8 | 0.25 | 4 |
| *Enterobacte r aerogenes* | 5658 | 161 | - | IMP-4 | 1 | ≤0.06 | ≤0.06 | ≤0.06 | 1 | >32 | 2 |
| *Escherichia coli* | 5659 | 162 | - | NDM-7 | 32 | ≤0.06 | ≤0.06 | ≤0.06 | 16 | >32 | 16 |
| *Enterobacte r cloacae* | 5660 | 163 | KPC-2 | - | 4 | ≤0.06 | ≤0.06 | ≤0.06 | ≤0.06 | 0.25 | ≤0.06 |
| *Enterobacte r cloacae complex* | 5661 | 164 | NMC -A | - | 32 | ≤0.06 | ≤0.06 | ≤0.06 | ≤0.06 | 0.5 | 2 |
| *Klebsiella pneumoniae* | 5757 | 361 | KPC-2 | - | 8 | ≤0.06 | ≤0.06 | ≤0.06 | ≤0.06 | 0.5 | 0.12 |
| *Klebsiella pneumoniae* | 5758 | 362 | KPC-2 | - | 8 | ≤0.06 | ≤0.06 | ≤0.06 | ≤0.06 | 0.5 | 0.12 |
| *Klebsiella pneumoniae* | 5759 | 363 | KPC-2 | - | 4 | ≤0.06 | ≤0.06 | ≤0.06 | ≤0.06 | 0.5 | ≤0.06 |
| *Klebsiella pneumoniae* | 5760 | 364 | - | - | 1 | ≤0.06 | ≤0.06 | ≤0.06 | ≤0.06 | 0.12 | 0.5 |
| *Enterobacte r cloacae* | 5761 | 365 | KPC-2 | - | 2 | <0.06 | ≤0.06 | ≤0.06 | ≤0.06 | 1 | 0.12 |
| *Enterobacte r cloacae* | 5762 | 366 | KPC-2 | - | 1 | ≤0.06 | ≤0.06 | ≤0.06 | ≤0.06 | 0.5 | 0.12 |
| *Enterobacte r cloacae* | 5763 | 367 | - | - | 0.5 | 0.12 | ≤0.06 | ≤0.06 | 0.12 | 1 | 0.12 |
| *Escherichia coli* | 5764 | 368 | - | - | ≤0.06 | ≤0.06 | ≤0.06 | ≤0.06 | ≤0.06 | ≤0.06 | 0.12 |
| *Escherichia coli* | 5765 | 369 | - | - | ≤0.06 | ≤0.06 | ≤0.06 | ≤0.06 | ≤0.06 | ≤0.06 | 0.12 |
| *Escherichia coli* | 5766 | 370 | - | - | ≤0.06 | ≤0.06 | ≤0.06 | ≤0.06 | ≤0.06 | 0.25 | 0.12 |
| *Escherichia coli* | 5767 | 371 | - | - | ≤0.06 | ≤0.06 | ≤0.06 | ≤0.06 | ≤0.06 | 1 | 0.12 |
| *Escherichia coli* | 5768 | 372 | - | - | ≤0.06 | ≤0.06 | ≤0.06 | ≤0.06 | ≤0.06 | 1 | ≤0.06 |
| *Escherichia coli* | 5769 | 373 | - | - | ≤0.06 | ≤0.06 | ≤0.06 | ≤0.06 | ≤0.06 | 0.25 | 0.12 |
| *Escherichia coli* | 5770 | 374 | - | - | ≤0.06 | ≤0.06 | ≤0.06 | ≤0.06 | ≤0.06 | 0.25 | 0.12 |
| *Klebsiella oxytoca* | 5771 | 375 | - | - | ≤0.06 | ≤0.06 | ≤0.06 | ≤0.06 | ≤0.06 | 0.12 | 0.12 |

(continued)

| Orgamism | KUB No. | AR Bank# | Harbored carbapenemase genes | | MIC (μg/mL) | | | | | | |
|---|---|---|---|---|---|---|---|---|---|---|---|
| | | | Serine -type | Metallo -type | Meropenem | Meropenem/ CompoundA(4 ) | Meropenem/ CompoundA(8 ) | Meropenem/ CompoundA(1 6) | Meropenem/ Vaborbactam( 8) | Ceftazidime/ Avibactam(4) | Imipenem/ Relebactam(4 ) |
| *Klebsiella pneumoniae* | 5772 | 376 | - | - | 0.12 | ≤0.06 | ≤0.06 | ≤0.06 | ≤0.06 | 1 | 0.12 |
| *Proteus mirabilis* | 5773 | 377 | KPC-3 | - | 1 | ≤0.06 | ≤0.06 | ≤0.06 | ≤0.06 | ≤0.06 | 0.12 |
| *Escherichia coli* | 5774 | 378 | - | - | ≤0.06 | ≤0.06 | ≤0.06 | ≤0.06 | ≤0.06 | ≤0.06 | ≤0.06 |
| *Proteus mirabilis* | 5775 | 379 | - | - | ≤0.06 | ≤0.06 | ≤0.06 | ≤0.06 | ≤0.06 | ≤0.06 | 0.25 |

[Table 8-6]

| Orgamism | KUB No. | AR Ban k# | Harbored carbapenemase genes | | MIC (µg/mL) | | | | | | |
|---|---|---|---|---|---|---|---|---|---|---|---|
| | | | Serine -type | Metallo -type | Merop enem | Merop enem/ Compo undA(4 ) | Merop enem/ Compo undA(8 ) | Merop enem/ Compo undA(1 6) | Merop enem/ Vaborb actam( 8) | Ceftazi dime/ Avibac tam(4) | Imipen em/ Releba ctam(4 ) |
| *Klebsiella oxytoca* | 5776 | 380 | - | - | 2 | 0.5 | 0.25 | 0.25 | 1 | 1 | 0.12 |
| *Enterobacte r aerogenes* | 5777 | 431 | - | - | ≤0.06 | ≤0.06 | ≤0.06 | ≤0.06 | ≤0.06 | 8 | 0.12 |
| *Enterobacte r cloacae* | 5778 | 432 | - | - | 0.12 | ≤0.06 | ≤0.06 | ≤0.06 | 0.25 | 32 | 0.25 |
| *Escherichia coli* | 5779 | 433 | - | - | ≤0.06 | ≤0.06 | ≤0.06 | ≤0.06 | ≤0.06 | 1 | 0.25 |
| *Escherichia coli* | 5780 | 434 | - | - | ≤0.06 | ≤0.06 | ≤0.06 | ≤0.06 | ≤0.06 | 16 | 0.12 |
| *Escherichia coli* | 5781 | 435 | - | NDM-1 | 32 | ≤0.06 | ≤0.06 | ≤0.06 | 32 | >32 | 32 |
| *Escherichia coli* | 5782 | 436 | - | - | ≤0.06 | ≤0.06 | ≤0.06 | ≤0.06 | ≤0.06 | 0.25 | 0.12 |
| *Escherichia coli* | 5783 | 437 | - | - | ≤0.06 | ≤0.06 | ≤0.06 | ≤0.06 | ≤0.06 | 8 | 0.25 |
| *Klebsiella pnemnonice* | 5784 | 438 | KPC-3 | - | >32 | 32 | 1 | 0.12 | 1 | 4 | 0.12 |
| *Enterobacte r cloacae* | 5792 | 448 | - | NDM-1 | 16 | 0.12 | ≤0.06 | ≤0.06 16 | | >32 | 8 |
| *Escherichia coli* | 5794 | 450 | - | - | 16 | 16 | 8 | 1 | 8 | 4 | 2 |
| *Escherichia coli* | 5795 | 451 | KPC-3 | - | 0.25 | ≤0.06 | ≤0.06 | ≤0.06 | ≤0.06 | 0.25 | 0.12 |
| *Escherichia coli* | 5796 | 452 | - | NDM-5 | 2 | ≤0.06 | ≤0.06 | ≤0.06 | 2 | >32 | 4 |
| *Klebsiella pneumoniae* | 5797 | 453 | KPC-3 | - | >32 | >32 | 16 | 0.5 | 32 | 16 | 1 |
| *Escherichia coli* | 5803 | 861 | - | NDM | 32 | 0.12 | 0.12 | ≤0.06 | 32 | >32 | 16 |
| *Enterobacte r cloacae* | 5807 | 501 | - | VIM-1 | 0.5 | ≤0.06 | ≤0.06 | ≤0.06 | 4 | >32 | 4 |
| *Enterobacte r cloacae* | 5808 | 502 | - | IMP-8 | 4 | 2 | 1 | 0.25 | 2 | >32 | 4 |
| *Klebsiella pneumoniae* | 5809 | 504 | OXA-48 | - | 4 | ≤0.06 | ≤0.06 | ≤0.06 | 1 | 0.25 | 1 |
| *Klebsiella pneumoniae* | 5810 | 505 | | NDM-1 | 16 | ≤0.06 | ≤0.06 | ≤0.06 | 16 | >32 | 8 |
| *Klebsiella pneumoniae* | 5811 | 506 | - | NDM-1 | 16 | ≤0.06 | ≤0.06 | ≤0.06 | 32 | >32 | 8 |
| *Klebsiella pneumoniae* | 5812 | 507 | OXA-232 | NDM-1 | >32 | >32 | >32 | 2 | >32 | >32 | >32 |
| *Serratia marcescens* | 5822 | 517 | KPC-3 | - | 8 | ≤0.06 | ≤0.06 | ≤0.06 | 0.12 | 1 | 1 |

(continued)

| Orgamism | KUB No. | AR Ban k# | Harbored carbapenemase genes | | MIC (μg/mL) | | | | | | |
|---|---|---|---|---|---|---|---|---|---|---|---|
| | | | Serine -type | Metallo -type | Merop enem | Merop enem/ Compo undA(4 ) | Merop enem/ Compo undA(8 ) | Merop enem/ Compo undA(1 6) | Merop enem/ Vaborb actam( 8) | Ceftazi dime/ Avibac tam(4) | Imipen em/ Releba ctam(4 ) |
| *Morganella morganii* | 5824 | 519 | - | - | 2 | 2 | 1 | 1 | 1 | 0.5 | 2 |
| *Serratia marcescens* | 5825 | 520 | - | - | ≤0.06 | ≤0.06 | ≤0.06 | ≤0.06 | ≤0.06 | 1 | 0.5 |
| *Serratia marcescens* | 5826 | 521 | - | - | ≤0.06 | ≤0.06 | ≤0.06 | ≤0.06 | ≤0.06 | 1 | 0.12 |
| *Klebsiella pneumoniae* | 5827 | 522 | KPC-2 | - | 8 | ≤0.06 | ≤0.06 | ≤0.06 | ≤0.06 | 0 | ≤0.06 |
| *Klebsiella* | 1 5828 | 523 | KPC- | - | 16 | 0.12 | ≤0.06 | ≤0.06 | 0.12 | 0.5 | 0.5 |

[Table 8-7]

| Orgamism | KUB No. | AR Ban k# | Harbored carbapenemase genes | | MIC (μg/mL) | | | | | | |
|---|---|---|---|---|---|---|---|---|---|---|---|
| | | | Serine -type | Metallo -type | Merop enem | Merop enem/ Compo undA(4 ) | Merop enem/ Compo undA(8 ) | Merop enem/ Compo undA(1 6) | Merop enem/ Vaborb actam( 8) | Ceftazi dime/ Avibac tam(4) | Imipen em/ Releba ctam(4 ) |
| *pneumoniae* | | | 2 | | | | | | | | |
| *Klebsiella pneumoniae* | 5829 | 524 | KPC-3 | - | 16 | ≤0.06 | ≤0.06 | ≤0.06 | ≤0.06 | 2 | 0.25 |
| *Klebsiella pneumoniae* | 5830 | 525 | KPC-11 | - | 8 | ≤0.06 | ≤0.06 | ≤0.06 | 0.12 | 1 | 0.12 |

[Table 8-8]

**Table 8 continued**

| Orgamism | K U B N o. | AR Bank # | MIC (μg/mL) | | | | | | | | | |
|---|---|---|---|---|---|---|---|---|---|---|---|---|
| | | | Ceftolo zane/ Tazoba ctam(4) | Cefid erocol | Piperac illin/ Tazoba ctam(4) | Aztre onam | Tigec yeline | Col isti n | Ami kaci n | Ciprofl oxacin | Levofl oxacin | Cefoz opran |
| *Enterobacter cloacae Enterobacter* | 55 29 | 32 | 8 | ≤0.0 6 | 32 | 32 | 1 | 0.5 | 1 | 0.25 | 1 | 4 |
| *Klebsiella pneumoniae* | 55 31 | 34 | >64 | 0.25 | 8 | 0.06 | 0.5 | 0.5 | 1 | 0.5 | 0.5 | 4 |
| *Enterobacter cloacae* | 55 35 | 38 | >64 | 0.5 | >64 | >64 | 4 | 0.5 | >64 | >64 | 64 | >64 |
| *Klebsiella pneumoniae* | 55 36 | 39 | >64 | 0.12 | >64 | >64 | 1 | 0.5 | 4 | 64 | 8 | >64 |
| *Klebsiella pneumoniae* | 55 37 | 40 | >64 | 0.5 | >64 | >64 | 2 | 4 | 32 | >64 | 32 | >64 |
| *Klebsiella pneumoniae* | 55 38 | 41 | >64 | 2 | >64 | >64 | 1 | 0.2 5 | >64 | 16 | 8 | >64 . |
| *Klebsiella pneumoniae* | 55 40 | 43 | 16 | 0.25 | >64 | >64 | 0.5 | 0.2 5 | 1 | 4 | 2 | >64 |
| *Klebsiella pneumoniae* | 55 41 | 44 | >64 | 0.5 | >64 | >64 | 0.5 | 0.2 5 | 16 | >64 | 8 | >64 |
| *Klebsiella pneumoniae* | 55 43 | 46 | >64 | 0.25 | >64 | >64 | 2 | 4 | 16 | >64 | 32 | >64 |
| *Klebsiella pneumoniae* | 55 | 47 | >64 | 1 2 | >64 | >64 | 0.5 | 64 | 32 | >64 | 64 | >64 |
| *Escherichia coli* | 55 45 | 48 | >64 | 2 | >64 | >64 | 0.25 | 0.5 | >64 | 64 | 32 | >64 |
| *Klebsiella pneumoniae* | 55 46 | 49 | >64 | 1 | >64 | >64 | 4 | 0.5 | >64 | >64 | >64 | >64 |
| *Enterobacter cloacae* | 55 47 | 50 | >64 | 2 | >64 | >64 | 0.25 | 0.2 5 | 1 | 8 | 8 | 8 |
| *Klebsiella ozaenae* | 55 48 | 51 | >64 | ≤0.0 6 | >64 | >64 | 1 | 0.5 | >64 | >64 | 16 | >64 |
| *Enterobacter cloacae* | 55 50 | 53 | >64 | 0.12 | >64 | >64 | 1 | 0.2 5 | 64 | 64 | 32 | >64 |
| *Escherichia coli* | 55 52 | 55 | >64 | 2 | >64 | 32 | 0.25 | 0.5 | >64 | >64 | 16 | >64 |
| *Morganella morganii* | 55 54 | 57 | >64 | ≤0.0 6 | 32 | 64 | 0.5 | >6 4 | 4 | >64 | 8 | >64 |
| *Escherichia coli* | 5555 | 58 | 4 | 0.5 | >64 | 8 | 0.25 | 0.5 | 0.5 | 64 | 16 | >64 |
| *Proteus mirabilis* | 55 56 | 59 | 16 | 0.25 | 2 | 0.25 | 1 | >6 4 | 4 | 16 | 8 | 2 |
| *Enterobacter cloacae* | 55 57 | 60 | 8 | 4 | >64 | 32 | 0.25 | 0.5 | 1 | ≤0.03 | 0.06 | 2 |

[Table 8-9]

| Orgamism | K U B N o. | AR Bank # | MIC (µg/mL) | | | | | | | | | |
|---|---|---|---|---|---|---|---|---|---|---|---|---|
| | | | Ceflolo zane/ Tazoba ctam(4) | Cefid erocol | Piperac illin/ Tazoba ctam(4) | Aztre onam | Tigec yeline | Col isti n | Ami kaci n | Ciprofl oxacin | Levofl exacin | Cefoz opran |
| *Escherichia coli* | 55 58 | 61 | 16 | ≤0.0 6 | >64 | >64 | 0.25 | 0.2 5 | 16 | ≤0.03 | 0.12 | 16 |
| *Enterobacter aerogenes* | 55 59 | 62 | 4 | ≤0.0 6 | 32 | 16 | 2 | 8 | 2 | 2 | 2 | 0.5 |
| *Enterobacter cloacae* | 55 62 | 65 | 16 | 0.25 | >64 | 64 | 0.5 | 0.5 | 1 | 0.06 | 0.12 | 16 |
| *Klebsiella pneumoniae* | 55 63 | 66 | >64 | 0.5 | >64 | >64 | 2 | 0.5 | 8 | >64 | 64 | >64 |
| *Escherichia coli* | 55 64 | 67 | 0.25 | 0.12 | 4 | 4 | 0.25 | 1 | 2 | >64 | 32 | 0.25 |
| *Klebsiella pneumoniae* | 55 65 | 68 | >64 | 2 | >64 | >64 | 2 | 1 | 64 | >64 | 64 | >64 |
| *Escherichia coli* | 55 66 | 69 | >64 | 1 | >64 | 4 | 0.25 | 0.2 5 | 16 | 0.06 | 0.12 | >64 |
| *Klebsiella oxytoca* | 55 68 | 71 | 16 | ≤0.0 6 | >64 | >64 | 1 | 0.5 | 0.5 | 0.12 | 0.5 | >64 |
| *Enterobacter cloacae* | 55 69 | 72 | 16 | 0.25 | >64 | >64 | 2 | 0.2 5 | 1 | 0.25 | 0.5 | 16 |
| *Enterobacter cloacae* | 55 70 | 73 | 1 | 0.25 | 8 | 4 | 1 | 8 | 0.25 | 0.06 | 0.25 | 0.25 |
| *Enterobacter aerogenes* | 55 71 | 74 | 1 | ≤0.0 6 | >64 | 0.12 | 0.25 | 0.5 | 2 | 0.06 | 0.12 | 0.5 |
| *Klebsiella pneumoniae* | 55 72 | 75 | 64 | 0.25 | >64 | >64 | 2 | 0.5 | >64 | >64 | >64 | >64 |
| *Klebsiella pneumoniae* | 55 73 | 76 | >64 | ≤0.0 6 | >64 | 0.25 | 0.25 | 0.2 5 | 8 | 0.5 | 1 | 64 |
| *Escherichia coli* | 55 74 | 77 | 0.25 | ≤0.0 6 | 1 | 0.06 | 0.12 | 0.2 5 | 1 | ≤0.03 | 0.06 | 0.06 |
| *Klebsiella pneumoniae* | 55 76 | 79 | >64 | 0.12 | >64 | >64 | 0.5 | 0.2 5 | >64 | >64 | 64 | >64 |
| *Klebsiella pneumoniae* | 55 77 | 80 | >64 | 2 | >64 | >64 | 0.25 | 0.5 | 4 | 0.12 | 0.12 | >64 |
| *Escherichia coli* | 55 78 | 81 | 1 | 0.25 | 4 | 8 | 0.12 | 0.5 | 2 | 64 | 32 | 0.5 |
| *Providencia rettgeri* | 55 79 | 82 | >64 | ≤0.0 6 | 16 | ≤0.0 3 | 2 | >6 4 | 1 | 0.06 | 0.25 | 16 |
| *Escherichia coli* | 55 81 | 84 | 1 | 0.5 | 4 | 0.5 | 0.12 | 0.5 | 0.5 | 64 | 64 | 0.25 |
| *Escherichia coli* | 55 82 | 85 | 4 | 0.12 | 16 | 16 | 0.25 | 0.2 5 | 1 | >64 | 32 | 8 |
| *Escherichia coli* | 55 83 | 86 | 0.5 | 0.12 | 1 | 4 | 0.5 | 0.2 5 | 1 | 32 | 8 | >64 |
| *Klebsiella pneumoniae* | 55 84 | 87 | 64 | 1 | >64 | >64 | 2 | 64 | 1 | >64 | >64 | 32 |
| *Escherichia coli* | 55 86 | 89 | 1 | ≤0.0 6 | 8 | 16 | 0.25 | 0.5 | 1 | ≤0.03 | 0.12 | 0.5 |
| *Serratia marcescens* | 55 88 | 91 | 1 | ≤0.0 6 | 2 | 32 | 0.5 | >6 4 | 1 | 0.06 | 0.25 | 0.12 |

(continued)

| Orgamism | K U B N o. | AR Bank # | MIC (µg/mL) | | | | | | | | | |
|---|---|---|---|---|---|---|---|---|---|---|---|---|
| | | | Ceflolo zane/ Tazoba ctam(4) | Cefid erocol | Piperac illin/ Tazoba ctam(4) | Aztre onam | Tigec yeline | Col isti n | Ami kaci n | Ciprofl oxacin | Levofl exacin | Cefoz opran |
| *Enterobacter cloacae* | 55 90 | 93 | 32 | 0.25 | >64 | >64 | 0.5 | 1 | 2 | 4 | 4 | 32 |
| *Klebsiella ozaenae* | 55 93 | 96 | 64 | ≤0.0 6 | >64 | >64 | 0.5 | 1 | 8 | 64 | 32 | >64 |
| *Klebsiella pneumoniae* | 55 94 | 97 | >64 | 1 | >64 | >64 | 1 | 8 | 64 | >64 | >64 | >64 |
| *Klebsiella pneumoniae* | 55 95 | 98 | 32 | 0.12 | >64 | >64 | 0.5 | 0.2 5 | 32 | >64 | 64 | 16 |

[Table 8-10]

| Orgamism | K U B N o. | AR Bank # | MIC (μg/mL) | | | | | | | | | |
|---|---|---|---|---|---|---|---|---|---|---|---|---|
| | | | Ceftolo zane/ Tazoba ctam(4) | Cefid erocol | Piperac illin/ Tazoba ctam(4) | Aztre onam | Tigec yeline | Col isti n | Ami kaci n | Ciprofl oxacin | Levofl oxacin | Cefoz opran |
| *Serratia marcescens* | 55 96 | 99 | 1 | 0.25 | 2 | 64 | 1 | >6 4 | 1 | 0.06 | 0.25 | 0.25 |
| *Escherichia coli* | 56 01 | 104 | >64 | 1 | >64 | >64 | 0.12 | 0.5 | 1 | 32 | 16 | 32 |
| *Klebsiella pneumoniae* | 56 03 | 106 | >64 | 1 | >64 | >64 | 2 | 32 | >64 | >64 | >64 | >64 |
| *Klebsiella pneumoniae* | 56 04 | 107 | 16 | ≤0.0 6 | >64 | >64 | 0.25 | 0.5 | >64 | >64 | 64 | >64 |
| *Klebsiella pneumoniae* | 56 06 | 109 | >64 | 0.5 | >64 | 64 | 1 | 32 | 8 | >64 | >64 | >64 |
| *Klebsiella pneumoniae* | 56 09 | 112 | >64 | 2 | >64 | >64 | 0.5 | *0.5* | 32 | 16 | 16 | 32 |
| *Klebsiella pneumoniae* | 56 10 | 113 | >64 | 0.5 | >64 | >64 | 0.5 | 0.5 | 32 | 32 | 16 | >64 |
| *Escherichia coli* | 56 11 | 114 | 64 | 0.25 | >64 | >64 | 0.12 | 0.5 | 1 | 64 | 32 | 16 |
| *Klebsiella pneumoniae* | 56 12 | 115 | 64 | 0.5 | >64 | >64 | 2 | 1 | 0.5 | 64 | 32 | 32 |
| *Citrobacter fremidii* | 56 13 | 116 | >64 | 0.5 | >64 | >64 | 0.5 | 1 | 1 | 32 | 16 | >64 |
| *Klebsiella pneumoniae* | 56 14 | 117 | >64 | 2 | >64 | >64 | 0.5 | 1 | 32 | 8 | 4 | >64 |
| *Escherichia coli* | 56 15 | 118 | >64 | 2 | >64 | 32 | 0.12 | 1 | >64 | 64 | 32 | >64 |
| *Escherichia coli* | 56 16 | 119 | >64 | 0.5 | >64 | >64 | 0.5 | 0.5 | >64 | >64 | 32 | >64 |
| *Klebsiella pneumoniae* | 56 17 | 120 | >64 | 0.25 | >64 | >64 | 0.5 | 1 | 32 | >64 | 64 | 32 |
| *Serratia marcescens* | 56 18 | 121 | 2 | 0.12 | 2 | 16 | 1 | >6 4 | 2 | 0.06 | 0.12 | 0.12 |
| *Serratia marcescens* | 56 19 | 122 | 1 | 0.12 | 2 | 16 | 1 | >6 4 | 1 | 0.06 | 0.5 | 0.12 |
| *Serratia marcescens* | 56 20 | 123 | 0.5 | <0.0 6 | 2 | 16 | 1 | >6 4 | 1 | 0.06 | 0.25 | 0.12 |
| *Serratia marcescens* | 56 21 | 124 | 0.5 | ≤0.0 | 1 | 8 | 1 | >6 4 | 2 | 0.06 | 0.5 | 0.12 |
| *Klebsiella pneumoniae* | 56 22 | 125 | >64 | 1 | >64 | >64 | 1 | 16 | 32 | >64 | 64 | 32 |
| *Klebsiella pneumoniae* | 56 23 | 126 | 4 | ≤0.0 6 | >64 | 32 | 0.5 | 0.2 5 | 4 | 0.12 | 0.12 | 64 |
| *Salmonella* Senftenberg | 56 24 | 127 | >64 | 1 | >64 | 64 | 0.5 | 0.5 | >64 | 32 | 32 | >64 |
| *Escherichia coli* | 56 25 | 128 | >64 | 2 | >64 | >64 | 0.12 | 0.2 5 | >64 | 64 | 32 | >64 |
| *Klebsiella pneumoniae* | 56 26 | 129 | >64 | 1 | >64 | >64 | 1 | 0.5 | 32 | >64 | 64 | 32 |

(continued)

| Orgamism | K U B N o. | AR Bank # | MIC (μg/mL) | | | | | | | | | |
|---|---|---|---|---|---|---|---|---|---|---|---|---|
| | | | Ceftolo zane/ Tazoba ctam(4) | Cefid erocol | Piperac illin/ Tazoba ctam(4) | Aztre onam | Tigec yeline | Col isti n | Ami kaci n | Ciprofl oxacin | Levofl oxacin | Cefoz opran |
| *Serratia marcescens* | 56 27 | 130 | 1 | 0.12 | 1 | 16 | 1 | >6 4 | 2 | 0.06 | 0.12 | 0.12 |
| *Serratia marcescens* | 56 28 | 131 | 0.5 | 50.0 6 | 2 | 16 | 1 | >6 4 | 1 | 0.06 | *0.5* | *0.12* |
| *Enterobacter cloacae group* | 56 29 | 132 | 0.5 | 0.5 | 2 | 2 | 0.5 | 1 | 1 | ≤0.03 | 0.06 | 0.25 |
| *Morganella morganii* | 56 30 | 133 | 16 | ≤0.0 6 | 64 | 64 | 4 | >6 4 | 1 | 64 | 64 | 64 |
| *Raoultella ornithinolvtica* | 56 31 | 134 | 8 | ≤0.0 6 | 32 | 8 | 0.25 | 8 | 1 | 0.12 | 0.5 | 8 |

[Table 8-11]

| Orgamism | K U B N o. | AR Bank # | MIC (μg/mL) | | | | | | | | | |
|---|---|---|---|---|---|---|---|---|---|---|---|---|
| | | | Ceftolo zane/ Tazoba ctam(4) | Cefid erocol | Piperac illin/ Tazoba ctam(4) | Aztre onam | Tigec yeline | Col isti 11 | Ami kaci n | Ciprofl oxacin | Levofl oxacin | Cefoz opran |
| *Klebsiella pneumoniae* | 56 32 | 135 | >64 | 1 | >64 | >64 | 1 | 1 | 8 | 64 | 64 | >64 |
| *Enterobacter clooreae* | 56 33 | 136 | >64 | 0.25 | >64 | >64 | 2 | 0.5 | 2 | 32 | 16 | >64 |
| *Escherichia coli* | 56 34 | 137 | >64 | 2 | >64 | >64 | 0.5 | 1 | 16 | >64 | 64 | >64 |
| *Klebsiella pneumoniae* | 56 35 | 138 | >64 | 1 | >64 | >64 | 2 | 0.5 | >64 | >64 | >64 | >64 |
| *Klebsiella pneumoniae* | 56 36 | 139 | 2 | 0.12 | 4 | >64 | 0.5 | 0.2 | >64 | 16 | 8 | 64 |
| *Klebsiella pneumoniae* | 56 37 | 140 | >64 | 0.12 | >64 | >64 | 1 | 0.5 | >64 | >64 | 16 | >64 |
| *Klebsiella pneumoniae* | 56 38 | 141 | >64 | ≤0.0 6 | >64 | >64 | 1 | 0.2 5 | >64 | >64 | 8 | >64 |
| *Klebsiella pneumoniae* | 56 39 | 142 | >64 | <0.0 6 | >64 | >64 | 1 | 0.5 | >64 | 64 | 8 | >64 |
| *Klebsiella pneumoniae* | 56 40 | 143 | >64 | 1 | >64 | >64 | 2 | 0.2 5 | >64 | 64 | 64 | >64 |
| *Kluyvera ascorbata* | 56 41 | 144 | 32 | ≤0.0 6 | >64 | >64 | 2 | 0.5 | 4 | 1 | 2 | 16 |
| *Klebsiella pneumoniae* | 56 42 | 145 | >64 | 0.25 | >64 | >64 | 0.5 | 0.2 5 | 16 | 64 | 32 | 64 |
| *Klebsiella pneumoniae* | 56 43 | 146 | >64 | 0.25 | >64 | >64 | 1 | 0.5 | 16 | >64 | 64 | >64 |
| *Klebsiella oxytoca* | 56 44 | 147 | 0.5 | 0.12 | 4 | 4 | 0.5 | 0.2 5 | 0.5 | ≤0.03 | 0.06 | 1 |
| *Klebsiella pneumoniae* | 56 45 | 148 | >64 | 0.5 | >64 | >64 | 1 | 0.2 5 | >64 | >64 | 64 | 64 |
| *Escherichia coli* | 56 46 | 149 | >64 | 16 | >64 | 0.5 | 0.25 | 0.5 | 2 | 16 | 8 | >64 |
| *Escherichia coli* | 56 47 | 150 | >64 | 4 | >64 | 32 | 0.25 | 0.2 5 | 0.5 | 64 | 32 | >64 |
| *Escherichia coli* | 56 48 | 151 | >64 | 4 | >64 | 32 | 0.25 | 0.2 5 | 1 | 64 | 32 | >64 |
| *Klebsiella pneumoniae* | 56 49 | 152 | >64 | 0.25 | >64 | >64 | 2 | 0.2 5 | 32 | 64 | 32 | >64 |
| *Klebsiella pneumoniae* | 56 50 | 153 | >64 | 1 | >64 | >64 | 1 | 0.5 | >64 | >64 | >64 | >64 |
| *Enterobacter cloaccae* | 56 51 | 154 | >64 | 4 | >64 | 64 | 0.5 | 0.2 5 | 8 | 0.5 | 1 | >64 |
| *Proteus mirabilis* | 56 52 | 155 | 16 | ≤0.0 6 | 16 | 4 | 2 | >6 4 | 1 | >64 | >64 | 64 |
| *Proteus mirabilis* | 56 53 | 156 | 0.25 | <0.0 6 | 0.25 | ≤0.0 3 | 1 | >6 4 | 4 | 4 | 2 | 0.5 |
| *Citrobacter freundii* | 56 54 | 157 | >64 | 1 | >64 | >64 | 0.5 | 0.5 | >64 | >64 | >64 | >64 |
| *Klebsiella pneumoniae* | 56 55 | 158 | >64 | 0.5 | >64 | >64 | 1 | 1 | 2 | 64 | 16 | >64 |

(continued)

| Orgamism | K U B N o. | AR Bank # | MIC (μg/mL) | | | | | | | | | |
|---|---|---|---|---|---|---|---|---|---|---|---|---|
| | | | Ceftolo zane/ Tazoba ctam(4) | Cefid erocol | Piperac illin/ Tazoba ctam(4) | Aztre onam | Tigec yeline | Col isti 11 | Ami kaci n | Ciprofl oxacin | Levofl oxacin | Cefoz opran |
| *Proteus mirabilis* | 56 56 | 159 | >64 | 0.5 | >64 | ≤0.0 3 | 2 | >6 4 | >64 | 1 | 2 | >64 |
| *Klebsiella pneumoniae* | 56 57 | 160 | 1 | 0.25 | >64 | 0.12 | 0.5 | 0.5 | 0.5 | 0.06 | 0.12 | 2 |
| *Enterobacter aerogenes* | 56 58 | 161 | >64 | 1 | 16 | 32 | 1 | 0.5 | 2 | 0.06 | 0.12 | 32 |
| *Escherichia coli* | 56 59 | 162 | >64 | 8 | >64 | >64 | 0.5 | 0.2 5 | 1 | 64 | 32 | >64 |

[Table 8-12]

| Orgamism | K U B N o. | AR Bank # | MIC (μg/mL) | | | | | | | | | |
|---|---|---|---|---|---|---|---|---|---|---|---|---|
| | | | Ceftolo zane/ Tazoba ctam(4) | Cefid erocol | Piperac illin/ Tazoba ctam(4) | Aztre onam | Tigec ycline | Col isti n | Ami kaci n | Ciprofl oxacin | Levofl oxacin | Cefoz opran |
| *Enterobacter cloacae* | 56 60 | 163 | 16 | 0.25 | >64 | >64 | 4 | 8 | 4 | 64 | 32 | >64 |
| *Enterobacter cloacae complex* | 56 61 | 164 | 8 | 0.5 | >64 | >64 | 0.5 | 0.5 | 1 | 0.06 | 0.12 | 1 |
| *Klebsiella pneumoniae* | 57 57 | 361 | 64 | 1 | >64 | >64 | 1 | 0.5 | 32 | >64 | 64 | 16 |
| *Klebsiella pnemnonine* | 57 58 | 362 | >64 | 0.5 | >64 | >64 | 1 | 0.2 5 | 32 | >64 | 64 | 32 |
| *Klebsiella pneumoniae* | 57 59 | 363 | 32 | 0.25 | >64 | >64 | 0.5 | 0.5 | 16 | 64 | 64 | 16 |
| *Klebsiella pneumoniae* | 57 60 | 364 | 8 | 2 | 64 | >64 | 1 | 64 | 16 | >64 | 64 | 4 |
| *Enterobacter cloacae* | 57 61 | 365 | 16 | 0.25 | >64 | >64 | 2 | 1 | 16 | 2 | 4 | 16 |
| *Enterobacter cloacae* | 57 62 | 366 | 8 | 0.25 | >64 | 64 | 2 | 0.2 5 | 4 | 0.12 | 0.5 | 16 |
| *Enterobacter cloacae* | 57 63 | 367 | 16 | 1 | >64 | >64 | 0.5 | 0.5 | 1 | 0.5 | 1 | 4 |
| *Escherichia coli* | 57 64 | 368 | 0.25 | ≤0.0 6 | 1 | 0.06 | 0.25 | 0.5 | 2 | ≤0.03 | 0.06 | 0.06 |
| *Escherichia coli* | 57 65 | 369 | 0.5 | 0.25 | 2 | 64 | 0.25 | 0.5 | 2 | 0.06 | 0.06 | 32 |
| *Escherichia coli* | 57 66 | 370 | 1 | 0.5 | 8 | >64 | 0.25 | 0.2 5 | 4 | >64 | 16 | 64 |
| *Escherichia coli* | 57 67 | 371 | 32 | 0.5 | >64 | 16 | 0.25 | 0.2 5 | 0.5 | 64 | 16 | 32 |
| *Escherichia coli* | 57 68 | 372 | 16 | 1 | 32 | >64 | 0.5 | 0.5 | 2 | >64 | 16 | >64 |
| *Escherichia coli* | 57 69 | 373 | 1 | ≤0.0 6 | 8 | 16 | 0.5 | 0.5 | 1 | 64 | 32 | 0.5 |
| *Escherichia coli* | 57 70 | 374 | 2 | <0.0 6 | 8 | 16 | 0.5 | 0.5 | 1 | >64 | 32 | 1 |
| *Klebsiella oxytoca* | 57 71 | 375 | 0.25 | 0.5 | 2 | 32 | 0.5 | 0.2 5 | 4 | ≤0.03 | 0.12 | 0.5 |
| *Klebsiella pneumoniae* | 57 72 | 376 | 8 | 1 | >64 | >64 | 1 | 0.5 | 2 | 64 | 16 | >64 |
| *Proteus mirabilis* | 57 73 | 377 | 8 | ≤0.0 6 | 4 | 4 | 4 | >6 4 | 2 | 16 | 16 | 16 |
| *Escherichia coli* | 57 74 | 378 | 0.25 | 0.12 | 0.5 | 0.12 | 0.25 | 0.2 5 | 1 | 32 | 8 | ≤0.0 3 |
| *Proteus mirabilis* | 57 75 | 379 | 0.5 | 2 | 0.5 | 0.25 | 0.5 | 64 | 8 | 4 | 4 | 4 |
| *Klebsiella oxytoca* | 57 76 | 380 | 8 | ≤0.0 6 | >64 | >64 | 2 | 0.5 | 1 | 0.12 | 0.5 | 32 |
| *Enterobacter aerogenes* | 57 77 | 431 | 32 | 1 | 16 | 16 | 1 | 0.2 5 | 0.5 | 1 | 1 | 4 |

(continued)

| Orgamism | K U B N o. | AR Bank # | MIC (μg/mL) | | | | | | | | | |
|---|---|---|---|---|---|---|---|---|---|---|---|---|
| | | | Ceftolo zane/ Tazoba ctam(4) | Cefid erocol | Piperac illin/ Tazoba ctam(4) | Aztre onam | Tigec ycline | Col isti n | Ami kaci n | Ciprofl oxacin | Levofl oxacin | Cefoz opran |
| *Enterobacter cloacae* | 57 78 | 432 | >64 | 4 | >64 | 32 | 1 | 0.5 | 2 | 0.06 | 0.12 | >64 |
| *Escherichia coli* | 57 79 | 433 | 32 | 0.5 | >64 | 32 | 0.5 | 0.2 5 | 0.5 | 64 | 32 | 16 |
| *Escherichia coli* | 57 80 | 434 | 32 | 0.5 | 2 | >64 | 0.5 | 0.5 | 16 | 64 | 16 | 64 |
| *Escherichia coli* | 57 81 | 435 | >64 | 4 | >64 | >64 | 0.5 | 0.2 5 | >64 | 64 | 32 | >64 |
| *Escherichia* | 57 | *436* | 2 | ≤0.0 | 16 | 32 | 0.5 | 0.2 | 1 | >64 | 32 | 1 |

[Table 8-13]

| Orgamism | K U B N o. | AR Bank # | MIC (µg/mL) | | | | | | | | | |
|---|---|---|---|---|---|---|---|---|---|---|---|---|
| | | | Ceftolo zine/ Tazoba ctam(4) | Cefid erocol | Piperac illin/ Tazoba ctam(4) | Aztre onam | Tigec yeline | Col isti n | Ami kaci n | Ciprofl oxacin | Levofl oxacin | Cefoz opran |
| *coli* | 82 | | | 6 | | | | 5 | | | | |
| *Escherichia coli* | 57 83 | 437 | 64 | 0.25 | >64 | >64 | 1 | 0.2 5 | 2 | >64 | 32 | >64 |
| *Klebsiella pneumoniae* | 57 84 | 438 | >64 | 0.5 | >64 | >64 | 1 | 0.5 | 32 | >64 | 64 | >64 |
| *Enterobacter cloacae* | 57 92 | 448 | >64 | 2 | >64 | >64 | 1 | 0.5 | 16 | >64 | >64 | >64 |
| *Escherichia coli* | 57 94 | 450 | >64 | 0.25 | >64 | >64 | 0.5 | 0.2 5 | 2 | 0.06 | 0.12 | >64 |
| *Escherichia coli* | 57 95 | 451 | 16 | 0.5 | 64 | 64 | 0.5 | 0.2 5 | 8 | 64 | 32 | 64 |
| *Escherichia coli* | 57 96 | 452 | >64 | 1 | 64 | 0.12 | 0.5 | 0.2 5 | 1 | >64 | 64 | >64 |
| *Klebsiella pneumoniae* | 57 97 . | 453 | >64 | 0.25 | >64 | >64 | 1 | 0.5 | 32 | >64 | 64 | >64 |
| *Escherichia coli* | 58 03 | 861 | >64 | 2 | >64 | 1 | 0.25 | 0.2 5 | 1 | 64 | 16 | >64 |
| *Enterobacter cloacae* | 58 07 | 501 | >64 | 1 | >64 | 1 | 1 | 0.5 | 4 | 0.25 | 1 | >64 |
| *Enterobacter cloacae* | 58 08 | 502 | >64 | 1 | >64 | >64 | 4 | 0.5 | 2 | 16 | 8 | >64 |
| *Klebsiella pneumoniae* | 58 09 | 504 | 16 | 0.25 | >64 | 32 | 1 | 1 | 2 | 2 | 0.5 | >64 |
| *Klebsiella pneumoniae* | 58 10 | 505 | >64 | 0.5 | >64 | 64 | 0.5 | 1 | 8 | 0.5 | 1 | >64 |
| *Klebsiella pneumoniae* | 58 11 | 506 | >64 | 2 | >64 | >64 | 2 | 0.5 | 32 | 2 | 4 | >64 |
| *Klebsiella pneumoniae* | 58 12 | 507 | >64 | 2 | >64 | >64 | 2 | 8 | >64 | >64 | >64 | >64 |
| *Serratia marcescens* | 58 22 | 517 | >64 | 2 | >64 | >64 | 1 | >6 4 | 8 | 1 | 0.5 | >64 |
| *Morganella morganii* | 58 24 | 519 | 64 | ≤0.0 6 | >64 | 32 | 0.5 | >6 4 | 4 | 0.06 | 0.12 | >64 |
| *Serratia marcescens* | 58 25 | 520 | 8 | ≤0.0 6 | 4 | 2 | 1 | >6 4 | 64 | 1 | 0.5 | 0.25 |
| *Serratia marcescens* | 58 26 | 521 | >64 | 0.5 | 32 | >64 | 2 | >6 4 | 2 | 16 | 16 | 8 |
| *Klebsiella pneumoniae* | 58 27 | 522 | >64 | 2 | >64 | >64 | 1 | 32 | 4 | 64 | 32 | >64 |
| *Klebsiella pneumoniae* | 58 28 | 523 | 32 | 1 | >64 | >64 | 1 | 4 | 8 | 4 | 4 | 32 |
| *Klebsiella pneumoniae* | 58 29 | 524 | >64 | 0.5 | >64 | >64 | 1 | 2 | 16 | 32 | 32 | 32 |
| *Klebsiella pneumoniae* | 58 30 | 525 | >64 | 2 | >64 | >64 | 1 | 16 | 32 | >64 | 64 | 32 |

[Table 9-1]

| Table 9 MIC of MEPM/Compound A and control agents against *A. baumannii* | | | | | | | | | | | | |
|---|---|---|---|---|---|---|---|---|---|---|---|---|
| KUB No | ARB ank# | Harbored carbapenemase genes | | MIC (μg/mL) | | | | | | | | |
| | | Serine-type | Metallo-type | Meropenem | Meropenem/ CompoundA(4) | Meropenem/ CompoundA(8) | Meropenem/ CompoundA(16) | Meropenem/ Vaborbactam(8) | Cefrazidime/ Avibactam(4) | Imipenem/ Relebactam(4) | Cefrolozane/ Tazobactam(4) |
| 55 30 | 3 3 | OXA-94 | NDM-1 | 128 | 4 | 2 | 1 | 128 | >32 | >32 | >64 |
| 55 32 | 3 5 | OXA-66, OXA-72 | - | 128 | 8 | 4 | 1 | 128 | 16 | >32 | 32 |
| 55 33 | 3 6 | OXA-24, OXA-65 | - | 64 | 4 | 2 | 1 | 64 | 16 | >32 | 32 |
| 55 34 | 3 7 | OXA-94 | NDM-1 | 128 | 4 | 2 | 1 | 128 | >32 | >32 | >64 |
| 55 42 | 4 5 | OXA-23, OXA-69 | - | 16 | 2 | 1 | 0.5 | 16 | >32 | 16 | >64 |
| 55 49 | 5 2 | OXA-58, OXA-100 | - | 4 | 2 | 1 | 1 | 4 | 4 | 8 | 2 |
| 55 53 | 5 6 | OXA-23, OXA-66 | - | 16 | 4 | 2 | 1 | 16 | 16 | 32 | >64 |
| 55 60 | 6 3 | OXA-23, OXA-24, OXA-65 | - | 64 | 4 | 2 | 1 | 64 | >32 | >32 | >64 |
| 55 67 | 7 0 | OXA-58, OXA-100 | - | 8 | 2 | 1 | ≤0.25 | 4 | 4 | 16 | 4 |
| 55 75 | 7 8 | OXA-71 | - | 32 | 16 | 8 | 4 | 32 | 16 | 32 | 8 |
| 55 80 | 8 3 | OXA-23, OXA-69 | NDM-1 | 128 | 16 | 1 | 1 | 128 | >32 | >32 | >64 |
| 55 85 | 8 8 | OXA-64 | NDM-1 | >128 | 4 | 2 | 0.5 | >128 | >32 | >32 | >64 |
| 55 98 | 1 0 1 | OXA-24, OXA-65 | - | 128 | 2 | 1 | 1 | 128 | >32 | >32 | 32 |
| 55 99 | 1 0 2 | OXA-66 | - | 2 | 1 | 1 | 1 | 2 | 32 | 0.5 | 8 |
| 57 06 | 2 7 3 | OXA-23, OXA-66 | - | 32 | 4 | 4 | 2 | 32 | 16 | 32 | 16 |
| 57 07 | 2 7 4 | OXA-66, OXA-72 | - | >128 | 64 | 8 | 4 | >128 | 32 | >32 | 32 |
| 57 08 | 2 7 5 | OXA-23, OXA-66 | - | 32 | 4 | 2 | 2 | 32 | >32 | 32 | 32 |
| 57 09 | 2 7 6 | - | - | 4 | 4 | 4 | 4 | 16 | >32 | 4 | 64 |
| 57 10 | 2 7 7 | OXA-24, OXA-65 | - | 128 | 8 | 2 | 2 | 128 | >32 | >32 | 64 |
| 57 11 | 2 7 8 | OXA-23, OXA-66 | - | 32 | 4 | 2 | 2 | 32 | >32 | 32 | 32 |

[Table 9-2]

| KUB No. | ARBank# | Harbored carbapenemase genes | | MIC (μg/mL) | | | | | | | |
|---|---|---|---|---|---|---|---|---|---|---|---|
| | | Serine-type | Metallo-type | Meropenem | Meropenem/ CompoundA(4) | Meropenem/ CompoundA(8) | Meropenem/ CompoundA(16) | Meropenem/ Vaborbactam(8) | Ceftazidime/ Avibactam(4) | Imipenem/ Relebactam(4) | Ceftolozane/ Tazobactam(4) |
| 57 12 | 2 7 9 | OXA-23, OXA-66 | - | 32 | 4 | 2 | 2 | 32 | 32 | 32 | 32 |
| 57 13 | 2 8 0 | OXA-66 | - | 2 | 2 | 2 | 2 | 2 | 32 | 0.5 | 16 |
| 57 14 | 2 8 1 | OXA-82 | - | 8 | 8 | 2 | 1 | 8 | 32 | 4 | 16 |
| 57 15 | 2 8 2 | OXA-23, OXA-66 | - | 32 | 16 | 4 | 2 | 64 | >32 | >32 | 32 |
| 57 16 | 2 8 3 | OXA-23, OXA-66 | - | 32 | 4 | 2 | 2 | 32 | 32 | 32 | 16 |
| 57 17 | 2 8 4 | OXA-24. OXA-65 | - | 128 | 8 | 4 | 2 | 128 | 16 | >32 | 16 |
| 57 18 | 28 5 | OXA-24, OXA-65 | - | 128 | 16 | 2 | 1 | 128 | 32 | >32 | 32 |
| 57 19 | 2 8 6 | OXA-24, OXA-66 | - | 128 | 16 | 4 | 2 | 128 | 32 | >32 | 64 |
| 57 20 | 2 8 7 | OXA-66, OXA-72 | - | >128 | 128 | 32 | 2 | >128 | 32 | >32 | 32 |
| 57 21 | 2 8 8 | OXA-23, OXA-66 | - | 16 | 4 | 4 | 2 | 8 | 16 | 8 | 16 |
| 57 22 | 2 8 9 | OXA-66, OXA-72 | - | 128 | 32 | 8 | 4 | >128 | 32 | >32 | 16 |
| 57 23 | 9 0 | OXA-23, OXA-66 | - | 32 | 8 | 4 | 4 | 32 | >32 | 32 | 32 |
| 57 24 | 2 9 1 | OXA-23, OXA-66 | - | 32 | 2 | 4 | 1 | 32 | 32 | >32 | 16 |
| 57 25 | 2 9 2 | OXA-66. OXA-72 | - | >128 | 128 | 16 | 1 | >128 | 32 | >32 | 32 |
| 57 26 | 2 9 3 | OXA-66, OXA-72 | - | >128 | 128 | 16 | 1 | >128 | 32 | >32 | 32 |
| 57 27 | 2 9 4 | OXA-23, OXA-65 | - | 32 | 8 | 4 | 1 | 32 | 32 | 32 | 16 |
| 57 28 | 29 5 | OXA-23, OXA-66 | - | 32 | 8 | 4 | 2 | 32 | 32 | >32 | 64 |
| 57 29 | 29 6 | OXA-23, OXA-223 | - | 64 | 16 | 4 | 2 | 64 | 32 | >32 | 32 |

[Table 9-3]

| KUB No. | ARB ank# | Harbored carbapenemase genes | | MIC (μg/mL) | | | | | | | |
|---|---|---|---|---|---|---|---|---|---|---|---|
| | | Serine-type | Metallo -type | Meropenem | Meropenem/ CompoundA(4) | Meropenem/ CompoundA(8) | Meropenem/ CompoundA(16) | Meropenem/ Vaborbactam(8) | Ceftazidime/ Avibactam(4) | Imipenem/ Relebactam(4) | Ceftolozane/ Tazobactam(4) |
| 57 30 | 2 9 7 | OXA-23, OXA-66 | - | 16 | 8 | 4 | 2 | 16 | 16 | 32 | 32 |
| 57 31 | 2 9 8 | OXA-66, OXA-237 | - | 16 | 8 | 4 | 2 | 16 | 32 | 8 | 16 |
| 57 32 | 2 9 9 | OXA-23, OXA-203 | - | 32 | 8 | 1 | 0.5 | 32 | 8 | 32 | >64 |
| 57 33 | 3 0 0 | OXA-66 | - | 2 | 2 | 2 | 2 | 2 | 32 | 0.5 | 8 |
| 57 34 | 3 0 1 | OXA-66, OXA-72 | - | >128 | 64 | 8 | 2 | >128 | >32 | >32 | >64 |
| 57 35 | 3 0 2 | OXA-23, OXA-82 | - | 32 | 16 | 4 | 2 | 32 | >32 | >32 | >64 |
| 57 36 | 3 0 3 | OXA-23, OXA-66 | - | 16 | 2 | 1 | 0.5 | 16 | 8 | 16 | 8 |
| 57 37 | 3 0 4 | OXA-66, OXA-72 | | 64 | 1 | 2 | 1 | 32 | 32 | 16 | 8 |
| 57 38 | 3 0 5 | OXA-24, OXA-65 | - | 128 | 4 | 4 | 1 | 128 | 32 | >32 | 32 |
| 57 39 | 3 0 6 | OXA-24, OXA-65 | - | 128 | 8 | 4 | 2 | 128 | >32 | >32 | 64 |
| 57 40 | 3 0 7 | OXA-66, OXA-237 | - | 8 | 2 | 2 | 1 | 8 | 32 | 2 | 16 |
| 57 41 | 3 0 8 | OXA-71 | - | 16 | 4 | 2 | 1 | 8 | 32 | 8 | 8 |
| 57 42 | 3 0 9 | OXA-23, OXA-82 | - | 32 | 8 | 4 | 2 | 32 | >32 | 32 | >64 |
| 57 43 | 3 1 0 | OXA-23, OXA-82 | - | 32 | 8 | 4 | 2 | 32 | >32 | 32 | >64 |
| 57 44 | 3 1 1 | OXA-23, OXA-82 | - | 32 | 8 | 4 | 2 | 64 | >32 | 32 | 64 |
| 57 45 | 3 1 2 | OXA-69 | - | 4 | 4 | 4 | 4 | 4 | >32 | 0.5 | 32 |
| 57 46 | 3 1 3 | OXA-23, OXA-69 | - | 16 | 2 | 1 | 0.5 | 16 | >32 | 16 | >64 |

[Table 9-4]

| Table 9 continued | | | | | | | | | | |
|---|---|---|---|---|---|---|---|---|---|---|
| KU B No. | AR Ba nk # | MIC (μg/mL) | | | | | | | | |
| | | Cefidero col | Piperacilli n/ Tazobacta m(4) | Aztreon am | Tigecycl ine | Colis tin | Amika cin | Ciproflox acin | Levotlox acin | Cefozop ran |
| 553 0 | 33 | 2 | >64 | >64 | 0.25 | 1 | 2 | 64 | 8 | >64 |
| 553 2 | 35 | 0.12 | >64 | 32 | 1 | 1 | 4 | 64 | 16 | 64 |
| 553 3 | 36 | 0.5 | >64 | 64 | 1 | 0.5 | 64 | >64 | 64 | 32 |
| 553 4 | 37 | 4 | >64 | >64 | 0.25 | 1 | 2 | >64 | 16 | >64 |
| 554 2 | 45 | 0.5 | >64 | >64 | 1 | 1 | 2 | 64 | 8 | 64 |
| 554 9 | 52 | ≤0.06 | >64 | 64 | 0.25 | 1 | 0.5 | 8 | 4 | 2 |
| 555 3 | 56 | 0.25 | >64 | >64 | 1 | 1 | 2 | 64 | 16 | >64 |
| 556 0 | 63 | 2 | >64 | 64 | 0.5 | 0.5 | 16 | 64 | 16 | >64 |
| 556 7 | 70 | ≤0.06 | >64 | 64 | 0.25 | 0.25 | 1 | 8 | 4 | 2 |
| 557 5 | 78 | 4 | >64 | >64 | 2 | 1 | 32 | >64 | 32 | >64 |
| 558 0 | 83 | 16 | >64 | >64 | 0.5 | 1 | >64 | 16 | 8 | >64 |
| 558 5 | 88 | 2 | >64 | 32 | 0.25 | 1 | 32 | 32 | 8 | >64 |
| 559 8 | 101 | 0.5 | >64 | 64 | 1 | 1 | 64 | >64 | 16 | 32 |
| 559 9 | 102 | 0.12 | >64 | 64 | 1 | 1 | >64 | 64 | 8 | 8 |
| 570 6 | 273 | 0.12 | >64 | 64 | 0.5 | 1 | >64 | >64 | 64 | 64 |
| 570 7 | 274 | 0.12 | >64 | 64 | 1 | 0.5 | 16 | >64 | 16 | 16 |
| 570 8 | 275 | 0.5 | >64 | >64 | 0.5 | 1 | >64 | 64 | 8 | 64 |
| 570 9 | 276 | 8 | >64 | 64 | 1 | 2 | 64 | 64 | 16 | >64 |
| 571 0 | 277 | 4 | >64 | >64 | 1 | 0.5 | 16 | >64 | 16 | 64 |
| 571 1 | 278 | 0.5 | >64 | >64 | 0.5 | 1 | >64 | 64 | 8 | 64 |
| 571 2 | 279 | 0.25 | >64 | >64 | 0.5 | 1 | >64 | 64 | 8 | 64 |
| 571 3 | 280 | 0.25 | >64 | 64 | 1 | 1 | 4 | >64 | 16 | 8 |
| 571 4 | 281 | 0.12 | >64 | 32 | 2 | 1 | 2 | 32 | 8 | 8 |
| 571 5 | 282 | 0.5 | >64 | >64 | 0.5 | 1 | >64 | 64 | 8 | 64 |
| 571 6 | 283 | 0.25 | >64 | >64 | 1 | 1 | >64 | 64 | 16 | 32 |
| 571 7 | 284 | 0.25 | >64 | 64 | 0.5 | 1 | 32 | 64 | 16 | 16 |
| 571 | 285 | 1 | >64 | >64 | 0.5 | 0.5 | 64 | >64 | 64 | 32 |

[Table 9-5]

| KU B No. | AR Ba nk # | MIC (μg/mL) | | | | | | | | |
|---|---|---|---|---|---|---|---|---|---|---|
| | | Cefidero col | Piperacilli n/ Tazobacta m(4) | Aztreon am | Tigecycl ine | Colis tin | Amika cin | Ciproflox acin | Levoflox acin | Cefozop ran |
| 8 | | | | | | | | | | |
| 571 9 | 286 | 0.5 | >64 | 32 | 1 | 1 | >64 | 64 | 8 | 32 |

(continued)

| KU B No. | AR Ba nk # | MIC (µg/mL) | | | | | | | | |
|---|---|---|---|---|---|---|---|---|---|---|
| | | Cefidero col | Piperacillin/ Tazobactam(4) | Aztreon am | Tigecycl ine | Colis tin | Amika cin | Ciproflox acin | Levoflox acin | Cefozop ran |
| 572 0 | 287 | 0.5 | >64 | 32 | 0.5 | 1 | 64 | 64 | 16 | 32 |
| 572 1 | 288 | 0.25 | >64 | 64 | 2 | >64 | >64 | >64 | >64 | 64 |
| 572 2 | 259 | 0.12 | >64 | 64 | 1 | 2 | 32 | >64 | 16 | 8 |
| 572 3 | 290 | 0.5 | >64 | >64 | 0.5 | 1 | >64 | 64 | 8 | >64 |
| 572 4 | 291 | 0.5 | >64 | >64 | 1 | 1 | >64 | >64 | 8 | 32 |
| 572 5 | 292 | 0.5 | >64 | 64 | 0.25 | 1 | 32 | 16 | 4 | 16 |
| 572 6 | 293 | 0.5 | >64 | 64 | 2 | 1 | 16 | 16 | 4 | 16 |
| 572 7 | 294 | 0.5 | >64 | 32 | 0.5 | 1 | 64 | >64 | 32 | >64 |
| 572 8 | 295 | 0.5 | >64 | >64 | 1 | 1 | 2 | >64 | 16 | 64 |
| 572 9 | 296 | 0.5 | >64 | >64 | 1 | 2 | >64 | >64 | 32 | >64 |
| 573 0 | 297 | 0.12 | >64 | >64 | 2 | 2 | 32 | >64 | 32 | 64 |
| 573 1 | 298 | 0.5 | >64 | >64 | 0.5 | 0.5 | >64 | 64 | 8 | 16 |
| 573 2 | 299 | 8 | >64 | >64 | 1 | 2 | >64 | >64 | 64 | >64 |
| 573 3 | 300 | 0.25 | 64 | 64 | 0.5 | 1 | >64 | >64 | 64 | 8 |
| 573 4 | 301 | 1 | >64 | >64 | 1 | 1 | 64 | >64 | 16 | >64 |
| 573 5 | 302 | 4 | >64 | >64 | 1 | 1 | >64 | >64 | >64 | >64 |
| 573 6 | 303 | 0.12 | >64 | >64 | 1 | >64 | >64 | 64 | 16 | 32 |
| 573 7 | 304 | ≤0.06 | >64 | 64 | 1 | 4 | 32 | >64 | 16 | 8 |
| 573 8 | 305 | 0.25 | >64 | 64 | 0.25 | 2 | 32 | >64 | 16 | 16 |
| 573 9 | 306 | 1 | >64 | >64 | 1 | 1 | 64 | >64 | 32 | 64 |
| 574 0 | 307 | 0.12 | >64 | 64 | 1 | 2 | >64 | 64 | 16 | 16 |
| 574 1 | 308 | 0.25 | >64 | 64 | 0.5 | 1 | >64 | >64 | 16 | 4 |
| 574 2 | 309 | 4 | >64 | 64 | 0.5 | 2 | >64 | >64 | 64 | 64 |
| 574 3 | 310 | 2 | >64 | 64 | 1 | 1 | >64 | >64 | 32 | 64 |
| 574 4 | 311 | 0.5 | >64 | 64 | 0.5 | 1 | >64 | 64 | 4 | >64 |
| 574 5 | 312 | 0.25 | >64 | >64 | 0.25 | 1 | 1 | 8 | 4 | 16 |

[Table 9-6]

| KU B No. | AR Ba nk # | MIC (µg/mL) | | | | | | | | |
|---|---|---|---|---|---|---|---|---|---|---|
| | | Cefidero col | Piperacillim/ Tazobactam(4) | Aztreon am | Tigecycl ine | Colis tin | Amika cin | Ciproflox acin | Levoflox acin | Cefozop ran |
| 574 6 | 313 | 1 | >64 | >64 | *0.5* | 2 | 2 | 64 | 8 | 64 |

[Table 10-1]

**Table 10 MIC of MEPM/Compound A and control agents against *P. aeruginosa***

| K U B N o. | A R Ba nk # | Harbored carbapene mase genes | | MIC (μg/mL) | | | | | | | |
|---|---|---|---|---|---|---|---|---|---|---|---|
| | | Seri ne-typ e | Met allo-type | Merop enem | Merope nem/ Compou ndA(4) | Merope nem/ Compou ndA(8) | Meropen em/ Compou ndA(16) | Merope nem/ Vaborba ctam(8) | Ceftazi dime/ Avibac tam(4) | Imipene m/ Relebac tam(4) | Ceftolo zane/ Tazobac tam(4) |
| 55 51 | 54 | - | VI M-4 | >128 | >128 | >128 | >128 | >128 | >32 | >32 | >64 |
| 55 61 | 64 | - | SP M-1 | 128 | 128 | 128 | 128 | 128 | >32 | >32 | >64 |
| 55 87 | 90 | KP C-5 | - | 32 | 4 | 0.5 | ≤0.25 | 2 | 2 | 1 | 32 |
| 55 89 | 92 | - | IMP -14 | >128 | >128 | >125 | >128 | >128 | >32 | >32 | >64 |
| 55 91 | 94 | - | - | 16 | 16 | 16 | 16 | 16 | 16 | 4 | 16 |
| 55 92 | 95 | - | - | 8 | 8 | 8 | 8 | 8 | 2 | 2 | 1 |
| 55 97 | 10 0 | - | VI M-2 | 128 | 64 | 64 | 32 | 128 | 32 | >32 | >64 |
| 56 00 | 10 3 | - | IMP -1 | >128 | >128 | >128 | >128 | >128 | >32 | >32 | >64 |
| 56 02 | 10 5 | - | - | 4 | 4 | 4 | 2 | 4 | 4 | 1 | 4 |
| 56 05 | 10 8 | - | VI M-2 | 128 | 64 | 32 | 16 | 128 | >32 | >32 | >64 |
| 56 07 | 11 0 | - | VI M-2 | 64 | 32 | 16 | 8 | 64 | 32 | >32 | >64 |
| 56 08 | 11 1 | - | VI M-2 | 32 | 32 | 8 | 8 | 32 | 16 | >32 | >64 |
| 56 62 | 22 9 | - | - | 32 | 32 | 32 | 32 | 32 | 8 | 4 | 2 |
| 56 63 | 23 0 | - | VI M-2 | 64 | 32 | 16 | 16 | 64 | 32 | >32 | >64 |
| 56 64 | 23 1 | KP C-5 | - | 128 | 32 | 16 | 16 | 64 | 16 | 16 | 64 |
| 56 65 | 23 2 | - | - | 4 | 2 | 2 | 2 | 4 | 1 | 0.5 | 1 |
| 56 66 | 23 3 | - | - | 16 | 8 | 8 | 8 | 8 | 4 | 1 | 1 |
| 56 67 | 23 4 | - | - | 2 | 1 | 1 | 1 | 2 | 1 | 0.5 | 0.5 |
| 56 68 | 23 5 | - | - | 16 | 16 | 16 | 16 | 16 | 32 | 4 | >64 |

[Table 10-2]

| K U B N o. | A R Ba nk # | Harbored carbapene mase genes | | MIC (μg/mL) | | | | | | | |
|---|---|---|---|---|---|---|---|---|---|---|---|
| | | Seri ne-typ e | Met allo-type | Merop enem | Merope nem/ Compou ndA(4) | Merope nem/ Compou ndA(8) | Meropen em/ Compou ndA(16) | Merope nem/ Vaborba ctam(8) | Ceftazi dime/ Avibac tam(4) | Imipene m/ Relebac tam(4) | Ceftolo zane/ Tazobac tam(4) |
| 56 69 | 23 6 | - | - | 2 | 1 | 2 | 2 | 2 | 1 | 1 | 1 |

(continued)

| K U B N o. | A R Ba nk # | Harbored carbapene mase genes | | MIC (µg/mL) | | | | | | | |
|---|---|---|---|---|---|---|---|---|---|---|---|
| | | Seri ne-typ e | Met allo-type | Merop enem | Merope nem/ Compou ndA(4) | Merope nem/ Compou ndA(8) | Meropen em/ Compou ndA(16) | Merope nem/ Vaborba ctam(8) | Ceftazi dime/ Avibac tam(4) | Imipene m/ Relebac tam(4) | Ceftolo zane/ Tazobac tam(4) |
| 56 70 | 23 7 | - | - | 2 | 1 | 1 | 1 | 2 | 1 | 0.5 | 0.5 |
| 56 71 | 23 8 | - | - | ≤0.25 | ≤0.25 | ≤0.25 | ≤0.25 | ≤0.25 | 2 | 0.25 | 1 |
| 56 72 | 23 9 | GE S-1 | VI M-11 | 64 | 64 | 64 | 32 | 64 | >32 | ->32 | >64 |
| 56 73 | 24 0 | - | VI M-2 | >128 | 128 | 128 | 64 | >128 | >32 | >32 | >64 |
| 56 74 | 24 1 | - | IMP -1 | >128 | >128 | 128 | 128 | >128 | >32 | >32 | >64 |
| 56 75 | 24 2 | - | VI M-2 | 4 | 2 | 1 | ≤0.25 | 4 | 32 | 8 | >64 |
| 56 76 | 24 3 | - | VI M-2 | 8 | 2 | 1 | 1 | 8 | 8 | 16 | >64 |
| 56 77 | 24 4 | - | - | 16 | 16 | 16 | 16 | 16 | 4 | 4 | 2 |
| 56 78 | 24 5 | - | VI M-2 | 8 | 4 | 4 | 2 | 8 | 32 | 16 | >64 |
| 56 79 | 24 6 | - | ND M-1 | >1.28 | >128 | >128 | >128 | >128 | >32 | >32 | >64 |
| 56 80 | 24 7 | - | - | 4 | 2 | 2 | 2 | 2 | 2 | 2 | 1 |
| 56 81 | 24 8 | - | VI M-2 | 4 | 4 | 2 | 0.5 | 8 | 32 | 8 | >64 |
| 56 82 | 24 9 | - | VI M-2 | >128 | >128 | >128 | 64 | >128 | >32 | >32 | >64 |
| 56 83 | 25 0 | - | ND M-1 | >128 | >128 | >128 | >128 | >128 | >32 | >32 | >64 |
| 56 84 | 25 1 | - | - | 1 | 1 | 1 | 1 | ≤0.25 | 4 | 0.25 | 2 |
| 56 85 | 25 2 | - | - | 2 | 2 | 2 | 1 | 2 | 4 | 0.5 | 4 |
| 56 86 | 25 3 | - | - | 0.5 | 0.5 | 1 | 0.5 | 0.5 | 1 | 0.25 | 2 |
| 56 87 | 25 4 | - | VI M-2 | 64 | 64 | 16 | 8 | 32 | 32 | >32 | >64 |
| 56 88 | 25 5 | - | - - VI M-2 | >128 | 128 | 64 | 32 | >128 | 32 | >32 | >64 |
| 56 89 | 25 6 | - | - | 2 | 2 | 4 | 2 | 4 | 2 | 1 | 1 |
| 56 90 | 25 7 | - | - | 4 | 4 | 4 | 4 | 4 | 4 | 1 | 2 |
| 56 91 | 25 8 | - | - | 2 | 2 | 1 | 1 | 2 | 2 | 0.5 | 0.5 |
| 56 92 | 25 9 | - | - | 0.5 | ≤0.25 | ≤0.25 | 0.5 | 0.5 | 1 | 0.5 | 0.5 |
| 56 93 | 26 0 | - | - | 4 | 4 | 8 | 4 | 8 | 2 | 1 | 1 |
| 56 94 | 26 1 | - | - | 0.5 | 0.5 | 0.5 | ≤0.25 | 0.5 | 2 | 0.5 | 2 |

[Table 10-3]

| K U B N o. | A R Ba nk # | Harbored carbapene mase genes | | MIC (µg/mL) | | | | | | | |
|---|---|---|---|---|---|---|---|---|---|---|---|
| | | Seri ne-typ e | Met allo-type | Merop enem | Merope nem/ Compou ndA(4) | Merope nem/ Compou ndA(8) | Meropen em/ Compou ndA(16) | Merope nem/ Vaborba ctam(8) | Ceftazi dime/ Avibac tam(4) | Imipene m/ Relebac tam(4) | Ceftolo zane/ Tazobac tam(4) |
| 56 95 | 26 2 | - | - | 0.5 | 0.5 | 0.5 | 0.5 | 1 | 2 | 0.5 | 1 |
| 56 96 | 26 3 | - | - | ≤0.25 | ≤0.25 | ≤0.25 | ≤0.25 | ≤0.25 | 0.5 | 0.25 | 0.5 |
| 56 97 | 26 4 | - | - | 2 | 2 | 2 | 2 | 2 | 1 | 1 | 1 |
| 56 98 | 26 5 | - | - | 8 | 8 | 8 | 8 | 8 | 4 | 2 | 2 |
| 56 99 | 26 6 | - | - | 8 | 8 | 8 | 4 | 8 | 2 | 2 | 2 |
| 57 00 | 26 7 | - | - | 2 | 2 | 2 | 2 | 2 | 2 | 2 | 2 |
| 57 01 | 26 8 | - | - | 16 | 16 | 8 | 8 | 16 | 8 | 4 | 8 |
| 57 02 | 26 9 | - | - | 8 | 8 | 8 | 8 | 8 | 4 | 1 | 1 |
| 57 03 | 27 0 | - | - | 2 | 2 | 2 | 2 | 2 | 1 | 2 | 2 |
| 57 04 | 27 1 | - | - | 8 | 8 | 8 | 8 | 8 | 4 | 2 | 1 |
| 57 05 | 27 2 | - | - | 2 | 1 | 2 | 2 | 2 | 2 | 1 | 1 |
| 57 47 | 35 1 | - | - | 16 | 16 | 16 | 16 | 16 | 16 | 2 | 4 |
| 57 48 | 35 2 | - | - | 128 | 128 | 128 | 128 | 128 | >32 | 16 | 32 |
| 57 49 | 35 3 | GE S-1 | - | 16 | 16 | 8 | 4 | 16 | 8 | 4 | >64 |
| 57 50 | 35 4 | - | - | 4 | 4 | 2 | 2 | 8 | 1 | 1 | 1 |
| 57 51 | 35 5 | - | - | 8 | 8 | 8 | 2 | 8 | 2 | 1 | 4 |
| 57 52 | 35 6 | KP C-2 | - | 32 | 8 | 2 | ≤0-25 | 2 | 1 | 1 | 16 |
| 57 53 | 35 7 | - | - | 4 | 4 | 4 | 4 | 4 | 4 | 2 | 64 |
| 57 54 | 35 8 | - | - | 8 | 8 | 8 | 4 | 8 | 2 | 1 | 2 |
| 57 55 | 35 9 | - | - | 8 | 8 | 8 | 4 | 8 | 2 | 1 | 2 |
| 57 56 | 36 0 | - | - | 16 | 8 | 8 | 8 | 32 | 2 | 1 | 1 |
| 57 85 | 43 9 | - | IMP -18 | 64 | 32 | 64 | 32 | 64 | >32 | >32 | >64 |
| 57 86 | 44 0 | - | - | 8 | 8 | 4 | 4 | 8 | 4 | 1 | 4 |
| 57 87 | 44 1 | KP C-2 | - | 32 | 16 | 4 | ≤0.25 | 4 | 1 | 1 | 16 |
| 57 88 | 44 3 | - | - | 16 | 16 | 16 | 16 | 16 | 32 | 4 | >64 |
| 57 89 | 44 4 | - | VI M-2 | >128 | >128 | >128 | 128 | >128 | 32 | >32 | >64 |
| 57 | 44 | - | - | 64 | 64 | 64 | 32 | 64 | 8 | 4 | 4 |

[Table 10-4]

| K U B N o. | A R Ba llk # | Harbored carbapene mase genes | | MIC (µg/mL) | | | | | | | |
|---|---|---|---|---|---|---|---|---|---|---|---|
| | | Seri ne-typ e | Met allo-type | Merop enem | Merope nem/ Compou ndA(4) | Merope nem/ Compon ndA(8) | Meropen em/ Compou ndA(16) | Merope nem/ Vaborba ctam(8) | Ceftazi dime/ Avibac tam(4) | Imipene m/ Relebac tam(4) | Ceftolo zane/ Tazobac tam(4) |
| 90 | 5 | | | | | | | | | | |
| 57 91 | 44 7 | - | - | 2 | 2 | 2 | 2 | 2 | 1 | 0.5 | 1 |
| 57 93 | 44 9 | - | - | 16 | 16 | 8 | 8 | 16 | 8 | 1 | >64 |
| 57 98 | 45 5 | - | - | 16 | 16 | 16 | 16 | 16 | 32 | | 8 |
| 57 99 | 45 6 | - | - | 64 | 64 | 64 | 64 | 64 | 8 | 4 | 2 |
| 58 00 | 45 7 | - | VI M-2 | 128 | 128 | 64 | 32 | 128 | 32 | >32 | >64 |
| 58 01 | 45 8 | - | - | 1 | 0.5 | 0.5 | ≤0.25 | 1 | 8 | 0.5 | 32 |
| 58 02 | 45 9 | | - | 16 | 16 | 16 | 16 | 16 | 16 | 4 | 4 |
| 58 04 | 86 2 | - | - | 8 | 16 | 8 | 8 | 64 | 32 | 32 | 16 |
| 58 05 | 86 3 | - | VI M | 128 | 128 | 64 | 32 | 128 | >32 | >32 | >64 |
| 58 06 | 86 4 | - | - | 64 | 64 | 64 | 64 | 64 | 16 | 2 | 4 |
| 58 13 | 50 8 | - | - | 16 | 16 | 16 | 16 | 16 | 32 | 8 | >64 |
| 58 14 | 50 9 | - | VI M-2 | >128 | >128 | >128 | 64 | >128 | >32 | >32 | >64 |
| 58 15 | 51 0 | - | - | 0.5 | 0.5 | ≤0.25 | ≤0.25 | 0.5 | 1 | 0.5 | 1 |
| 58 16 | 51 1 | - | - | 4 | 4 | 4 | 4 | 4 | 1 | 1 | 0.5 |
| 58 17 | 51 2 | - | - | 16 | 16 | 16 | 16 | 16 | 4 | 2 | 1 |
| 58 18 | 51 3 | - | - | 32 | 32 | 16 | 16 | 32 | 8 | 1 | 2 |
| 58 19 | 51 4 | - | - | 4 | 4 | 4 | 4 | 4 | 2 | 1 | 1 |
| 58 20 | 51 5 | - | - | 32 | 32 | 32 | 16 | 1 32 | >32 | 4 | 32 |
| 58 21 | 51 6 | KP C-2 | - | 64 | 32 | 16 | 1 | 8 | 4 | 1 | 32 |
| 58 23 | 51 8 | KP C-2 | - | >128 | 64 | 32 | 16 | 128 | 4 | 32 | 16 |
| 58 31 | 52 6 | - | - | 8 | 4 | 4 | 2 | 8 | 1 | 2 | 1 |
| 58 32 | 52 7 | - | - | 16 | 16 | 16 | 16 | 16 | 4 | 4 | 1 |
| 58 33 | 52 8 | - | - | 8 | 8 | 8 | 4 | 8 | 4 | 2 | 4 |
| 58 34 | 52 9 | - | - | 8 | 8 | 8 | 4 | 8 | 1 | 1 | 1 |

[Table 10-5]

| KU B No. | AR Ba nk # | MIC (µg/mL) | | | | | | | | |
|---|---|---|---|---|---|---|---|---|---|---|
| | | Cefider ocol | Piperacilli n/ Tazobacta m(4) | Aztreon am | Tigecyc line | Colis tin | Amika cin | Ciproflox acin | Levoflox acin | Cefozop ran |
| 555 1 | 54 | 0.5 | >64 | 32 | 16 | 2 | 4 | 32 | 64 | >64 |
| 556 1 | 64 | 2 | 64 | 16 | 2 | 1 | 1 | 16 | 32 | >64 |

(continued)

| KU B No. | AR Ba nk # | MIC (μg/mL) | | | | | | | | |
|---|---|---|---|---|---|---|---|---|---|---|
| | | Cefiderocol | Piperacillin/ Tazobactam(4) | Aztreonam | Tigecycline | Colistin | Amikacin | Ciprofloxacin | Levofloxacin | Cefozopran |
| 558 7 | 90 | 1 | >64 | >64 | 2 | 1 | 32 | 64 | 32 | >64 |
| 558 9 | 92 | 2 | >64 | >64 | 4 | 1 | >64 | 64 | 64 | >64 |
| 559 1 | 94 | 2 | >64 | >64 | 8 | 1 | 32 | 32 | 64 | 64 |
| 559 2 | 95 | 0.5 | 16 | 16 | 2 | 1 | 0.5 | 16 | 32 | 1 |
| 559 7 | 100 | 0.5 | 64 | 32 | 16 | 1 | >64 | 64 | >64 | >64 |
| 560 0 | 103 | 1 | 16 | 16 | 16 | 1 | 32 | 64 | 64 | >64 |
| 560 2 | 105 | 1 | 64 | 32 | 8 | 1 | 8 | >64 | >64 | 16 |
| 560 5 | 108 | 0.5 | >64 | 8 | 8 | 1 | >64 | 32 | 64 | >64 |
| 560 7 | 110 | 0.25 | 64 | 4 | 8 | 1 | >64 | 32 | 32 | >64 |
| 560 8 | 111 | 0.5 | >64 | 2 | 8 | 1 | >64 | 16 | 32 | >64 |
| 566 2 | 229 | 0.12 | >64 | 64 | 16 | 1 | 16 | 32 | 64 | 16 |
| 566 3 | 230 | 0.5 | >64 | 4 | 4 | 1 | >64 | 32 | 32 | >64 |
| 566 4 | 231 | 2 | >64 | >64 | 16 | 1 | 8 | 64 | 64 | >64 |
| 566 5 | 232 | 0.12 | 2 | 4 | 4 | 1 | 8 | 2 | 8 | 4 |
| 566 6 | 23 | 0.12 | 16 | 16 | 8 | 1 | 4 | 0.5 | 2 | 2 |
| 566 7 | 234 | 0.25 | 2 | 2 | 2 | 1 | 8 | 2 | 8 | 2 |
| 566 8 | 235 | 1 | 64 | >64 | 8 | 0.5 | 64 | 32 | 64 | >64 |
| 566 9 | 236 | 0.25 | 4 | 2 | 4 | 1 | 8 | 4 | 16 | 4 |
| 567 0 | 237 | 0.25 | 2 | 4 | 2 | 0.5 | 2 | 0.12 | 0.5 | 0.5 |
| 567 1 | 238 | 1 | 4 | 4 | 4 | 1 | 4 | 0.25 | 0.5 | 1 |
| 567 2 | 239 | 2 | >64 | 32 | 4 | 1 | >64 | 32 | 64 | >64 |
| 567 3 | 240 | 1 | >64 | *32* | 8 | 1 | 64 | 64 | >64 | >64 |
| 567 4 | 241 | 0.25 | 64 | 32 | 4 | 1 | 32 | 16 | 32 | >64 |
| 567 5 | 242 | 0.5 | >64 | 4 | 2 | 0.5 | 64 | 8 | 16 | >64 |
| 567 | 243 | 0.5 | 64 | 2 | 1 | 1 | 32 | 8 | 16 | >64 |

[Table 10-6]

| KU B No. | AR Ba nk # | MIC (μg/mL) I | | | | | | | | |
|---|---|---|---|---|---|---|---|---|---|---|
| | | Cefiderocol | Piperacillin/ Tazobactam(4) | Aztreonam | Tigecycline | Colistin | Amikacin | Ciyprofloxacin | Levofloxacin | Cefozopran |
| 6 | | | | | | | | | | |
| 567 7 | 244 | 0.25 | 16 | 32 | 16 | 1 | 8 | 64 | 64 | 8 |
| 567 8 | 245 | 0.25 | 32 | 16 | 16 | 1 | >64 | 64 | >64 | >64 |
| 567 9 | 246 | 4 | >64 | >64 | 4 | 0.5 | >64 | 16 | 32 | >64 |
| 568 0 | 247 | 0.12 | 4 | 4 | 4 | 0.5 | 4 | 0.25 | 1 | 4 |

(continued)

| KU B No. | AR Ba nk # | MIC (μg/mL) I | | | | | | | | |
|---|---|---|---|---|---|---|---|---|---|---|
| | | Cefider ocol | Piperacilli n/ Tazobacta m(4) | Aztreon am | Tigecyc line | Colis tin | Amika cin | Ciyproflox acin | Levoflox acin | Cefozop ran |
| 568 1 | 248 | 0.5 | >64 | 4 | 2 | 1 | 64 | 8 | 16 | >64 |
| 568 2 | 249 | 0.5 | >64 | 32 | 4 | 0.5 | >64 | 32 | 64 | >64 |
| 508 3 | 250 | 4 | >64 | >64 | | 1 | >64 | 16 | 64 | >64 |
| 568 4 | 251 | 0.12 | 16 | 32 | 4 | 0.5 | 8 | 4 | 8 | 8 |
| 568 5 | 252 | 2 | >64 | 32 | 0.5 | 1 | 0.5 | 8 | 16 | 16 |
| 568 6 | 253 | 0.12 | 16 | 16 | 2 | 1 | | 0.25 | 1 | 4 |
| 568 7 | 254 | *0.5* | 64 | 4 | | 1 | >64 | 16 | 16 | >64 |
| 568 8 | 255 | 0.25 | >64 | 8 | 2 | 1 | >64 | 32 | 32 | >64 |
| 568 9 | 256 | 2 | 4 | 4 | | 0.5 | 2 | 0.12 | 0.5 | 0.5 |
| 569 0 | 257 | 0.25 | 8 | 8 | 8 | 1 | 8 | 32 | 64 | 8 |
| 569 1 | 258 | 0.25 | 4 | 4 | 2 | 0.5 | 4 | 0.12 | 0.5 | 1 |
| 569 2 | 259 | 0.12 | 2 | 4 | 4 | 1 | 2 | 0.12 | 0.5 | 1 |
| 569 3 | 260 | 0.25 | 8 | 8 | 2 | 1 | 64 | 32 | 32 | 4 |
| 5694 | 261 | ≤0.06 | 8 | 4 | 8 | 1 | 16 | 2 | 4 | 4 |
| 569 5 | 262 | 0.12 | 4 | 16 | 2 | 1 | 4 | 0.5 | 2 | 2 |
| 569 6 | 263 | 0.12 | 2 | 2 | 1 | 0.5 | 32 | 1 | 2 | 2 |
| 569 7 | 264 | 0.25 | 2 | 2 | 2 | 1 | 2 | 16 | 32 | 0.5 |
| 569 8 | 265 | 0.5 | 8 | 8 | 8 | 1 | 8 | 32 | 64 | 8 |
| 569 9 | 266 | 0.5 | 8 | 4 | 8 | 1 | 8 | 32 | 32 | 8 |
| 570 0 | 267 | 0.12 | 4 | 4 | 8 | 1 | 16 | 8 | 32 | 4 |
| 570 1 | 268 | 0.25 | >64 | 32 | 2 | 1 | 8 | 4 | 8 | 32 |
| 570 2 | 269 | 1 | 8 | 16 | 4 | 1 | 4 | 16 | 32 | 1 |
| 570 3 | 270 | 0.25 | 2 | 2 | 8 | 1 | 16 | 8 | 32 | 4 |

[Table 10-7]

| KU B No. | AR Ba nk # | MIC (μg/mL) | | | | | | | | |
|---|---|---|---|---|---|---|---|---|---|---|
| | | Cefider ocol | Piperacilli n/ Tazobacta m(4) | Aztreon am | Tigecyc line | Colis tin | Amika cin | Ciproflox acin | Levoflox acin | Cefozop ran |
| 570 4 | 271 | 0.25 | 8 | 16 | 4 | 1 | 4 | 16 | 32 | 1 |
| 570 5 | 272 | 0.25 | 4 | 4 | 2 | 1 | 4 | 8 | 8 | 0.5 |
| 574 7 | 351 | 0.25 | 64 | 64 | 8 | 1 | 64 | 2 | 8 | >64 |
| 574 8 | 352 | 0.5 | >64 | >64 | 8 | 0.5 | 4 | 4 | 32 | >64 |
| 574 9 | 353 | 1 | 64 | 8 | 2 | 0.5 | 64 | 16 | 32 | >64 |
| 575 0 | 354 | 0.5 | >64 | 16 | 4 | 1 | 8 | 4 | 8 | 8 |
| 575 1 | 355 | 0.25 | >64 | 64 | 8 | 1 | 8 | 16 | 32 | 16 |

(continued)

| KU B No. | AR Ba nk # | MIC (µg/mL) | | | | | | | | |
|---|---|---|---|---|---|---|---|---|---|---|
| | | Cefider ocol | Piperacilli n/ Tazobacta m(4) | Aztreon am | Tigecyc line | Colis tin | Amika cin | Ciproflox acin | Levoflox acin | Cefozop ran |
| 575 2 | 356 | 1 | >64 | >64 | 2 | 0.5 | 2 | 0.12 | 0.5 | >64 |
| 575 3 | 357 | 4 | 32 | >64 | 8 | 1 | >64 | 16 | 32 | >64 |
| 575 4 | 358 | 0.5 | >64 | 32 | 4 | 1 | 4 | 4 | 8 | 16 |
| 575 5 | 359 | 0.25 | >64 | 32 | 4 | 1 | 4 | 4 | 8 | 16 |
| 575 6 | 360 | 0.25 | >64 | 16 | 4 | 1 | 4 | 0.12 | 1 | 8 |
| 578 5 | 439 | 0.5 | 4 | 4 | 4 | 0.5 | >64 | 16 | 32 | >64 |
| 578 6 | 440 | 0.25 | >64 | 32 | 8 | 1 | 8 | 16 | 32 | 32 |
| 578 7 | 441 | 1 | >64 | >64 | 4 | 0.5 | 2 | 0.12 | 0.5 | >64 |
| 578 8 | 443 | 1 | 32 | >64 | 4 | 1 | 64 | 32 | 64 | >64 |
| 578 9 | 444 | 0.5 | >64 | 8 | 4 | 1 | >64 | 32 | 32 | >64 |
| 579 0 | 445 | 0.5 | >64 | >64 | 4 | 1 | 64 | 16 | 32 | 32 |
| 579 | 447 | 0.12 | 4 | 4 | 4 | 1 | 8 | 0.12 | 0.5 | 0.5 |
| 579 3 | 449 | 0.5 | >64 | >64 | 4 | 1 | 8 | 16 | 32 | >64 |
| 579 8 | 455 | 0.25 | >64 | >64 | 1 | 0.25 | 0.25 | 1 | 4 | 32 |
| 579 9 | 456 | 0.25 | 64 | 64 | 8 | 1 | 8 | 64 | >64 | 32 |
| 580 0 | 457 | 0.25 | 64 | 32 | 2 | 0.5 | 2 | 32 | 64 | >64 |
| 580 1 | 458 | 2 | 64 | 64 | 8 | 0.5 | 16 | 0.5 | 2 | 64 |
| 580 2 | 459 | 0.12 | >64 | 64 | 4 | 0.5 | 8 | 4 | 8 | 32 |
| 580 4 | 862 | 0.5 | >64 | 64 | 8 | 1 | 4 | 1 | 4 | 32 |
| 580 5 | 863 | 0.5 | 64 | 16 | 8 | 1 | >64 | 32 | 64 | >64 |
| 580 | 864 | 0.12 | >64 | >64 | 2 | 1 | 0.5 | >64 | >64 | 64 |

[Table 10-8]

| KU B No. | AR Ba nk # | MIC (ng/mL) I | | | | | | | | |
|---|---|---|---|---|---|---|---|---|---|---|
| | | Cefider ocol | Piperacilli n/ Tazobacta m(4) | Aztreon am | Tigecyc line | Colis tin | Amika cin | Ciproflox acin | Levoflox acin | Cefozop ran |
| 6 | | | | | | | | | | |
| 581 3 | 508 | 1 | 64 | >64 | 16 | 0.5 | 64 | 2 | 8 | >64 |
| 581 4 | 509 | 0.5 | >64 | 8 | 8 | 1 | >64 | 32 | 32 | >64 |
| 581 5 | 510 | ≤0.06 | 0.5 | 0.5 | 2 | 0.5 | 8 | 4 | 8 | 4 |
| 581 6 | 511 | 0.25 | 8 | 4 | 4 | 0.5 | 4 | 0.12 | 1 | 0.5 |
| 581 7 | 512 | 1 | 16 | 32 | 16 | 1 | 4 | 1 | 4 | 2 |
| 581 8 | 513 | 0.25 | >64 | 32 | 8 | 1 | 32 | 64 | >64 | 16 |
| 581 9 | 514 | 0.12 | 4 | 4 | 4 | 1 | 4 | 0.12 | 1 | 0.5 |
| 582 0 | 515 | 1 | 64 | 32 | 8 | 1 | 8 | >64 | >64 | 64 |

(continued)

| KU B No. | AR Ba nk # | MIC (ng/mL) I | | | | | | | | |
|---|---|---|---|---|---|---|---|---|---|---|
| | | Cefider ocol | Piperacilli n/ Tazobacta m(4) | Aztreon am | Tigecyc line | Colis tin | Amika cin | Ciproflox acin | Levoflox acin | Cefozop ran |
| 582 1 | 516 | 0.5 | >64 | >64 | 4 | 1 | 2 | 0.12 | 1 | >64 |
| 582 3 | 518 | 0.25 | >64 | *>64* | 16 | 1 | 16 | 64 | 64 | >64 |
| 583 1 | 526 | 0.12 | 32 | 4 | 4 | 1 | 4 | 16 | 32 | 8 |
| 583 2 | 527 | 0.25 | 64 | 32 | 2 | 0.5 | 0.5 | 16 | 32 | 8 |
| 583 3 | 528 | 0.25 | >64 | 64 | 4 | 1 | 8 | 16 | 32 | 32 |
| 583 4 | 529 | ≤0.06 | 64 | 8 | 4 | 1 | 4 | 16 | 16 | 8 |

(Discussion)

Compound A enhanced an in vitro antibacterial activity of MEPM against Enterobacterales and A. baumannii including carbapenem-resistant strains in a dose-dependent manner. The results indicate that the Compound A also inhibits various β-lactamases other than the serine- or metallo-β-lactamases tested in Example 1. In other words, Inhibitory activity against OXA-58, 64, 69, 71, 82, 94, 100, 181, 203, 223, 232, 237, KPC-4, 5, 6, 11, GES-1, SME-3, NMC-A for serine carbapenemases, and IMP-4, 8, VIM-11, 27 for metallo-carbapenemases was demonstrated.

MEPM/Compound A showed higher efficacy against Enterobacterales than all control agents and a superior effect against A. baumannii to all other control agents than cefiderocol, tigecycline, or colistin. A detail thereof will be described below. That is, Tables 11 and 12 were made based on individual data in Table 8, Tables 13 and 14 were made using individual data in Table 9, and Table 15 was made using individual data in Table 10, which are discussed below.

Discussion of antibacterial activity against Enterobacterales

The $MIC_{50}$, the $MIC_{80}$, and the $MIC_{90}$ of MEPM against 154 strains of Enterobacterales including E. coli, K. pneumoniae, and E. cloacae were 2, 16, and 32 μg/mL, respectively (Table 11). When Compound A at fixed concentrations of 4, 8, and 16 μg/mL was used in combination with the MEPM, the $MIC_{50}$, the $MIC_{80}$, and the $MIC_{90}$ of the MEPM were decreased to ≤0.06, ≤0.06 to 0.12, and 4 to 0.25 μg/mL, respectively. Addition of the Compound A at 8 ug/mL or more reduced the $MIC_{90}$ of the MEPM to equal to or lower than a susceptibility breakpoint (1 μg/mL) for the MEPM alone, which was lower than the $MIC_{90}$ of all control agents such as MEPM/vaborbactam, ceftazidime/avibactam, imipenem/relebactam, ceftolozane/tazobactam, and cefiderocol. The $MIC_{90}$s of the control agents were above the respective susceptibility breakpoints except for cefiderocol, tigecycline, and cefozopran for which no breakpoint MIC was defined.

[Table 11]

| **Table 11. Antibacterial activity of MEPM/Compound A and control agents against 154 strains of *Enterobacterales*** | | | | | | | |
|---|---|---|---|---|---|---|---|
| **Antibacterial agent** | **MIC (μg/mL)** | | | | **CLSI breakpoint(μg/mL)** | | |
| | $MIC_{range}$ | $MIC_{50}$ | $MIC_{80}$ | $MIC_{90}$ | **Susce ptible** | **Interm ediate** | **Resistant** |
| **MEPM** | ≤0.06 - >32 | 2 | 16 | 32 | ≤1 | 2 | >4 |
| **MEPM/Compoun d A(4)** | <0.06 - >32 | ≤0.06 | 0.12 | 4 | - | - | - |
| **MEPM/Compoun d A(8)** | ≤0.06 - >32 | ≤0.06 | ≤0.06 | 1 | - | - | - |
| **MEPM/Compoun d A(16)** | ≤0.06 - >32 | <0.06 | ≤0.06 | 0.25 | - | - | - |
| **MEPM/ Vaborbactam(8)** | ≤0.06 - >32 | 0.12 | 8 | 32 | ≤4 | 8 | ≥16 |
| **Ceftazidime/ avibactam(4)** | 0.25 - >32 | 1 | >32 | >32 | ≤8 | - | ≥16 |
| **Imipenem/ relebactam(4)** | ≤0.06 - >32 | 0.5 | 8 | 16 | ≤1* | 2* | ≥4* |

(continued)

| Table 11. Antibacterial activity of MEPM/Compound A and control agents against 154 strains of *Enterobacterales* | | | | | | | |
|---|---|---|---|---|---|---|---|
| **Antibacterial agent** | **MIC (μg/mL)** | | | | **CLSI breakpoint(μg/mL)** | | |
| | $MIC_{range}$ | $MIC_{50}$ | $MIC_{80}$ | $MIC_{90}$ | **Susceptible** | **Intermediate** | **Resistant** |
| **Ceftolozane/ tazobactam(4)** | 0.25 - >64 | >64 | >64 | >64 | ≤2 | 4 | ≥8 |
| **Cefiderocol** | ≤0.06 - 16 | 0.5 | 1 | 2 | ≤4 | 8 | ≥16 |
| **Piperacillin/ tazobactam(4)** | 0.25 - >64 | >64 | >64 | >64 | ≤16 | 32 - 64 | ≥128 |
| **Aztreonam** | ≤0.03 - >64 | >64 | >64 | >64 | ≤4 | 8 | ≥16 |
| **Tigecycline** | 0.12 - 4 | 0.5 | 1 | 2 | ≤2* | 4* | ≥8* |
| **Colistin** | 0.25 - >64 | 0.5 | 8 | >64 | - | ≤2 | ≥4 |
| **Amikacin** | 0.25 - >64 | 4 | 32 | >64 | ≤16 | 32 | ≥64 |
| **Ciprofloxacin** | ≤0.03 - >64 | 64 | >64 | >64 | ≤0.25 | 0.5 | ≥1 |
| **Levofloxacin** | 0.06 - >64 | 16 | 64 | 64 | ≤0.5 | 1 | ≥2 |
| **Cefozopran** | ≤0.03 - >64 | 64 | >64 | >64 | - | - | - |

**Concentration of β -lactamase inhibitor in combination with β-lactam is shown in parentheses.**
* **Breakpoint provided by the FDA was referenced.(** Antibacterial Susceptibility Test Interpretive Criteria. FDA. https://www.fda.gov/drugs/development-resources/antibacterial-susceptibilitytestinterpretive-criteria)

The MIC of MEPM/Compound A against serine-carbapenemase- and metallo-β-lactamase-producing strains showed results with a similar trend to those against all test strains (Table 12). The $MIC_{90}$ of MEPM against the serine carbapenemase-producing strains when Compound A at 4 ug/mL was added thereto was lower than the $MIC_{90}$ of all control agents, and the $MIC_{90}$ of cefiderocol (4 μg/mL), tigecycline (2 μg/mL), and colistin (8 μg/mL) against the metallo-β-lactamase-producing strains were lower than the $MIC_{90}$ of the MEPM when the Compound A at 4 ug/mL was added thereto. The results are shown in Table 12.

[Table 12-1]

| Table 12. Antibacterial activity of MEPM/Compound A and control agents against carbapenemase-producing *Enterobacterales* | | | | | | |
|---|---|---|---|---|---|---|
| | **Antibacterial agent** | **$MIC_{50}$ (μg/mL)** | **$MIC_{90}$ (μg/mL)** | **Antibacterial agent** | **$MIC_{50}$ (μg/mL)** | **$MIC_{90}$ (μg/mL)** |
| **Serine carbape nemase [a] (N=61)** | MEPM | 4 | 32 | | | |
| | MEPM/Compound A (4) | ≤0.06 | ≤0.06 | **Piperacillin/ Tazobactam (4)** | >64 | >64 |
| | MEPM/Compound A (8) | ≤0.06 | ≤0.06 | **Aztreonam** | >64 | >64 |
| | MEPM/Compound A (16) | ≤0.06 | ≤0.06 | **Tigecycline** | 1 | 2 |
| | MEPM/ **vaborbactam** (8) | ≤0.06 | 1 | **Colistin** | 0.5 | >64 |
| | **Ceftazidimel Avibactam(4)** | 0.5 | 2 | **Amikacin** | 4 | 64 |
| | **Imipenem/ Relebactam(4)** | 0.25 | 2 | **Ciprofloxa cin** | 32 | >64 |
| | **Ceftolozane/ Tazobactam(4)** | 32 | >64 | **Levofloxa cin** | 16 | 64 |
| | **Cefiderocol** | 0.25 | l | **Cefozopran** | 32 | >64 |
| **Metallo -*β*-lactam ase [b]** | MEPM | 16 | >32 | | | |
| | MEPM/Compound A (4) | ≤0.06 | 32 | **Piperacillin /** | >64 | >64 |

[Table 12-2]

| | **Antibacterial agent** | **$MIC_{50}$ (μg/mL)** | **$MIC_{90}$ (μg/mL)** | **Antibacterial agent** | **$MIC_{50}$ (μg/mL)** | **$MIC_{90}$ (μg/mL)** |
|---|---|---|---|---|---|---|
| **(N = 47)** | | | | **Tazobactam (4)** | | |
| | MEPM/Compound A (8) | ≤0.06 | 8 | **Aztreonam** | >64 | >64 |
| | MEPM/Compound A (16) | ≤0.06 | 1 | **Tigecycline** | 0.5 | 2 |
| | MEPM/ **vaborbactam** (8) | 16 | >32 | **Colistin** | 0.5 | 8 |
| | **Ceftazidime/ Avibactam(4)** | >32 | >32 | **Amikacin** | 16 | >64 |
| | **Imipenem/ Relebactam(4)** | 8 | >32 | **Ciprofloxa cin** | 64 | >64 |
| | **Ceftolozane/ Tazobactam(4)** | >64 | >64 | **Levofloxa cin** | 32 | >64 |
| | **Cefiderocol** | 1 | 4 | **Cefozopran** | >64 | >64 |

Discussion of antibacterial activity against A. baumannii

The $MIC_{50}$, the $MIC_{80}$, and the $MIC_{90}$ of MEPM against 55 strains of A. baumannii were 32, 128, and >128 μg/mL, respectively (Table 13). When Compound A at fixed concentrations of 4 to 16 ug/mL was used in combination, the Compound A restored an activity of MEPM, and the $MIC_{50}$, the $MIC_{80}$, and the $MIC_{90}$ were decreased to 4, 16, and 32 ug/mL for MEPM/Compound A (4 μg/mL), 4, 4, and 8 μg/mL for MEPM/Compound A (8 μg/mL), and 2, 2, and 4 μg/mL for MEPM/Compound A (16 μg/mL), respectively. Addition of the Compound A at 16 μg/mL reduced the $MIC_{80}$ of the MEPM to a susceptibility breakpoint of the MEPM (2 μg/mL). The $MIC_{80}$ of cefiderocol and colistin were lower than susceptibility and intermediate breakpoints, while the $MIC_{80}$ of MEPM/vaborbactam, ceftazidime/avibactam, imipenem/relebactam, and ceftolozane/tazobactam were equal to or higher than the susceptibility breakpoint. Almost all (54/55) strains of A. baumannii possessed at least one OXA-carbapenemase gene, and 4 of the 55 strains possessed NDM-1, a metallo-carbapenemase. A distribution of MIC values for each agent against the 4 NDM-1-producing strains is shown in Table 14. A MIC distribution of MEPM/Compound A (8 μg/mL) was lower than those of the comparators including MEPM/vaborbactam, ceftazidime/avibactam, imipenem/relebactam, ceftolozane/tazobactam, and cefiderocol and only tigecycline and colistin showed lower MIC distributions.

[Table 13-1]

**Table 13. Antibacterial activity of MEPM/Compound A and control agents against 55 isolates of *A. baumannii***

| Antibacterial agent | MIC (μg/mL) | | | | CLSI Breakpoints (μg/mL) | | |
|---|---|---|---|---|---|---|---|
| | $MIC_{range}$ | $MIC_{50}$ | $MIC_{80}$ | $MIC_{90}$ | Susce ptible | Interm ediate | Resistant |
| **MEPM** | 2 - >128 | 32 | 128 | >128 | ≤2 | 4 | ≥8 |
| **MEPM/Comp ound A(4)** | 1 - 128 | 4 | 16 | 32 | - | - | - |
| **MEPM/Comp ound A(8)** | 1 -32 | 4 | 4 | 8 | - | - | - |
| **MEPM/Comp ound A(16)** | ≤0.25 - 4 | 2 | 2 | 4 | - | - | - |
| **MEPM/ Vaborbactam(8)** | 2 - >128 | 32 | 128 | >128 | - | - | - |
| **Ceftazidime/ Avibactam(4)** | 4 - >32 | 32 | >32 | >32 | - | - | - |
| **Imipenem/ Relebactam (4)** | **0.5** - >32 | 32 | >32 | >32 | ≤2 * | 4 * | ≥8* |
| **Ceftolozane/ Tazobactam (4)** | 2 - >64 | 32 | >64 | >64 | - | - | - |
| **Cefiderocol** | ≤0.06 - 16 | 0.5 | 2 | 4 | ≤4 | 8 | ≥16 |
| **Piperacillin/ Tazobactam (4)** | 64 - >64 | >64 | >64 | >64 | <16 | 32-64 | >128 |
| **Aztreonam** | 32 - >64 | 64 | >64 | >64 | - | - | - |
| **Tigecycline** | 0.25 - 2 | 1 | 1 | 1 | - | - | - |
| **Colistin** | 0.25 - >64 | 1 | 2 | 2 | - | ≤2 | ≥4 |
| **Amikacin** | 0.5 - >64 | 64 | >64 | >64 | ≤16 | 32 | ≥64 |
| **Ciprofloxacin** | 8 - >64 | 64 | >64 | >64 | ≤1 | 2 | ≥4 |

[Table 13-2]

| Antibacterial agent | MIC (μg/mL) | | | | CLSI Breakpoints (μg/mL) | | |
|---|---|---|---|---|---|---|---|
| | $MIC_{range}$ | $MIC_{50}$ | $MIC_{80}$ | $MIC_{90}$ | Susce ptible | Interm ediate | Resistant |
| | | | | | | | |
| **Levofloxacin** | 4 - >64 | 16 | 32 | 64 | ≤2 | 4 | ≥8 |
| **Cefozopran** | 2 - >64 | 64 | >64 | >64 | - | - | - |

**Concentration of *β*-lactamase inhibitor (μg/mL) in combination with *β* -lactam is shown in parentheses. * Breakpoint of imipenem provided by the FDA was referenced since breakpoint is not described in the CLSI [4].**

[Table 14]

**Table 14. MIC distribution against metallo- *β* -lactamase-producing *A. baumannii***

| **Number of isolates at MIC** | | | | | | | | | | | |
|---|---|---|---|---|---|---|---|---|---|---|---|
| **MIC (μg/mL)** | ≤0.25 | 0.5 | 1 | 2 | 4 | 8 | 16 | 32 | >32 | >64 | ≥128 |
| **MEPM** | - | - | - | - | - | - | - | - | - | - | 4 |
| **MEPM/Compound A(4)** | - | - | - | - | 3 | - | 1 | - | - | - | - |
| **MEPM/Compound A(8)** | - | - | - | 4 | - | - | - | - | - | - | - |

(continued)

| Table 14. MIC distribution against metallo- β -lactamase-producing *A. baumannii* | | | | | | | | | | |
|---|---|---|---|---|---|---|---|---|---|---|
| **Number of isolates at MIC** | | | | | | | | | | |
| **MEPM/Compound A(16)** | - | 1 | 3 | - | - | - | - | - | - | - | - |
| **MEPM/Vaborbactam(8)** | - | - | - | - | - | - | - | - | - | - | 4 |
| **Ceftazidime/avibactam(4)** | - | - | - | - | - | - | - | - | 4 | n.t. | n.t |
| **Imipenem/relebactam(4)** | - | - | - | - | - | - | - | - | 4 | n.t. | n.t. |
| **Ceftolozane/tazobactam(4)** | - | - | - | - | - | - | - | - | - | 4 | n.t. |
| **Cefiderocol** | - | - | - | 2 | 1 | - | 1 | - | - | - | n.t. |
| **Piperacillin/tazobactam(4)** | - | - | - | - | - | - | - | - | - | 4 | n.t. |
| **Aztreonam** | - | - | - | - | - | - | - | 1 | - | 3 | n.t. |
| **Tigecycline** | 3 | 1 | - | - | - | - | - | - | - | - | n.t. |
| **Colistin** | - | - | 4 | - | - | - | - | - | - | - | n.t. |
| **Amikacin** | - | - | - | 2 | - | - | - | 1 | - | 1 | n.t.- |
| **Ciprofloxacin** | - | - | - | - | - | - | 1 | 1 | 1 | 1 | n.t. |
| **Levofloxacin** | - | - | - | - | - | 3 | 1 | - | - | - | n.t. |
| **Cefozopran** | - | - | - | - | - | - | - | - | - | 4 | n.t. |

Discussion of antibacterial activity against P. aeruginosa

The $MIC_{50}$, the $MIC_{80}$, and the $MIC_{90}$ of MEPM against 96 strains of P. aeruginosa were 16, 64, and >128 μg/mL, respectively. Addition of 16 ug/mL of Compound A resulted in a two-times decrease in the $MIC_{90}$. Only cefiderocol and colistin showed low MIC values against P. aeruginosa (Table 15).

An in vitro antibacterial activity of MEPM/Compound A was analyzed for the MIC distribution, the $MIC_{range}$, the $MIC_{50}$, the $MIC_{80}$, and the $MIC_{90}$ against metallo-carbapenemase gene-harboring strains, MEPM/Compound A showed low MIC values against some strains but moderate to high MIC values against many strains, and a combined effect of MEPM with Compound A was only about two times decrease regardless of presence or absence of the metallo-carbapenemase gene.

[Table 15-1]

**Table 15. Activity of MEPM/Compound A and control agents against 96 strains of *P. aeruginosa and* 26 metallo-carbapenemase-producing strains**

| Antibacterial agent | MIC against 96 strains of *P. aeruginosa* μg(mL) | | | | MIC against 26 metallo-carbapenemase-producing strains μg(mL) | | | | CLSI breakpoint (μg/mL) | | |
|---|---|---|---|---|---|---|---|---|---|---|---|
| | $MIC_{range}$ | $MIC_{50}$ | $MIC_{80}$ | $MIC_{90}$ | $MIC_{rang\ e}$ | $MIC_{50}$ | $MIC_{80}$ | $MIC_{90}$ | Susce Interm Resis ptible ediate tent | | |
| **MEP M** | ≤0.25 - >128 | 16 | 64 | >128 | 4 - >128 | 128 | >128 | >128 | ≤2 | 4 | ≥8 |
| **MEP M/Co mpoun d A (4)** | ≤0.25 - >128 | 8 | 64 | 128 | 2 - >128 | 128 | >128 | >128 | - | - | - |
| **MEP M/Co mpoun d A (8)** | ≤0.25 - >128 | 8 | 64 | 128 | 1 - >128 | 64 | >128 | >128 | - | - | - |
| **MEP M/Co mpoun d A** | ≤0.25 - >128 | 8 | 32 | 64 | ≤0.25 - >128 | 32 | 128 | >128 | - | - | - |

[Table 15-2]

| | | | | | | | | | | | |
|---|---|---|---|---|---|---|---|---|---|---|---|
| **(16)** | | | | | | | | | | | |
| **MEP M/Va borba ctam (8)** | ≤0.25 - >128 | 8 | 64 | >128 | 4 - >128 | 128 | >128 | >128 | - | - | - |
| **Ceftazid ime/Avi bactam (4)** | 0.5 - >32 | 4 | 32 | >32 | 8 - >32 | >32 | >32 | >32 | <8 | | ≥16 |
| **Imipene m/Rele bactam (4)** | 0.25 - >32 | 2 | >32 | >32 | 8 - >32 | >32 | >32 | >32 | ≤2 | 4 | ≥8 |
| **Ceftoloz ane/-Taz obactam (4)** | 0.5 - >64 | 4 | >64 | >64 | >64 | >64 | >64 | >64 | <4 | 8 | ≥16 |
| **Cefiderocol** | ≤0.06 - 4 | 0.5 | 1 | 2 | 0.25 - 4 | 0.5 | 1 | 2 | ≤4 | 8 | ≥16 |
| **Piperacil lin/-Tazo bactam (4)** | 0.5 - >64 | 64 | >64 | >64 | 4 - >64 | >64 | >64 | >64 | <16 | 32-64 | >128 |
| **Aztreonam** | 0.5 - >64 | 16 | 64 | >64 | 2 - >64 | 16 | 32 | >64 | <8 | 16 | ≥32 |
| **Tigecycline** | 0.5 - 16 | 4 | 8 | 16 | 1 - 16 | 4 | 8 | 16 | - | - | - |
| **Colistin** | 0.25 - 2 | 1 | 1 | 1 | 0.5 - 2 | 1 | 1 | 1 | - | ≤2 | ≥4 |
| **Amikacin** | 0.25 - >64 | 8 | 64 | >64 | 1 - >64 | >64 | >64 | >64 | ≤16 | 32 | ≥64 |
| **Ciprofloxacin** | 0.12 - >64 | 16 | 32 | 64 | 8 - >64 | 32 | 32 | 64 | ≤0.5 | 1 | ≥2 |
| **Levofloxacin** | 0.5 - >64 | 32 | 64 | 64 | 16 - >64 | 32 | 64 | >64 | ≤1 | 2 | ≥4 |
| **Cefozopran** | 0.5 - >64 | 32 | >64 | >64 | >64 | >64 | >64 | >64 | - | - | - |
| **Concentration of $\beta$-lactamase inhibitor ($\mu$g/mL) in combination with $\beta$-lactam is shown in parentheses.** | | | | | | | | | | | |

(Example 8) In vitro antibacterial activity against Gram-negative bacteria clinically isolated in the United States

(Method and Result)

An antibacterial activity of MEPM/Compound A was evaluated against Klebsiella pneumoniae, E. coli, A. baumannii, and P. aeruginosa clinically isolated in the United States according to the method of the Clinical and Laboratory Standards Institute (CLSI) using the minimal inhibitory concentration (MIC) as an indicator (Table 16). No enhancement of an MEPM activity of Compound A against E. coli was observed because MEPM alone inhibited proliferation of many isolates (73/80) at 0.125 μg/mL, which was the lowest concentration of MEPM in this study. Addition of the Compound A enhanced an activity of the MEPM against Klebsiella pneumoniae, with the $MIC_{50}$ and the $MIC_{90}$ of the MEPM/Compound A (both 4 and 8 μg/mL) being ≤0.125 μg/mL, which was equal to or less than the susceptibility breakpoint MIC (1 μg/mL) of the MEPM alone described in the CLSI M100. For A. baumannii, the Compound A at both 4 μg/mL and 8 μg/mL reduced the $MIC_{50}$ and the $MIC_{90}$ of the MEPM to 1 and 4 μg/mL, respectively, which was equal to or lower than the intermediate breakpoint MIC (4 μg/mL) for MEPM alone. The MEPM/Compound A at 4 ug/mL and the MEPM/Compound A at 8 μg/mL had limited activity against P. aeruginosa ($MIC_{50}$/$MIC_{90}$, 2/64 and 2/32 μg/mL), but the $MIC_{50}$ was the susceptibility breakpoint MIC for the MEPM alone (2 μg/mL).

[Table 16-1]

**Table 16 Antibacterial activity of MEPM/Compound A against Gram-negative bacterial strains isolated in the United States**

| Agent | MIC (ug/mL) | | | CLSI breakpoint (ug/mL) | | |
|---|---|---|---|---|---|---|
| | $MIC_{range}$ | $MIC_{50}$ | $MIC_{90}$ | Susceptible | Intermediate | Resistant |
| ***E. coli*** **(80 strains)** | | | | | | |
| **MEPM** | ≤0.125 - 16 | ≤0.125 | ≤0.125 | ≤1 | 2 | ≥4 |
| **MEPM/Compound A (4)** | ≤0.125 - 16 | ≤0.125 | ≤0.125 | | | |
| **MEPM/Compound A (8)** | ≤0.125 - 16 | ≤0.125 | ≤0.125 | | | |
| ***K. pneumoniae*** **(80 strains)** | | | | | | |

[Table 16-2]

| | | | | | | |
|---|---|---|---|---|---|---|
| **MEPM** | ≤0.125 - >64 | ≤0.125 | 4 | ≤1 | 2 | ≥4 |
| **MEPM/Compound A (4)** | ≤0.125 - 64 | ≤0.125 | ≤0.125 | | | |
| **MEPM/Compound A (8)** | ≤0.125 - 2 | ≤0.125 | ≤0.125 | | | |
| ***A. baumannii*** **(80 strains)** | | | | | | |
| **MEPM** | ≤0.125 - >64 | 2 | 64 | ≤2 | 4 | ≥8 |
| **MEPM/Compound A (4)** | 0.25 - 8 | 1 | 4 | | | |
| **MEPM/Compound A (8)** | ≤0.125 - 8 | 1 | 4 | | | |
| *P. aeruginosa* **(80 strains)** | | | | | | |
| **MEPM** | ≤0.125 - >64 | 2 | 32 | ≤2 | 4 | ≥8 |
| **MEPM/Compound A (4)** | ≤0.125 - >64 | 2 | 64 | | | |
| **MEPM/Compound A (8)** | ≤0.125 - >64 | 2 | 32 | | | |
| **Concentration of Compound A (μg/mL) in combination with MEPM is shown in parentheses.** | | | | | | |

(Example 9) Antibacterial activity of MEPM/Compound A against anaerobic bacteria

An antibacterial activity of meropenem/Compound A against anaerobic bacteria was evaluated by an agar dilution method according to Clinical and Laboratory Standards Institute (CLSI) M11 9th ed. (2018) using the minimal inhibitory concentration (MIC) as an indicator.

A total of 196 anaerobic bacterial strains from the American Type Culture Collection and clinically isolated in Japan between 2010 and 2015 were used. Test strains were cultured on a Brucella agar supplemented with vitamin $K_1$, hemin, and sheep defibrinated blood at 35 to 36°C for 2 to 4 days under an anaerobic environment. The thus-grown test strains were smeared on the same fresh agar medium and stationary cultured under an anaerobic environment at 35 to 36°C for 1 to 2 days. The resultant was then suspended in a Brucella broth and adjusted to 0.2 to 0.6 McFarland. Meropenem was dissolved in distilled water, Compound A was dissolved in distilled water with sodium carbonate, and metronidazole was dissolved in dimethyl sulfoxide and the resultants were diluted to target concentrations with distilled water. One milliliter of each of a meropenem solution, a mixed solution of meropenem and Compound A, and a metronidazole solution at various concentrations was mixed with 9 mL of a Brucella agar supplemented with vitamin $K_1$, hemin, and sheep defibrinated blood and allowed to solidify at room temperature. Each bacterial suspension was inoculated onto an agar plate at approximately 2 μL/spot using a microplanter and cultured at 35 to 36°C for 42 to 48 hours under an anaerobic environment. The MIC was defined as the lowest concentration at which growth on a test agar medium was significantly reduced compared to growth on an anaerobic positive growth control agar medium according to the CLSI.

An antibacterial activity of meropenem against each anaerobic bacterium was as described below (Tables 17 to 26). Bacteroides spp. (n=56; $MIC_{50}$, 0.5 μg/mL; $MIC_{90}$, 4 μg/mL), Prevotella spp. (n=54; $MIC_{50}$, 0.06 μg/mL; $MIC_{90}$, 0.12 μg/mL), Porphyromonas spp. (n=26; $MIC_{50}$, ≤0.03 μg/mL; $MIC_{90}$, ≤0.03 μg/mL), Fusobacterium spp. (n=20; $MIC_{50}$, ≤0.03 μg/mL; $MIC_{90}$, ≤0.03 μg/mL), and Gram-positive bacteria (n=40; $MIC_{50}$, 0.06 μg/mL; $MIC_{90}$, 2 μg/mL). Except for

Porphyromonas spp. ($MIC_{50}$, ≤0.03 μg/mL; $MIC_{90}$, ≤0.03 μg/mL) against which meropenem alone exhibits sufficient efficacy, addition of Compound A at 4 ug/mL enhanced efficacy of meropenem against Bacteroides spp. ($MIC_{50}$, 0.12 μg/mL; $MIC_{90}$, 1 μg/mL), Prevotella spp. ($MIC_{50}$, ≤0.03 μg/mL; $MIC_{90}$, 0.06 μg/mL), Fusobacterium spp. ($MIC_{50}$, ≤0.03 μg/mL; $MIC_{90}$, ≤0.03 μg/mL), and Gram-positive bacteria (n=40; $MIC_{50}$, ≤0.03 μg /mL; $MIC_{90}$, 2 μg/mL). The Compound A did not antagonize an anti-anaerobic bacterial activity of the meropenem. The Compound A at 4, 8, and 16 μg/mL alone showed an anti-anaerobic bacterial activity against some anaerobic bacteria in a dose-dependent manner and enhanced an activity of the meropenem.

[Table 17-1]

| Table 17 Antibacterial activity of meropenem/Compound A against *Bacteroides* spp. | | | | | | |
|---|---|---|---|---|---|---|
| KUB No. | Organism | MIC (μg/mL) | | | | |
| | | Meropenem | Meropenem/ CompoundA( 4) | Meropenem/ CompoundA( 8) | Meropenem/ CompoundA( 16) | Metronidazole |
| 6458 | *Bacteroides fragilis* | 0.5 | 0.06 | 0 | 0 | 0.5 |
| 6459 | *Bacteroides stercoris* | 0.25 | 0.12 | 0.06 | ≤0.03 | 1 |
| 6460 | *Bacteroides thetaiotaomicron* | 2 | 1 | 0.5 | 0.25 | 2 |
| 6461 | *Bacteroides fragilis* | 0.5 | 0.06 | 0 | 0 | 0.5 |
| 6463 | Bacteroides fragilis | 0.12 | 0.06 | ≤0.03 | 0 | 0.5 |
| 6464 | *Bacteroides vulgarus* | 0.12 | 0.06 | ≤0.03 | 0 | 0.5 |
| 6465 | *Bacteroides thetaiotaomicron* | 0.5 | 0.25 | 0.12 | ≤0.03 | 2 |
| 6466 | *Bacteroides fragilis* | 4 | 0.25 | 0.25 | ≤0.03 | 1 |
| 6467 | *Bacteroides fragilis* | 0.12 | ≤0.03 | 0 | 0 | 0.5 |
| 6468 | *Bacteroides fragilis* | 4 | 0.12 | 0.06 | 0 | 1 |
| 6469 | *Bacteroides fragilis* | 0.25 | 0.06 | ≤0.03 | ≤0.03 | 1 |
| 6470 | Bacteroides fragilis | 0.25 | 0.12 | 0.12 | 0.12 | 2 |
| 6471 | Bacteroides *fragilis* | 0.12 | 0.06 | 0 | 0 | 1 |

[Table 17-2]

| KUB No. | Organism | MIC (μg/mL) | | | | |
|---|---|---|---|---|---|---|
| | | Meropenem | Meropenem/ CompoundA( 4) | Meropenem/ CompoundA( 8) | Meropenem/ CompoundA( 16) | Metronidazole |
| 6472 | *Bacteroides stercoris* | 1 | 0.06 | 0.06 | ≤0.03 | 0.5 |
| 6473 | *Bacteroides fragilis* | 0.25 | ≤0.03 | 0 | 0 | 1 |
| 6474 | *Bacteroides fragilis* | 1 | 0.12 | 0 | 0 | 0.5 |

(continued)

| KUB No. | Organism | MIC (μg/mL) | | | | |
|---|---|---|---|---|---|---|
| | | Meropenem | Meropenem/ CompoundA( 4) | Meropenem/ CompoundA( 8) | Meropenem/ CompoundA( 16) | Metronidazole |
| 6475 | *Bacteroides the-taiotaomicron* | 2 | 1 | 0.5 | 0.25 | 1 |
| 6476 | *Bacteroides vulgatus* | 0.5 | 0.25 | 0.12 | 0.12 | 1 |
| 6477 | *Bacteroides fragilis* | 4 | 1 | 0.12 | 0 | ≤0.12 |
| 6478 | *Bacteroides fragilis* | 0.12 | ≤0.03 | 0 | 0 | 0.25 |
| 6480 | *Bacteroides thetaioraomicron* | 1 | 0.5 | 0.25 | 0.12 | 1 |
| 6481 | *Bacteroides fragilis* | 0.12 | ≤0.03 | 0 | 0 | 1 |
| 6483 | *Bacteroides fragilis* | 0.12 | ≤0.03 | 0 | 0 | 0.25 |
| 6484 | *Bacteroides fragilis* | 0.25 | 0.06 | ≤0.03 | 0 | 0.5 |
| 6485 | *Bacteroides vulgatus* | 8 | 2 | 0.12 | 0 | 0.25 |
| 6486 | *Bacteroides fragilis* | 0.12 | ≤0.03 | 0 | 0 | 1 |
| 6487 | *Bacteroides thetaiotamicron* | 2 | 1 | 0.25 | 0.12 | 0.25 |
| 6490 | *Bacteroides fragilis* | 64 | 1 | 0 | 0 | 0.5 |
| 6491 | *Bacteroides fragilis* | 16 | 0.12 | 0 | 0 | 1 |
| 6493 | *Bacteroides thetaiotaomicron* | 0.25 | 0.25 | 0.12 | 0.12 | 1 |
| 6496 | *Bacteroides fragilis* | 0.12 | ≤0.03 | 0 | 0 | 1 |
| 6497 | *Bacteroides vulgatus* | 0.12 | 0.06 | ≤0.03 | 0 | 1 |
| 6502 | *Bacteroides fragilis* | 16 | 4 | 0 | 0 | 0.25 |
| 6503 | *Bacteroides thetaiotaomicron* | 0.5 | 0.25 | 0.25 | 0.12 | 1 |
| 6504 | *Bacteroides caccae* | 0.25 | 0.25 | 0.25 | 0.12 | 1 |
| 6506 | *Bacteroides thetaiotaomicron* | 4 | 2 | 1 | 0.5 | 1 |
| 6509 | *Bacteroides fragilis* | >512 | 64 | 16 | ≤0.03 | ≤0.12 |
| 6511 | *Bacteroides salyersiae* | 1 | 0.25 | 0.12 | 0.06 | 1 |
| 6513 | *Bacteroides fragilis* | 0.25 | 0.06 | 0 | 0 | 0.5 |
| 6515 | *Bacteroides thetaiotaomicron* | 2 | 0.5 | 0.25 | 0.12 | 1 |
| 6518 | *Bacteroides fragilis* | 0.06 | ≤0.03 | 0 | 0 | 0.5 |
| 6522 | *Bacteroides fragilis* | 0.25 | 0.06 | 0.06 | 0 | 1 |
| 6523 | *Bacteroides fragilis* | 4 | 0.12 | 0.12 | 0.12 | 1 |
| 6525 | *Bacteroides fragilis* | 4 | 1 | 0.06 | 0 | 1 |

(continued)

| KUB No. | Organism | MIC (μg/mL) | | | | |
|---|---|---|---|---|---|---|
| | | Meropenem | Meropenem/ CompoundA( 4) | Meropenem/ CompoundA( 8) | Meropenem/ CompoundA( 16) | Metronidazole |
| 6527 | *Bacteroides the-taiotaomicron* | 4 | 2 | 1 | 0.5 | 1 |
| 6530 | *Bacteroides thetaiotaomicron* | 2 | 1 | 0.25 | 0.06 | 1 |
| 6535 | *Bacteroides fragilis* | 0.12 | ≤0.03 | 0 | 0 | 0.5 |
| 6544 | *Bacteroides vulgatus* | 1 | 0.5 | 0.12 | ≤0.03 | 1 |
| 6545 | *Bacteroides thetaiotaomicron* | 2 | 1 | 0.5 | 0.12 | 0.5 |
| 6556 | *Bacteroides vulgatus* | 1 | 0.5 | 0.25 | 0.12 | 0.5 |
| 3003 | *Bacteroides fragilis* ATCC 25285 | 0.12 | ≤0.03 | ≤0.03 | ≤0.03 | 0.5 |
| 2982 | *Bacteroidesfragilis* | 0.12 | 0.06 | 0.06 | 0 | 1 |
| 2983 | *Bacteroides fragilis* | 4 | 1 | 0.25 | 0 | 0.25 |
| 2977 | *Bacteroides thetaiotaomicron* ATCC 29741 | 0.25 | 0.25 | 0.12 | 0.12 | 2 |
| 2984 | *Bacteroides thetaiotaomicron* | 0.25 | 0.25 | 0.25 | 0.12 | 1 |
| 3009 | *Bacteroides ovatus* | 1 | 0.25 | 0.25 | 0.12 | 1 |
| **Concentration of Compound A added is shown in parentheses**<br>**The strain with a MIC value of 0 means Compound A itself showed an antibacterial activity as a single agent** | | | | | | |

[Table 18-1]

| **Table 18 Antibacterial activity of meropenem/Compound A against *Prevotella* spp.** | | | | | | |
|---|---|---|---|---|---|---|
| KUB No. | Organism | MIC (μg/mL) | | | | |
| | | Meropenem | Meropenem/ CompoundA(4 ) | Meropenem/ CompoundA(8 ) | Meropenem/ CompoundA(1 6) | Metronidazole |
| 6558 | *Prevotella heparinolytica* | 0.06 | ≤0.03 | ≤0.03 | 0 | 0.25 |
| 6559 | *Prevotella intermedia* | ≤0.03 | 0 | 0 | 0 | 2 |
| 6560 | *Prevotella nigrescens* | ≤0.03 | ≤0.03 | 0 | 0 | 0.5 |
| 6561 | *Prevotella melaninogenica* | 0.12 | ≤0.03 | 0 | 0 | 1 |
| 6562 | *Prevotella salivae* | ≤0.03 | ≤0.03 | 0 | 0 | 1 |
| 6563 | *Prevotella nigrescens* | ≤0.03 | ≤0.03 | 0 | 0 | 0.5 |
| 6564 | *Prevotella buccae* | 0.12 | 0.06 | ≤0.03 | 0 | 2 |

(continued)

| Table 18 Antibacterial activity of meropenem/Compound A against *Prevotella* spp. | | | | | | |
|---|---|---|---|---|---|---|
| KUB No. | Organism | MIC (μg/mL) | | | | |
| | | Meropenem | Meropenem/ CompoundA(4 ) | Meropenem/ CompoundA(8 ) | Meropenem/ CompoundA(1 6) | Metronidazole |
| 6565 | *Prevotella nigrescens* | 0.06 | ≤0.03 | 0 | 0 | 0.5 |

[Table 18-2]

| KUB No. | Organism | MIC (μg/mL) | | | | |
|---|---|---|---|---|---|---|
| | | Meropenem | Meropenem/ CompoundA(4 ) | Meropenem/ CompoundA(8 ) | Meropenem/ CompoundA(1 6) | Metronidazole |
| 6566 | *Prevotella oralis* | ≤0.03 | 0 | 0 | 0 | 0.25 |
| 6567 | *Prevotella melaninogenica* | ≤0,03 | 0 | 0 | 0 | 0.5 |
| 6568 | *Prevotella intermedia* | 0.06 | 0 | 0 | 0 | 1 |
| 6569 | *Prevotella nanceiensis* | 0.5 | 0.25 | 0.06 | 0 | 2 |
| 6570 | *Prevotella intermedia* | 0.06 | ≤0.03 | 0 | 0 | 2 |
| 6571 | *Prevotella denticola* | 0.06 | 0 | 0 | 0 | 0.25 |
| 6572 | *Prevotella melaninogenica* | 0.06 | ≤0.03 | 0 | 0 | 0.5 |
| 6574 | *Prevotella oris* | ≤0.03 | ≤0.03 | 0 | 0 | 0.5 |
| 6575 | *Prevotella nigrescens* | ≤0.03 | ≤0.03 | 0 | 0 | 1 |
| 6576 | *Prevotella intermedia* | ≤0.03 | ≤0.03 | 0 | 0 | 0.25 |
| 6579 | *Prevotella pallens* | 0.06 | ≤0.03 | 0 | 0 | 0.5 |
| 6581 | *Prevotella intermedia* | 0.06 | ≤0.03 | 0 | 0 | 2 |
| 6582 | *Prevotella melaninogenica* | 0.06 | ≤0.03 | 0 | 0 | 0.5 |
| 6583 | *Prevotella nonceiensis* | 0.12 | 0.06 | 0 | 0 | 2 |
| 6584 | *Prevotella salivae* | ≤0.03 | ≤0.03 | 0 | 0 | 1 |
| 6585 | *Prevotella baroniae* | 0.06 | 0.06 | 0.06 | ≤0.03 | 1 |
| 6587 | *Prevotella melaninogenica* | 0.06 | 0 | 0 | 0 | ≤0.12 |
| 6588 | *Prevotella nigrescens* | ≤0.03 | ≤0.03 | 0 | 0 | 0.5 |

(continued)

| KUB No. | Organism | MIC (μg/mL) | | | | |
|---|---|---|---|---|---|---|
| | | Meropenem | Meropenem/ CompoundA(4 ) | Meropenem/ CompoundA(8 ) | Meropenem/ CompoundA(1 6) | Metronidazole |
| 6589 | *Prevotella buccae* | 0.12 | ≤0.03 | 0 | 0 | 0.5 |
| 6590 | *Prevotella buccae* | 0.32 | ≤0.03 | 0 | 0 | 0.5 |
| 6591 | *Prevotella intermedia* | 0.06 | ≤0.03 | 0 | 0 | 0.5 |
| 6592 | *Prevotella jejuni* | 0.06 | ≤0.03 | 0 | 0 | 0.5 |
| 6600 | *Prevotella intermedia* | ≤0.03 | ≤0.03 | ≤0.03 | ≤0.03 | 0.5 |
| 6601 | *Prevotella melaninogenica* | 0.12 | 0.06 | ≤0.03 | 0 | 0.5 |
| 6603 | *Prevotella melaninogenica* | 0.12 | 0 | 0 | 0 | 0.25 |
| 6604 | *Prevotella nonceiensis* | 0.25 | 0.12 | 0.06 | 0 | 1 |
| 6609 | *Prevotella buccae* | 0.12 | ≤0.43 | 0 | 0 | 0.5 |
| 6611 | *Prevotella nigrescens* | 0.06 | ≤0.03 | 0 | 0 | 1 |
| 6612 | *Prevotella oris* | 0.25 | ≤0.03 | ≤0.03 | 0 | 0.5 |
| 6613 | *Prevotella buccae* | 0.12 | ≤0.03 | 0 | 0 | 0.5 |
| 6614 | *Prevotella intermedia* | ≤0.03 | 0 | 0 | 0 | 1 |
| 6615 | *Prevotella buccae* | 0.06 | ≤0.03 | ≤0.03 | 0 | 1 |
| 6617 | *Prevotella buccae* | 0.06 | 0 | 0 | 0 | 2 |
| 6618 | *Prevotella melaninogenica* | 0.06 | ≤0.03 | 0 | 0 | 0.25 |
| 6622 | *Prevotella loescheii* | 0.06 | ≤0.03 | 0 | 0 | 1 |
| 6623 | *Prevotella nigrescens* | ≤0.03 | ≤0.03 | 0 | 0 | 1 |
| 6624 | *Prevotella salivae* | ≤0.03 | ≤0.03 | 0 | 0 | 0.5 |
| 6625 | *Prevotella melaninogenica* | ≤0.03 | 0 | 0 | 0 | 0.5 |
| 6626 | *Prevotella loescheii* | ≤0.03 | 0 | 0 | 0 | 1 |
| 6629 | *Prevotella bivia* | 0.06 | ≤0.03 | 0 | 0 | 8 |
| 6630 | *Prevotella nigrescens* | ≤0.03 | ≤0.03 | 0 | 0 | 0.5 |

(continued)

| KUB No. | Organism | MIC (μg/mL) | | | | |
|---|---|---|---|---|---|---|
| | | Meropenem | Meropenem/ CompoundA(4 ) | Meropenem/ CompoundA(8 ) | Meropenem/ CompoundA(1 6) | Metronidazole |
| 6631 | *Prevotella jejuni* | 0.06 | ≤0.03 | 0 | 0 | 0.5 |
| 2989 | *Prevotella histicola* | 0.06 | ≤0.03 | 0 | 0 | 0.5 |
| 2991 | *Prevotella intermedia* | 0.06 | 0 | 0 | 0 | 0.5 |
| 3005 | *Prevotella melaninogenica* | ≤0.03 | 0 | 0 | 0 | 1 |
| 2992 | *Prevotella buccae* | 0.12 | ≤0.03 | ≤0.03 | 0 | 1 |
| **Concentration of Compound A added is shown in parentheses**<br>**Strain with MIC value of 0 means that Compound A itself showed antibacterial activity as a single agent** | | | | | | |

[Table 19]

| **Table 19 Antibacterial activity of meropenem/Compound A against *Porphyromonas* spp.** | | | | | | |
|---|---|---|---|---|---|---|
| KUB No. | Organism | MIC (ug/mL) | | | | |
| | | Meropenem | Meropenem/ CompoundA(4 ) | Meropenem/ CompoundA(8 ) | Meropenem/ CompoundA(1 6) | Metronidazole |
| 6663 | *Porphyromonas gingivalis* | ≤0.03 | ≤0.03 | ≤0.03 | 0 | ≤0.12 |
| 6664 | *Porphyromonaa gingivalis* | ≤0.03 | ≤0.03 | 0 | 0 | ≤0.12 |
| 6665 | *Porphyromonas assaccharolytica* | ≤0.03 | ≤0.03 | 0 | 0 | ≤0.12 |
| 6666 | *Porphyromonas gingivalis* | ≤0.03 | ≤0.03 | 0 | 0 | ≤0.12 |
| 6667 | *Porphyromonas gingivalis* | ≤0.03 | ≤0.03 | 0 | 0 | ≤0.12 |
| 6668 | *Porphyromonas gingivalis* | ≤0.03 | 0 | 0 | 0 | ≤0.12 |
| 6669 | *Porphyromonas gingivalis* | ≤0.03 | ≤0.03 | ≤0.03 | ≤0.03 | ≤0.12 |
| 6670 | *Porphyromonas gingivalis* | ≤0.03 | ≤0.03 | 0 | 0 | ≤0.12 |
| 6671 | *Porphyromonas gingivalis* | ≤0.03 | ≤0.03 | ≤0.03 | 0 | ≤0.12 |
| 6672 | *Porphyromonas gingivalis* | ≤0.03 | ≤0.03 | ≤0.03 | 0 | ≤0.12 |
| 6673 | *Porphyromonas gingivalis* | ≤0.03 | ≤0.03 | ≤0.03 | 0 | ≤0.12 |
| 6674 | *Porphyromonas gingivalis* | ≤0.03 | ≤0.03 | ≤0.03 | ≤0.03 | ≤0.12 |
| 6675 | *Porphynromonas gingivalis* | 50.03 | ≤0.03 | ≤0.03 | ≤0.03 | ≤0.12 |

(continued)

| Table 19 Antibacterial activity of meropenem/Compound A against *Porphyromonas* spp. | | | | | | |
|---|---|---|---|---|---|---|
| KUB No. | Organism | MIC (ug/mL) | | | | |
| | | Meropenem | Meropenem/ CompoundA(4 ) | Meropenem/ CompoundA(8 ) | Meropenem/ CompoundA(1 6) | Metronidazole |
| 6676 | *Porphyromonas gingivalis* | ≤0.03 | ≤0.03 | ≤0.03 | 0 | ≤0.12 |
| 6677 | *Porphyromonas gingivalis* | ≤0.03 | ≤0.03 | ≤0.03 | 0 | ≤0.12 |
| 6678 | *Porphyromonas gingivalis* | ≤0.03 | ≤0.03 | 0 | 0 | ≤0.12 |
| 6679 | *Porphyromonas gingivalis* | ≤0.03 | ≤0.0 | 0 | 0 | ≤0.12 |
| 6680 | *Porphyromonas gingivalis* | ≤0.03 | ≤0.03 | 0 | 0 | ≤0.12 |
| 6681 | Porphyromonas *gingivalis* | ≤0.03 | 0 | 0 | 0 | ≤0.12 |
| 6682 | *Porphyromonas* gingivalis | ≤0.03 | 0 | 0 | 0 | ≤0.12 |
| 6683 | *Porphyromonas* gingivalis | ≤0.03 | ≤0.03 | ≤0.03 | 0 | ≤0.12 |
| 6684 | *Porphyromonas gingivalis* | ≤0.03 | ≤0.03 | ≤0.03 | 0 | ≤0.12 |
| 6685 | *Porphyromonas gingivalis* | ≤0.03 | ≤0.03 | ≤0.03 | 0 | ≤0.12 |
| 6686 | *Porphyromonas gingivalis* | ≤0.03 | ≤0.03 | ≤0.03 | 0 | ≤0.12 |
| 6687 | *Porphyromonas* gingivalis | ≤0.03 | 0 | 0 | 0 | ≤0.12 |
| 6688 | *Porphyromonas gingivalis* | 0.03 | 0 | 0 | 0 | ≤0.12 |
| **Concentration of Compound A added is shown in parentheses**<br>**Strain with MIC value of 0 means that Compound A itself showed antibacterial activity as a single agent** | | | | | | |

[Table 20]

| Table 20 Antibacterial activity of meropenem/Compound A against *Fusobacterium* spp. | | | | | | |
|---|---|---|---|---|---|---|
| KUB No. | Organism | MIC (μg/mL) | | | | |
| | | Meropenem | Meropenem/ CompoundA(4 ) | Meropenem/ CompoundA(8 ) | Meropenem/ CompoundA(1 6) | Metronidazole |
| 6692 | *Fusobacterium* sp. | ≤0.03 | 0 | 0 | 0 | ≤0.12 |
| 6693 | Fusobacterium *nucleatium* | ≤0.03 | 0 | 0 | 0 | 0.25 |
| 6694 | *Fusobacterium necrophorum* | ≤0.03 | 0 | 0 | 0 | ≤0.12 |
| 6695 | *Fusobacterium necrophorum* | ≤0.03 | ≤0.03 | 0 | 0 | 0.5 |

(continued)

| Table 20 Antibacterial activity of meropenem/Compound A against *Fusobacterium* spp. | | | | | | |
|---|---|---|---|---|---|---|
| KUB No. | Organism | MIC (µg/mL) | | | | |
| | | Meropenem | Meropenem/ CompoundA(4 ) | Meropenem/ CompoundA(8 ) | Meropenem/ CompoundA(1 6) | Metronidazole |
| 6696 | *Fusobacterium necrophorum* | ≤0.03 | ≤0.03 | 0 | 0 | ≤0.12 |
| 6697 | *Fusobacterium* nucleatum | ≤0.03 | ≤0.03 | ≤0.03 | ≤0.03 | ≤0.12 |
| 6698 | *Fusobacterium nucleatum* | ≤0.03 | 0 | 0 | 0 | ≤0.12 |
| 6699 | *Fusobacterium necrophorum* | ≤0.03 | 0 | 0 | 0 | ≤0.12 |
| 6700 | *Fusobacterium nucleatum* | ≤0.03 | ≤0.03 | 0 | 0 | ≤0.12 |
| 6702 | Fusobacterium necrophorum | ≤0.03 | 0 | 0 | 0 | 0.25 |
| 6708 | *Fusobacterium necrophorun* | ≤0.03 | ≤0.03 | 0 | 0 | ≤0.12 |
| 6710 | Fusobacterium *necrophorum* | ≤0.03 | ≤0.03 | 0 | 0 | 0,25 |
| 6715 | *Fusobacterium* sp. | ≤0.03 | ≤0.03 | ≤0.03 | ≤0.03 | ≤0.12 |
| 6716 | *Fusobacterium nuncleatum* | ≤0.03 | ≤0.03 | 0 | 0 | ≤0.12 |
| 6719 | Fusobacterium sp. | ≤0.03 | ≤0.03 | 0 | 0 | ≤0.12 |
| 6720 | Fusobacterium *nucleatum* | 0.06 | ≤0.03 | 0 | 0 | ≤0.12 |
| 6724 | *Fusobacterium nucleatu*m | ≤0.03 | ≤0.03 | 0 | 0 | ≤0.12 |
| 6725 | *Fusobacterium sp.* | 0.06 | ≤0.03 | ≤0.03 | 0 | ≤0.12 |
| 2741 | *Fusobacterium varium* ATCC 27725 | 0.12 | 0.12 | 0.06 | 0.06 | 0.25 |
| 2742 | *Fusobacterium nucleatum* ATCC 25586 | ≤0.03 | ≤0.03 | ≤0.03 | 0 | ≤0.12 |
| Concentration of Compound A added is shown in parentheses<br>Strain with MIC value of 0 means that Compound A itself showed antibacterial activity as a single agent | | | | | | |

[Table 21]

| Table 21 Antibacterial activity of meropenem/Compound A against Gram-positive bacteria | | | | | | |
|---|---|---|---|---|---|---|
| KUB No. | Organism | MIC (μg/mL) | | | | |
| | | Meropenem | Meropenem/ CompoundA(4 ) | Meropenem/ CompoundA(8 ) | Meropenem/ CompoundA(1 6) | Metronidazole |
| 6740 | *Parvimonas micra* | 0.06 | ≤0.03 | ≤0.03 | ≤0.03 | 0.5 |
| 6741 | *Parvimonas micra* | 0.06 | 0.06 | 0.06 | 0.06 | ≤0.12 |
| 6742 | *Parvimonas micra* | ≤0.03 | ≤0.03 | 0 | 0 | 0.25 |
| 6743 | *Parvimonas micra* | ≤0.03 | ≤0.03 | ≤0.03 | 0 | 0.25 |
| 6744 | *Parvimonas micra* | ≤0 03 | ≤0.03 | 0 | β | ≤0.12 |
| 6745 | *Parvimonas micra* | 0.06 | ≤0.03 | ≤0.03 | 0 | 0.5 |
| 6746 | *Parvimonas micra* | ≤0.03 | ≤0.03 | ≤0.03 | ≤0.03 | 0.25 |
| 6747 | *Parvimonas micra* | 0.06 | 0.06 | ≤0.03 | ≤0.03 | 0.25 |
| 6748 | *Parvimonas micra* | ≤0.03 | ≤0.03 | 0 | 0 | 0.25 |
| 6749 | *Parvimonas micra* | ≤0.03 | 0 | 0 | 0 | 0.25 |
| 6750 | *Parvimonas micra* | ≤0.03 | ≤0.03 | 0 | 0 | 0.25 |
| 6756 | *Parvimonas micra* | 0.5 | 0.5 | 0.5 | 0.5 | 0.5 |
| 6758 | *Peptoniphilus indolicus* | ≤0.03 | 0 | 0 | 0 | 0.25 |
| *6759* | *Peprostrentococcus stomatis* | 0.12 | 0.12 | 0.12 | 0.06 | ≤0.12 |
| 6761 | *Parvimonas micra* | 0.25 | 0.25 | 0.25 | 0.25 | 0.25 |
| 6762 | *Parvimonas micra* | ≤0.03 | ≤0.03 | ≤0,03 | ≤0,03 | ≤0.12 |
| 6769 | *Peprostreptococcus stomatis* | 0.12 | 0.12 | 0.12 | 0.12 | ≤0.12 |
| 6772 | *Pervimonas micra* | 0.06 | 0.06 | ≤0.03 | ≤0.03 | 0.25 |
| 6773 | *Parvimonas micra* | ≤0.03 | ≤0.03 | ≤0.03 | ≤0.03 | 0.25 |
| 6775 | *Peptostreptococcus anaerobius* | 2 | 2 | 2 | 2 | 0.5 |
| 6776 | *Parvimonas micra* | ≤0.03 | ≤0.03 | ≤0.03 | 0 | 0.5 |
| 6778 | *Parvimonas micra* | 0.25 | 0.25 | 0.25 | 0.25 | 0.5 |
| 6779 | *Parvimonas micra* | ≤0.03 | ≤0.03 | ≤0.03 | 0 | 0.25 |
| 6780 | *Parvimonas micra* | ≤0.03 | ≤0.03 | ≤0.03 | 0 | 0.5 |

(continued)

**Table 21 Antibacterial activity of meropenem/Compound A against Gram-positive bacteria**

| KUB No. | Organism | MIC (μg/mL) | | | | |
|---|---|---|---|---|---|---|
| | | Meropenem | Meropenem/ CompoundA(4 ) | Meropenem/ CompoundA(8 ) | Meropenem/ CompoundA(1 6) | Metronidazole |
| 6781 | *Parvimonas micra* | ≤0.03 | ≤0.03 | ≤0.03 | 0 | 0.5 |
| 2978 | *Clostridioides difficile* ATCC 700057 | 2 | 2 | 2 | 2 | 0.5 |
| 3518 | *Clostridioides difficile* ATCC 43255 | 2 | 2 | 2 | 2 | 0.5 |
| 3511 | *Clostridioides difficile* ATCC BAA-1803 | 4 | 4 | 4 | 4 | 1 |
| 3385 | *Clostridioides difficile* ATCC BAA-1870 | 4 | 4 | 4 | 4 | 0.5 |
| 2981 | *Clostridium perfringeus* ATCC 13124 | ≤0.03 | 0 | 0 | 0 | 1 |
| 2980 | *Peprostreptococcus anaerobius* ATCC 27337 | 0.25 | 0.25 | 0.25 | 0.12 | 0.25 |
| 3496 | *Peptoniphilus asaccharolyticus* ATCC 14963 | ≤0.03 | ≤0.03 | ≤0.03 | ≤0.03 | 0.5 |
| 3000 | *Parvimonas micra* | 0.06 | 0.06 | 0.06 | ≤0.03 | 0.5 |
| 3494 | *Finegoldia magna* ATCC 29328 | 0.06 | 0.06 | 0.06 | 0.06 | 1 |
| 2979 | *Eggerthella lenta* ATCC 43055 | 0.5 | 0.5 | 0.5 | 0.5 | 1 |
| 3012 | *Olsenella uli* | ≤0.03 | ≤0.03 | ≤0.03 | ≤0.03 | 2 |
| 3013 | *Bulleidia extructa* | 0.12 | 0.12 | 0.12 | 0.12 | 2 |
| 3014 | *Atropobium rimae* | 0.06 | 0.06 | ≤0.03 | ≤0.03 | 2 |
| 3377 | *Propionibacterium acnes* ATCC 11827 | ≤0.03 | ≤0.03 | ≤0.03 | 0 | >64 |
| 3488 | *Actinomyces odontolyticus* ATCC 17929 | 0.12 | 0.12 | 0.12 | 0.12 | 32 |

**Concentration of Compound A added is shown in parentheses**
**Strain with MIC value of 0 means that Compound A itself showed antibacterial activity as a single agent**

[Table 22]

| Table 22 $MIC_{range}$, $MIC_{50}$, and $MIC_{90}$ of meropenem/Compound A against *Bacteroides* spp. | | | |
|---|---|---|---|
| MIC (μg/mL) | $MIC_{range}$ | $MIC_{50}$ | $MIC_{90}$ |
| Meropenem | 0.06 - >512 | 0.5 | 4 |
| Meropenem/CompoundA(4) | ≤0.03 - 64 | 0.12 | 1 |
| Meropenem/CompoundA(8) | ≤0.03 - 16 | 0.06 | 0.5 |
| Meropenem/CompoundA(16 ) | ≤0.03 - 0.5 | ≤0.03 | 0.12 |
| Metronidazole | ≤0.12 - 2 | 1 | 1 |
| **Concentration of Compound A added is shown in parentheses** | | | |

[Table 23]

| Table 23 $MIC_{range}$, $MIC_{50}$, and $MIC_{90}$ of meropenem/Compound A against *Prevotella* spp. | | | |
|---|---|---|---|
| MIC (μg/mL) | $MIC_{range}$ | $MIC_{50}$ | $MIC_{90}$ |
| Meropenem | ≤0.03 - 0.5 | 0.06 | 0.12 |
| Meropenem/CompoundA(4) | ≤0.03 - 0.25 | ≤0.03 | 0.06 |
| Meropenem/CompoundA(8) | ≤0.03 - 0.06 | ≤0.03 | ≤0.03 |
| Meropenem/CompoundA(16) | ≤0.03 | ≤0.03 | ≤0.03 |
| Metronidazole | ≤0.12 - 8 | 0.5 | 2 |
| **Concentration of Compound A added is shown in parentheses** | | | |

[Table 24]

| Table 23 $MIC_{range}$, $MIC_{50}$, and $MIC_{90}$ of meropenem/Compound A against *Prevotella* spp. | | | |
|---|---|---|---|
| MIC (μg/mL) | $MIC_{range}$ | $MIC_{50}$ | $MIC_{90}$ |
| Meropenem | ≤0.03 | ≤0.03 | ≤0.03 |
| Meropenem/CompoundA(4) | ≤0.03 | ≤0.03 | ≤0.03 |
| Meropenem/CompoundA(8) | ≤0.03 | ≤0.03 | ≤0.03 |
| Meropenem/CompoundA(16) | ≤0.03 | ≤0.03 | ≤0.03 |
| Metronidazole | ≤0.12 | ≤0.12 | ≤0.12 |
| **Concentration of Compound A added is shown in parentheses** | | | |

[Table 25]

| Table 25 $MIC_{range}$, $MIC_{50}$, and $MIC_{90}$ of meropenem/Compound A against *Fusobacterium* spp. | | | |
|---|---|---|---|
| MIC (μg/mL) | $MIC_{range}$ | $MIC_{50}$ | $MIC_{90}$ |
| Meropenem | ≤0.03 - 0.12 | ≤0.03 | 0.06 |
| Meropenem/CompoundA(4) | ≤0.03 - 0.12 | ≤0.03 | ≤0.03 |
| Meropenem/CompoundA(8) | ≤0.03 - 0.06 | ≤0.03 | ≤0.03 |
| Meropenem/CompoundA(16) | ≤0.03 - 0.06 | ≤0.03 | ≤0.03 |
| Metronidazole | ≤0.12 - 0.5 | ≤0.12 | 0.25 |
| **Concentration of Compound A added is shown in parentheses** | | | |

[Table 26]

| Table 26 MICrange, $MIC_{50}$, and MIC90 of meropenem/Compound A against Gram-positive bacteria | | | |
|---|---|---|---|
| MIC (μg/mL) | $MIC_{range}$ | $MIC_{50}$ | $MIC_{90}$ |
| Meropenem | ≤0.03 - 4 | 0.06 | 2 |
| Meropenem/CompoundA(4) | ≤0.03 - 4 | ≤0.03 | 2 |
| Meropenem/CompoundA(8) | ≤0.03 - 4 | ≤0.03 | 2 |
| Meropenem/CompoundA(16) | ≤0.03 - 4 | ≤0.03 | 2 |
| Metronidazole | ≤0.12 - >64 | 0.5 | 2 |
| **Concentration of Compound A added is shown in parentheses** | | | |

(Example 10) Bactericidal activity of meropenem (MEPM)/Compound A against carbapenemase-producing Enterobacteriaceae (CPE) using bactericidal curve as indicator

A bactericidal activity of MEPM/Compound A against CPE was examined using a bactericidal curve method. Four strains were evaluated: E. coli ATCC BAA-2469 (NDM-1-producing strain), E. coli NCTC 13476 (IMP-1-producing strain), Klebsiella pneumoniae ATCC BAA-2344 (KPC-2-producing strain), and Klebsiella pneumoniae CDC-505 (NDM-1-producing strain). The MIC of MEPM against these four strains were 32, 4, 16, and 64 μg/mL, respectively, and the MIC of MEPM/Compound A (4 μg/mL) were 0.03, 0.06, 0.03, and 0.03 μg/mL, respectively.

Test strains were cultured overnight at 35°C in a cation-adjusted Mueller Hinton broth (CAMHB). The test strains were then suspended in a fresh CAMHB to 0.5 McFarland. The resulting bacterial suspension was diluted 900-fold with the CAMHB and cultured at 35 to 36°C for 1 hour with agitation at 180 rpm. Compound A was dissolved in a sodium carbonate aqueous solution to prepare a 3.2 mg/mL solution, which was diluted with the CAMHB to 80 or 320 μg/mL. MEPM was dissolved in the CAMHB to 2.56 mg/mL and diluted in the CAMHB to 0.6 to 1280 μg/mL. MEPM/Compound A at 0.3/40 to 10/40 μg/mL or 0.3/16 to 10/160 μg/mL was prepared by mixing equal amounts of MEPM at 0.6 to 20 μg/mL and Compound A at 80 or 320 μg/mL. Then, 400 μL of the CAMHB (positive control), the MEPM, or the MEPM/Compound A at each concentration were added to an L-shaped test tube, and 3.6 mL of the bacterial solution which had been cultured for 1 hour was added thereto. The resulting test bacterial solution was cultured at 36°C with agitation at 40 rpm, and a culture solution was collected at each time point. After about 10-fold serial dilution with sterile saline, 100 μL of each of the resultants was smeared on a Mueller Hinton agar. These agar media were cultured at 35°C for 1 day and the number of colonies was measured. A bactericidal activity was defined as a reduction in the number of bacteria by 3 $log_{10}$ or more (99.9% or more) compared to that in the inoculum.

When a concentration of MEPM was ≥1 x MIC, the number of bacteria could be reduced by 3 $log_{10}$ compared to a preculture level within 1 to 2 hours after culture for all four strains. When MEPM/Compound A (4 μg/mL) was used in combination at ≥4 x MIC, ≥99.9% reduction in the number of bacteria was observed against all strains within 2 hours after culture. A combination of MEPM at 0.03 or 0.06 μg/mL and Compound A at 16 μg/mL showed a reduction by 3 $log_{10}$ or more for all strains by 2 hours after culture. These results confirm that MEPM/Compound A exhibits a bactericidal activity against serine- and metallo-carbapenemase-producing Enterobacteriaceae.

(Example 11) In vitro antibacterial activity of Compound A against anaerobic bacteria

An in vitro antibacterial activity of Compound A alone against anaerobic bacteria was evaluated using the minimal inhibitory concentration (MIC) by an agar dilution method according to the CLSI M11-A8 (metronidazole (MNZ) was included as a positive control). Prevotella histicola KUB2989 and the other test strains were cultured at 36°C under an anaerobic condition for 1 and 2 days, respectively, on a Brucella agar supplemented with vitamin $K_1$, hemin, and sheep defibrinated blood. The strains were further smeared on the same fresh agar medium and cultured at 36°C for 19 hours under an anaerobic condition. The test strains on the agar medium were then suspended in a Brucella broth and adjusted to about 0.5 McFarland. Compound A was dissolved in distilled water supplemented with sodium carbonate and MNZ was dissolved in DMSO, and each of them was diluted to a target concentration with distilled water. One milliliter of each of the Compound A and the MNZ at each concentration was mixed with 9 mL of a Brucella agar supplemented with vitamin $K_1$, hemin, and sheep defibrinated blood and allowed to solidify at room temperature. The agar medium was inoculated with each of the bacterial suspensions at approximately 2 μL/spot using a microplanter and cultured at 36°C for 2 days under an anaerobic condition. The MIC was defined as the lowest concentration at which growth on a test agar medium was significantly reduced compared to growth on a positive growth control agar medium. The MIC of the Compound A against 20 anaerobic bacteria strains tested ranged from 2 to 256 μg/mL (Table 27). The MIC of the Compound A against the other

seven strains was higher than the maximum concentration of 256 μg/mL. The Compound A showed intrinsic antibacterial activity against some anaerobic bacteria.

[Table 27-1]

| Table 27 Antibacterial activity of Compound A and MNZ against anaerobic bacteria | | |
|---|---|---|
| **Organism** | **Compound A (MIC μg/mL)** | **MNZ (MIC μg/mL)** |
| ***Clostridioides difficile* ATCC 700057 (QC strain)** | >256 | 0.5 (0.125 to 0.5)* |
| ***Clostridioides difficile* ATCC 43255** | >256 | 1 |
| ***Clostridioides difficile* ATCC BAA-1803** | >256 | 2 |
| ***Clostridioides difficile* ATCC BAA-1870** | >256 | 1 |
| ***Clostridium perfringens* ATCC 13124** | 2 | 2 |
| ***Peptostreptococcus anaerobius* ATCC 27337** | >256 | 0.5 |

[Table 27-2]

| | | |
|---|---|---|
| ***Peptoniphilus asaccharolyticus* ATCC 14963** | 32 | 1 |
| ***Parvimonas micra* KUB3000** | 64 | 0.5 |
| ***Finegoldia magna* ATCC 29328** | 128 | 2 |
| ***Eggerthella lenta* ATCC 43055** | >256 | 1 |
| ***Olsenella uli* KUB3012** | 64 | 4 |
| ***Bulleidia extructa* KUB3013** | 32 | 2 |
| ***Atopobium rimae* KUB3014** | 8 | 1 |
| ***Propionibacterium acnes* ATCC 11827** | 32 | >64 |
| ***Actinomyces odontolyticus* ATCC 17929** | 256 | >64 |
| ***Bacteroides fragilis* ATCC 25285 (QC strain)** | 8 | 1 (0.25 to 1)* |
| ***Bacteroides fragilis* KUB2982** | 16 | 2 |
| ***Bacteroides fragilis* KUB2983** | 16 | 0.5 |
| ***Bacteroides thetaiotaomicron* ATCC 29741 (QC strain)** | 64 | 2 (0.5 to 2)* |
| ***Bacteroides thetaiotaomicron* KUB2984** | 32 | 1 |
| ***Bacteroides ovatus* KUB3009** | 128 | 1 |
| ***Prevotella histicola* KUB2989** | 8 | 2 |
| ***Prevotella intermedia* KUB2991** | 4 | 1 |
| ***Prevotella melaninogenica* KUB3005** | 4 | 1 |
| ***Prevotella buccae* KUB2992** | 8 | 2 |
| ***Fusobacterium varium* ATCC 27725** | >256 | 0.5 |
| ***Fusobacterium nucleatum* ATCC 25586** | 32 | ≤0.125 |
| *** Acceptable MIC range of quality control (QC) strain** | | |

(Example 12) Effect of addition of human plasma and human serum albumin on in vitro antibacterial activity of meropenem (MEPM) /Compound A against carbapenemase-producing Enterobacteriaceae (CPE)

To examine an effect of addition of human plasma and human serum albumin on an in vitro activity of MEPM/Compound A against CPE, the minimal inhibitory concentration (MIC) was determined in a cation-adjusted Muer Hinton broth (CAMHB) supplemented with human plasma and human serum albumin using a broth microdilution method according to the Clinical and Laboratory Standards Institute. Test strains were cultured on a Mueller Hinton agar at 35°C for

21.3 hours. The test strains were then suspended in saline to 0.5 McFarland. The resulting bacterial suspension was diluted 2-fold with saline, which was used as an inoculum. Compound A was dissolved in a sodium carbonate aqueous solution to prepare a 1.6 mg/mL solution, which was diluted with 2x CAMHB to 16 μg/mL. MEPM was dissolved in water to 6.4 mg/mL and diluted with 2x CAMHB to a concentration of 0.015 to 128 μg/mL. MEPM/Compound A at 0.015/8 to 8/8 μg/mL was prepared by mixing equal amounts of the meropenem at 0.03 to 16 μg/mL and the Compound A at 16 μg/mL. Then, 50 μL of the MEPM or MEPM/Compound A solution at each concentration was added into a 96-well round-bottom microplate, and 50 μL of water, 8% human serum albumin, 40% heat-inactivated human plasma, or 100% heat-inactivated human plasma was added so as to give 1x CAMHB, human serum albumin at a final concentration of 4%, or human plasma at final concentrations of 20% or 50%. Final concentrations of the MEPM and the MEPM/Compound A ranged from 0.008 to 64 and 0.008/4 to 4/4 μg/mL. Each well of the microplate was inoculated with 1 μL of the inoculum and cultured at 35°C for 18 hours. The lowest concentration exhibiting no visible growth was determined as MIC. The MIC of the MEPM alone against 8 CPE strains ranged from 1 to 32 μg/mL under a standard condition (CAMHB), and addition of the Compound A at 4 μg/mL markedly reduced the MIC of the MEPM to 0.03 μg/mL or less (Table 28). The MIC of the MEPM/Compound A (4 μg/mL) in the presence of 20% human plasma, 50% human plasma, or 4% human serum albumin was increased within one dilution of the MIC in the absence thereof, indicating that an in vitro activity of the MEPM/Compound A against the CPE used in this study was not affected by addition of human plasma or human serum albumin.

[Table 28]

| Organisms (Carbapenemases) | MICs of MEPM (μg/mL) | | | | | | | |
|---|---|---|---|---|---|---|---|---|
| | MEPM alone | | | | MEPM with CompoundA at 4 μg/mL | | | |
| | Albumin | | Plasma | | Albumin | | Plasma | |
| | 0%* | 4% | 20% | 50% | 0%* | 4% | 20% | 50% |
| *E. coli* ATCC BAA-2340 (KPC-3) | 4 | 4 | 4 | 0.5 | 0.015 | 0.03 | 0.03 | 0.015 |
| *K. pneumoniae* ATCC BAA-2342 (KPC-3) | 16 | 32 | 16 | 16 | 0.03 | 0.06 | 0.06 | 0.06 |
| *K. pneumoniae* ATCC BAA-2344 (KPC-2) | 16 | 32 | 16 | 16 | 0.03 | 0.03 | 0.06 | 0.06 |
| *K. pneumoniae* ATCC BAA-2524 (OXA-48) | 1 | 0.5 | 2 | 4 | 0.03 | 0.03 | 0.03 | 0.03 |
| *E. coli* ATCC BAA-2469 (NDM-1) | 32 | 16 | ≤0.12. | ≤0.12 | 0.03 | 0.03 | 0.03 | 0.03 |
| *K. pneumoniae* ATCC BAA-2470 (NDM-1) | 16 | 8 | 0.5 | 0.5 | 0.03 | 0.06 | 0.06 | 0.06 |
| *K pneumoniae* NCTC13439 (VIM-1) | 4 | 2 | ≤0.12 | ≤0.12 | 0.03 | 0.06 | 0.06 | 0.06 |
| *E. coli* NCTC 13476 (IMP-1) | 4 | 4 | 1 | 0.5 | 0.03 | 0.06 | 0.06 | 0.03 |
| *E. coli* ATCC 25922 (quality control strain) | 0.015 | 0.03 | 0.03 | 0.03 | 0.015 | 0.03 | 0.03 | 0.03 |

***E. coli, Escherichia coli ; K. pneumoniae, Klebsiella pneumoniae***
*** Standard condition**

(Example 13) Effect of human urine and medium pH on in vitro antibacterial activity of MEPM/Compound A against carbapenemase-producing Enterobacteriaceae (CPE).

To identify an effect of human urine and a medium pH on an in vitro activity of MEPM/Compound A against CPE, the minimal inhibitory concentration (MIC) was determined using human urine and a cation-adjusted Muer Hinton broth (CAMHB) adjusted to pH 5.0 to 8.0 by a broth microdilution method according to the Clinical and Laboratory Standards Institute (CLSI).

Test strains were cultured on a Mueller Hinton agar at 35°C for 18 hours. The test strains were then suspended in saline to 0.5 McFarland. The resulting bacterial suspension was diluted 20-fold with saline, which was used as an inoculum. The pH of CAMHB was adjusted with hydrochloric acid or sodium hydroxide to 5.0, 6.0, 7.2, or 8.0. Human pooled urine was thawed at 37°C and centrifuged at 1,300 g for 5 minutes at room temperature, and the supernatant was refrigerated until use. When used, the pH of the human pooled urine was adjusted to 5.0, 6.0, 7.0, or 8.0 with hydrochloric

acid or sodium hydroxide and sterilized by filtration. Compound A was dissolved in a sodium carbonate aqueous solution to prepare a 0.8 mg/mL solution, which was diluted with CAMHB at each pH or the human pooled urine at each pH to 8 μg/mL. MEPM was dissolved in water to 1.6 to 12.8 mg/mL and diluted with CAMHB at each pH or the human pooled urine at each pH to a concentration of 0.008 to 128 μg/mL. MEPM/Compound A at 0.008/4 to 32/4 μg/mL was prepared by mixing equal amounts of the MEPM at 0.015 to 64 μg/mL and the Compound A at 8 μg/mL. One hundred microliters of a MEPM or MEPM/Compound A solution at each concentration was added to a 96-well round-bottom microplate, and 10 μL of the bacterial suspension was inoculated thereinto and cultured at 35°C for 18 hours. The MIC was defined as the lowest concentration exhibiting no visible growth.

The MIC of the MEPM alone against 8 CPE strains was 1 to 32 μg/mL under standard conditions (CAMHB, pH 7.2), and the Compound A markedly reduced the MIC of the MEPM against these strains to 0.06 μg/mL or less (Table 29, 30). For CAHMB at pH 5.0, 6.0, or 8.0, an activity of the MEPM/Compound A changed within about 2-fold dilution relative to that under the standard conditions. For the human pooled urine at pH 7.0, an antibacterial activity of the MEPM/Compound A was comparable to that in the CAMHB. For the human pooled urine adjusted to pH 5.0 to 7.0, there was no change in the antibacterial activity of the MEPM/Compound A against almost all strains, while the MIC was increased over them for the human pooled urine at pH 8.0. An in vitro activity of the MEPM/Compound A against the CPE used in this study was not affected by the human urine or the medium pH, except for the human pooled urine at pH 8.0.

[Table 29]

| | MICs of MEPM ( μg/mL) | | | | | | | |
|---|---|---|---|---|---|---|---|---|
| | Standard condition* | CAMHB | | | Human urine | | | |
| | | pH5.0 | pH6.0 | pH8.0 | pH5.0 | pH6.0 | pH7.0 | pH8.0 |
| *E. coli ATCC* BAA-2340 (KPC-3) | 8 | 2 | 8 | 4 | 1 | 1 | 2 | 8 |
| *K. pneumoniae* ATCC BAA-2342 (KPC-3) | 16 | 16 | 16 | 16 | 4 | 8 | 16 | 32 |
| *K. pneumoniae* ATCC BAA-2344 (KPC-2) | 32 | 8 | 32 | 32 | 2 | 8 | 32 | 32 |
| *K. pneumoniae* ATCC BAA-2524 (OXA-48) | 1 | 0.25 | 0.25 | 4 | 0.25 | 0.25 | 0.25 | 1 |
| *E. coli* ATCC BAA-2469 (NDM-1) | 32 | 0.12 | 8 | 64 | 0.12 | 0.12 | 16 | 8 |
| *K. pmeumoniae* ATCC BAA-2470 (NDM-1) | 16 | 0.12 | 8 | 64 | 0.12 | 0.12 | 16 | 8 |
| *K. pneumoniae* NCTC13439 (VIM-1) | 4 | 0.12 | 1 | 4 | 0.12 | 0.06 | 2 | 1 |
| *E. coli* NCTC 13476 (IMP-1) | 4 | 0.25 | 1 | 16 | 0.25 | 0.12 | 1 | 8 |
| *E. coli* ATCC 25922 (QC strain) | 0.03 | 0.12 | 0.06 | 0.015 | 0.12 | 0.03 | 0.015 | 0.015 |

***E. coli, Escherichia coli ; K. pneumoniae, Klebsiella pneumoniae***
*CAMHB pH7. 2

[Table 30]

| | MICs of MEPM with CompoundA at 4 μg/mL ( μg/mL) | | | | | | | |
|---|---|---|---|---|---|---|---|---|
| | Standard condition* | CAMHB | | | Human urine | | | |
| | | pH5.0 | pH6.0 | pH8.0 | pH5.0 | pH6.0 | pH7.0 | pH8.0 |
| *E. coli* ATCC BAA-2340 (KPC-3) | 0.015 | 0.12 | 0.06 | ≦0.03 | 0.25 | 0.03 | 0.015 | 0.015 |
| *K. pneumoniae* ATCC BAA-2342 (KPC-3) | 0.03 | 0.12 | 0.06 | 0.06 | 0.12 | 0.06 | 0.06 | 2 |
| *K. pneumoniae* ATCC BAA-2344 (KPC-2) | 0.03 | 0.12 | 0.06 | ≦0.03 | 0.12 | 0.06 | 0.03 | 0.5 |
| *K. pneumoniae* ATCC BAA-2524 (OXA-48) | 0.03 | 0.12 | 0.06 | ≦0.03 | 0.12 | 0.06 | 0.03 | 0.03 |

(continued)

| | MICs of MEPM with CompoundA at 4 μg/mL ( μg/mL) | | | | | | | |
|---|---|---|---|---|---|---|---|---|
| | Standard condition* | CAMHB | | | Human urine | | | |
| | | pH5.0 | pH6.0 | pH8.0 | pH5.0 | pH6.0 | pH7.0 | pH8.0 |
| *E. coli* ATCC BAA-2469 (NDM-1) | 0.03 | 0.12 | 0.03 | 0.06 | 0.12 | 0.03 | 0.03 | 0.5 |
| *K pneumoniae* ATCC BAA-2470 (NDM-1) | 0.06 | 0.12 | 0.06 | 0.5 | 0.06 | 0.06 | 0.25 | 2 |
| *K. pneumoniae* NCTC13439 (VIM-1) | 0.06 | 0.12 | 0.06 | 0.12 | 0.06 | 0.03 | 0.06 | 0.5 |
| *E. coli* NCTC 13476 (IMP-1) | 0.03 | 0.06 | 0.06 | 0.25 | 0.12 | 0.03 | 0.12 | 1 |
| *E. coli* ATCC 25922 (QC strain) | 0.015 | 0.06 | 0.03 | 0.015 | 0.12 | 0.03 | 0.015 | 0.015 |

***E. coli, Escherichia coli ; K. pneumoniae, Klebsiella pneumoniae***
*CAMHB pH7. 2

(Example 14) Activity of meropenem (MEPM)/Compound A in systemic infection lethal mouse model by NDM- or KPC-producing carbapenem-resistant Enterobacteriaceae

In vivo activities of MEPM/Compound A and a control were compared in a systemic infection lethal mouse model by carbapenemase-producing carbapenem-resistant Enterobacteriaceae. Test strains were cultured on a Mueller Hinton agar (MHA) at 35°C for 9 to 16 hours twice. The test strains on the agar medium were suspended in saline and adjusted to a Klett unit of about 200 using a Klett Photoelectric Colorimeter. The resulting bacterial suspension was further diluted with saline and diluted 2-fold with a 16% mucin suspension to prepare a bacterial suspension for infection. Dissolved a Meropen for Intravenous Infusion vial in saline and adjusted to a target concentration. Compound A, vaborbactam, and cilastatin were dissolved in saline with sodium carbonate and adjusted with saline to a target concentration. Avibactam sodium was dissolved in saline and adjusted to a target concentration. These solutions were mixed to prepare an MEPM/cilastatin mixed solution, an MEPM/Compound A/cilastatin mixed solution, an MEPM/vaborbactam/cilastatin mixed solution, and a MEPM/avibactam/cilastatin mixed solution. Note that, for the purpose of suppressing degradation of MEPM by a dehydropeptidase-I in a mouse, the agent at each concentration was administered in combination with cilastatin at 100 mg/kg, which can inhibit this enzyme. Male ICR mice were inoculated intraperitoneally with 0.2 mL of the bacterial suspension. Two hours after the inoculation, saline or a solution for administering the agent at each concentration was administered subcutaneously to the mice in a single dose. The number of mice that survived from Day 1 to Day 7 after infection was recorded. A 50% survival dose ($ED_{50}$) and a 95% confidence intervals for each agent were calculated from the number of mice alive on Day ·BR.> V for the MEPM at each dose using the probit method. A 50% lethal inoculum dose ($LD_{50}$) was calculated using the probit method based on the number of mice alive on Day 7 at each infectious dose.

An $ED_{50}$ of the MEPM in combination with 1/4 dose of Compound A for a KPC-producing bacterium infection model was 6.48 mg/kg, which was comparable to those of MEPM/vaborbactam (MEPM 9.08 mg/kg; dose ratio 4:1) and MEPM/avibactam (MEPM 6.18 mg/kg; dose ratio 4:1) and significantly lower than that of MEPM alone (205 mg/kg) (Table 31). An $ED_{50}$ of MEPM/Compound A (8.93 mg/kg) for a NDM-producing bacterium infection model was significantly lower than those of the MEPM alone, the MEPM/vaborbactam at a dose ratio of 4:1, and the MEPM/avibactam at a dose ratio of 4:1 (64.8 to 103 mg/kg) (Table 32). An activity of the MEPM was improved for both infection models only in combination with the Compound A.

[Table 31]

**Table 31. Activity of MEPM/Compound A in systemic infection lethal mouse model by *K. pneumoniae* ATCCBAA-2344 producing KPC-2**

| Antibacterial agent | Dose(mg/kg) | Survival rate (%)* | $ED_{50}$ of MEPM(mg/kg) (95% confidence intervals) |
|---|---|---|---|
| **MEPM** | 10, 30, 60, 100 | 10, 10, 10, 50 | 205 (ND) |
| **MEPM / Compound A[a]** | 1/0.25, 3/0.75, 10/2.5, 30/7.5 | 0, 10, 90, 90 | 6.48 (3.78 - 10.5) |
| **MEPM /vaborbactam[a]** | 1/0.25, 3/0.75, 10/2.5, 30/7.5 | 10, 30, 30, 90 | 9.08 (4.4 - 27.0) |

(continued)

| Table 31. Activity of MEPM/Compound A in systemic infection lethal mouse model by *K. pneumoniae* ATCCBAA-2344 producing KPC-2 | | | |
|---|---|---|---|
| **Antibacterial agent** | **Dose(mg/kg)** | **Survival rate (%)*** | **$ED_{50}$ of MEPM(mg/kg) (95% confidence intervals)** |
| **MEPM/avibactam[a]** | 1/0.25, 3/0.75,10 2.5, 30/7.5 | 0, 10, 80, 100 | 6.18 (3.90 - 9.69) |
| **All mice were infected intraperitoneally with *K. pneumoniae* ATCC BAA-2344 at 4.75 x $10^6$ CFU (25.0 times $LD_{50}$). Ten infected mice for each group were subcutaneously treated with each agent in a single dose 2 hours after infection. ND, Not determined. * 7 days after infection. a, Coadministration with cilastatin at 100 mg/kg.** | | | |

[Table 32]

**Table 32. Activity of MEPM/Compound A in systemic infection lethal mouse model by E. *coli* ATCCBAA-2469 producing NDM-1**

| Antibacterial agent | Dose(mg/kg) | Survival rate (%)* | $ED_{50}$ of MEPM (mg/kg) (95% confidence intervals) |
|---|---|---|---|
| **MEPM** | 10, 30, 60, 100 | 10, 0, 0, 80 | 103 (ND) |
| **MEPM/Compound A[a]** | 3/0.75, 6/1.5, 10/2.5, 30/7.5 | 10, 20, 70, 90 | 8.93 (5.90-14.5) |
| **MEPM /vaborbactam[a]** | 10/2.5,30/7.5, 60/15, 100/25 | 0.0. 10, 100 | 64.8 (60.8 - 69.1) |
| **MEPM/avibactam[a]** | 10/2.5,30/7.5, 60/15, 100/25 | 0, 0, 10, 90 | 77.5 (64.4 - 93.1) |

**All mice were infected intraperitoneally with *E. coli* ATCC BAA-2469 at 1.98 x $10^6$ CFU (20.1 times $LD_{50}$). Ten infected mice for each group were subcutaneously treated with each agent in a single dose 2 hours after infection. ND, Not determined. * 7 days after infection. a, Coadministration with cilastatin at 100 mg/kg.**

(Example 15) Activity of meropenem (MEPM)/Compound A in a systemic infection lethal mouse model by carbapenemase-producing carbapenem-resistant Acinetobacter baumannii and P. aeruginosa

An in vivo activity of MEPM alone and MEPM in combination with Compound A in a systemic infection lethal mouse model by serine- and metallo-carbapenemase-producing carbapenem-resistant Acinetobacter baumannii and P. aeruginosa was examined.

Test strains were cultured on a Mueller Hinton agar (MHA) at 35 to 36°C for 10 to 17 hours twice. The test strains on the agar medium were suspended in saline and adjusted to a Klett unit of about 200 using a Klett Photoelectric Colorimeter. The resulting bacterial suspension was further diluted with saline and diluted 2-fold with a 16% mucin suspension to prepare a bacterial suspension for infection. Dissolved a Meropen for Intravenous Infusion vail in saline and adjusted to a target concentration. Compound A and cilastatin were dissolved in saline with sodium carbonate and adjusted with saline to a target concentration. These solutions were mixed to prepare an MEPM/cilastatin mixed solution and an MEPM/Compound A/cilastatin mixed solution. Note that, for the purpose of suppressing degradation of MEPM by a dehydropeptidase-I in a mouse, the agent at each concentration was administered in combination with cilastatin at 100 mg/kg, which can inhibit this enzyme. Male ICR mice were inoculated intraperitoneally with 0.2 mL of the bacterial suspension. Two hours after the inoculation, saline or a solution for administering the agent of each concentration was administered subcutaneously to the mice in a single dose. The number of mice that survived from Day 1 to Day 7 after infection was recorded. A 50% survival dose ($ED_{50}$) and a 95% confidence intervals for each agent were calculated from the number of mice alive on Day 7 at each dose of the MEPM using the probit method. A 50% lethal inoculum dose ($LD_{50}$) was calculated using the probit based on the number of mice alive on Day 7 at each infectious dose.

The $ED_{50}$ of the MEPM alone against Acinetobacter baumannii CDC-83 (NDM-1, OXA-23, OXA-69), CDC-303 (OXA-23, OXA-66), P. aeruginosa CDC-90 (KPC-5), and CDC-110 (VIM-2) infection models were 300 mg/kg or more, 300 mg/kg or more, 54.8 mg/kg, and 218 mg/kg, respectively (Tables 33 and 34). When being used in combination with the Compound A, the $ED_{50}$ of MEPM decreased against Acinetobacter baumannii CDC-83 and CDC-303 to 16.3 mg/kg and 73.7 mg/kg, respectively, and against P. aeruginosa CDC-90 and CDC-110 to 6.03 mg /kg and 50.9 mg/kg, respectively. The Compound A reduced an effective dose of the MEPM to 1/9 for serine-carbapenemase-producing P. aeruginosa, 1/4 for metallo-carbapenemase-producing P. aeruginosa, 1/25 for serine-carbapenemase-producing Acinetobacter baumannii, and 1/89 for metallo-carbapenemase-producing Acinetobacter baumannii.

[Table 33]

| Table 33. Activity of MEPM/Compound A in systemic infection lethal mouse model by *P. aeruginosa* | | | |
|---|---|---|---|
| **Antibacterial agent** | **Dose*(mg/kg)*** | **Survival rate (%)*** | **$ED_{50}$ of MEPM *(mg/kg (95%* confidence intervals)** |
| ***CDC-90*** **(Serine)** | | | |
| ***MEPM[a]*** | *10, 30, 100, 300* | *0, 10, 90, 100* | 54.8 (35.8-83.9) |
| ***MEPM / Compound A[a]*** | *3/3, 10/10, 30/30, 100/100* | *20, 70, 100, 100* | *6.03* (3.28 - 1*0)* |
| ***CDC-110*** (**Metallo**) | | | |
| ***MEPM[a]*** | *100, 150, 200, 300* | *0, 30, 60, 60* | *218 (170-346)* |
| ***MEPM / Compound A[a]*** | *30 30, 60/60, 100/100, 150/150* | *10, 70, 90, 100* | *50.9 (36.5 -- 65.3)* |
| **All mice were infected intraperitoneally with *P. aeruginosa* CDC-90 at 5.43 x $10^4$ CFU (32.9 times $LD_{50}$) or P. aeruginosa CDC-110 at 1.81 x $10^5$ CFU (17.1 times $LD_{50}$). Ten infected mice for each group were subcutaneously treated with each agent in a single dose 2 hours after infection. * 7 days after infection. a, Coadministration with cilastatin at 100 mg/kg.** | | | |

[Table 34]

| Table 34. Activity of MEPM/Compound A in systemic infection lethal mouse model by *A. baumannii* | | | |
|---|---|---|---|
| **Antibacterial agent** | **Dose *(mg/kg)*** | **Survival rate (%)*** | **$ED_{50}$ of MEPM *(mg/kg (95%* confidence intervals)** |
| ***CDC-83 (Serine and metallo)*** | | | |
| ***MEPM[a]*** | *30, 100, 200, 300* | *0, 10, 20, 40* | *ND [401^]* |
| ***MEPM / Compound A[a]*** | *10/10, 20/20, 30/30, 100/100* | *20, 60, 90, 100* | *16.3 (10.7 - 22.0)* |
| ***CDC-303*** **(Serine)** | | | |
| ***MEPM[a]*** | *50, 100, 200, 300* | *10, 0, 10, 20* | *ND [6550^]* |
| ***MEPM / Compound A[a25/25]*** | *25/25, 50/50, 100/100, 200/200* | *10, 10, 70, 100* | *73.7 (53.5 - 105)* |
| **All mice were infected intraperitoneally with *A. baumannii* CDC-83 at 1.32 x $10^5$ CFU (9.2 times $LD_{50}$) or *A. baumannii* CDC-303 at 2.35 x $10^6$ CFU (37.1 times $LD_{50}$). Ten infected mice for each group were subcutaneously treated with each agent in a single dose 2 hours after infection. ND, Not determined. * 7 days after infection. a, Coadministration with cilastatin at 100 mg/kg. ^, Value estimated by probit analysis.** | | | |

(Example 16) In vivo antibacterial activity of meropenem (MEPM)/Compound A against carbapenemase-producing Enterobacteriaceae of complicated urinary tract infection model of neutropenic mouse

An in vivo activity of MEPM in combination with Compound A against NDM-5-producing Escherichia coli CDC-150 and KPC-2-producing Klebsiella pneumoniae ATCC BAA-2344 was evaluated in a complicated urinary tract infection model of a neutropenic mouse.

The test strains were cultured on a Mueller Hinton agar (MHA) at 35°C for 22 hours. The thus-cultured test strains were then suspended in 25 mL of a Luria-Bertani medium and cultured at 35°C for 18.5 hours. The resulting culture solution was diluted with sterile saline to a bacterial concentration of 5 x $10^5$ to 6 x $10^5$ CFU/mL. Dissolved a Meropen for Intravenous Infusion vial in saline and adjusted to a target concentration. Compound A was dissolved in saline with sodium carbonate. These solutions were mixed to prepare a MEPM/Compound A mixed solution.

In order to induce neutropenia, cyclophosphamide was administered intraperitoneally to a female ICR mouse at 150 mg/kg and 100 mg/kg 4 days and 1 day before infection, respectively. Under isoflurane anesthesia, 3 to 5 hours before treatment on the day of infection, the skin over the left kidney of a neutropenic mouse was shaved and incised, the left renal pelvis was inoculated with 0.05 mL of a bacterial suspension (2 x $10^4$ to 3 x $10^4$ CFU/mouse), and the incision was sutured. The thus-infected mouse was administered subcutaneously with MEPM at 30 mg/kg and Compound A at 30 mg/kg every 3 hours for 24 hours. The number of bacteria in the kidney was measured at about 4 hours and 28 to 29 hours after infection. The mouse was euthanized and the kidney was removed therefrom. Sterile saline was added thereto and the kidney was

homogenized using a Multi-Beads shocker (Yasui Kikai Corporation). Approximately 10-fold serial dilutions of the kidney homogenate were prepared using sterile saline, and 0.1 mL of each dilution was smeared on an MHA. The agar medium was cultured at 35°C for 1 day and colonies were counted. A decrease of less than 1 $log_{10}$ CFU/kidney and a decrease of 1 $log_{10}$ CFU/kidney or more relative to the number of bacteria in the kidney at the start of administration were defined as a bacteriostatic activity and a bactericidal activity, respectively.

In both an Escherichia coli CDC-150 infection model and a Klebsiella pneumoniae ATCC BAA-2344 infection model, a control group (saline), a 30 mg/kg MEPM every 3 hours (q3h) administration group, and a 30 mg/kg MEPM and 30 mg/kg Compound A q3h administration group showed growth of about 3 logs, growth of 1 log, and a decrease of 1 log or more relative to the number of bacteria in the kidney at the start of administration, respectively (FIG. 2). These results confirm that the Compound A enhances an in vivo antibacterial activity of the MEPM in these infection models.

(Example 17) In vivo antibacterial activity of meropenem (MEPM)/Compound A against carbapenemase-producing Acinetobacter baumannii in respiratory infection model of neutropenic mouse

In vivo effects of MEPM alone and in combination with Compound A were examined in a MEPM-resistant Acinetobacter baumannii CDC-277 respiratory infection model of a neutropenic mouse using the number of bacteria in the lungs as an indicator. The Compound A was administered to the same infection model mouse in a single dose, and a concentration of the Compound A in plasma and alveolar epithelial lining fluid (ELF) was measured.

Test strains were cultured in 20 mL of a Brain Heart Infusion medium for 6 hours at 35 to 37°C with shaking. The resulting culture solution was centrifuged to remove supernatant and resuspended in cooled phosphate-buffered saline, which was used as an inoculum. Dissolved a Meropenem injection vial in saline and adjusted to a target concentration. Cilastatin and Compound A were dissolved in saline with equal molar sodium carbonate and adjusted to a target concentration with saline. These solutions were mixed to prepare an MEPM/cilastatin mixed solution and an MEPM/-Compound A/cilastatin mixed solution. Note that, for the purpose of suppressing degradation of MEPM by a dehydropeptidase-I in a mouse, the agent at each concentration was administered in combination with cilastatin at 100 mg/kg, which can inhibit this enzyme. Colistin sulfate was dissolved in water and adjusted to a target concentration with saline. In order to induce neutropenia, cyclophosphamide was administered intraperitoneally to female BALB mice at 150 mg/kg and 100 mg/kg 4 days and 1 day before infection, respectively. Two hours before the start of treatment on the day of infection, 0.01 mL of the inoculum was inoculated into each of the right and left nasal cavities of a neutropenic mouse under anesthesia (about 1 x $10^6$ CFU/mouse). Then, 100 mg/kg MEPM/100 mg/kg cilastatin was administered subcutaneously to the thus-infected mouse for 8 doses at 3-hour intervals starting 2 hours after infection, and when the Compound A was used in combination, it was administered subcutaneously for 2 doses (12-hour intervals), 4 doses (6-hour intervals), or 8 doses (3-hour intervals) with the total dose kept constant at 240 and 480 mg/kg. As a positive control, colistin at 30 mg/kg was administered subcutaneously every 12 hours. Saline serving as a control was administered subcutaneously for 8 doses at 3-hour intervals (10 mL/kg/dose). The number of bacteria in the lungs was measured at 2 and 26 hours after infection. The mouse was euthanized and the lungs were removed therefrom. Phosphate-buffered saline was added thereto and the lungs were homogenized using a polytron homogenizer. Ten-fold serial dilutions of the lung homogenate were prepared using phosphate-buffered saline, and 0.1 mL of each dilution was smeared on a MacConkey II agar. The agar medium was cultured for 1 day and colonies were counted. ANOVA was used to test for a significant difference in the number of bacteria in the lungs. In the same infection mouse model, the Compound A was administered subcutaneously in a single dose at 30 to 240 mg/kg 2 hours after infection, and cardiac blood samples were taken under anesthesia at 5, 15, 30, 60, 120, 240, 480, and 1440 minutes after administration. Bronchoalveolar lavage fluid (BALF) was collected with 0.5 mL of phosphate-buffered saline immediately after blood collection. Plasma and the BALF were analyzed by liquid chromatography-tandem mass spectrometry (LC-MS/MS) to determine a concentration of the Compound **A.** Measurement conditions for LC-MS/MS are as follows.

HPLC: ExionLC series (SHIMADZU CORPORATION)
MS/MS: Triple Quad 5500+ (SCIEX)
Column: Atlantis T3, 3 μm, 50 x 2.1 mm (Waters Corp.)
Mobile phase: A: water containing 0.2% formic acid, B: methanol containing 0.2% formic acid
Detection: ESI (positive mode)
Monitor ion (Q1 -> Q3, m/z): 387.3 -> 342.4

A concentration of the Compound A in the ELF was corrected by measuring urea concentrations in the BALF and the plasma. Various PK parameters in the plasma and the ELF were calculated by non-compartmental analysis using WinNonlin.

The number of bacteria in the lungs of the control group at 26 hours after infection increased by 3 logs compared to the start of treatment (FIG. 3). The number of bacteria in the lungs of the colistin-treated group serving as the positive

control decreased by 0.5 logs compared to the start of treatment, indicating a bacteriostatic effect. The number of bacteria in the lungs of the MEPM alone group increased by 0.1 logs compared to the start of treatment. There was a significant difference to the control in the number of bacteria in the lungs for both treatment groups. When the Compound A was used in combination with the MEPM, the number of bacteria in the lungs was decreased by 3 logs or more compared to the start of treatment for both regimens of the Compound A, that is, a bactericidal effect was observed, and there were significant differences to the control group and the MEPM alone group ($p < 0.05$). The PK parameters of the Compound A in the plasma and the ELF after subcutaneous administration of the Compound A are shown (Tables 35 and 36). An ELF penetration was approximately 70 to 100% at 30 to 240 mg/kg, suggesting that the Compound A, concomitantly with the MEPM, migrated well to an infected region in the lung and showed excellent efficacy.

[Table 35]

**Table 35. Plasma PK parameters of Compound A after administration of Compound A to *A. baumannii* lung infection model**

| Compound A (mg/kg) | $t_{1/2}$ (h) | $C_{max}$(μg/mL) | $AUC_{0-inf}$ (μg.h/mL) |
|---|---|---|---|
| 30 | 1.35 | 61.1 | 47.0 |
| 60 | 1.39 | 88.4 | 71.0 |
| 120 | 4.45 | 193.5 | 146.4 |
| 240 | 2.69 | 294.4 | 297.2 |

[Table 36]

**Table 36. ELF PK parameters of Compound A after administration of Compound A to *A. baumannii* lung infection model**

| Compound A (mg/kg) | $t_{1/2}$ (h) | $C_{max}$(μg/mL) | $AUC_{0-info}$ (μg.h/mL) |
|---|---|---|---|
| 30 | 1.76 | 18.2 | 33.0 |
| 60 | 2.81 | 28.6 | 68.0 |
| 120 | 3.78 | 61.1 | 131.0 |
| 240 | 3.87 | 141.5 | 299.2 |

(Example 18) In vivo antibacterial activity of meropenem (MEPM)/Compound A against carbapenemase-producing Klebsiella pneumoniae in intramuscular infection model of neutropenic mouse

An in vivo activity of Compound A at various dosage and administration in combination with MEPM was evaluated in a neutropenic mouse model intramuscularly infected with Klebsiella pneumoniae harboring a serine- or metallo-carbapenemase (KPC-2, KPC-3, KPC-38, NDM-1, IMP-1).

Test strains were cultured overnight at 36°C in a cation-adjusted Mueller Hinton medium, and then added to the same fresh medium to 1.5 McFarland and cultured at 36°C for 2 hours with shaking. The resulting culture solution was diluted with saline, which was used as an inoculum (about 1 x $10^7$ CFU/mL). Dissolved a Meropenem for Intravenous Infusion vial in saline and adjusted to a target concentration. Cilastatin sodium was dissolved with saline and mixed with MEPM to prepare a MEPM/cilastatin mixed solution having a target concentration. Compound A was dissolved in saline with sodium carbonate or the MEPM/cilastatin mixed solution and diluted with the MEPM/cilastatin mixed solution to prepare a MEPM/Compound A/cilastatin mixed solution with a target concentration. Note that, for the purpose of suppressing degradation of MEPM by a dehydropeptidase-I in a mouse, the agent at each concentration was administered in combination with cilastatin at 100 mg/kg, which can inhibit this enzyme. In order to induce neutropenia, cyclophosphamide was administered intraperitoneally to a male ICR mouse at 150 mg/kg and 100 mg/kg 4 days and 1 day before infection, respectively. Two hours before the start of treatment on the day of infection, 0.1 mL of the inoculum was inoculated into the left gastrocnemius muscle of a neutropenic mouse under anesthesia (about 1 x $10^6$ CFU/mouse). Then, MEPM at 5 or 100 mg/kg and cilastatin at 100 mg/kg were administered subcutaneously to the thus-infected mouse for 8 doses at 3-hour intervals starting 2 hours after infection, and when the Compound A was used in combination, it was administered subcutaneously for 1 dose, 2 doses (12-hour intervals), 4 doses (6-hour intervals), or 8 doses (3-hour intervals) with the total dose kept constant at 1, 4, 15, 16, 60, 240, or 960 mg/kg. Saline serving as a control was administered subcutaneously for 8 doses at 3-hour intervals (10 mL/kg/dose). The number of bacteria in the muscle was measured at 2 and 26 hours after infection. The mouse was euthanized and the left gastrocnemius muscle was removed

therefrom. Saline was added thereto and the muscle was homogenized using a Multi-Beads shocker. Approximately 10-fold serial dilutions of the muscle homogenate were prepared using saline, and 0.1 mL of each dilution was smeared on a Mueller Hinton agar. The agar medium was cultured for 1 day and colonies were counted. A decrease of less than 1 log or 1 log or more in the number of bacteria in muscle in the drug-treated group compared to that at the start of treatment was considered to indicate a bacteriostatic effect and a bactericidal effect, respectively.

For the below-described four bacterial infection models in which MEPM at 5 mg/kg was administered every 3 hours (FIG. 6A to D), a bacteriostatic effect was observed for Compound A at 16 mg/kg/day in a KPC-2-producing bacterial infection model. A 1-log bactericidal effect was observed for Compound A at 60 mg/kg/day or more in a KPC-3-producing bacterial infection model. A 1-log bactericidal effect and a bacteriostatic effect were observed for Compound A at 240 mg/kg/day or more and 60 mg/kg/day, respectively, in a NDM-1-producing bacterial infection model. A bacteriostatic effect and a 1-log bactericidal effect were observed for Compound A at 60 mg/kg/day or more and 960 mg/kg/day, respectively, in an IMP-1-producing bacterial infection model. A 2-log bactericidal effect and a bacteriostatic effect were observed for Compound A at 240 mg/kg/day or more and 60 mg/kg/day, respectively, in a KPC-38-producing bacterial infection model in which MEPM at 100 mg/kg was administrated every 3 hours (FIG. 6E). A series of test results indicate that for the same total daily dose of the Compound A, an antibacterial activity of the regimen with more divided doses of the Compound A is not attenuated compared to that of the single dose, and a peak concentration of the Compound A does not affect enhancement of a MEPM activity. The Compound A was suggested to enhance an in vivo antibacterial activity of MEPM against serine- and metallo-carbapenemase-producing bacteria in a dose-dependent manner in the present infection model.

(Example 19) In vivo antibacterial activity of meropenem (MEPM)/Compound A against carbapenemase-producing Acinetobacter baumannii in intramuscular infection model of neutropenic mouse

An in vivo activity of Compound A at various dosage and administration in combination with MEPM was evaluated in an intramuscular infection model of a neutropenic mouse against Acinetobacter baumannii CDC-83 (producing NDM-1, OXA-23, OXA-69), CDC-277 (producing OXA-24, OXA-65), CDC-301 (producing OXA-66, OXA-72), CDC-293 (producing OXA-66, OXA-72), and CDC-289 (producing OXA-66, OXA-72).

Test strains were cultured overnight at 35 to 36°C in a cation-adjusted Mueller Hinton medium, and then added to the same fresh medium to 1.5 McFarland and cultured at 36°C for 2 hours with shaking. The resulting culture solution was diluted with saline, which was used as an inoculum (approximately 1 x $10^7$ CFU/mL). Dissolved a Meropenem for Intravenous Infusion vial in saline and adjusted to a target concentration. Cilastatin was dissolved in saline with sodium carbonate and mixed with MEPM to prepare a MEPM/cilastatin mixed solution having a target concentration. Compound A was dissolved in a MEPM/cilastatin mixed solution with sodium carbonate and diluted with the MEPM/cilastatin mixed solution to prepare a MEPM/Compound A/cilastatin mixed solution with a target concentration. Note that, for the purpose of suppressing degradation of meropenem by a dehydropeptidase-I in a mouse, the agent at each concentration was administered in combination with cilastatin at 100 mg/kg, which can inhibit this enzyme. In order to induce neutropenia, cyclophosphamide was administered intraperitoneally to a male ICR mouse at 150 mg/kg and 100 mg/kg 4 days and 1 day before infection, respectively. Two hours before the start of treatment on the day of infection, 0.1 mL of the inoculum was inoculated into the left gastrocnemius muscle of a neutropenic mouse under anesthesia (about 1 x $10^6$ CFU/mouse). Then, 100 mg/kg MEPM/100 mg/kg cilastatin were administered subcutaneously to the thus-infected mouse for 8 doses at 3-hour intervals starting 2 hours after infection, and when the Compound A was used in combination, it was administered subcutaneously for 1 dose, 2 doses (12-hour intervals), 4 doses (6-hour intervals), or 8 doses (3-hour intervals) with the total dose kept constant at 60, 240, 480, or 960 mg/kg (except for 960 mg/kg in a single dose). Saline serving as a control was administered subcutaneously for 8 doses at 3-hour intervals (10 mL/kg/dose). The number of bacteria in the muscle was measured at 2 and 26 hours after infection. The mouse was euthanized and the left gastrocnemius muscle was removed therefrom. Saline was added thereto and the muscle was homogenized using a Multi-Beads shocker. Approximately 10-fold serial dilutions of the muscle homogenate were prepared using saline, and 0.1 mL of each dilution was smeared on a Mueller Hinton agar. The agar medium was cultured for 1 day and colonies were counted. A decrease of less than 1 log or 1 log or more in the number of bacteria in the muscle in the drug-treated group compared to that at the start of treatment was considered to indicate a bacteriostatic effect and a bactericidal effect, respectively. A 2-log or more bactericidal effect and a bacteriostatic effect were observed for Compound A at 240 mg/kg/day and 60 mg/kg/day, respectively, in CDC-83, CDC-277, and CDC-301 infection models (FIGs. 7A to 7C). A 2-log or more bactericidal effect was observed for Compound A at 240 mg/kg/day in a CDC-293 infection model (FIG. 7D). A 2-log or more bactericidal effect and a 1-log bactericidal effect were observed for Compound A at 240 mg/kg/day and 60 mg/kg/day, respectively, in a CDC-289 infection model (FIG. 2E). A series of test results indicate that for the same total daily dose of Compound A, an antibacterial activity of the regimen with more divided doses of the Compound A is not attenuated compared to that of the single dose, and a peak concentration of the Compound A does not affect enhancement of a MEPM activity. The Compound A was suggested to enhance an in vivo antibacterial activity of MEPM against serine- and metallo-carbapenemase-producing bacteria in a dose-dependent manner in the present infection model.

(Example 20) Measurement of plasma concentration of Compound A in intramuscular infection model of neutropenic mouse

To evaluate a PK/PD relationship for efficacy of Compound A against carbapenem-resistant bacteria, a plasma concentration of the Compound A in an intramuscular infection model with Klebsiella pneumoniae KUB3606 (KPC-38-producing) of a neutropenic mouse was measured. The Klebsiella pneumoniae KUB3606 was cultured overnight at 36°C in a cation-adjusted Mueller Hinton medium, and then added to the same fresh medium to 1.5 McFarland and cultured at 36°C for 2 hours with shaking. The resulting culture solution was diluted with saline, which was used as an inoculum (approximately 1 x $10^7$ CFU/mL). The Compound A was dissolved in saline with sodium carbonate and diluted with saline to prepare a Compound A solution with a target concentration. In order to induce neutropenia, cyclophosphamide was administered intraperitoneally to a male ICR mouse at 150 mg/kg and 100 mg/kg 4 days and 1 day before infection, respectively. On the day of infection, 0.1 mL of the inoculum was inoculated into the left gastrocnemius muscle of a neutropenic mouse under anesthesia (about 1 x $10^6$ CFU/mouse). The Compound A at 5, 20, and 100 mg/kg was administered subcutaneously to the thus-infected mouse in a single dose starting 2 hours after infection. Cardiac blood was collected under anesthesia at 5 minutes, 15 minutes, 30 minutes, 1 hour, 2 hours, 4 hours, 8 hours, and 24 hours after administration and plasma was prepared.

A plasma concentration of the Compound A was measured using LC-MS/MS. Measurement conditions for LC-MS/MS are as follows.

HPLC: Shimadzu 20A series (SHIMADZU CORPORATION)
MS/MS: API 4000 (SCIEX)
Column: Atlantis T3, 3 μm, 50 x 2.1 mm (Waters Corp.)
Column temperature: 40°C
Mobile phase: A: water containing 0.1% formic acid, B: acetonitrile
A/B (min): 95/5 (0.01) -> 95/5 (1) -> 5/95 (2) -> 5/95 (3.5) -> 95/5 (3.51) -> 95/5 (7)
Flow rate: 0.4 mL/min
Detection: ESI (positive mode)
Monitor ion (Q1 -> Q3, m/z): 387.02 -> 341.94

The Compound A was rapidly absorbed and excreted, and its concentration increased with increasing dose. Plasma concentration data were well described by the two-compartment model. Estimated parameters were V1/F of 0.911 to 1.27L/kg, Ka of 8.39 to 14.4/hr, Ke of 1.91 to 2.35/hr, K12 of 0.104 to 0.210/hr, and K21 of 0.609/hr, and the standard deviation of proportional residual error was 0.0641 to 0.104.

(Example 21) Measurement of plasma concentration of Compound A in rat, dog, and monkey

To evaluate a PK of Compound A in each animal species, the Compound A was administered intravenously to a rat, a dog, and a monkey at 5 mg/kg, 1 mg/kg, and 1 mg/kg, respectively, in a single dose. The blood was collected at 5 minutes, 15 minutes, 30 minutes, 1 hour, 2 hours, 4 hours, 6 hours, and 24 hours after administration and plasma was prepared. A plasma concentration of the Compound A was measured using LC-MS/MS. Measurement conditions for LC-MS/MS are as follows.

HPLC: Shimadzu 20A series (SHIMADZU CORPORATION)
MS/MS: API 4000 (SCIEX)
Column: CAPCELL PAK ADME, 3 μm, 50 x 2.1 mm (OSAKA SODA Co., Ltd.) or Atlantis T3, 3 μm, 50 x 2.1 mm (Waters Corp.)
Column temperature: 40°C
Mobile phase: A: water containing 0.1% formic acid, B: acetonitrile
A/B(min): 95/5 (0) -> 95/5 (1) -> 5/95 (3.5) -> 5/95 (5) -> 95/5 (5) -> 95/5 (5.01) -> 95/5 (7) or 95/5 (0) -> 95/5 (1) -> 5/95 (2) -> 5/95 (3.5) -> 95/5 (3.51) -> 95/5 (7)
Flow rate: 0.4 mL/min
Detection: ESI (positive mode)
Monitor ion (Q1 -> Q3, m/z): 387.02 -> 341.94
PK parameters obtained by the noncompartmental analysis are shown below.

[Table 37]

| | Rats (n = 3) | Dogs (n = 2) | Monkeys (n = 2) |
|---|---|---|---|
| $C_0$ (μg/mL) | 18.0 ± 0.7 | 6.68 | 11.4 |
| $AUC_{0-\infty}$ (μg·h/mL) | 13.6 ± 3.6 | 6.59 | 7.19 |
| CL (L/h/kg) | 0.384 ± 0.102 | 0.153 | 0.141 |
| $V_{ss}$ (L/kg) | 0.380 ± 0.011 | 0.373 | 0.193 |
| $t_{1/2}$ (h) | 0.819 ± 0.101 | 2.50 | 1.30 |

(Example 22) Plasma protein binding rate of Compound A

Samples of mouse, rat, dog, monkey, and human plasma spiked with [14C]Compound A at 3 to 3000 μmol/L (3 to 300 μmol/L for human plasma only) were incubated at 37°C for 5 min, transferred to an ultrafilter (Centrifree (Nominal Molecular Weight Limit: 30000, Merck)), and centrifuged. A radioactivity concentration in the resulting filtrate was measured using a liquid scintillation counter (Tri-carb 4910TR (PerkinElmer)). Protein binding rates in mouse, rat, dog, monkey, and human plasma were 15.1% to 23.1%, 22.0% to 45.9%, 18.1% to 36.1%, 38.3% to 60.7%, and 70.9% to 78.6%, respectively.

(Example 23) PK prediction of Compound A in a clinical setting

A PK in a clinical setting was predicted based on the allometric scaling method from data on PK parameters and various plasma protein binding rates in a rat, a dog, and a monkey. Predicted values for the parameters were 0.620 mL/min/kg for CL, 0.116 L/kg for Vss, and 2.2 h for t1/2. A predicted half-life of Compound A was similar to that of meropenem (Reference: Carbapenem antibiotic, the Japanese Pharmacopoeia package insert, Meropenem for I.V. Infusion), and was estimated to support administration of the Compound A and the meropenem every 8 hours.

(Example 24) Measurement of plasma concentration of meropenem (MEPM)/cilastatin of intramuscular infection model in neutropenic mouse

To evaluate a PK/PD relationship for efficacy of Compound A against carbapenem-resistant bacteria, plasma concentrations of MEPM and cilastatin in an intramuscular infection model with Klebsiella pneumoniae KUB3606 (KPC-38-producing) of a neutropenic mouse was measured.

The Klebsiella pneumoniae KUB3606 was cultured overnight at 36°C in a cation-adjusted Mueller Hinton medium, and then added to the same fresh medium to 1.5 McFarland and cultured at 36°C for 2 hours with shaking. The resulting culture solution was diluted with saline, which was used as an inoculum (approximately 1 x $10^7$ CFU/mL). Dissolved a Meropenem for Intravenous Infusion vial in saline and adjusted to a target concentration. Cilastatin sodium was dissolved with saline and mixed with meropenem to prepare a MEPM/cilastatin mixed solution having a target concentration. In order to induce neutropenia, cyclophosphamide was administered intraperitoneally to a male ICR mouse at 150 mg/kg and 100 mg/kg 4 days and 1 day before infection, respectively. On the day of infection, 0.1 mL of the inoculum was inoculated into the left gastrocnemius muscle of a neutropenic mouse under anesthesia (about 1 x $10^6$ CFU/mouse). Meropenem at 5, 20, and 100 mg/kg was administered subcutaneously to the thus-infected mouse in a single dose in combination with cilastatin at 100 mg/kg starting 2 hours after infection. Cardiac blood was collected under anesthesia at 5 minutes, 15 minutes, 30 minutes, 1 hour, 1.5 hours, 2 hours, 3 hours, and 4 hours after administration and plasma was prepared.

Plasma concentrations of meropenem and cilastatin were measured using LC-MS/MS. Measurement conditions for LC-MS/MS are as follows.

HPLC: Shimadzu 20A series (SHIMADZU CORPORATION)
MS/MS: API 4000 (SCIEX)
Column: CAPCELL PAK C18 MGII, 3 μm, 50 x 2 mm (Shiseido Co., Ltd.)
Column temperature: 40°C
Mobile phase: A: water containing 0.1% formic acid, B: methanol
A/B (min): 95/5 (0.01) -> 95/5 (1) -> 5/95 (5) -> 5/95 (7) -> 95/5 (7.01) -> 95/5 (9)
Flow rate: 0.4 mL/min
Detection: ESI (positive mode)
Monitor ion (Q1 -> Q3, m/z): MEPM 384.183 -> 67.940, cilastatin: 359.239 -> 97.016

MEPM was rapidly absorbed and excreted, and its concentration increased with increasing dose. An exposure amount to cilastatin at 100 mg/kg was similar when combined with meropenem at doses of 5, 20, and 100 mg/kg. Plasma concentration data for the meropenem were well described by the 1-compartment model. Estimated parameters were V1/F of 0.452 to 0.669 L/kg, Ka of 6.50 to 11.2/hr, Ke of 2.53 to 2.91/hr, and the standard deviation of proportional residual error of 0.238 to 0.270.

(Example 25) Evaluation of in vivo pharmacodynamic target of Compound A against carbapenemase-producing Enterobacteriaceae (CPE) in intramuscular infection model of immunocompromised mouse

Pharmacokinetics/Pharmacodynamics (PK/PD) parameters correlating with efficacy of Compound A in combination with MEPM against CPE were analyzed using PK parameters of Compound A and data on change in the number of bacteria in the muscle in an intramuscular infection model of an immunocompromised mouse. Phoenix WinNonlin and Microsoft Excel were used to simulate a plasma concentration of the Compound A from PK parameters at 100 mg/kg and calculate a plasma concentration of the Compound A at the dose used for assessing the number of bacteria in the muscle. The below-described PK/PD parameters were then calculated for each dosage and administration and examined for PK/PD parameters that correlated with the change in the number of bacteria in the muscle. An unbound area under a plasma concentration-time curve from 0 to 24 hours (fAUC), an unbound maximum plasma concentration ($fC_{max}$), fAUC/MEPM-Compound A (M-K) MIC, $fC_{max}$/M-K MIC, a proportion of time for which a free form concentration is above M-K MIC in a dosing interval (%fT > M-K MIC), and a proportion of time for which a free form concentration is above a threshold concentration in a dosing interval (%fT > $C_T$) were used as PK/PD parameters.

Efficacy data on a change in the number of bacteria in the muscle in 5 strains of Klebsiella pneumoniae were pooled and analyzed. Correlation between the efficacy data and each PK/PD parameter was evaluated with a sigmoidal $E_{max}$ model. $E = (E_{max} \times D^N) / (ED_{50}{}^N + D^N)$ in which $E_{max}$ is the maximum reduction in the number of bacteria in the muscle, D is each PK/PD parameter, $ED_{50}$ is a D value required for 50% of $E_{max}$, and N is the Hill coefficient indicating sigmoidality of a dose-response curve. For %fT > $C_T$, 0.25, 0.5, 1, 2, and 4 ug/mL were set as CT. The M-K MIC for Compound A at fixed concentrations of 4, 8, or 16 ug/mL was expressed as M-K MIC 4 μg/mL, M-K MIC 8 μg/mL, or M-K MIC 16 μg/mL, respectively. Analysis using each of the above PK/PD parameters showed the highest correlation ($R^2 > 0.7$) between %fT > 1 μg/mL or fAUC, and efficacy as indexed by the change in the number of bacteria in the muscle (FIG. 8). Values required for bacteriostatic and bactericidal effects (1 log kill) for %fT > 1 ug/mL were 12.2 and 36.4%, respectively. Values required for bacteriostatic and bactericidal effects (1 log kill) for fAUC were 15 and 134 μg·h/mL, respectively.

(Example 26) Evaluation of in vivo pharmacodynamic target of Compound A against carbapenemase-producing Acinetobacter baumannii in intramuscular infection model of immunocompromised mouse

Pharmacokinetics/Pharmacodynamics (PK/PD) parameters correlating with efficacy of Compound A in combination with MEPM against carbapenemase-producing Acinetobacter baumannii were analyzed using PK parameters of the Compound A and data on change in the number of bacteria in the muscle in an intramuscular infection mouse model. Phoenix WinNonlin and Microsoft Excel were used to simulate a plasma concentration of Compound A from PK parameters at 100 mg/kg and calculate a plasma concentration of the Compound A at the dose used for assessing the number of bacteria in the muscle. The below-described PK/PD parameters were then calculated for each dosage and administration and examined for PK/PD parameters that correlated with the change in the number of bacteria in the muscle. An unbound area under a plasma concentration-time curve from 0 to 24 hours (fAUC), an unbound maximum plasma concentration ($fC_{max}$), fAUC/MEPM-Compound A (M-K) MIC, $fC_{max}$/M-K MIC, a proportion of time for which a free form concentration is above M-K MIC in a dosing interval (%fT > M-K MIC), and a proportion of time for which a free form concentration is above a threshold concentration in a dosing interval (%fT > $C_T$) were used as PK/PD parameters.

Efficacy data on a change in the number of bacteria in the muscle in 5 strains of Acinetobacter baumannii were pooled and analyzed. Correlation between the efficacy data and each PK/PD parameter was evaluated with a sigmoidal $E_{max}$ model. $E = E_0 + (E_{max} \times D^N) / (ED_{50}{}^N + D^N)$ in which $E_0$ is a change in the number of bacteria in the muscle from baseline at which D is 0, $E_{max}$ is a maximum reduction in the number of bacteria in the muscle, D is each PK/PD parameter, $ED_{50}$ is a D value required for 50% of $E_{max}$, and N is the Hill coefficient indicating sigmoidality of a dose-response curve. For %fT > $C_T$, 0.5, 1, 2, 4, and 8 μg/mL were set as $C_T$. The M-K MIC for Compound A at fixed concentrations of 4, 8, or 16 ug/mL was expressed as M-K MIC 4 μg/mL, M-K MIC 8 μg/mL, or M-K MIC 16 μg/mL, respectively. Analysis using each of the above PK/PD parameters showed the highest correlation ($R^2 > 0.6$) between %fT > 4 ug/mL or fAUC, and efficacy as indexed by the change in the number of bacteria in the muscle (FIG. 9). Values required for bacteriostatic and bactericidal effects (1 log kill) for %fT > 4 μg/mL were 7.2 and 13.3%, respectively. Values required for bacteriostatic and bactericidal effects (1 log kill) for fAUC were 32 and 74 μg·h/mL, respectively. Note that, values required for a 2-log kill in %fT > 4 μg/mL and fAUC were 22.3% and 142 μg·h/mL, respectively. Although the %fT > 4 μg/mL and the fAUC had comparable correlation coefficients ($R^2$ of 0.61 and 0.63, respectively), the %fT > 4 μg/mL was determined as the best PK/PD

parameter correlating with drug efficacy of Compound A after taking into account visual evaluation of a plot against the change in the number of bacteria in the muscle.

For the purpose of suppressing degradation of meropenem by rodent dehydropeptidase-I in an intramuscular infection model of a neutropenic mouse, a dehydropeptidase-I inhibitor cilastatin at 100 mg/kg was administered in combination for each dose of the meropenem (Reference 1). In the above infection model, ·BR>$fC_{max}$ and $fAUC_{0\text{-}24h}$ were 85.4 μg/mL and 424 μg·h/mL, respectively, when the meropenem at 100 mg/kg was administered every 3 hours for 8 doses in combination with the cilastatin at 100 mg/kg. A human protein binding rate of the meropenem is 2% (Reference 2), and the $C_{max}$ and the $fAUC_{0\text{-}24h}$ in a human have been reported to be 46.0 μg/mL (Reference 3) and 402 μg·h/mL (Reference 2), respectively, when 2 g of the meropenem is administered intravenously for 3 hours every 8 hours. Therefore, unbound plasma pharmacokinetics of the meropenem in a human at this dosage and administration were considered to be similar to the unbound plasma pharmacokinetics of the meropenem in the above mouse infection model. The %fT > MIC required for a bactericidal activity of a carbapenem antibiotic against Gram-negative bacterial infection is recognized to be 40% (Reference 4). When the meropenem at 100 mg/kg was administered to the above mouse infection model every 3 hours for 8 doses in combination with the cilastatin at 100 mg/kg, %fT > 8 μg/mL (the intermediate breakpoint MIC for the meropenem at 6 g/day and vaborbactam at 6 g/day is 8 ug/mL (Reference 5)) reached 40% or more, similar to 56% when 2 g of the meropenem was administered by intravenous infusion for 3 hours to a human every 8 hours (Reference 2). This supports that administration of the meropenem at 100 mg/kg to the mouse infection model every 3 hours for 8 doses in combination with the cilastatin at 100 mg/kg corresponds to 6 g/day in a human.

(Reference 1) Fukasawa M, Sumita Y, Harabe ET, Tanio T, Nouda H, Kohzuki T, Okuda T, Matsumura H, Sunagawa M. 1992. Stability of Meropenem and Effect of 1β-Methyl Substitution on Its Stability in the Presence of Renal Dehydropeptidase I. Antimicrob Agents Chemother 36:1577-1579

(Reference 2) Sabet M, Tarazi Z, Nolan T, Parkinson J, Rubio-Aparicio D, Lomovskaya O, Dudley MN, Griffith DC. 2018. Activity of Meropenem-Vaborbactam in Mouse Models of Infection Due to KPC-Producing Carbapenem-Resistant Enterobacteriaceae. Antimicrob Agents Chemother 62:e01446-17

(Reference 3) Rubino CM, Bhavnani SM, Loutit JS, Morgan EE, White D, Dudley MN, Griffith DC. 2018. Phase 1 Study of the Safety, Tolerability, and Pharmacokinetics of Vaborbactam and Meropenem Alone and in Combination Following Single and Multiple Doses in Healthy Adult Subjects. Antimicrob Agents Chemother 62:e02228-17

(Reference 4) Crandon JL, Nicolau DP. 2011. Pharmacodynamic Approaches to Optimizing β-Lactam Therapy. Crit Care Clin 27:77-93

(Reference 5) CLSI. Performance Standards for Antimicrobial Susceptibility Testing. 31st ed. CLSI supplement M100. Clinical and Laboratory Standards Institute; 2021

(Example 27)

Compound A at 16 μg/mL can be said to be an excellent concentration since, in Example 7, when the Compound A at a fixed concentration of 16 μg/mL was used in combination with MEPM, the $MIC_{90}$ against Enterobacteriaceae and Acinetobacter baumannii were 0.25 and 4 μg/mL, respectively, which indicates restoration of an activity to the susceptible or intermediate breakpoint MIC of the MEPM described in the CLSI M100. In addition, in Example 3, when MEPM was used in combination with Compound A at 16 μg/mL, acquisition of resistance can be suppressed in all strains used in that experiment. Since an in vitro activity of MEPM in combination with Compound A in Example 12 was not affected by addition of human plasma or human serum albumin, it was believed that the in vitro activity could be considered in terms of a total plasma concentration rather than a free form plasma concentration. Furthermore, a PK/PD parameter that correlates with drug efficacy of MEPM is %fT > MIC, which is considered 40 to 50% as a value required to develop a bactericidal activity (considered to be 45% of an intermediate value). In other words, if the total plasma concentration of Compound A can be maintained at 16 μg/mL for a period corresponding to 45% of the dosing interval, the MIC of MEPM against carbapenem-resistant bacteria can be maintained low similar to a susceptible bacteria level.

(Example 28)

A preliminary population PK analysis and subsequent simulation were performed using data from a subset of the ongoing Phase I clinical trial of Compound A with the goal of estimating a clinically effective dose from the PK/PD index and target value obtained in the non-clinical study. NONMEM was used as an analysis software. PK parameter estimates obtained by the 2-compartment model are shown below.

[Table 38]

| Fixed effect | Estimated value (Relative standard error %) | 95% confidence intervals |
|---|---|---|
| CL (L/h) | 4.28(3.25) | 4.01-4.55 |
| V1 (L) | 11.2(3.01) | 10.5-11.9 |
| Q (L/h) | 1.12(7.42) | 0.957-1.28 |
| V2 (L) | 5.85(11.8) | 4.50-7.2 0 |
| **Interindividual variability** | **Coefficient of variation % (Shrinkage %)** | **%Relative standard error** |
| IIV CL | 22.6(0.0395) | 20.2 |
| IIV V1 | 20.8(5.3) | 27.5 |
| IIV Q | 37.0(19.6) | 29.2 |
| IIV V2 | 64.1(10.7) | 31.4 |
| **Residual variability** | **(Shrinkage %)** | **Relative standard error %** |
| **Proportional error, Coefficient of variation %** | 8.54(14.7) | 12.4 |
| **Additive error, Standard deviation** | 34.9 (14.7) | 22.8 |

The thus-obtained PK parameter estimates were used to simulate plasma concentrations of Compound A in 1000 hypothetical subjects, and the PK/PD index and an achievement rate of the target value (40% of fT > 1 μg/mL or fAUC > 134 μg·h/mL for CPE and 15% of fT > 4 ug/mL or fAUC > 74 μg·h/mL for Acinetobacter baumannii) were examined. For q8h administration at 500 mg, 90% or more of the subjects were expected to achieve 40% of fT > 1 μg/mL and 15% of fT > 4 μg/mL. For q8h administration at 1000 mg, 90% or more and 80% or more of the subjects were expected to achieve fAUC > 74 μg·h/mL and fAUC > 134 μg·h/mL, respectively. Based on these analysis results, the clinically effective dose of Compound A was estimated to be 500 mg to 1000 mg every 8 hours.

(Example 29)

In vivo antibacterial activity of meropenem (MEPM)/Compound A against carbapenemase-producing Klebsiella pneumoniae in intramuscular infection model of neutropenic mouse

An in vivo activity of Compound A at various dosage and administration in combination with MEPM was evaluated in a neutropenic mouse model intramuscularly infected with Klebsiella pneumoniae harboring serine- or metallo-carbapenemase (KPC-3, NDM-7, VIM-27, NDM-1, and OXA-232).

Test strains were cultured overnight at 35°C in a cation-adjusted Mueller Hinton medium, and then added to the same fresh medium to 1.5 McFarland and cultured at 36°C for 2 hours with shaking. The resulting culture solution was diluted with saline, which was used as an inoculum (approximately 1 x $10^7$ CFU/mL). Dissolved a Meropenem for Intravenous Infusion vial in saline and adjusted to a target concentration. Cilastatin sodium was dissolved with saline and mixed with MEPM to prepare a MEPM/cilastatin mixed solution having a target concentration. Compound A was dissolved in saline with sodium carbonate or the MEPM/cilastatin mixed solution and diluted with the MEPM/cilastatin mixed solution to prepare a MEPM/Compound A/cilastatin mixed solution with a target concentration. Note that, for the purpose of suppressing degradation of MEPM by a dehydropeptidase-I in a mouse, the agent at each concentration was administered in combination with cilastatin at 100 mg/kg, which can inhibit this enzyme. In order to induce neutropenia, cyclophosphamide was administered intraperitoneally to a male ICR mouse at 150 mg/kg and 100 mg/kg 4 days and 1 day before infection, respectively. Two hours before the start of treatment on the day of infection, 0.1 mL of the inoculum was inoculated into the left gastrocnemius muscle of a neutropenic mouse under anesthesia (about 1 x $10^6$ CFU/mouse). Then, the MEPM at 50 or 100 mg/kg and cilastatin at 100 mg/kg were administered subcutaneously to the thus-infected mouse for 8 doses at 3-hour intervals starting 2 hours after infection, and when the Compound A was used in combination, it was administered subcutaneously for 2 doses (12-hour intervals), 4 doses (6-hour intervals), or 8 doses (3-hour intervals) with the total dose kept constant at 15, 60, 120, 240, or 480 mg/kg. Saline serving as a control was administered subcutaneously for 8 doses at 3-hour intervals (10 mL/kg/dose). The number of bacteria in the muscle was measured at 2 and 26 hours after infection. The mouse was euthanized and the left gastrocnemius muscle was removed therefrom. Saline

was added thereto and the muscle was homogenized using a Multi-Beads shocker. Approximately 10-fold serial dilutions of the muscle homogenate were prepared using saline, and 0.1 mL of each dilution was smeared on a Mueller Hinton agar. The agar medium was cultured for 1 day and colonies were counted. A decrease of less than 1 log or 1 log or more in the number of bacteria in the muscle in the drug-treated group compared to that at the start of treatment was considered to indicate a bacteriostatic effect and a bactericidal effect, respectively.

For a VIM-27 infection model in which the MEPM at 50 mg/kg was administered every 3 hours (FIG. 10A), a bacteriostatic effect was observed for the Compound A at 120 mg/kg/day or more. For the below-described three bacterial infection models in which the MEPM at 100 mg/kg was administered every 3 hours (FIG. 10B to D), a 1-log bactericidal effect was observed for the Compound A at 60 mg/kg/day or more in a KPC-3-producing bacterial infection model. A 1-log bactericidal effect and a bacteriostatic effect were observed for the Compound A at 120 mg/kg/day or more and 60 mg/kg/day, respectively, in an NDM-7-producing bacterial infection model. A bacteriostatic effect and a 1-log bactericidal effect were observed at 120 mg/kg/day and 480 mg/kg/day in NDM-1 and OXA-232-producing bacterial infection models. A series of test results indicate that for the same total daily dose of the Compound A, an antibacterial activity of the regimen with more divided doses of the Compound A is not attenuated compared to that of the single dose, and a peak concentration of the Compound A does not affect enhancement of a MEPM activity. The Compound A was suggested to enhance an in vivo antibacterial activity of the MEPM against serine- and metallo-carbapenemase-producing bacteria in a dose-dependent manner in the present infection model.

(Example 30)

Evaluation of in vivo pharmacodynamic target of Compound A against carbapenemase-producing Enterobacteriaceae (CPE) in intramuscular infection model of immunocompromised mouse

Pharmacokinetics/Pharmacodynamics (PK/PD) parameters correlating with efficacy of Compound A in combination with MEPM against CPE were analyzed using PK/PD parameters of Compound A and data on change in the number of bacteria in the muscle in an intramuscular infection model of an immunocompromised mouse. An unbound area under a plasma concentration-time curve from 0 to 24 hours (fAUC), an unbound maximum plasma concentration ($fC_{max}$), fAUC/MEPM-Compound A (M-K) MIC, $fC_{max}$/M-K MIC, a proportion of time for which a free form concentration is above M-K MIC in a dosing interval (%fT > M-K MIC), and a proportion of time for which a free form concentration is above a threshold concentration in a dosing interval (%fT > $C_T$) were used as PK/PD parameters.

Efficacy data on change in the number of bacteria in the muscle in 9 strains of carbapenemase-producing Klebsiella pneumoniae were pooled and analyzed. Correlation between the efficacy data and each PK/PD parameter was evaluated with a sigmoidal $E_{max}$ model.

$$E = E_0 + (E_{max} \times D^N) / (ED_{50}{}^N + D^N)$$

in which $E_0$ is a change in the number of bacteria in the muscle from baseline at which D is 0, $E_{max}$ is the maximum reduction in the number of bacteria in the muscle, D is each PK/PD parameter, $ED_{50}$ is a D value required for 50% of $E_{max}$, and N is the Hill coefficient indicating sigmoidality of a dose-response curve.

For %fT > $C_T$, 0.25, 0.5, 1, 2, and 4 ug/mL were set as $C_T$. The M-K MIC for Compound A at fixed concentrations of 4, 8, or 16 ug/mL was expressed as M-K MIC 4 μg/mL, M-K MIC 8 μg/mL, or M-K MIC 16 μg/mL, respectively. Analysis using each of the above PK/PD parameters showed the highest correlation ($R^2 > 0.58$) between %fT > 2 ug/mL and efficacy as indexed by the change in the number of bacteria in the muscle (FIG. 11). Values required for bacteriostatic and bactericidal effects (1 log kill) for %fT > 2 ug/mL were 14.1 and 35.8%, respectively. Efficacy of Compound A against 4 starains of serine-CPE or 5 strains of metallo-CPE in combination with MEPM also correlated well with %fT > 2 μg/mL, with values required for a bacteriostatic effect being 7.7 or 20.0% and values required for a bactericidal (1-log kill) effect being 20.4 or 51.9%, respectively.

(Example 31)

Results of the Phase 1 study of Compound A performed in the United States showed that after an intravenous infusion of the Compound A for 3 hours, a plasma concentration of the Compound A reached the maximum near the end of the infusion, with the majority excreted in the urine. Main pharmacokinetic parameters calculated are shown in the tables below.

[Table 39]

| Pharmacokinetic parameters upon administration of Compound A in single dose | | | | |
|---|---|---|---|---|
| **Dosage** (mg) | **Number of cases** | Cmax ($\mu$g/mL) | $AUC_{0\text{-}inf}$ ($\mu$g·h/mL) | $T_{1/2}$ (1/h) |
| 50 | 6 | 2.71 (0.798) | 11.1 (3.45) | 2.66 (0.361) |
| 125 | 6 | 7.44 (1.16) | 31.9 (5.71) | 3.51 (0.989) |
| 250 | 6 | 13.1 (3.88) | 59.7 (12.6) | 3.87 (0.268) |
| 750 | 6 | 32.4 (4.76) | 135 (25.8) | 3.49 (0.303) |
| 1500 | 6 | 71.3 (13. 8) | 353 (79.1) | 4.05 (0.918) |
| 2000 | 6 | 96.6 (9.99) | 496 (86. 1) | 4.83 (1.13) |
| **Average (standard deviation)** | | | | |

[Table 40]

**Pharmacokinetic parameters on day 5 of repeated administration of Compound A**

| **Dosage** (mg) | **Number of cases** | Cmax ($\mu$g/mL) | $AUC_{0\text{-}tau}$ ($\mu$g·h/mL) | Cmax **ratio (Day 5 / Day 1)** | $AUC_{0\text{-}8}$ **ratio (Day 5 / Day 1)** |
|---|---|---|---|---|---|
| 500 | 6 | 24.1 (2.85) | 99.2 (14. 9) | 0.975 (0.0947) | 1.07 (0.137) |
| 1000 | 5 | 52.1 (6.32) | 243 (53.7) | 1.11 (0.105) | 1.32 (0.101) |
| 1500 | 6 | 97.0 (22.8) | 463 (96.0) | 1.21 (0.155) | 1.45 (0.123) |

**Average (standard deviation)**
**After administration in morning on day 5 of administration every 8 hours**

[Table 41]

**Pharmacokinetic parameters on day 5 of repeated administration of Compound A in combination with meropenem**

| **Dosage** (mg) | **Number of cases** | Cmax ($\mu$g/mL) | $AUC_{0\text{-}tau}$ ($\mu$g·h/mL) | Cmax **ratio (Day 5 / Day 1)** | $AUC_{0\text{-}8}$ **ratio (Day 5 / Day 1)** |
|---|---|---|---|---|---|
| 500 | 8 | 32.0 (10.5) | 143 (61.5) | 1.13 (0.191) | 1.24 (0.219) |
| 1500 | 8 | 117 (17.3) | 547 (87.5) | 1.60 (0.286) | 1.77 (0.257) |

**Average (standard deviation)**
**After administration in morning on day 5 of administration every 8 hours in combination with 2 g/dose of meropenem**

[Table 42]

**Pharmacokinetic parameters upon administration of Compound A in single dose to Japanese**

| **Dosage** (mg) | **Number of cases** | Cmax ($\mu$g/mL) | $AUC_{0\text{-}inf}$ ($\mu$g·h/mL) | $T_{1/2}$ (1/h) |
|---|---|---|---|---|
| 250 | 6 | 13.3(1.27) | 55.0(7.51) | 2.77(0.659) |
| 750 | 6 | 42.0(5.71) | 188(38.3) | 3.40(0.291) |
| 1500 | 6 | 93.5(21.8) | 461(128) | 4.27(0.82) |
| **Average (standard deviation)** | | | | |

(Example 32)

Results of the Phase 1 study of Compound A conducted in the United States showed that the Compound A in a range of 50 mg to 2000 mg was safe and tolerated under intravenous infusion for 3 hours. The Compound A in a range of 50 mg to 1500 mg was confirmed to be safe and tolerated under intravenous infusion of Compound A for 3 hours every 8 hours for 5 days.

(Example 33) A population PK analysis was performed using data of Phase I clinical trial of Compound A to create

a population pharmacokinetic model to be used to simulate a change in drug concentration necessary for estimating a clinically effective dose from PK/PD indices and target values obtained in a nonclinical study. NONMEM was used as an analysis software. PK parameter estimates obtained by the 2-compartment model are shown below. The eGFR and body weight, which were indicators of renal function, were confirmed to affect pharmacokinetics of the Compound A. Effects of gender and combination with meropenem were also detected, but their effects were small.

[Table 43]

| PK parameters | | Estimated value (95% confidence interval) | | |
|---|---|---|---|---|
| CL (L/h) | | | | |
| | $\theta_{CL}$ | 4.54 (4.27, 4.82) | | |
| | $\theta_{eGFR\ on\ CL}$ | 0.455 (0.353, 0.636) | | |
| | $\theta_{BW\ on\ CL}$ | 0.561 (0.299, 0.810) | | |
| | $\theta_{Sex\ on\ CL}$ | -0.120 (-0.204, -0.0314) ; | **0 for male** | |
| | $\theta_{meropenem\ on\ CL}$ | -0.174 (-0.262, -0.0905) ; | **0 for non-combination** | |
| $V_c$ (L) | | | | |
| | $\theta_{Vc}$ | 11.0 (10.6, 11.6) | | |
| | $\theta_{BW\ on\ Vc}$ | 0.455 (0.270, 0.658) | | |
| | $\theta_{Sex\ on\ Vc}$ | -0.114 (-0.192, -0.0434) ; | **0 for male** | |
| Q (L/h) | | | | |
| | $\theta_{Q}$ | 1.08 (0.953, 1.17) | | |
| | $\theta_{BW\ on\ Q}$ | 1.37 (0.948, 1.69) | | |
| $V_p$ (L) | | | | |
| | $\theta_{Vp}$ | 4.12 (3.70, 4.61) | | |
| | $\theta_{BW\ on\ Vp}$ | 1.37 (0.948, 1.69) | | |
| **Interindividual variability** | | | **Coefficient of variation** | **Shrinkage** |
| | $\omega_{CL}^2$ | 0.0323 (0.0223, 0.0391) | 18.1% | 1.0% |
| | $\omega_{Vc}^2$ | 0.0210 (0.0133, 0.0263) | 14.6% | 9.9% |
| | $\omega_{Vp}^2$ | 0.240 (0.0046, 0.5300) | 52.1% | 12.8% |

(continued)

| | | | |
|---|---|---|---|
| **Intraindividual variability** | | | |
| $\sigma^2$ | 0.00831 (0.00676, 0.0111) | 9.13% | 11.4% |

$$CL_i = \theta_{CL} \times \left(\frac{eGFR_i}{108}\right)^{\theta_{eGFR\ on\ CL}} \times \left(\frac{BW_i}{75.2}\right)^{\theta_{BW\ on\ CL}} \times (1 + \theta_{Sex\ on\ CL}) \times (1 + \theta_{meropenem\ on\ CL})$$

$$Vc_i = \theta_{Vc} \times \left(\frac{BW_i}{75.2}\right)^{\theta_{BW\ on\ Vc}} \times (1 + \theta_{sex\ on\ Vc})$$

$$Q_i = \theta_Q \times \left(\frac{BW_i}{75.2}\right)^{\theta_{BW\ on\ Vp}}$$

$$Vp_i = \theta_{Vp} \times \left(\frac{BW_i}{75.2}\right)^{\theta_{BW\ on\ Vp}}$$

***eGFRi*** **denotes estimated glomerular filtration rate for each subject and** ***BWi*** **denotes body weight for each subject.**

(Example 34)

The PK/PD indices and target values obtained in evaluation of in vivo pharmacodynamic targets of Compound A against carbapenemase-producing Enterobacteriaceae (CPE) performed in an intramuscular infection model of an immunocompromised mouse for the purpose of estimating clinically effective doses against CPE and Acinetobacter baumannii and a population pharmacokinetic model of the Compound A were used to simulate a probability of target attainment when the Compound A was administered repeatedly at various dosages and administration. Plasma concentrations of the Compound A were simulated in 1000 hypothetical subjects, and the PK/PD index and an achievement rate of the target value (40% of fT > 2 ug/mL or 20% of fT > 2 ug/mL or 55% of fT > 2 μg/mL for CPE and 15% of fT > 4 ug/mL or 25% of fT > 4 ug/mL for Acinetobacter baumannii) were examined. For 3-hour intravenous infusion, 90% or more of the subjects were expected to achieve 20% of fT > 2 μg/mL for q8h administration at 250 mg. For q8h administration at 500 mg, 90% or more of the subjects were expected to achieve all of these target values. Based on these analysis results, the clinically effective dose of the Compound A was estimated to be 500 mg or more for 3-hour infusion every 8 hours. Tolerability at this dose was confirmed in the Phase 1 study conducted in the United States.

(Example 35)

PK/PD simulations were performed to estimate efficacy against meropenem-non-susceptible Pseudomonas aeruginosa. Using MIC distribution data of meropenem in 76 strains of Pseudomonas aeruginosa with MIC of the meropenem of 4 ug/mL or more in combination with Compound A at concentrations of 0, 4, 8, and 16 μg/mL, %T>MIC was used as the PK/PD index for meropenem and target values of bacteriostatic and 1-log and 2-log bactericidal effects were determined as 30, 35, and 45%. Data from 76 pairs of 76 strains were multiplied by 14 to yield 1064 pairs of data, which were randomly combined with 1064 hypothetical subjects. A population pharmacokinetic model for Compound A and meropenem was used to simulate a change in plasma concentration of the Compound A and the meropenem in the 1064 hypothetical subjects when the Compound A and the meropenem were administered repeatedly in combination. The MIC of meropenem at each time point was changed according to a concentration of Compound A at that time point, and the % T>MIC of meropenem in combination with the Compound A was calculated based on the change in concentration of the meropenem. An achievement rate of 45% T>MIC was 32% or 49% for 1 g or 2 g of meropenem alone administered as 3-hour infusion every 8 hours, respectively, but increased to 69% when 2 g of meropenem was used in combination with 750 mg of the Compound A. The rate was 73% for 4-hour infusion. Based on the above, it was estimated that when 750 mg of the Compound A/2 g of the meropenem was administered in combination by intravenous infusion for 3 to 4 hours every 8 hours, the achievement rate against meropenem-non-susceptible Pseudomonas aeruginosa was about 20% higher than when meropenem was used alone. Tolerability at this dose is also expected and will be confirmed in a Phase 1 study in the future.

(Example 36)

PK/PD simulations were performed to estimate efficacy against meropenem-non-susceptible Enterobacteriaceae. Using MIC distribution data of meropenem in 92 strains of Enterobacteriaceae with MIC of meropenem of 2 ug/mL or more in combination with Compound A at concentrations of 0, 4, 8, and 16 μg/mL, %T>MIC was used as the PK/PD index for meropenem and target values of bacteriostatic and 1-log and 2-log bactericidal effects were determined as 30, 35, and 45%. Data from 92 pairs of 92 strains were multiplied by 11 to yield 1012 pairs of data, which were randomly combined with 1012 hypothetical subjects. A population pharmacokinetic model of Compound A and meropenem was used to simulate a change in plasma concentration of the Compound A and the meropenem in the 1012 hypothetical subjects when the Compound A and the meropenem were administered repeatedly in combination. The MIC of meropenem at each time point was changed according to a concentration of the Compound A at that time point, and the %T>MIC of meropenem in combination with the Compound A was calculated based on the change in concentration of the meropenem. An achievement rate of 45% T>MIC was 50% or 69% for 1 g or 2 g of the meropenem administered alone as a 3-hour infusion every 8 hours, respectively, but increased to 90% or more when 2 g of the meropenem was used in combination with 750 mg of the Compound A. Based on the above, it was estimated that administration of 750 mg of the Compound A/2 g of the meropenem in combination by intravenous infusion for 3 hours every 8 hours was effective against meropenem-non-susceptible Enterobacteriaceae. Tolerability at this dose is also expected and will be confirmed in a Phase 1 study in the future.

(Example 37)

PK/PD simulations were performed to estimate efficacy against meropenem-non-susceptible Acinetobacter baumannii. Using MIC distribution data of meropenem in 52 strains of Acinetobacter baumannii with MIC of meropenem of

4 μg/mL or more in combination with Compound A at concentrations of 0, 4, 8, and 16 μg/mL, %T>MIC was used as the PK/PD index for meropenem and target values of bacteriostatic and 1-log and 2-log bactericidal effects were determined as 30, 35, and 45%. Data from 52 pairs of 52 strains were multiplied by 20 to yield 1040 pairs of data, which were randomly combined with 1040 hypothetical subjects. A population pharmacokinetic model of the Compound A and the meropenem was used to simulate a change in plasma concentration of the Compound A and the meropenem in the 1040 hypothetical subjects when the Compound A and the meropenem were administered repeatedly in combination. The MIC of the meropenem at each time point was changed according to a concentration of the Compound A at that time point, and the % T>MIC of meropenem in combination with the Compound A was calculated based on the change in concentration of the meropenem. An achievement rate of 45% T>MIC was 14% or 29% for 1 g or 2 g of the meropenem administered alone as a 3-hour infusion every 8 hours, respectively, but increased to 90% or more when 2 g of the meropenem was used in combination with 750 mg of the Compound A. Based on the above, it was estimated that administration of 750 mg of the Compound A/2 g of the meropenem in combination by intravenous infusion for 3 hours every 8 hours was effective against meropenem-non-susceptible Acinetobacter baumannii. Tolerability at this dose is also expected and will be confirmed in a Phase 1 study in the future.

(Reference Example 1) Lyophilized product containing 168 mg of Compound A

In the Phase 1 study of Compound A, lyophilized product were prepared using the following formulation.

[Table 44]

| | Item name | Amount per vial |
|---|---|---|
| Drug Substance | Compound A | 168 mg |
| Bulking agent | Sucrose | 420 mg |
| pH modifier | Dried sodium carbonate | 46.12 mg |

The drug substance and excipients in the above formulations were as follows unless otherwise noted.

Compound A: Sumitomo Pharma Co., Ltd.
Sucrose: Merck KGaA
Dried sodium carbonate: FUJIFILM Wako Pure Chemical Corporation

(Manufacturing procedure)

(1) Preparation of solution for lyophilization containing Compound A

First, 80 L of water for injection was added to a 230 L dissolution tank, and the solution was cooled by circulating chiller water at 10°C or less. After comfirming the temperature of the water for injection in the dissolution tank, nitrogen bubbling was performed, and 2100 g of Compound A predissolved in water for injection in advance was added thereto. Then, 576.5 g of dried sodium carbonate and 5250 g of Sucrose were added, and after confirming complete dissolution, water for injection was added up to 107762 g, which corresponded to a weight of 105.0 L of the solution. This solution was sterilized using two PVDF filters with a pore size of 0.22 μm, microorganism retaining filter and sterile filter, resulting in the solution for lyophilization.

(2) Manufacturing of lyophilized product containing Compound A

The solution for lyophilization (1) was filled into 20 mL glass vials which had been washed and dry heat sterilized with 8.4 mL each. The vials were then pertially stoppered with butyl rubber stopperes which had been washed, steam sterilized, and dried.

A shelf temperature of a lyophilizer was set to 5°C. The pertially stoppered vials were loaded in the lyophilizer for lyophilization under conditions shown in Table 45. After the lyophilization, the vials were backfilled with nitrogen gas, fully stoppered and capped to produce a lyophilized product containing Compound A

[Table 45]

| Step | | Shelf temperature | Degree of vacuum | Time |
|---|---|---|---|---|
| 1 | **Waiting** | 5°C | *N/A* | N/A |

(continued)

| Step | | Shelf temperature | Degree of vacuum | Time |
|---|---|---|---|---|
| 2 | **Cooling** | -40°C | N/A | 90分 |
| 3 | **Pre-freezing** | -40°C | N/A | 120分 |
| 4 | **Heating** | -18°C | 6.9 Pa | 44分 |
| 5 | **Primary drying** | -18°C | 6.9 Pa | 4500分 |
| 6 | **Heating** | 40°C | 6.9 Pa | 580分 |
| 7 | **Secondary drying** | 40°C | 6.9 Pa | 600分 |
| 8 | **Cooling** | 5°C | 6.9 Pa | 70分 |
| 9 | **Holding** | 5°C | 6.9 Pa | N/A |
| 10 | **First repressurization** | 5°C | 76.3 kPa | N/A |
| 11 | **Capping** | 5°C | 76.3 kPa | N/A |
| 12 | **Second repressurization** | 5°C | 101.3 kPa | N/A |
| **N/A: Not applicable** | | | | |

[Industrial Applicability]

A medicament of the present disclosure includes Compound A or a pharmaceutically acceptable salt thereof and meropenem or a pharmaceutically acceptable salt thereof, and may be used as a medicament for treating or preventing a bacterial infection.

## Claims

**1.** A medicament for treating or preventing a bacterial infection, the medicament comprising:

Compound A or a pharmaceutically acceptable salt thereof; and
meropenem or a pharmaceutically acceptable salt thereof,
the Compound A being of Formula (1a), (1b), or (2): or

[Chem. 1]

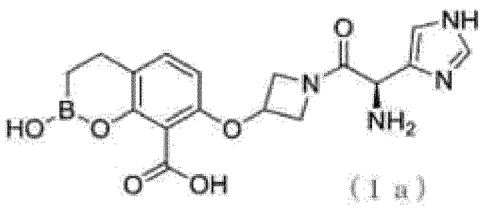
(1 a)

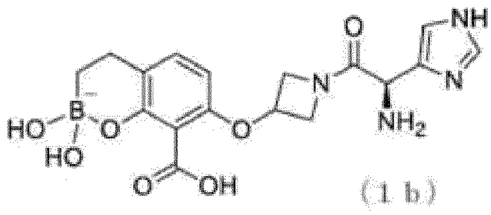
(1 b)

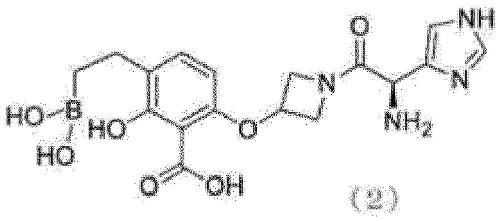
(2)

and

the medicament being administered intravenously to a subject.

**2.** A medicament for treating or preventing a bacterial infection, the medicament comprising:

Compound A or a pharmaceutically acceptable salt thereof; and
meropenem or a pharmaceutically acceptable salt thereof,
the Compound A being of Formula (1a), (1b), or (2):

[Chem. 2]

or

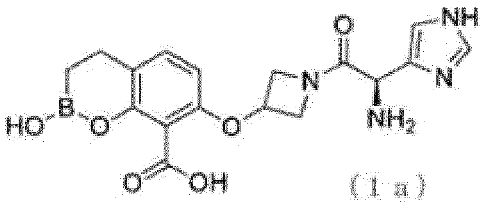

(1 a)

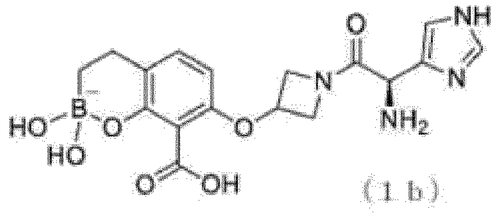

(1 b)

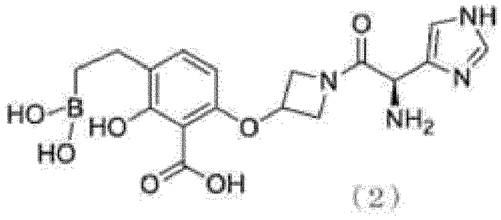

(2)

and
the Compound A or the pharmaceutically acceptable salt thereof being administered to a subject so as to enhance an activity of the meropenem or the pharmaceutically acceptable salt thereof as compared to when not being used in combination.

**3.** The medicament according to claim 1 or 2, wherein the medicament is administered intravenously to the subject at a frequency of at least once every 24 hours.

**4.** The medicament according to claim 3, wherein the medicament is administered intravenously to the subject at a frequency of at least once every 8 hours.

**5.** The medicament according to claim 3, wherein the medicament is administered intravenously to the subject at a frequency of at least once every 6 hours.

**6.** The medicament according to claim 3, wherein the Compound A or the pharmaceutically acceptable salt thereof is administered intravenously to the subject at a frequency of at least once every 8 hours.

**7.** The medicament according to claim 3, wherein the Compound A or the pharmaceutically acceptable salt thereof is administered intravenously to the subject at a frequency of at least once every 6 hours.

**8.** The medicament according to claim 3, wherein the meropenem or the pharmaceutically acceptable salt thereof is administered intravenously to the subject at a frequency of at least once every 8 hours.

**9.** The medicament according to claim 3, wherein the meropenem or the pharmaceutically acceptable salt thereof is administered intravenously to the subject at a frequency of at least once every 6 hours.

**10.** The medicament according to claim 3, wherein the medicament is administered by intravenous infusion for 3 hours or more.

**11.** The medicament according to claim 3, wherein the medicament is administered by intravenous infusion for 4 hours or

more.

12. The medicament according to claim 3, wherein the medicament is administered by intravenous infusion for 3 hours or less.

13. The medicament according to claim 3, wherein the medicament is administered by intravenous infusion for 3 hours.

14. The medicament according to claim 3, wherein the medicament is administered by intravenous infusion for 4 hours.

15. The medicament according to claim 3, wherein the Compound A or the pharmaceutically acceptable salt thereof and the meropenem or the pharmaceutically acceptable salt thereof are administered concomitantly or separately.

16. The medicament of claim 3, wherein the Compound A or the pharmaceutically acceptable salt thereof and the meropenem or the pharmaceutically acceptable salt thereof are comprised in a same agent or separate agents.

17. The medicament according to any one of claims 1 to 5, wherein the Compound A or the pharmaceutically acceptable salt thereof and the meropenem or the pharmaceutically acceptable salt thereof are comprised in a ratio of 1:1 to 1:10 by mass on a free form basis.

18. The medicament according to any one of claims 1 to 5, wherein the Compound A or the pharmaceutically acceptable salt thereof and the meropenem or the pharmaceutically acceptable salt thereof are comprised in a ratio of 1:2 by mass on a free form basis.

19. The medicament according to any one of claims 1 to 5, wherein the Compound A or the pharmaceutically acceptable salt thereof and the meropenem or the pharmaceutically acceptable salt thereof are comprised in a ratio of 1:4 on a free form basis.

20. The medicament according to any one of claims 1 to 5, wherein the Compound A or the pharmaceutically acceptable salt thereof and the meropenem or the pharmaceutically acceptable salt thereof are comprised in a ratio of 1:8 on a free form basis.

21. The medicament according to any one of claims 1 to 20, wherein the medicament comprises 500 to 3000 mg of the Compound A.

22. The medicament according to any one of claims 1 to 20, wherein the medicament comprises 500 to 1000 mg of the Compound A.

23. The medicament according to any one of claims 1 to 22, wherein the medicament comprises 200 to 6000 mg of the meropenem.

24. The medicament according to any one of claims 1 to 22, wherein the medicament comprises 250 to 3000 mg of the meropenem.

25. The medicament according to any one of claims 1 to 22, wherein the medicament comprises 1000 mg of the meropenem.

26. The medicament according to any one of claims 1 to 22, wherein the medicament comprises 1500 mg of the meropenem.

27. The medicament according to any one of claims 1 to 22, wherein the medicament comprises 2000 mg of the meropenem.

28. The medicament according to any one of claims 1 to 27, wherein the medicament comprises 500 mg of the Compound A.

29. The medicament according to any one of claims 1 to 27, wherein the medicament comprises 750 mg of the Compound A.

**30.** The medicament according to any one of claims 1 to 27, wherein the medicament comprises 1000 mg of the Compound A.

**31.** The medicament according to any one of claims 1 to 20, wherein the medicament comprises 750 mg of the Compound A and 2000 mg of the meropenem.

**32.** The medicament according to any one of claims 1 to 20, wherein the medicament comprises 500 mg of the Compound A and 1500 mg of the meropenem.

**33.** The medicament according to any one of claims 1 to 20, wherein the medicament comprises 750 mg of the Compound A and 1500 mg of the meropenem.

**34.** The medicament according to any one of claims 1 to 33, wherein the bacterial infection is a bacterial infection involving a bacterium that can possess a β-lactamase.

**35.** The medicament according to any one of claims 1 to 33, wherein the bacterial infection is a bacterial infection involving a bacterium that can possess a serine-β-lactamase.

**36.** The medicament according to claim 24, wherein the serine-β-lactamase is an Ambler class A, Ambler class C, or Ambler class D β-lactamase.

**37.** The medicament according to any one of claims 1 to 33, wherein the bacterial infection is a bacterial infection involving a bacterium that can possess a metallo-β-lactamase.

**38.** The medicament according to claim 37, wherein the metallo-β-lactamase is an Ambler class B β-lactamase.

**39.** The medicament according to any one of claims 1 to 38, wherein a therapeutically effective amount of the Compound A or the pharmaceutically acceptable salt thereof and the meropenem or the pharmaceutically acceptable salt thereof results in %fT > 1 μg/mL of 30% or more in terms of a plasma concentration of the Compound A.

**40.** The medicament according to any one of claims 1 to 38, wherein a therapeutically effective amount of the Compound A or the pharmaceutically acceptable salt thereof and the meropenem or the pharmaceutically acceptable salt thereof results in %fT > 2 μg/mL of 20% or more in terms of a plasma concentration of the Compound A.

**41.** The medicament according to any one of claims 1 to 38, wherein a therapeutically effective amount of the Compound A or the pharmaceutically acceptable salt thereof and the meropenem or the pharmaceutically acceptable salt thereof results in %fT > 4 μg/mL of 10% or more in terms of a plasma concentration of the Compound A.

**42.** The medicament according to any one of claims 1 to 38, wherein a therapeutically effective amount of Compound A or a pharmaceutically acceptable salt thereof and meropenem or a pharmaceutically acceptable salt thereof results in a fAUC of 70 μg·h/mL or more in terms of a plasma concentration of the Compound A.

**43.** The medicament according to any one of claims 1 to 42, wherein the bacterial infection is a bacterial infection involving a bacterium selected from Enterobacteriales, Acinetobacter baumannii complex, Pseudomonas aeruginosa, and Bacteroides spp..

**44.** The medicament according to any one of claims 1 to 42, wherein the bacterial infection is a bacterial infection involving Escherichia coli, Klebsiella pneumoniae, Enterobacter cloacae complex, or Acinetobacter baumannii complex.

**45.** The medicament according to any one of claims 1 to 44, wherein the bacterial infection is a complicated urinary tract infection, a complicated intra-abdominal infection, hospitalacquired pneumonia, or ventilator-associated pneumonia.

**46.** The medicament according to any one of claims 1 to 45, wherein the medicament is used for a patient suffering from a bacterium susceptible to a combination of the Compound A or the pharmaceutically acceptable salt thereof and the meropenem or the pharmaceutically acceptable salt thereof.

**47.** The medicament according to any one of claims 1 to 46, wherein the medicament has a metallo-β-carbapenemase inhibitory activity.

**48.** The medicament according to any one of claims 1 to 47, wherein the medicament has an antibacterial activity against Escherichia coli, Klebsiella pneumoniae, Enterobacter cloacae complex, or Acinetobacter baumannii complex.

**49.** The medicament according to any one of claims 1 to 47, wherein the medicament has an antibacterial activity against Acinetobacter baumannii complex.

[FIG. 1]

**Types of β-lactamases and inhibition spectra of various β-lactamase inhibitors**

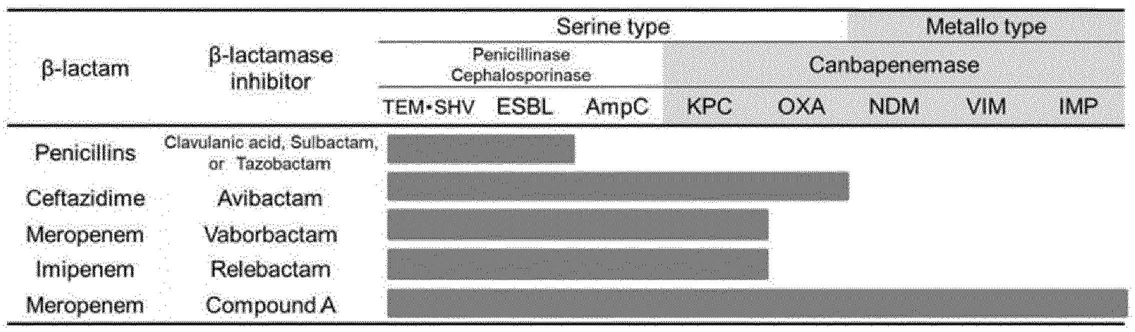


[FIG. 2]

**Fig.2 *in vivo* antibacterial activity of MEPM/Compound A against NDM-5-producing *E. coli* and KPC-2-producing *K. pneumoniae* in complicated urinary tract infection model of neutropenic mouse**

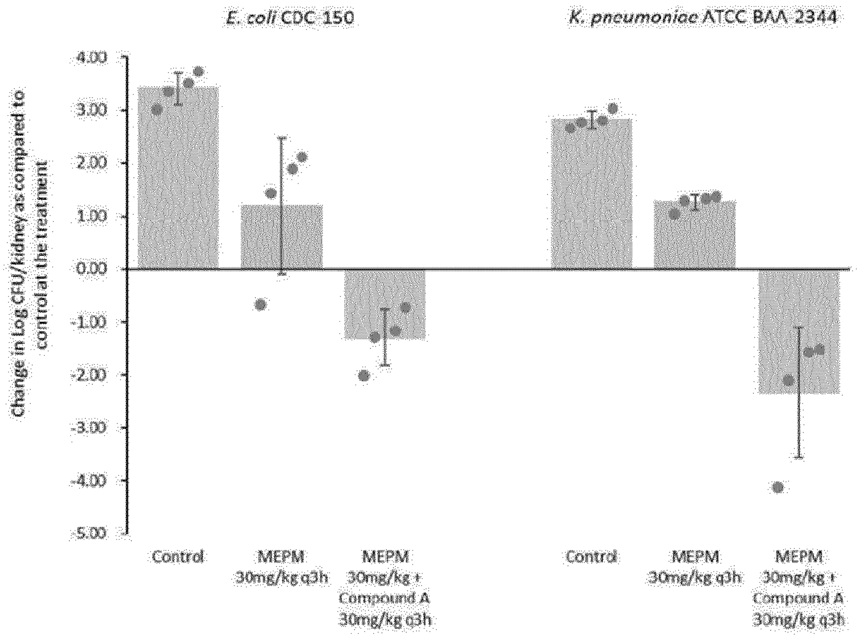

[FIG. 3]

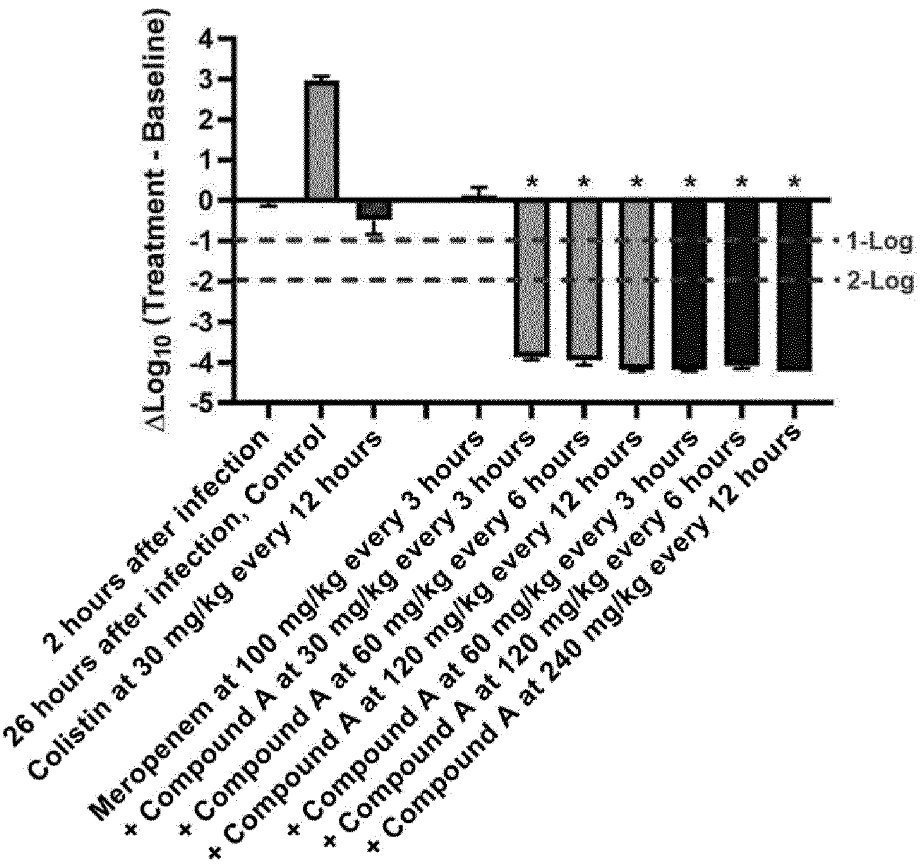
ΔLog10 (Treatment - Baseline)
4
3
2
1
0
-1
-2
-3
-4
-5
*
1-Log
2-Log
2 hours after infection
26 hours after infection, Control
Colistin at 30 mg/kg every 12 hours
Meropenem at 100 mg/kg every 3 hours
+ Compound A at 30 mg/kg every 3 hours
+ Compound A at 60 mg/kg every 6 hours
+ Compound A at 120 mg/kg every 12 hours
+ Compound A at 60 mg/kg every 3 hours
+ Compound A at 120 mg/kg every 6 hours
+ Compound A at 240 mg/kg every 12 hours

[FIG. 4]

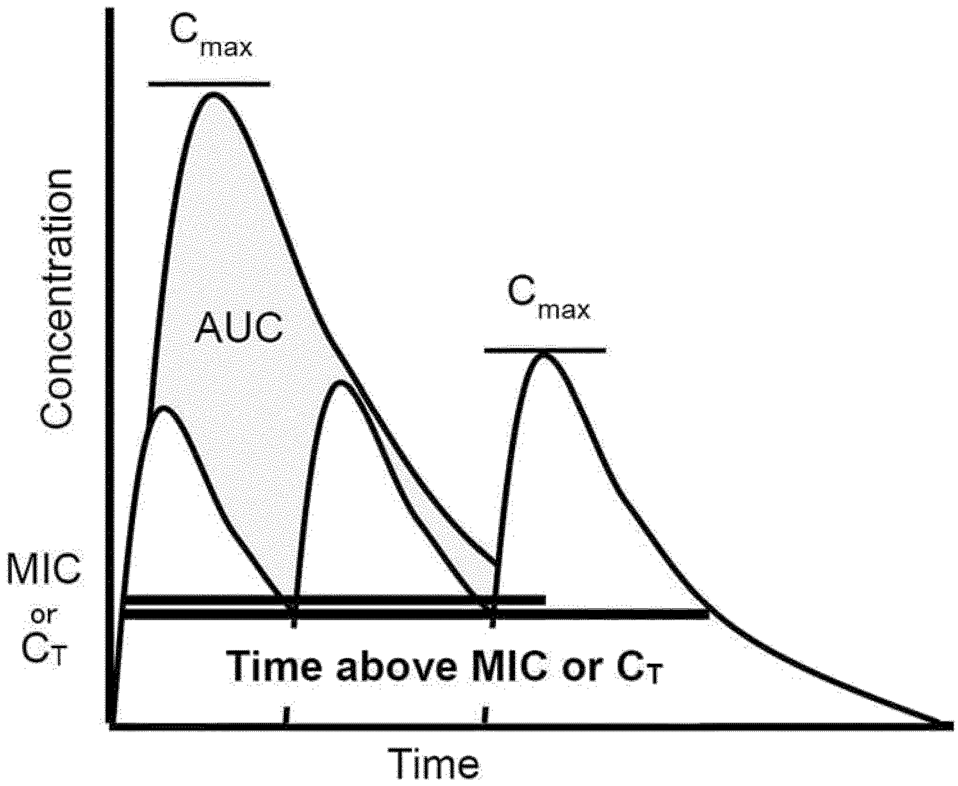


[FIG. 5]

**Summary of test method in intramuscular infection model of neutropenic mouse**

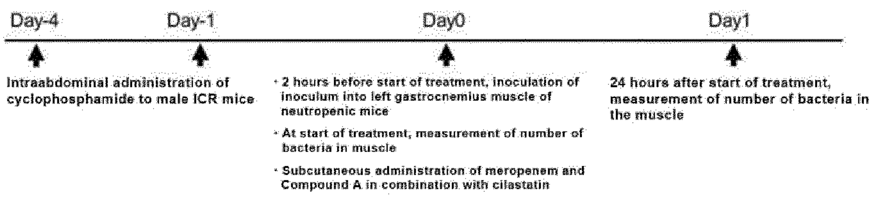


| Treatment | | At start of treatment | After 3 hours | After 6 hours | After 9 hours | After 12 hours | After 15 hours | After 18 hours | After 21 hours |
|---|---|---|---|---|---|---|---|---|---|
| Meropenem alone | | ✓ | ✓ | ✓ | ✓ | ✓ | ✓ | ✓ | ✓ |
| Meropenem and Compound A | | ✓ Administration of total dose | ✓ | ✓ | ✓ | ✓ | ✓ | ✓ | ✓ |
| Various dosage and administration (e.g., 60, 240, 960 mg.kg) of Compound A at constant total dosage | | ✓ Administration of 1/2 dose | ✓ | ✓ | ✓ | ✓ Administration of 1/2 dose | ✓ | ✓ | ✓ |
| | | ✓ Administration of 1/4 dose | ✓ | ✓ Administration of 1/4 dose | ✓ | ✓ Administration of 1/4 dose | ✓ | ✓ Administration of 1/4 dose | ✓ |
| | | ✓ Administration of 1/8 dose | ✓ Administration of 1/8 dose | ✓ Administration of 1/8 dose | ✓ Administration of 1/8 dose | ✓ Administration of 1/8 dose | ✓ Administration of 1/8 dose | ✓ Administration of 1/8 dose | ✓ Administration of 1/8 dose |

[FIG. 6-1]

A) KPC－2— producing bacterial infection model

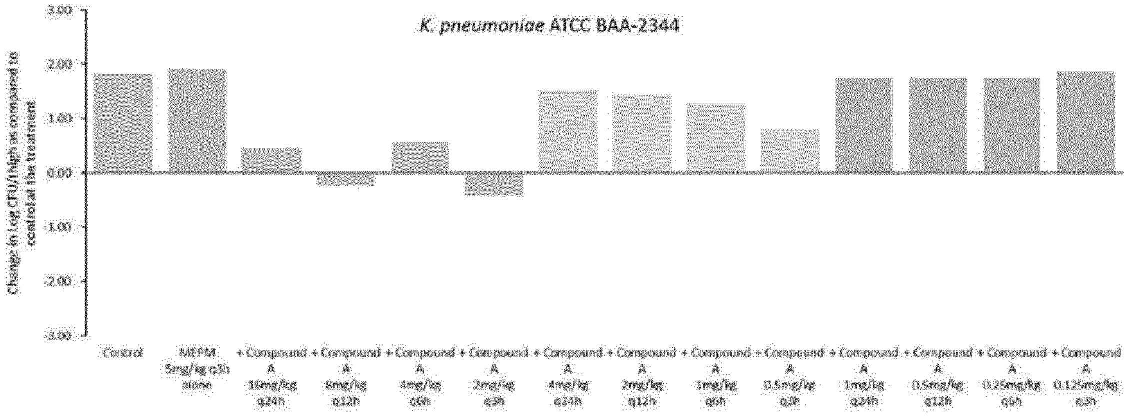


B) KPC－3— producing bacterial infection model

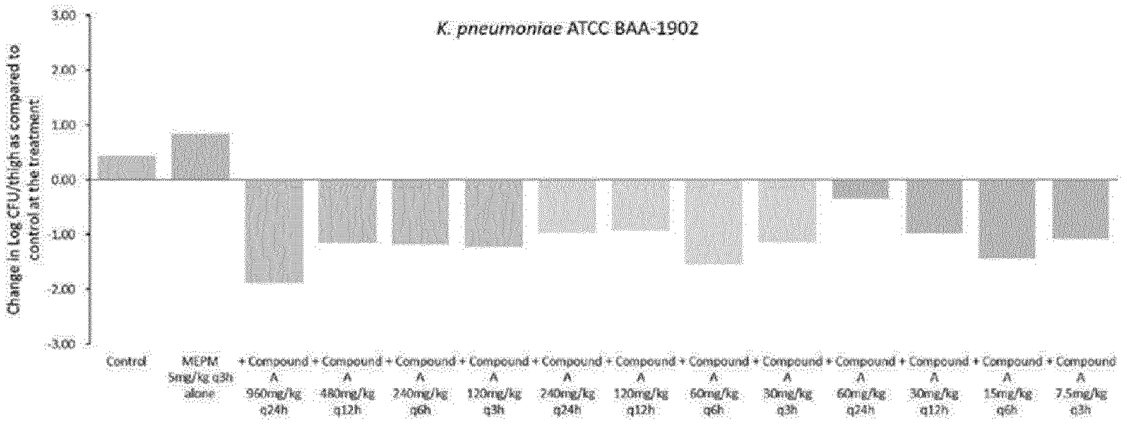


C) NDM－1— producing bacterial infection model

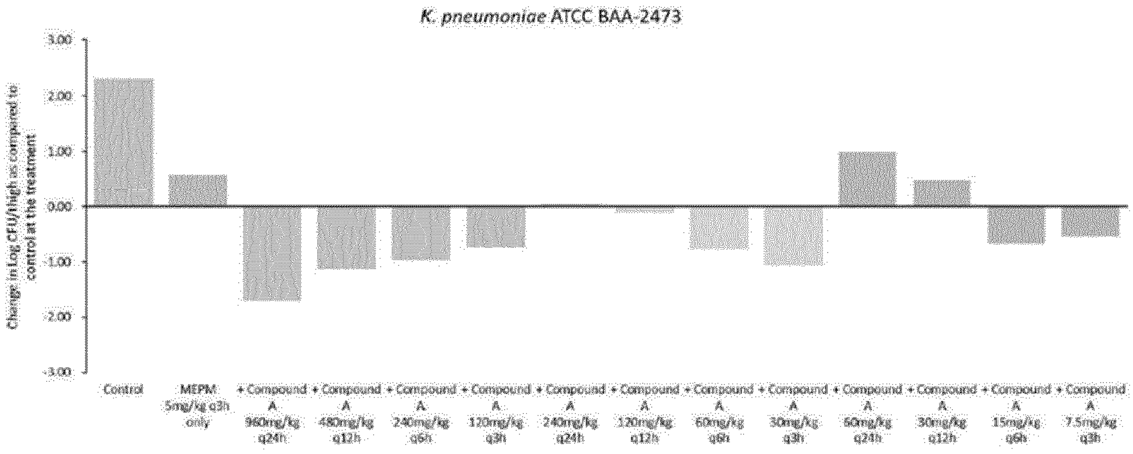

[FIG. 6-2]

D) IMP-1-producing bacterial infection model

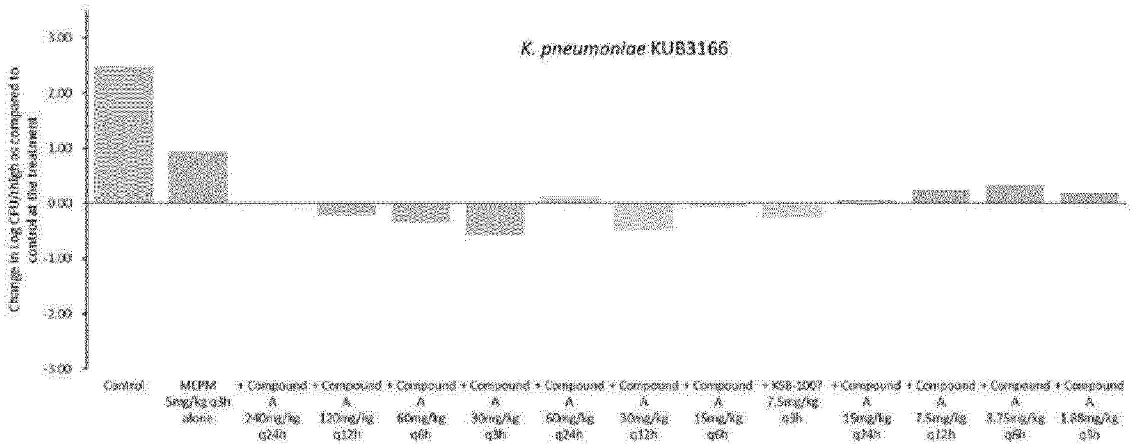


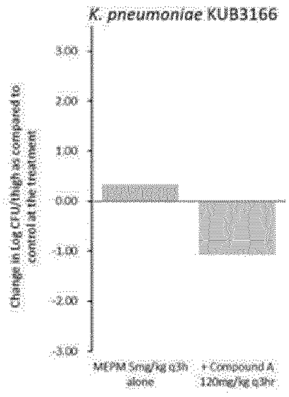


E) KPC-38-producing bacterial infection model

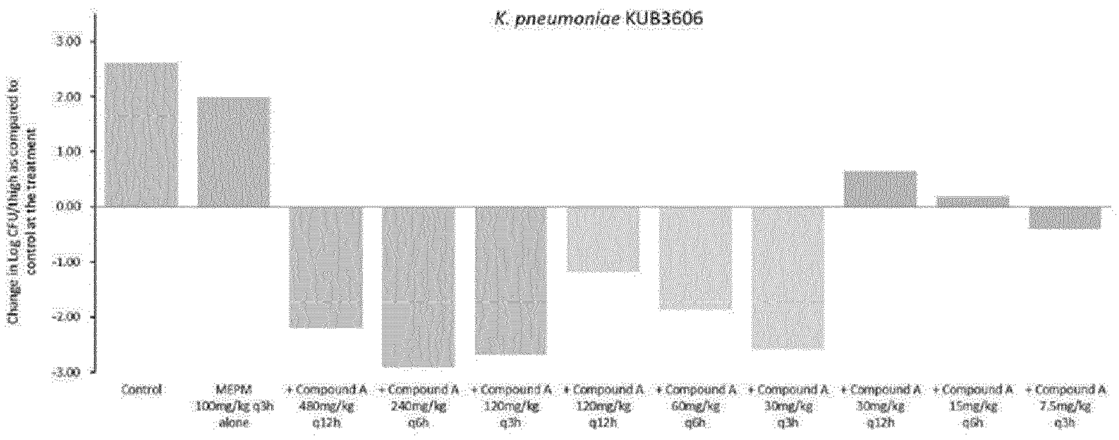

[FIG. 7-1]

A) CDC-83 infection model

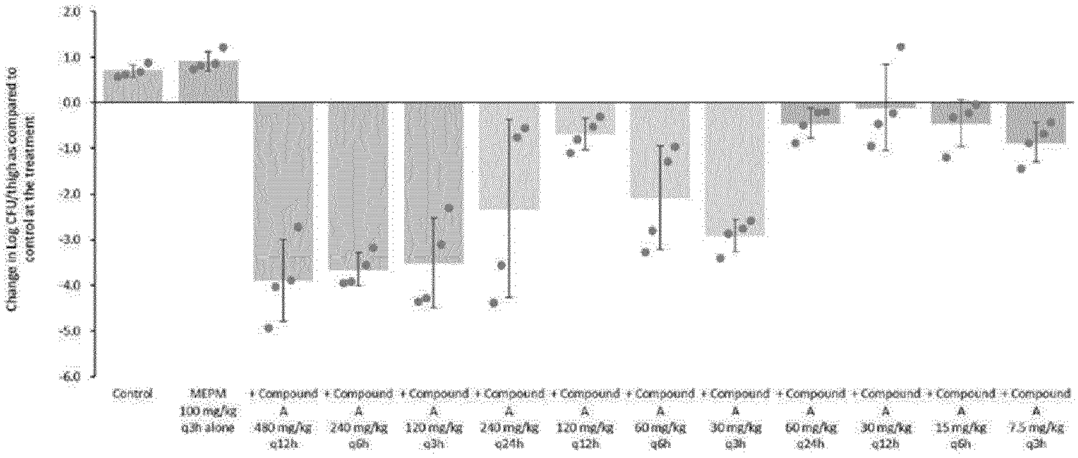


B) CDC-277 infection model

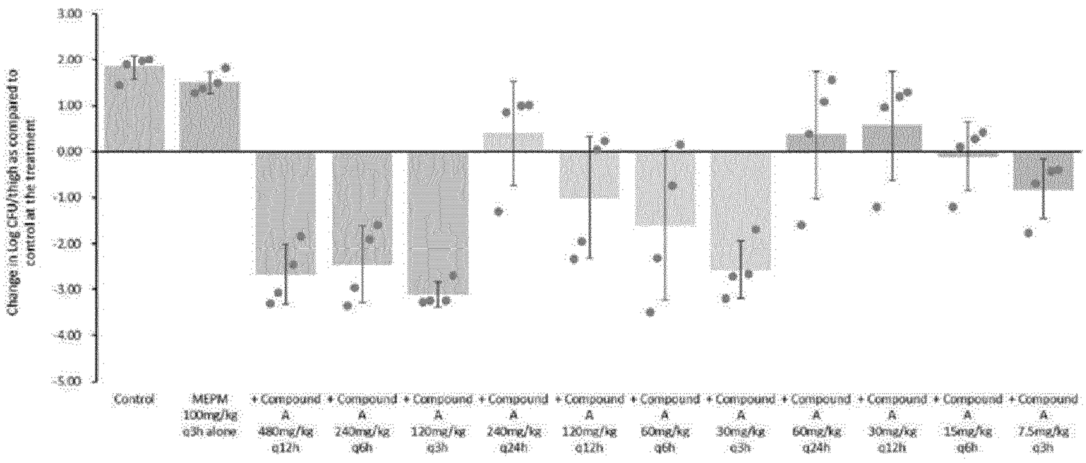


C) CDC-301 infection model

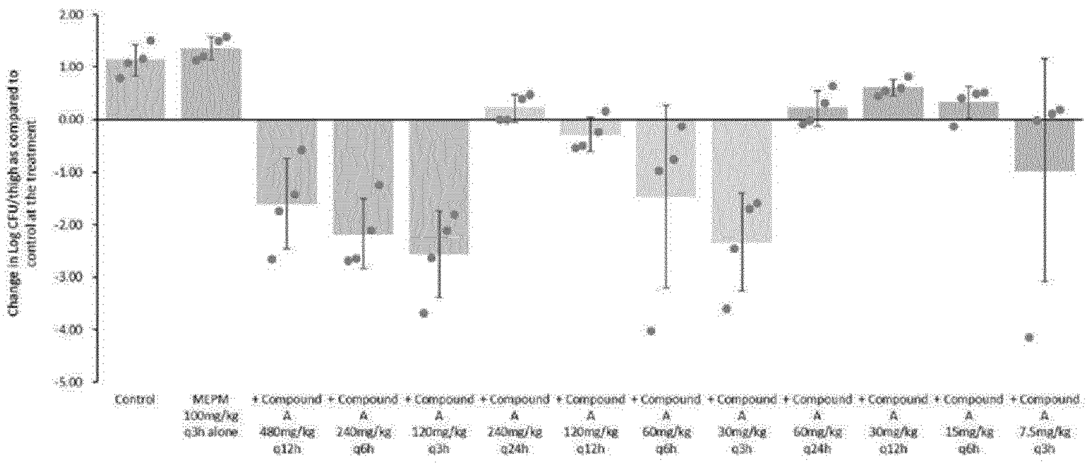

[FIG. 7-2]

D) CDC－２９３ infection model

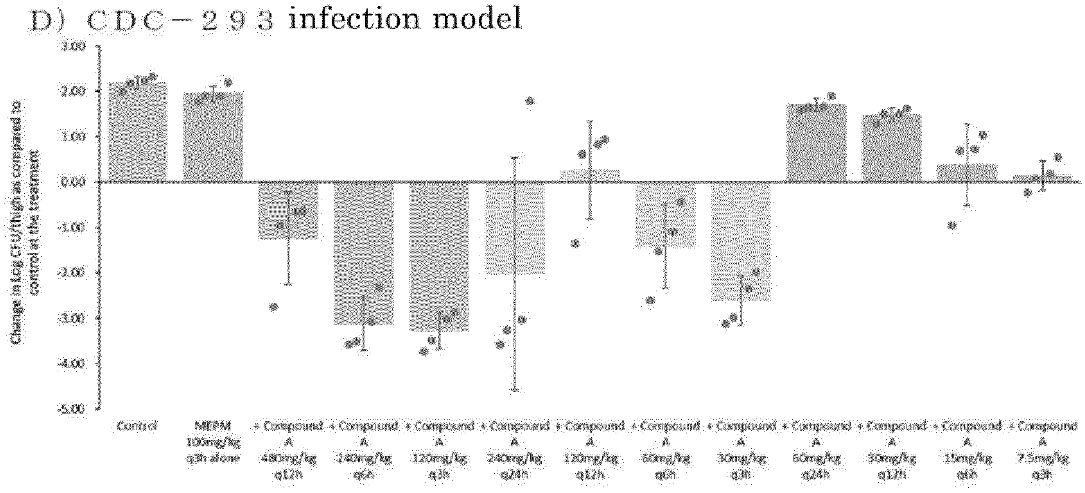


E) CDC－２８９ infection model

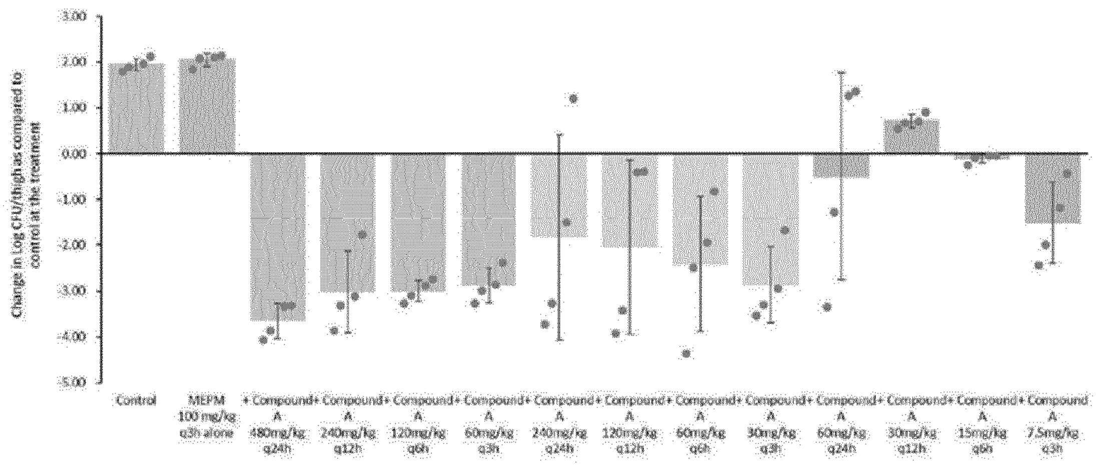

[FIG. 8]

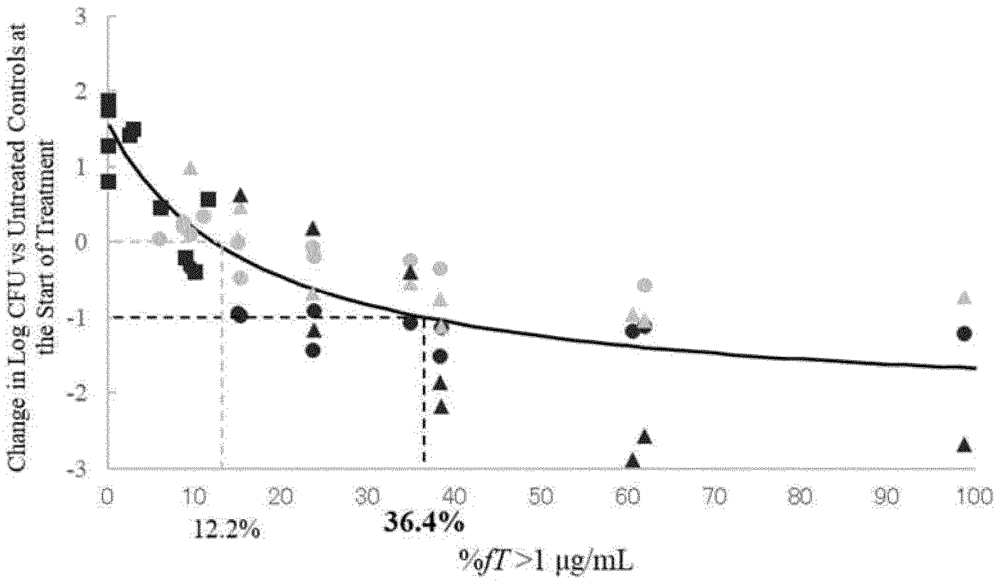
Change in Log CFU vs Untreated Controls at the Start of Treatment
3
2
1
0
-1
-2
-3
0
10
20
30
40
50
60
70
80
90
100
12.2%
36.4%
%fT >1 μg/mL

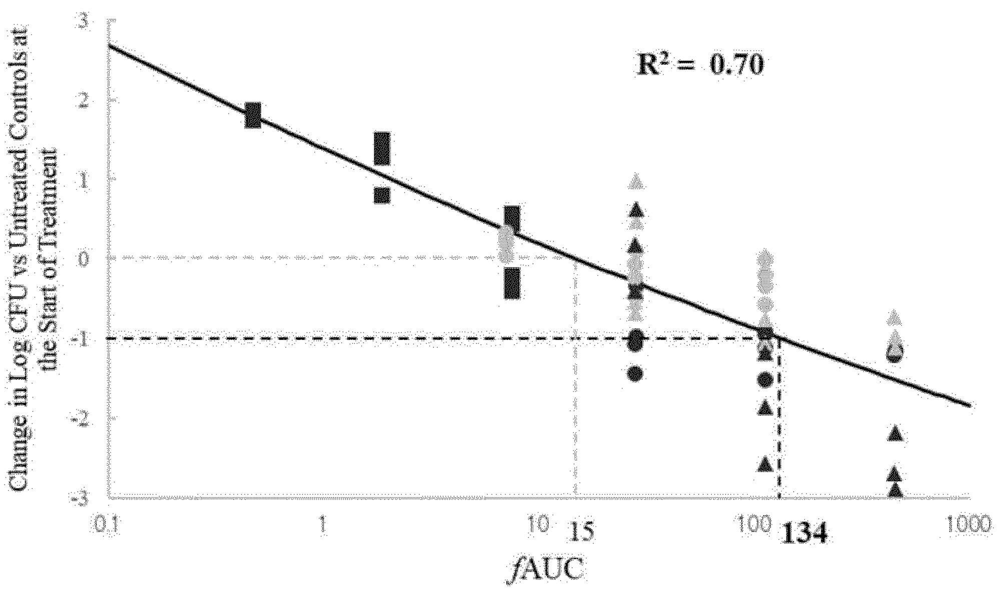
Change in Log CFU vs Untreated Controls at the Start of Treatment
$R^2$ = 0.70
3
2
1
0
-1
-2
-3
0.1
1
10
15
100
134
1000
fAUC

[FIG. 9]

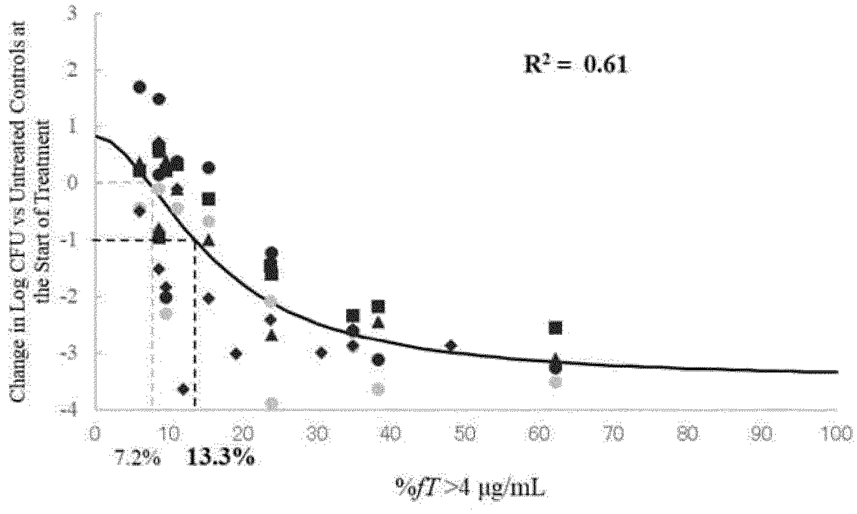
R$^2$ = 0.61
Change in Log CFU vs Untreated Controls at the Start of Treatment
3
2
1
0
-1
-2
-3
-4
0
10
20
30
40
50
60
70
80
90
100
7.2%
13.3%
%fT>4 µg/mL

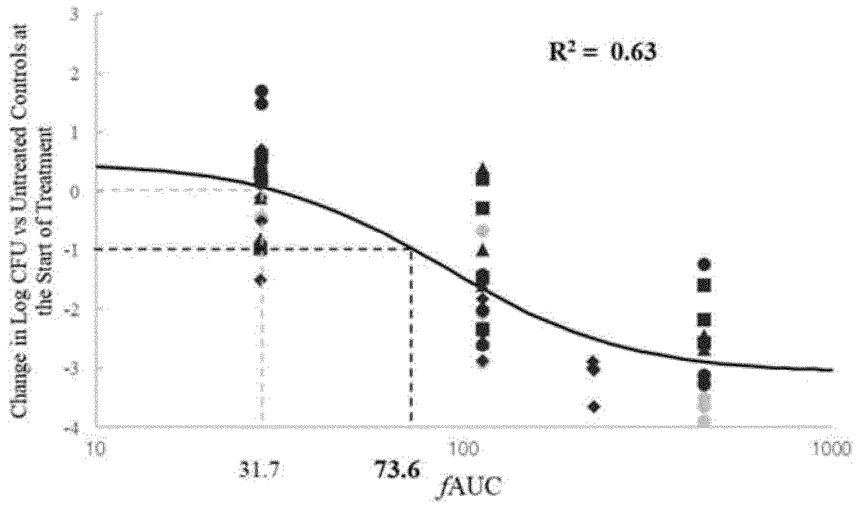
R$^2$ = 0.63
Change in Log CFU vs Untreated Controls at the Start of Treatment
3
2
1
0
-1
-2
-3
-4
10
100
1000
31.7
73.6
fAUC

[FIG. 10-1]

A) CDC－40 infection model (VIM-27-producing bacterium)

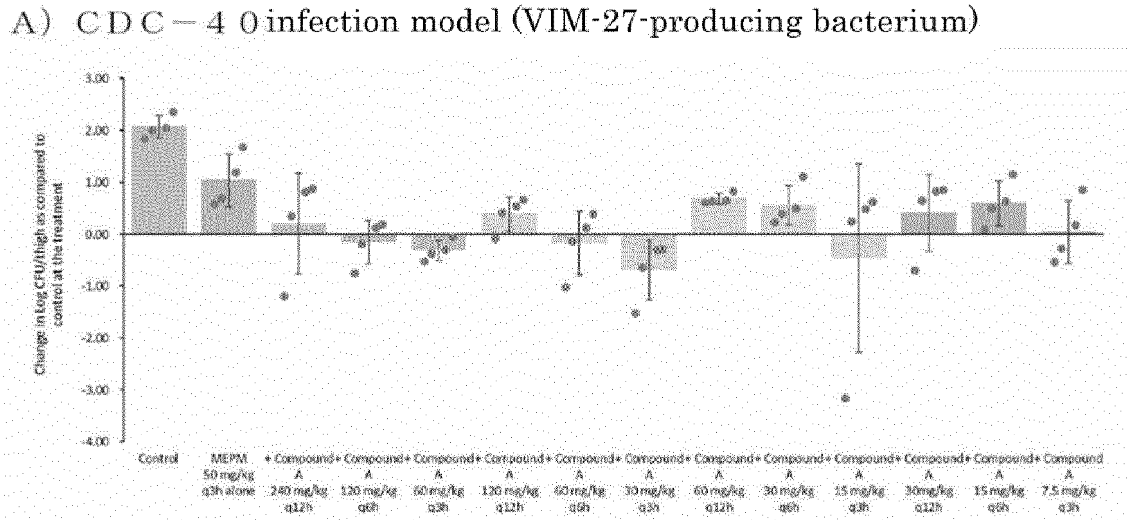


B) CDC－113 infection model (KPC-3-producing bacterium)

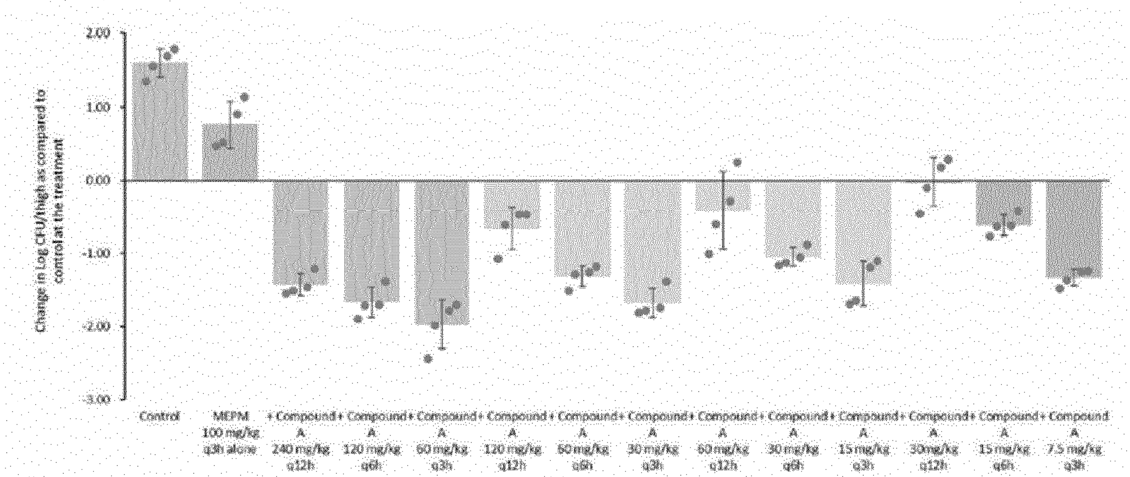


C) CDC－138 infection model (NDM-7-producing bacterium)

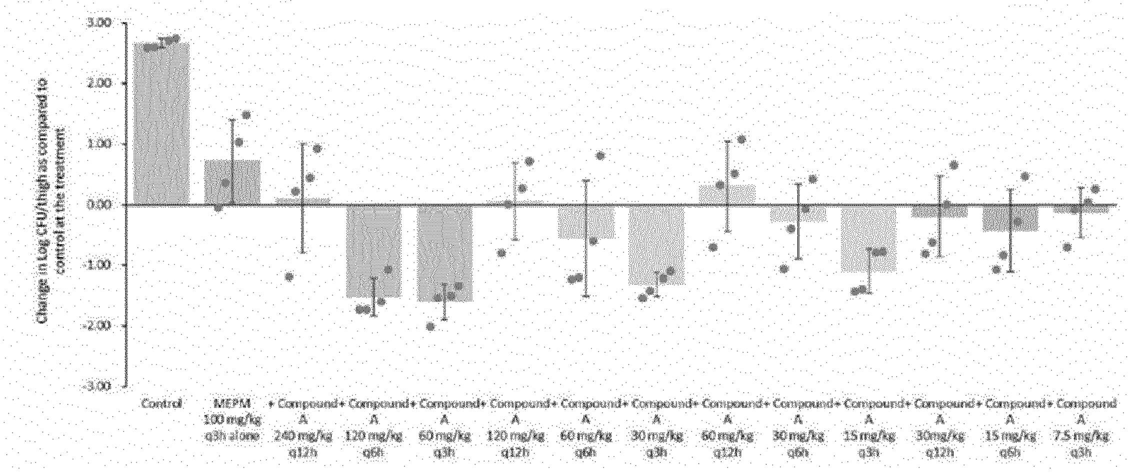

[FIG. 10-2]

D) CDC－68 infection model (NDM-1- and OXA-232-producing bacterium)

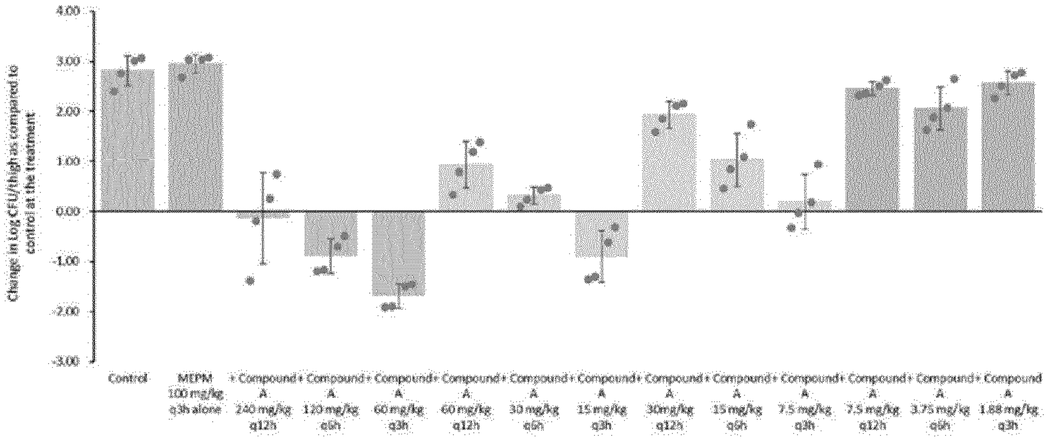

[FIG. 11]

A) **Evaluation in 9 strains of carbapenemase-producing *Klebsiella pneumoniae***

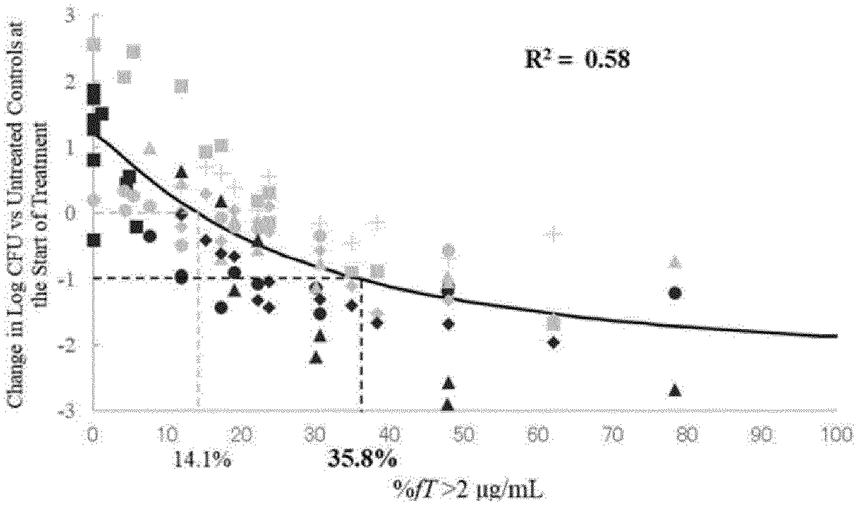


B) **Evaluation in 4 strains of serine-carbapenemase-producing *Klebsiella pneumoniae***

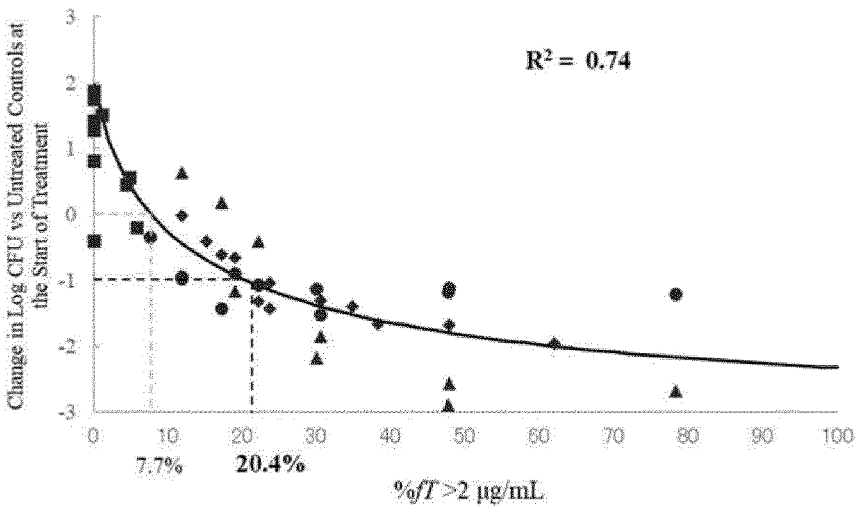


C) **Evaluation in 5 strains of metallo-carbapenemase-producing *Klebsiella pneumoniae***

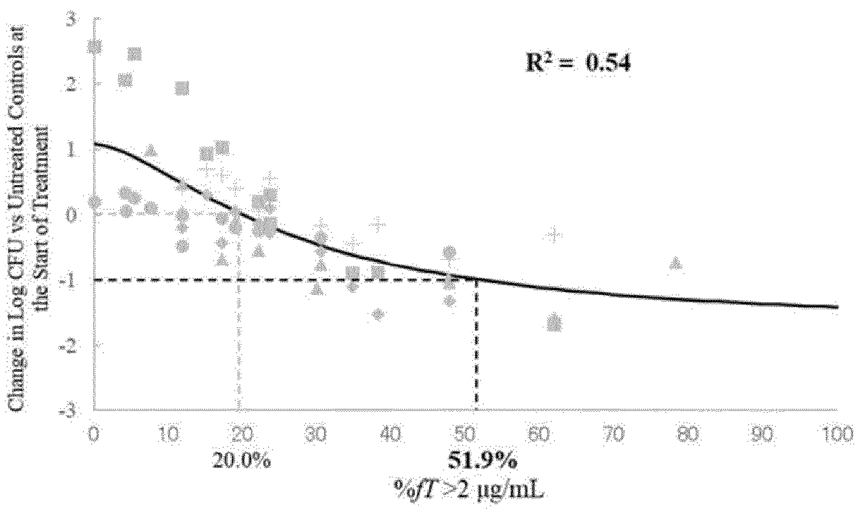

## INTERNATIONAL SEARCH REPORT

International application No.
**PCT/JP2023/044535**

### A. CLASSIFICATION OF SUBJECT MATTER

***A61K 31/69***(2006.01)i; ***A61K 31/407***(2006.01)i; ***A61P 11/00***(2006.01)i; ***A61P 13/02***(2006.01)i; ***A61P 31/04***(2006.01)i; ***A61P 43/00***(2006.01)i
FI: A61K31/69; A61P31/04; A61P43/00 121; A61K31/407; A61P13/02; A61P13/02 105; A61P11/00; A61P43/00 111

According to International Patent Classification (IPC) or to both national classification and IPC

### B. FIELDS SEARCHED

Minimum documentation searched (classification system followed by classification symbols)

A61K31/69; A61K31/407; A61P11/00; A61P13/02; A61P31/04; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | WO 2019/208797 A1 (SUMITOMO DAINIPPON PHARMA CO., LTD.) 31 October 2019 (2019-10-31) claims, paragraphs [0002], [0012], [0141]-[0147], example 36, test example 3, table 5-1 | 1-49 |
| X | JP 2021-70691 A (SUMITOMO DAINIPPON PHARMA CO., LTD.) 06 May 2021 (2021-05-06) claims, paragraphs [0002], [0012], [0141]-[0147], example 36, test example 3, table 5-1 | 1-49 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2024** | **20 February 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2023/044535**

| Patent document cited in search report | Publication date (day/month/year) | Patent family member(s) | Publication date (day/month/year) |
|---|---|---|---|
| WO 2019/208797 A1 | 31 October 2019 | US 2021/0147448 A1<br>claims, paragraphs [0003], [0930], [1168]-[1173], example 36, test example 3, table 5-1<br>EP 3786168 A1<br>CN 112135830 A<br>KR 10-2021-0005910 A | |
| JP 2021-70691 A | 06 May 2021 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019208797 A
- WO 8705297 A, Johnston


### Non-patent literature cited in the description


- **BUYNAK. JD.** *Expert Opinion on Therapeutic Patents*, 2013, vol. 23 (11), 1469-1481
- **MONOGUE. ML.** *Expert Review of Anti-infective Therapy*, 2019, vol. 17 (8), 571-582
- **BUSH. K.** *Nature Reviews Microbiology*, 2019, vol. 17, 295-306
- **ADEOLU M.** *International Journal of Systematic and Evolutionary Microbiology*, 2016, vol. 66, 5575-5599
- **PELEG AY.** *Clinical Microbiology Reviews.*, 2008, vol. 21, 538-582
- **RINO MATSUI.** *The Journal of the Japanese Society for Clinical Microbiology.*, 2021, vol. 31 (2), 113-115
- Evidence-based CKD Clinical Practice Guidelines. Japanese Society of Nephrology. 2018, vol. 2018
- Clinical Practice Guidelines. Japanese Society of Nephrology. 2012, vol. 2012
- Goodman and Gilman's. The Pharmacological Basis of Therapeutics. Pergamon Press, 1990
- **JOHN F. MORRISON** ; **CHRISTOPHER T. WALSH**. The Behavior AND Significance OF Slow-Binding Enzyme Inhibitors. *Adv Enzymol Relat Areas Mol Biol.*, 1988, vol. 61, 201-301
- **ROBERT A COPELAND** ; **ARAVIND BASAVAPATHRUNI** ; **MIKEL MOYER** ; **MARGARET PORTER SCOTT**. Impact of enzyme concentration and residence time on apparent activity recovery in jump dilution analysis.. *Anal Biochem.*, 2011, vol. 416 (2), 206-10
- **FUKASAWA M** ; **SUMITA Y** ; **HARABE ET** ; **TANIO T** ; **NOUDA H** ; **KOHZUKI T** ; **OKUDA T** ; **MATSUMURA H** ; **SUNAGAWA M.** Stability of Meropenem and Effect of 1β-Methyl Substitution on Its Stability in the Presence of Renal Dehydropeptidase I.. *Antimicrob Agents Chemother*, 1992, vol. 36, 1577-1579
- **SABET M** ; **TARAZI Z** ; **NOLAN T** ; **PARKINSON J** ; **RUBIO-APARICIO D** ; **LOMOVSKAYA O** ; **DUDLEY MN** ; **GRIFFITH DC.** Activity of Meropenem-Vaborbactam in Mouse Models of Infection Due to KPC-Producing Carbapenem-Resistant Enterobacteriaceae.. *Antimicrob Agents Chemother*, 2018, vol. 62, e01446-17
- **RUBINO CM** ; **BHAVNANI SM** ; **LOUTIT JS** ; **MORGAN EE** ; **WHITE D** ; **DUDLEY MN** ; **GRIFFITH DC.** Phase 1 Study of the Safety, Tolerability, and Pharmacokinetics of Vaborbactam and Meropenem Alone and in Combination Following Single and Multiple Doses in Healthy Adult Subjects.. *Antimicrob Agents Chemother*, 2018, vol. 62, e02228-17
- **CRANDON JL** ; **NICOLAU DP.** Pharmacodynamic Approaches to Optimizing β-Lactam Therapy.. *Crit Care Clin*, 2011, vol. 27, 77-93
- CLSI. Performance Standards for Antimicrobial Susceptibility Testing.. Clinical and Laboratory Standards Institute. 2021